(19) Europäisches Patentamt
European Patent Office
Office européen des brevets

(11) EP 4 539 933 B1

(12) **EUROPEAN PATENT SPECIFICATION**

(45) Date of publication and mention of the grant of the patent:
**05.08.2026 Bulletin 2026/32**

(21) Application number: **23738605.7**

(22) Date of filing: **14.06.2023**

(51) International Patent Classification (IPC):
*A61P 3/00* (2006.01) *A61P 25/28* (2006.01)
*A61P 29/00* (2006.01) *A61P 35/00* (2006.01)
*A61P 37/00* (2006.01) *C07D 215/14* (2006.01)
*C07D 215/20* (2006.01) *C07D 239/74* (2006.01)
*C07D 401/12* (2006.01) *C07D 401/14* (2006.01)
*C07D 405/12* (2006.01) *C07D 413/12* (2006.01)
*C07D 417/12* (2006.01) *C07D 471/04* (2006.01)
*C07D 519/00* (2006.01) *A61K 31/4375* (2006.01)
*A61K 31/47* (2006.01) *A61K 31/4709* (2006.01)
*A61K 31/444* (2006.01) *A61K 31/501* (2006.01)

(52) Cooperative Patent Classification (CPC):
**C07D 215/14; A61P 3/00; A61P 25/28; A61P 29/00; A61P 35/00; A61P 37/00; C07D 215/20; C07D 239/74; C07D 401/12; C07D 401/14; C07D 405/12; C07D 413/12; C07D 417/12; C07D 471/04; C07D 519/00**

(86) International application number:
**PCT/CN2023/100107**

(87) International publication number:
**WO 2023/241608 (21.12.2023 Gazette 2023/51)**

(54) **CSF-1R INHIBITORS AND USES THEREOF**

CSF-1R-HEMMER UND VERWENDUNGEN DAVON

INHIBITEURS DE CSF-1R ET LEURS UTILISATIONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.06.2022 PCT/CN2022/098943**
**10.05.2023 PCT/CN2023/093222**

(43) Date of publication of application:
**23.04.2025 Bulletin 2025/17**

(73) Proprietor: **Myrobalan Therapeutics, Inc.**
**Medford, Massachusetts 02155 (US)**

(72) Inventors:
- **WANG, Hao**
  **Nanjing, Jiangsu 210032 (CN)**
- **ZHANG, Enge**
  **Nanjing, Jiangsu 210032 (CN)**
- **ZHAO, Shuhai**
  **Nanjing, Jiangsu 210032 (CN)**
- **YANG, Minmin**
  **Nanjing, Jiangsu 210032 (CN)**
- **LI, Peng**
  **Nanjing, Jiangsu 210032 (CN)**
- **GENG, Bolin**
  **Medford, Massachusetts 02155 (US)**
- **SHLEVKOV, Evgeny**
  **Medford, Massachusetts 02155 (US)**
- **ONG, Ta Ren**
  **Medford, Massachusetts 02155 (US)**
- **WANG, Jing Yu**
  **Medford, Massachusetts 02155 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- LIN, Yen-Chen
  Medford, Massachusetts 02155 (US)
- YOUNG, Michael Nakamura
  Medford, Massachusetts 02155 (US)
- HARVEY, Lauren Marie
  Medford, Massachusetts 02155 (US)
- CHEN, Yi Alex
  Medford, Massachusetts 02155 (US)
- HAN, Xiao
  Medford, Massachusetts 02155 (US)

(74) Representative: **HGF**
**HGF Limited**
**4th Floor, 1 City Square**
**Leeds LS1 2ES (GB)**



(56) References cited:
**EP-A1- 3 738 961**
**WO-A1-2018/119387**
**WO-A1-2021/144360**
**WO-A1-2021/170078**
**US-A- 5 236 952**

## Description

CROSS REFERENCE TO RELATED APPLICATION

This application claims the benefit of priority to International Patent Application Number PCT/CN2022/098943, filed on June 15, 2022, and International Patent Application Number PCT/CN2023/093222, filed on May 10, 2023.

BACKGROUND

CSF-1R, *i.e.* CSF-1 receptor (colony stimulating factor 1 receptor), is encoded by the oncogene c-fms. The human c-fms gene is located at 5q33.3 of chromosome 5, downstream of the β-type platelet-derived growth factor receptor (PDGF_Rβ) gene, and the two genes are connected end to end. Human CSF-1R is a single-chain, transmembrane receptor tyrosine kinase, a transmembrane glycoprotein composed of 972 amino acids, with a molecular weight of 150 Kd. It consists of an extramembrane region with 512 amino acids, a transmembrane region with 25 amino acids, and an intracellular cytoplasmic region with 435 amino acids. The extracellular region has 5 disulfide bonds and 11 possible glycosylation sites, and the intracellular region has a Gly-X-Gly-X-X-Gly motif. Lysine at position 616 is a binding site for ATP, flanked by a kinase insertion region with 72 amino acids. It is speculated that it has the function of recognizing specific substrates (Cold Spring Harb Perspect Biol. 2014, 6 (6)).

CSF-1, also called M-CSF (macrophage colony stimulating factor), is encoded by the CSF-1 gene. CSF-1 exerts its biological effects by binding to the only cell surface receptor CSF-1R thereof. After binding to CSF-1, CSF-1R undergoes changes in its conformation and forms a dimer or polymer. After dimerization, the tyrosine kinase activity of the receptor is activated, and the tyrosines at positions 544, 559, 699, 708, 723, 809, 923, *etc.* are phosphorylated, and subsequently interact with multiple intracellular signaling pathways such as Ras, MAPK, PI3K, JAK, *etc.* to produce various biological effects in cells (J Cell Biochem. 1988, 38 (3):179-87).

The tumor microenvironment is a complex ecosystem, and provides support for the occurrence, growth and metastasis of tumors. Macrophages are particularly abundant in immune cells that migrate to the tumor site, and exist in all stages of tumor development. Studies have shown that tumor-associated macrophages (TAMs) play an important role in the occurrence, growth and metastasis of tumors. For primary tumors, macrophages can stimulate the neovascularization, aid the extravasation, survival and continuous growth of tumor cells, thereby promoting tumor cell metastasis. TAM also exerts an immunosuppressive effect, preventing natural killer cells and T cells from attacking tumor cells (Immunity. 2014, 41 (1):49-61). CSF-1R is expressed in macrophages, and the survival and differentiation of macrophages depend on the CSF-1/CSF-1R signaling pathway. The CSF-1/CSF-1R signaling pathway interferes with tumor progression by regulating TAMs to reduce tumor invasiveness and proliferation, as a consequence, the CSF1/CSF1R signaling pathway is a potential target for cancer treatment. Overexpression of CSF-1 or CSF-1R is related to tumor malignant invasiveness and poor prognosis. Studies have shown that the application of CSF-1R inhibitors can affect the exchange of inflammatory factors between TAMs and glioma cells, which significantly reduces the volume of glioblastoma, and reduces tumor invasiveness and proliferation (Nat Med. 2013, 19 (10):1264-72). In addition, aberrantly high expression of CSF-1 is the main pathogenesis of tenosynovial giant cell tumor (a type of rare non-metastatic tumor with giant cell tumor and pigmented villonodular synovitis in tendon sheath). Patients with tenosynovial giant cell tumor have obvious clinical benefits after using CSF-1R inhibitors (N Engl J Med. 2015, 373 (5):428-37).

In addition to tumors, the CSF-1R signaling pathway plays an important role in autoimmune diseases and inflammatory diseases, including systemic lupus erythematosus, arthritis, atherosclerosis and obesity (Arthritis Res Ther. 2016, 18: 75; Nat Rev Immunol. 2008, 8 (7):533-44; J Immunother Cancer. 2017, 5 (1):53). Therefore, the development of CSF-1R inhibitors may also be used to treat such diseases.

Furthermore, increasing studies have shown that inflammation in the nervous system and abnormal activation of brain microglia cells are important pathogenic factors of related neurodegenerative diseases, especially Alzheimer's disease (Neurobiol Aging. 2000, 21: 383-421) . Among others, the signaling pathway mediated by CSF-1R plays a leading role in the activation and proliferation of microglia cells in the brain. Studies have shown that the expression of CSF-1R in tissue samples from Alzheimer's patients is significantly upregulated, accompanied by the abnormal activation and proliferation of microglia (Brain Res., 1994, 639: 171-4) . Animal model studies have shown that blocking CSF-1R signaling can effectively inhibit microglial proliferation, thereby effectively alleviating the disease progression in mouse models of Alzheimer's disease (Brain. 2016, 139: 891-907). In models of other neurodegenerative diseases, such as amyotrophic lateral sclerosis, the therapeutic effects of targeting CSF-1R on the disease has been preliminarily demonstrated in a proof-of-concept study (Sci Rep., 2016, 6: 25663). Currently, a number of CSF-1R inhibitor candidates are in clinical trials on neurodegenerative diseases.

Therefore, it remains a need to discover novel CSF-1R inhibitors for the treatment of diseases, such as cancer, autoimmune diseases, inflammatory diseases or neurodegenerative diseases.

SUMMARY

The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy. Described herein are compounds of Formula (I) (including subgenus encompassed by Formula (I)) that inhibit the activity of CSF-1R, or pharmaceutically acceptable salts thereof. More specifically, the compounds disclosed herein not only effectively inhibit the activity of CSF-1 R, but also selectively inhibit CSF-1R over cKit, FLT3, PDGFR-β (see Tables 2 and 3), possess desired pk profile (see Table 5), and have high exposure in brain and high blood-brain barrier (BBB) penetration compard to other known CSF-1R inhibtors (see Table 6).

In one aspect, the present disclosure provides a compound of Formula (I), or a pharmaceutically acceptable salt thereof:

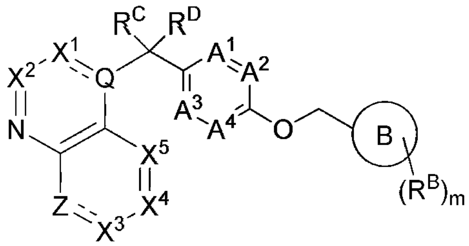

(I);

wherein the variables shown in the formula are defined herein.

Also provided are pharmaceutical compositions comprising a compound of Formula (I), or a pharmaceutically acceptable salt thereof and a pharmaceutically acceptable carrier or excipient.

The present disclosure further provides methods of inhibiting CSF-1 R in a subject, comprising administering to the subject a compound of Formula (I), or a pharmaceutically acceptable salt thereof.

The present disclosure also provides methods of treating a disease or condition mediated by CSF-1R or at least in part by CSF-IR in a subject, comprising administering to the subject a therapeutically effective amount of a compound of Formula (I), a pharmaceutically acceptable salt, or a tautomer thereof. In certain embodiments, the disease is an autoimmune disease, an inflammatory disease, a neurodegenerative disease, cancer, a metabolic disease, obesity, or an obesity-related disease.

The present disclosure further provides a method of treating Alzheimer’s disease in a subject, comprising administering to the subject an effective amount of (1) a compound of Formula (I), or a pharmaceutically acceptable salt thereof; or (2) a pharmaceutically acceptable composition comprising a compound of Formula (I), or a pharmaceutically acceptable salt thereof, and a pharmaceutically acceptable carrier.

The present disclosure further provides a method of treating progressive supranuclear palsy in a subject, comprising administering to the subject an effective amount of (1) a compound of Formula (I), or a pharmaceutically acceptable salt thereof; or (2) a pharmaceutically acceptable composition comprising a compound of Formula (I), or a pharmaceutically acceptable salt thereof, and a pharmaceutically acceptable carrier.

The present disclosure further provides a method of treating a tau-mediated neurodegenerative disorder in a subject, comprising administering to the subject an effective amount of (1) a compound of Formula (I), or a pharmaceutically acceptable salt thereof; or (2) a pharmaceutically acceptable composition comprising a compound of Formula (I), or a pharmaceutically acceptable salt thereof, and a pharmaceutically acceptable carrier.

The present disclosure further provides a method of treating a disease or disorder involving microglia-mediated inflammation in a subject, comprising administering to the subject an effective amount of (1) a compound of Formula (I), or a pharmaceutically acceptable salt thereof; or (2) a pharmaceutically acceptable composition comprising a compound of Formula (I), or a pharmaceutically acceptable salt thereof, and a pharmaceutically acceptable carrier.

The present disclosure also provides a use of a compound of Formula (I), or a pharmaceutically acceptable salt thereof or a pharmaceutical composition comprising the same in any of the methods described herein. In one embodiment, provided is a compound of Formula (I) or a pharmaceutically acceptable salt thereof or a pharmaceutical composition comprising the same for use in any of the methods described herein. In another embodiment, provided is use of a compound of Formula (I) or a pharmaceutically acceptable salt thereof or a pharmaceutical composition comprising the same for the manufacture of a medicament for any of the methods described herein.

BRIEF DESCRIPTION OF DRAWINGS

Figures 1A-1C show effects of CSFIR inhibitors on CSF1 induced CSF1R and ERK1/2 phosphorylation in BV2 cells. BV2 cells were stimulated with recombinant mouse CSF1 (rmCSF1) after being pretreated with the following CSF1R inhibitors GW2580 (Figure 1A), Comparator A (Figure 1B), or Example 16 (Figure 1C). Stimulation was terminated by cell lysis followed by SDS-PAGE, western blotting, and immunoblotting for epitopes as stated in the figure.

Figures 2A-2C: Quantification of western blots from Figure 1. The small molecule inhibitors of CSF1R: GW2580 (Figure 2A), Comparator A (Figure 2B), and Example 16 (Figure 2C), demonstrate a dose dependent inhibition of CSF1 directed CSF1R and ERK1/2 phosphorylation. The corresponding values represent the average of two experimental replicates.

Figures 3A-3D show effects of CSF1R inhibitors on CSF1R phosphorylation in THP1 cells stimulated with CSF1. Graphs show a dose-dependent decrease in CSF1 induced CSF1R phosphorylation in the presence of GW2580 (Figure 3A), BLZ946 (Figure 3B), Comparator A (Figure 3C), and Example 16 (Figure 3D) using a sandwich ELISA in detected phosphorylated CSF1R.

Figures 4A-4D show effects of CSF1R inhibitors on CSF1 induced BV2 cell proliferation. The following experiment measures BV2 cell proliferation in response to CSF1 using a Cell Titer GLO assay. Figure 4A shows CSF1 induced cell proliferation is dampened in the presence of 500nM of CSF1R inhibitors GW2580 or BLZ945. Numbers above each column represent the percent (%) change in proliferation. P-value was obtained from a Dunnett's multiple comparison test. CSFIR inhibitors GW2580 (Figure 4B), BLZ945 (Figure 4C), and Example 16 (Figure 4D), demonstrate a dose dependent inhibition of CSF1 directed cell proliferation.

Figures 5A and 5B show the effect of CSF1R inhibitors on the survival of in vitro cultured primary rat microglia. This experiment measures the potency of CSF1R inhibitors using a primary brain glia from rat cortices in-vitro. Figure 5A shows that CSF1R inhibitor, Example 16 demonstrates a dose-dependent inhibition of primary rat microglia survival after 3 days of exposure. Figure 5B shows that CSF1R inhibitors, Examples 73, 134, and 136 demonstrate a dose-dependent inhibition of primary rat microglia survival after 3 days of exposure.

Figure 6 shows the effects of CSF1R inhibitors on the level of phosphorylated CSF1R (pCSF1R) in a human monocyte-like cell line, THP-1 cells, stimulated by CSF1R. In Figure 6, Example 16 shows a dose-dependent inhibition of CSF1R phosphorylation in CSF1R-stimulated THP-1 monocytes.

Figure 7A and 7B shows the effect of CSF1R inhibitor on human iPSC-derived microglia (hiPSC-MG) survival. Figure 7A shows that CSF1R inhibitor Example 16 demonstrates a dose-dependent inhibition of hiPSC-MG survival (IC50= 31.5 nM). Figure 7B shows that CSF1R inhibitors BLZ945, Example 73, Example 136, and Example 134 demonstrate a dose-dependent inhibition of hiPSC-MG survival.

Figure 8A and 8B shows the depletion effect of Example 134 on Iba1+ cell density (iba1 is a marker for microglia) in the cortex (Figure 8A) and the hippocampus (Figure 8B) of female C57BL/6J mice following a 7-day oral gavage BID dosing regimen.

DETAILED DESCRIPTION

1. *Compounds*

In a first embodiment, the present disclosure provides a compound represented by Formula (I):

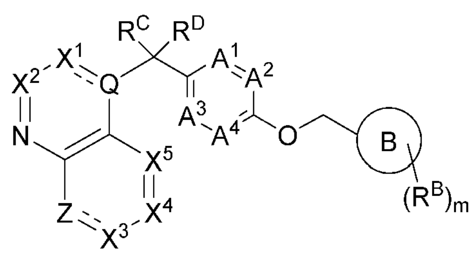

(I);

or a pharmaceutically acceptable salt, or a tautomer thereof, wherein:

Q is C or N;
when Q is N, Q-$X^1$ is a single bond and $X^1$ is C=O;
when Q is C, Q-$X^1$ is a double bond and $X^1$ is N or $CR^1$;
Z is CH or NH;
when Z is NH, Z-$X^3$ is a single bond and $X^3$ is C=O;
when Z is CH, Z-$X^3$ is a double bond and $X^3$ is N or $CR^3$;
$X^2$ is N or $CR^2$;
$X^4$ is N or $CR^4$;
$X^5$ is N or $CR^5$
each $R^1$, $R^2$, $R^3$, $R^4$, and $R^5$ is independently selected from H, halogen, -CN, -OH, $C_{1-6}$alkyl optionally substituted with one to three deuterium, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\ or\ 1}$-3-12 membered carbocyclyl, $-(CH_2)_{0\ or\ 1}$-3-12 membered heterocyclyl, $-(CH_2)_{0\ or\ 1}$-6-10 membered aryl, or $-(CH_2)_{0\ or\ 1}$-5-10 membered heteroaryl; wherein said carbocyclyl, heterocyclyl, aryl, or heteroaryl in the group represented by $R^1$, $R^2$, $R^3$, $R^4$, or $R^5$ is optionally substituted by one or two groups selected from CN, halogen, $C_{1-6}$alkyl, $C_{1-6}$haloalkyl, $C_{1-6}$alkoxy, and $C_{1-6}$haloalkoxy;
each $A^1$, $A^2$, $A^3$, and $A^4$ is independently N or $CR^A$; and no more than two of $A^1$, $A^2$, $A^3$, and $A^4$ are N;
each $R^A$ is independently selected from H, halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\ or\ 1}$-3-12 membered carbocyclyl, $-(CH_2)_{0\ or\ 1}$-3-12 membered heterocyclyl, $-(CH_2)_{0\ or\ 1}$-6-10 membered aryl, or $-(CH_2)_{0\ or\ 1}$-5-10 membered heteroaryl; wherein said carbocyclyl, heterocyclyl, aryl, or heteroaryl in the group represented by $R^A$ is optionally substituted by one or two groups selected from CN, halogen, $C_{1-6}$alkyl, $C_{1-6}$haloalkyl, $C_{1-6}$alkoxy, and $C_{1-6}$haloalkoxy;
Ring B is phenyl, or 5 or 6-membered monocyclic heteroaryl;
each $R^B$ is independently selected from halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\ or\ 1}$-3-12 membered carbocyclyl, $-(CH_2)_{0\ or\ 1}$-3-12 membered heterocyclyl, $-(CH_2)_{0\ or\ 1}$-6-10 membered aryl, or $-(CH_2)_{0\ or\ 1}$-5-10 membered heteroaryl; wherein said carbocyclyl, heterocyclyl, aryl, or heteroaryl in the group represented by $R^B$ is optionally substituted by one or two groups selected from CN, halogen, $C_{1-6}$alkyl, $C_{1-6}$haloalkyl, $C_{1-6}$alkoxy, and $C_{1-6}$haloalkoxy; or
two $R^B$ together with the ring B atoms which they attached, form 5-7 membered monocyclic ring or 6-9 membered bicyclic ring fused to Ring B, said 5-7 membered monocyclic or 6-9 membered bicyclic ring is optionally substituted with one or two groups selected from CN, halogen, $C_{1-6}$alkyl, $C_{1-6}$haloalkyl, $C_{1-6}$alkoxy, $C_{1-6}$haloalkoxy, and 3-6 membered cycloalkyl;
each $R^C$ and $R^D$ is independently selected from hydrogen, deuterium, F, and methyl;
each R* is independently selected from the group consisting of H, $C_{1-6}$alkyl, $C_{1-6}$haloalkyl, $C_{2-6}$alkenyl, $C_{2-6}$alkynyl, 3-6 membered carbocyclyl, or 4-6 membered heterocyclyl; and
m is 0, 1, 2, 3, or 4.

In a second embodiment, the present disclosure provides a compound, or pharmaceutically acceptable salt thereof, according to the first embodiment, wherein:

each $R^1$, $R^2$, $R^3$, $R^4$, and $R^5$ is independently selected from H, halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\ or\ 1}$-3-12 membered carbocyclyl, $-(CH_2)_0$ or $_1$-3-12 membered heterocyclyl, $-(CH_2)_{0\ or\ 1}$-6-10 membered aryl, or $-(CH_2)_{0\ or\ 1}$-5-10 membered heteroaryl; wherein said carbocyclyl, heterocyclyl, aryl, or heteroaryl in the group represented by R', $R^2$, $R^3$, $R^4$, or $R^5$ is optionally substituted by one or two groups selected from CN, halogen, $C_{1-6}$alkyl, $C_{1-6}$haloalkyl, $C_{1-6}$alkoxy, and $C_{1-6}$haloalkoxy;
each $R^A$ is independently selected from H, halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\ or\ 1}$-3-12 membered carbocyclyl, $-(CH_2)_{0\ or\ 1}$-3-12 membered heterocyclyl, $-(CH_2)_{0\ or\ 1}$-6-10 membered aryl, or $-(CH_2)_{0\ or\ 1}$-5-10 membered heteroaryl; wherein said carbocyclyl, heterocyclyl, aryl, or heteroaryl in the group represented by $R^A$ is optionally substituted by one or two groups selected from CN,

halogen, $C_{1-6}$alkyl, $C_{1-6}$haloalkyl, $C_{1-6}$alkoxy, and $C_{1-6}$haloalkoxy;
each $R^B$ is independently selected from halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, $-C(O)R^*$, $-C(O)OR^*$, $-C(O)NR^*R^*$, $-SO_2R^*$, $-(CH_2)_{0-4}NR^*R^*$, $-NR^*C(O)R^*$, $-NR^*C(O)OR^*$, $-NR^*SO_2R^*$, $-NR^*SO_2NR^*R^*$, $-P(O)R^*R^*$, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^B$ is optionally substituted by one or two groups selected from CN, halogen, $C_{1-6}$alkyl, $C_{1-6}$haloalkyl, $C_{1-6}$alkoxy, and $C_{1-6}$haloalkoxy; or
two $R^B$ together with the ring B atoms which they attached, form 5,6-membered ring fused to Ring B, said 5,6-membered ring is optionally substituted with one or two groups selected from CN, halogen, $C_{1-6}$alkyl, $C_{1-6}$haloalkyl, $C_{1-6}$alkoxy, and $C_{1-6}$haloalkoxy; and
each $R^C$ and $R^D$ is independently selected from hydrogen, F, and methyl. The definitions of the remaining variables are provided in the first embodiment.

In a third embodiment, the present disclosure provides a compound according to the second embodiment, wherein the compound is represented by Formula (II-A):

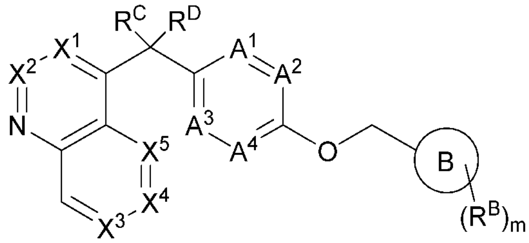
(II-A);

or a pharmaceutically acceptable salt thereof. The definitions of the variables are provided in the second embodiment. Alternatively, the definitions of the variables are provided in the first embodiment.

In a fourth embodiment, the present disclosure provides a compound according to the second embodiment, wherein the compound is represented by any one of Formula (III-A-1), Formula (III-A-2), Formula (III-A-3), Formula (III-A-4), Formula (III-A-5), Formula (III-A-6), or Formula (III-A-7):

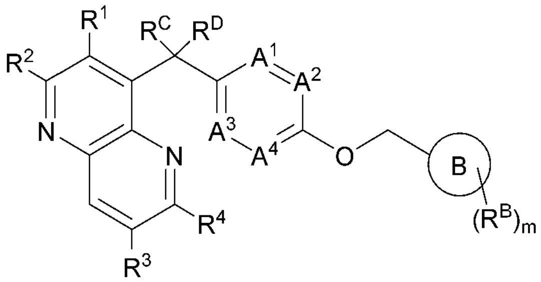
(III-A-1),

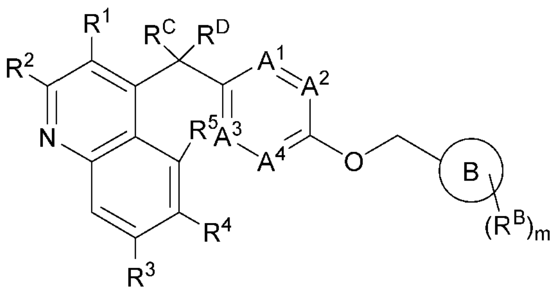
(III-A-2),

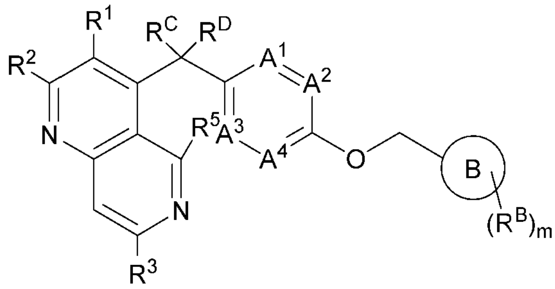

(III-A-3),

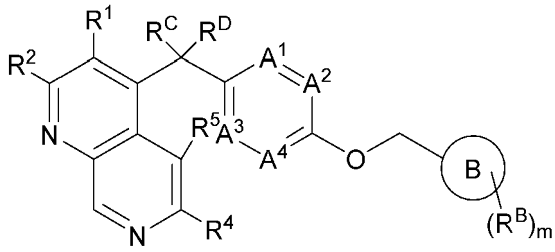

(III-A-4),

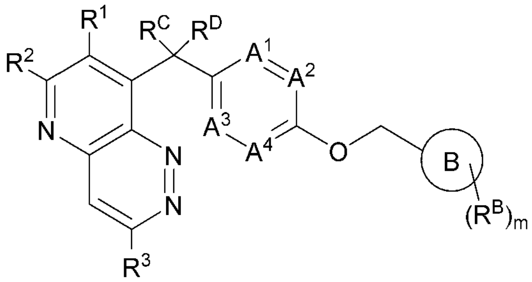

(III-A-5),

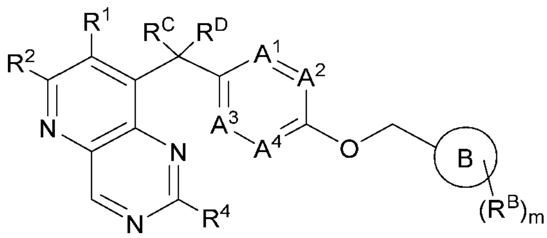

(III-A-6),

or

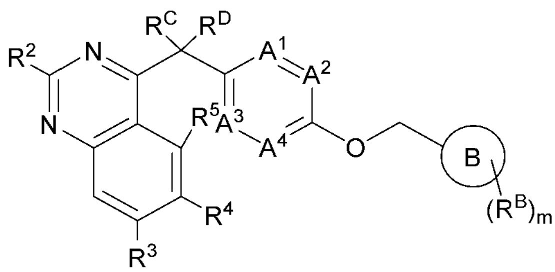

(III-A-7),

or a pharmaceutically acceptable salt thereof. The definitions of the variables are provided in the second embodiment. Alternatively, the definitions of the variables are provided in the first embodiment.

In a fifth embodiment, the present disclosure provides a compound according to the second embodiment, wherein the compound is represented by any one of Formula (III-B-1), Formula (III-B-2), Formula (III-C-1), Formula (III-C-2), or Formula (III-C-3):

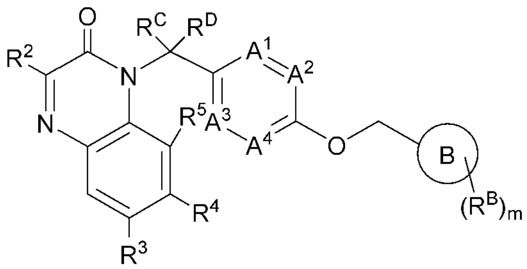

(III-B-1),

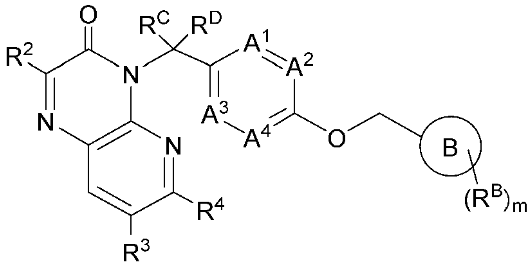

(III-B-2);

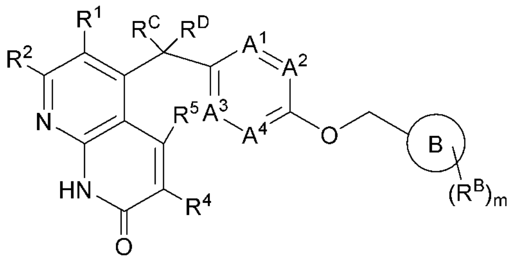

(III-C-1),

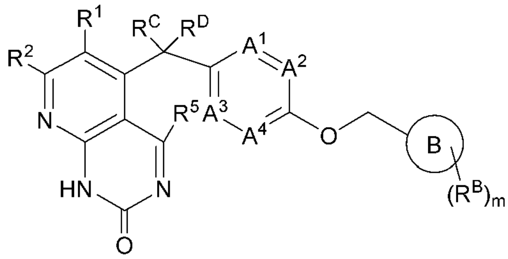

(III-C-2),

or

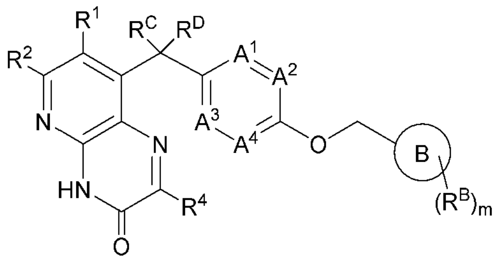

(III-C-3);

or a pharmaceutically acceptable salt thereof. The definitions of the remaining variables are provided in the second embodiment. Alternatively, the definitions of the variables are provided in the first embodiment.

In a sixth embodiment, the present disclosure provides a compound according to any one of the second through fifth embodiments, or a pharmaceutically acceptable salt thereof, wherein

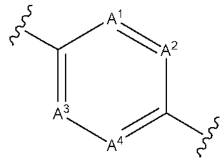

is phenyl or pyridyl, each of which is optionally substituted by one or two $R^A$. The definitions of the remaining variables are provided in any one of the second through fifth embodiments. Alternatively, the definitions of the variables are provided in the first embodiment.

In a seventh embodiment, the present disclosure provides a compound according to any one of the second through sixth embodiments, or a pharmaceutically acceptable salt thereof, wherein ring B is phenyl optionally substituted by one or two $R^B$, each $R^B$ is independently selected from halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, -OR*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*,$-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^B$ is optionally substituted by one or two halogen or $C_{1-6}$alkyl. The definitions of the remaining variables are provided in any one of the second through sixth embodiments. Alternatively, the definitions of the variables are provided in the first embodiment.

In an eigth embodiment, the present disclosure provides a compound according to any one of the second through sixth embodiments, or a pharmaceutically acceptable salt thereof, wherein ring B is 5, 6-membered monocyclic heteroaryl optionally substituted by one or two $R^B$, each $R^B$ is independently selected from halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, -OR*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^B$ is optionally substituted by one or two halogen or $C_{1-6}$alkyl. The definitions of the remaining variables are provided in any one of the second through sixth embodiments. Alternatively, the definitions of the variables are provided in the first embodiment.

In a ninth embodiment, the present disclosure provides a compound according to any one of the second through sixth embodiments, or a pharmaceutically acceptable salt thereof, wherein ring B is phenyl or 5, 6-membered monocyclic heteroaryl, each of which is optionally substituted by one to four $R^B$, and two $R^B$ together with the Ring B atoms to which they attached, form 5,6-membered ring fused to Ring B, said 5,6-membered fused ring is optionally substituted with one or two CN, halogen, $C_{1-4}$alkyl, $C_{1-4}$haloalkyl, $C_{1-4}$alkoxy, or $C_{1-4}$haloalkoxy. The definitions of the remaining variables are provided in any one of the second through sixth embodiments. Alternatively, the definitions of the variables are provided in the first embodiment.

In a tenth embodiment, the present disclosure provides a compound according to any one of the second through sixth embodiments, or a pharmaceutically acceptable salt thereof, wherein ring B is pyrazolyl, isoxazolyl, 1,2,4-oxadiazolyl, thiazolyl, phenyl, pyridyl, pyrimidyl, pyrazinyl, imidazo[1,2-a]pyridinyl, benzo[d]imidazolyl, pyrazolo[1,5-a]pyridinyl, indazolyl, [1,2,4]triazolo[1,5-a]pyridinyl, 4,5,6,7-tetrahydropyrazolo[1,5-a]pyrazinyl, 6,7-dihydro-4H-pyrazolo[5,1-c][1,4]oxazinyl, 5,6-dihydro-4H-pyrrolo[1,2-b]pyrazolyl, or benzo[d][1,3]dioxolyl. The definitions of the remaining variables are provided in any one of the second through sixth embodiments. Alternatively, the definitions of the variables are provided in the first embodiment.

In an eleventh embodiment, the present disclosure provides a compound according to any one of the second through tenth embodiments, or a pharmaceutically acceptable salt thereof, wherein each $R^1$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, -C(O)R*, -C(O)OR*, -C(O)NR*R*, $-(CH_2)_{0-4}NR^*R^*$, -NR*C(O)R*, and -NR*C(O)OR*. The definitions of the remaining variables are provided in any one of the second through tenth embodiments.

In a twelfth embodiment, the present disclosure provides a compound according to any one of the second through eleventh embodiments, or a pharmaceutically acceptable salt thereof, wherein each $R^2$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, -C(O)R*, -C(O)OR*, -C(O)NR*R*, $-(CH_2)_{0-4}NR^*R^*$, -NR*C(O)R*, and -NR*C(O)OR*. The definitions of the remaining variables are provided in any one of the second through eleventh embodiments. Alternatively, the definitions of the variables are provided in the first embodiment.

In a thirteenth embodiment, the present disclosure provides a compound according to any one of the second through twelfth embodiments, or a pharmaceutically acceptable salt thereof thereof, wherein each $R^3$ is independently selected from H, halogen, -CN, -OH, $C_{1-4}$alkyl, $C_{1-4}$haloalkyl, $C_{1-4}$hydroxylalkyl, $-(CH_2)_{0-2}C_{1-4}$alkoxy optionally substituted with one to three deuterium, $C_{1-4}$haloalkoxy, $C_{1-4}$hydroxyalkoxy, -C(O)R*, -C(O)OR*, -C(O)NR*R*, $-(CH_2)_{0-4}NR^*R^*$,

$-NR^*C(O)R^*$, and $-NR^*C(O)OR^*$. The definitions of the remaining variables are provided in any one of the second through twelfth embodiments. Alternatively, the definitions of the variables are provided in the first embodiment.

In a fourteenth embodiment, the present disclosure provides a compound according to any one of the second through thirteenth embodiments, or a pharmaceutically acceptable salt thereof thereof, wherein each $R^4$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, $-C(O)R^*$, $-C(O)OR^*$, $-C(O)NR^*R^*$, $-(CH_2)_{0-4}NR^*R^*$, $-NR^*C(O)R^*$, and $-NR^*C(O)OR^*$. The definitions of the remaining variables are provided in any one of the second through thirteenth embodiments. Alternatively, the definitions of the variables are provided in the first embodiment.

In a fifteenth embodiment, the present disclosure provides a compound according to any one of the second through fourteenth embodiments, or a pharmaceutically acceptable salt thereof, wherein each $R^5$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, $-C(O)R^*$, $-C(O)OR^*$, $-C(O)NR^*R^*$, $-NR^*R^*$, $-NR^*C(O)R^*$, and $-NR^*C(O)OR^*$. The definitions of the remaining variables are provided in any one of the second through fourteenth embodiments. Alternatively, the definitions of the variables are provided in the first embodiment.

In a sixteenth embodiment, the present disclosure provides a compound according to any one of the second through fifteenth embodiments, or a pharmaceutically acceptable salt thereof, wherein each $R^A$ is independently selected from H, halogen, -CN, -OH, $C_{1-4}$alkyl, $C_{1-4}$haloalkyl, $C_{1-4}$hydroxylalkyl, $C_{1-4}$alkoxy optionally substituted with one to three deuterium, $C_{1-4}$haloalkoxy, $C_{1-4}$hydroxyalkoxy, $-C(O)R^*$, $-C(O)OR^*$, $-C(O)NR^*R^*$, $-(CH_2)_{0-4}NR^*R^*$, $-NR^*C(O)R^*$, and $-NR^*C(O)OR^*$. The definitions of the remaining variables are provided in any one of the second through fifteenth embodiments. Alternatively, the definitions of the variables are provided in the first embodiment.

In a seventheenth embodiment, the present disclosure provides a compound according to any one of the second through sixteenth embodiments, wherein each $R^B$ is independently selected from halogen, -CN, -OH, $C_{1-4}$alkyl, $C_{1-4}$haloalkyl, $C_{1-4}$hydroxylalkyl, $-(CH_2)_{0-2}C_{1-4}$alkoxy optionally substituted with one to three deuterium, $C_{1-4}$haloalkoxy, $C_{1-4}$hydroxyalkoxy, $-C(O)R^*$, $-C(O)OR^*$, $-C(O)NR^*R^*$, $-(CH_2)_{0-4}NR^*R^*$, $-NR^*C(O)R^*$, $-NR^*C(O)OR^*$, and $-(CH_2)_{0\ or\ 1}$-3-6 membered cycloalkyl optionally substituted with one or two halogen. The definitions of the remaining variables are provided in any one of the second through sixteenth embodiments. Alternatively, the definitions of the variables are provided in the first embodiment.

In an eighteenth embodiment, the present disclosure provides a compound according to any one of the second through seventeenth embodiments, wherein each $R^*$ is independently H or $C_{1-4}$alkyl. The definitions of the remaining variables are provided in any one of the second through seventeenth embodiments. In certain embodiments, each $R^*$ is independently H or $C_{1-2}$alkyl. Alternatively, the definitions of the variables are provided in the first embodiment.

In a nineteenth embodiment, the present disclosure provides a compound according to any one of the second through eighteenth embodiments, wherein each $R^1$ is independently selected from H, halogen, $C_{1-4}$alkyl, $C_{1-4}$haloalkyl, $C_{1-4}$alkoxy, and $C_{1-4}$haloalkoxy (preferably H); and each $R^2$ is independently selected from H, halogen, $C_{1-4}$alkyl, $C_{1-4}$haloalkyl, $C_{1-4}$alkoxy, and $C_{1-4}$haloalkoxy (preferably H). The definitions of the remaining variables are provided in any one of the second through eighteenth embodiments. In certain embodiments, R' is H and $R^2$ is H. Alternatively, the definitions of the variables are provided in the first embodiment.

In a twentieth embodiment, the present disclosure provides a compound according to any one of the second through nineteenth embodiments, wherein each $R^3$ is independently selected from H, halogen, $C_{1-4}$alkyl, $C_{1-4}$haloalkyl, $C_{1-4}$alkoxy optionally substituted with one to three deuterium, and $C_{1-4}$haloalkoxy. The definitions of the remaining variables are provided in any one of the second through nineteenth embodiments. Alternatively, the definitions of the variables are provided in the first embodiment. In certain embodiments, each $R^3$ is independently H, CN, halogen, $C_{1-4}$alkyl, or $C_{1-4}$alkoxy optionally substituted with one to three deuterium. In a specific embodiment, $R^3$ is H, CN, F, Cl, $-CH_3$, or $-OCH_3$.

In a twenty-first embodiment, the present disclosure provides a compound according to any one of the second through twentieth embodiments, wherein each $R^4$ is independently selected from H, halogen, $C_{1-4}$alkyl, $C_{1-4}$haloalkyl, $C_{1-4}$alkoxy, and $C_{1-4}$haloalkoxy. The definitions of the remaining variables are provided in any one of the second through twentieth embodiments. Alternatively, the definitions of the variables are provided in the first embodiment. In certain embodiments, each $R^4$ is H, F, or $C_{1-4}$alkoxy. In certain embodiments, $R^4$ is H, F, or $-OCH_3$.

In a twenty-second embodiment, the present disclosure provides a compound according to any one of the second through twenty-first embodiments, wherein each $R^5$ is independently selected from H, halogen, $C_{1-4}$alkyl, $C_{1-4}$haloalkyl, $C_{1-4}$alkoxy, and $C_{1-4}$haloalkoxy. The definitions of the remaining variables are provided in any one of the second through twenty-first embodiments. Alternatively, the definitions of the variables are provided in the first embodiment. In certain embodiments, each $R^5$ is H.

In a twenty-third embodiment, the present disclosure provides a compound according to any one of the second through twenty-second embodiments, wherein each $R^A$ is independently selected from H, $C_{1-2}$alkyl, or $C_{1-2}$alkoxy optionally substituted with one to three deuterium. The definitions of the remaining variables are provided in any one of the second through twenty-second embodiments. Alternatively, the definitions of the variables are provided in the first

embodiment. In certain embodiments, each $R^A$ is H, $-OCH_3$, or $-OCD_3$. In certain embodiments, each $R^A$ is H or $-OCH_3$.

In a twenty-fourth embodiment, the present disclosure provides a compound according to any one of the second through twenty-third embodiments, wherein each $R^B$ is independently selected from halogen, -OH, $C_{1-3}$alkyl, $C_{1-3}$haloalkyl, $-(CH_2)_{0-2}C_{1-2}$alkoxy optionally substituted with one to three deuterium, $C_{1-2}$haloalkoxy, $-(CH_2)_{0-2}NR^*R^*$, or 3-6 membered cycloalkyl optionally substituted with one or two halogen (preferably). The definitions of the remaining variables are provided in any one of the second through twenty-third embodiments. Alternatively, the definitions of the variables are provided in the first embodiment. In certain embodiments, each $R^B$ is independently selected from F, Cl, -OH, $-CH_3$, $-CH_2CH_3$, $-CH(CH_3)_2$, $-CHF_2$, $-CF_3$, $-CH_2N(CH_3)_2$,$-CH_2OCH_3$, $-OCH_3$, $-OCHF_2$, $-OCD_3$, and cyclopropyl optionally substituted with one or two fluoro. In certain embodiments, each $R^B$ is independently selected from F, Cl, -OH, $-CH_3$, $-CHF_2$, $-CF_3$, $-CH_2CH_3$, $-CH(CH_3)_2$, $-OCH_3$, $-OCHF_2$, and cyclopropyl.

In a twenty-fifth embodiment, the present disclosure provides a compound according to any one of the second through twenty-fourth embodiments,
wherein

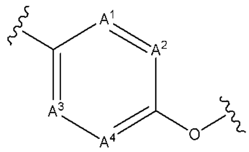 is 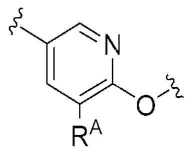 or 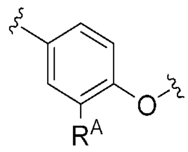 .

The definitions of the remaining variables are provided in any one of the second through twenty-fourth embodiments. Alternatively, the definitions of the variables are provided in the first embodiment.

In a twenty-sixth embodiment, the present disclosure provides a compound according to any one of the second, thirteenth, fourteenth, sixteenth through eighteenth, twentieth, twenty-first, twenty-third, and twenty-fourth embodiments, wherein the compound is represented by Formula (IV-A-1):

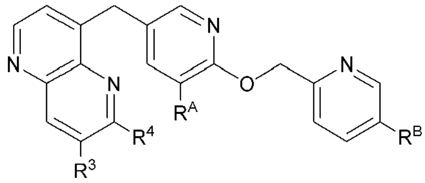
(IV-A-1),

or a pharmaceutically acceptable salt thereof. The definitions of the remaining variables are provided in any one of the second, thirteenth, fourteenth, sixteenth through eighteenth, twentieth, twenty-first, twenty-third, and twenty-fourth embodiments. Alternatively, the definitions of the variables are provided in the first embodiment.

In a twenty-seventh embodiment, the present disclosure provides a compound according to any one of the second, thirteenth, fourteenth, sixteenth through eighteenth, twentieth, twenty-first, twenty-third, and twenty-fourth embodiments, wherein the compound is represented by Formula (IV-A-2):

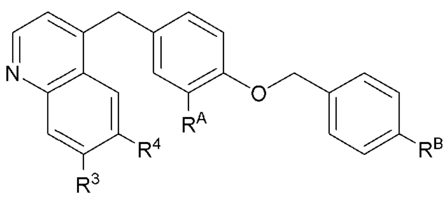
(IV-A-2),

or a pharmaceutically acceptable salt thereof. The definitions of the remaining variables are provided in any one of the second, thirteenth, fourteenth, sixteenth through eighteenth, twentieth, twenty-first, twenty-third, and twenty-fourth embodiments. Alternatively, the definitions of the variables are provided in the first embodiment.

In a twenty-eighth embodiment, the present disclosure provides a compound according to any one of the first through twelfth, fourteenth through nineteenth, and twenty-first through twenty-seventh embodiments, a pharmaceutically acceptable salt, or a tautomer thereof, wherein each $R^3$ is independently selected from H, halogen, -CN, $C_{1-6}$alkyl optionally substituted with one to three deuterium, $C_{1-6}$haloalkyl, $C_{1-6}$hydroxylalkyl, $-C_{1-6}$alkoxy optionally substituted with one to three deuterium, $C_{1-6}$haloalkoxy, $C_{1-6}$hydroxyalkoxy, $-C(O)R^*$, $-C(O)OR^*$, $-C(O)NR^*R^*$, $-NR^*R^*$, 3-6 membered carbocyclyl, 3-6 membered monocyclic heterocyclyl, 7-10 membered bridged heterocyclyl, phenyl, or 5-6 membered heteroaryl; wherein said carbocyclyl, heterocyclyl, phenyl, or heteroaryl in the group represented by $R^3$ is optionally

substituted by one or two CN, halogen, $C_{1-4}$alkyl, $C_{1-4}$haloalkyl, $C_{1-4}$alkoxy, or $C_{1-4}$haloalkoxy. The definitions of the remaining variables are provided in any of the first through twelfth, fourteenth through nineteenth, and twenty-first through twenty-seventh embodiments.

In a twenty-ninth embodiment, the present disclosure provides a compound of the twenty-eighth embodiment, a pharmaceutically acceptable salt, or a tautomer thereof, wherein each $R^3$ is independently selected from H, halogen, CN, $C_{1-4}$alkyl optionally substituted with one to three deuterium, $C_{1-4}$alkoxy optionally substituted with one to three deuterium, $NR^*R^*$, $-C(O)NR^*R^*$, 3-6 membered cycloalkyl, 3-6 membered monocyclic heterocyclyl, 5-6 membered heteroaryl, or 6-oxa-3-azabicyclo[3.1.1]heptanyl, wherein said cycloalkyl, heterocyclyl, or heteroaryl in the group represented by $R^3$ is optionally substituted by one or two halogen, $C_{1-2}$alkyl, $C_{1-2}$haloalkyl, $C_{1-2}$alkoxy, or $C_{1-2}$haloalkoxy. The definitions of the remaining variables are provided in the twenty-eighth embodiment.

In a thirtieth embodiment, the present disclosure provides a compound of the twenty-ninth embodiment, a pharmaceutically acceptable salt, or a tautomer thereof, wherein each $R^3$ is independently selected from H, F, Cl, CN, $CH_3$, $OCH_3$, $OCD_3$, $-N(CH_3)_2$, $-CON(CH_3)_2$, cyclopropanyl, azetidinyl optionally substituted with one or two fluoro or $OCH_3$, imidazole optionally substituted with $CH_3$, morpholinyl, pyridyl, piperazinyl optionally substituted with $CH_3$, pyrrolidinyl optionally substituted with $OCH_3$, pyrazolyl optionally substituted with $CH_3$, thiazole optionally substituted with $CH_3$, or 6-oxa-3-azabicyclo[3.1.1]heptanyl. The definitions of the remaining variables are provided in the twenty-ninth embodiment.

In a thirty-first embodiment, the present disclosure provides a compound of the thirtieth embodiment, a pharmaceutically acceptable salt, or a tautomer thereof, wherein each $R^3$ is independently selected from H or $OCH_3$. The definitions of the remaining variables are provided in the thirtieth embodiment.

In a thirty-second embodiment, the present disclosure provides a compound of any one of the first through sixth, eleventh through twenty-fifth, and twenty-eighth through thirty-first embodiments, a pharmaceutically acceptable salt, or a tautomer thereof, wherein ring B is phenyl, 5 or 6-membered monocyclic heteroaryl optionally substituted by one or two $R^B$, each $R^B$ is independently selected from halogen, -OH, $C_{1-4}$alkyl, $C_{1-4}$haloalkyl, $C_{1-4}$alkoxyalkyl, $C_{1-4}$alkoxy optionally substituted with one to three deuterium, $C_{1-4}$haloalkoxy, $-C(O)NR^*R^*$, $-(CH_2)_{0-1}NR^*R^*$, $-NR^*C(O)R^*$, -3-6 membered monocyclic carbocyclyl, -3-6 membered monocyclic heterocyclyl; wherein said carbocyclyl or heterocyclyl in the group represented by $R^B$ is optionally substituted by one or two halogen or $C_{1-4}$alkyl. The definitions of the remaining variables are provided in the first through sixth, eleventh through twenty-fifth, and twenty-eighth through thirty-first embodiments.

In a thirty-third embodiment, the present disclosure provides a compound of the thirty-second embodiment, a pharmaceutically acceptable salt, or a tautomer thereof, wherein each $R^B$ is independently selected from F, Cl, -OH, $C_{1-4}$alkyl, $C_{1-4}$haloalkyl, $C_{1-4}$alkoxyalkyl, $C_{1-4}$alkoxy optionally substituted with one to three deuterium, $C_{1-4}$haloalkoxy, $-C(O)NR^*R^*$, $-CH_2NR^*R^*$, $-NR^*C(O)R^*$, -3-5 membered monocyclic cycloalkyl, -3-5 membered monocyclic heterocyclyl; wherein said cycloalkyl or heterocyclyl in the group represented by $R^B$ is optionally substituted by one or two F or $_{1-2}$alkyl. The definitions of the remaining variables are provided in the thirty-second embodiment.

In a thirty-fourth embodiment, the present disclosure provides a compound of any one of the first through sixth, eleventh through twenty-fifth, and twenty-seventh through thirty-first embodiments, a pharmaceutically acceptable salt, or a tautomer thereof, wherein ring B is phenyl or 5, 6-membered monocyclic heteroaryl, each of which is optionally substituted by one to four $R^B$, and two $R^B$, together with the ring B atoms to which they attached, form 5-7 membered monocyclic ring or 6-9 membered bicyclic ring fused to ring B, said 5-7 membered monocyclic ring or 6-9 membered bicyclic ring is optionally substituted with one or two CN, halogen, $C_{1-4}$alkyl, $C_{1-4}$haloalkyl, $C_{1-4}$alkoxy, $C_{1-4}$haloalkoxy, or 3-6 membered cycloalkyl. The definitions of the remaining variables are provided in the first through sixth, eleventh through twenty-fifth, and twenty-seventh through thirty-first embodiments.

In a thirty-fifth embodiment, the present disclosure provides a compound of the thirty-fourth embodiment, a pharmaceutically acceptable salt, or a tautomer thereof, wherein two $R^B$, together with the ring B atoms to which they attached, form a ring selected from

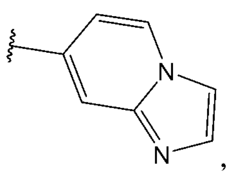,
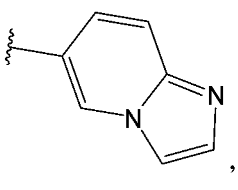,
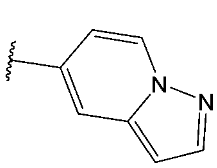,
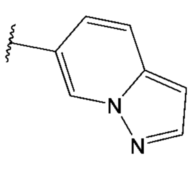,
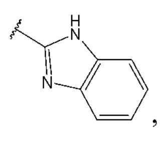,

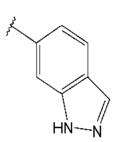,
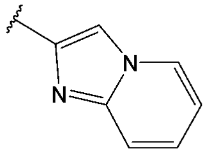,
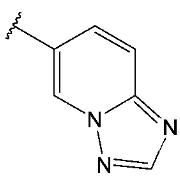,
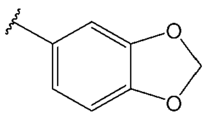,
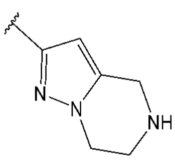,

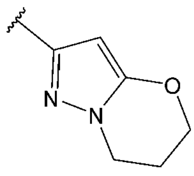, 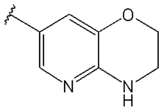, 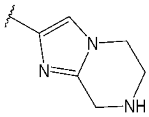, 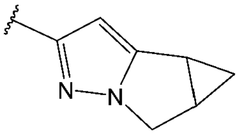, 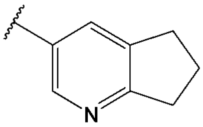,

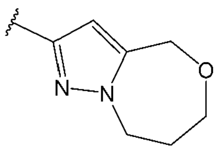, 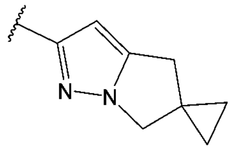, 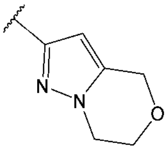, 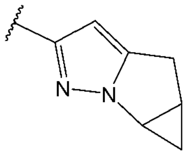, 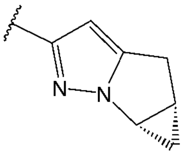,

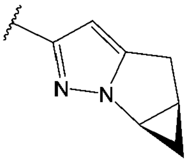, 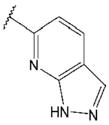, 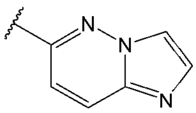, 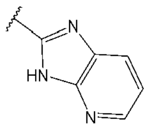, 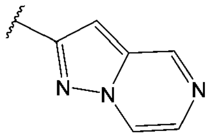,

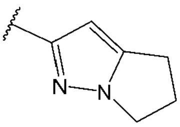, and 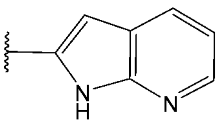,

each of which is optionally substituted with one or two groups selected from halogen, $C_{1-4}$alkyl, $C_{1-4}$haloalkyl, $C_{1-4}$alkoxy, and $C_{1-4}$haloalkoxy. The definitions of the remaining variables are provided in the thirty-fourth embodiment.

In a thirty-sixth embodiment, the present disclosure provides a compound of the thirty-fifth embodiment, a pharmaceutically acceptable salt, or a tautomer thereof, wherein two $R^B$, together with the ring B atoms to which they attached, form a ring selected from

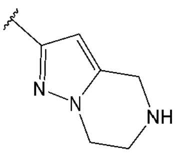, 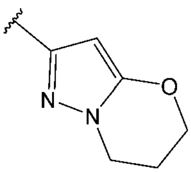, 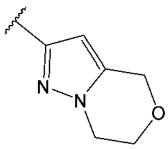, 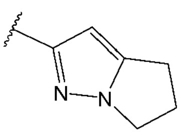 and 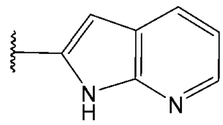.

The definitions of the remaining variables are provided in the thirty-fifth embodiment.

In a thirty-seventh embodiment, the present disclosure provides a compound of any one of the first to thirty-sixth embodiments, a pharmaceutically acceptable salt, or a tautomer thereof, wherein $R^C$ is H, and $R^D$ is H. The definitions of the remaining variables are provided in the first to thirty-sixth embodiments.

In a thirty-eighth embodiment, the present disclosure provides a compound of any of the first, third through twenty-fifth, and twenty-eighth through thirty-sixth embodiments, a pharmaceutically acceptable salt, or a tautomer thereof, wherein $R^C$ is H, F, or deuterium, and $R^D$ is H or deuterium. The definitions of the remaining variables are provided in the first, third through twenty-fifth, and twenty-eighth through thirty-sixth embodiments.

In a thirty-ninth embodiment, the present disclosure provides a compound represented by Formula (I'):

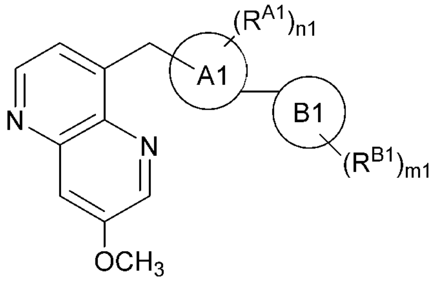

(I')

or a pharmaceutically acceptable salt thereof, wherein:

ring A1 is 5,6 or 6,6-membered nitrogen containing bicyclic heteroaryl;

ring B1 is 3-6 membered monocyclic carbocyclyl or 3-6 membered monocyclic heterocyclyl;

each $R^{A1}$ is independently selected from halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl,-$(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, -$SO_2R^*$, -$(CH_2)_{0-4}NR^*R^*$, -NR*C(O)R*, -NR*C(O)OR*, -$NR^*SO_2R^*$, -$NR^*SO_2NR^*R^*$, -P(O)R*R*, -$(CH_2)_{0\ or\ 1}$-3-12 membered carbocyclyl, -$(CH_2)_{0\ or\ 1}$-3-12 membered heterocyclyl, -$(CH_2)_{0\ or\ 1}$-6-10 membered aryl, or -$(CH_2)_{0\ or\ 1}$-5-10 membered heteroaryl; wherein said carbocyclyl, heterocyclyl, aryl, or heteroaryl in the group represented by $R^{A1}$ is optionally substituted by one or two CN, halogen, $C_{1-6}$alkyl, $C_{1-6}$haloalkyl, $C_{1-6}$alkoxy, or $C_{1-6}$haloalkoxy;

each $R^{B1}$ is independently selected from halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl,-$(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, -$SO_2R^*$, -$(CH_2)_{0-4}NR^*R^*$, -NR*C(O)R*, -NR*C(O)OR*, -$NR^*SO_2R^*$, -$NR^*SO_2NR^*R^*$, -P(O)R*R*, -$(CH_2)_{0\ or\ 1}$-3-12 membered carbocyclyl, -$(CH_2)_{0\ or\ 1}$-3-12 membered heterocyclyl, -$(CH_2)_{0\ or\ 1}$-6-10 membered aryl, or -$(CH_2)_{0\ or\ 1}$-5-10 membered heteroaryl; wherein said carbocyclyl, heterocyclyl, aryl, or heteroaryl in the group represented by $R^{B1}$ is optionally substituted by one or two CN, halogen, $C_{1-6}$alkyl, $C_{1-6}$haloalkyl, $C_{1-6}$alkoxy, or $C_{1-6}$haloalkoxy; and/or

two $R^{B1}$ together with the ring B1 atoms which they attached, form 5,6-membered monocyclic ring or 6-9-membered bicyclic ring fused to ring B1, said 5,6-membered ring or 6-9-membered bicyclic ring is optionally substituted with one or two CN, halogen, $C_{1-6}$alkyl, $C_{1-6}$haloalkyl, $C_{1-6}$alkoxy, $C_{1-6}$haloalkoxy, or 3-6 membered cycloalkyl;

each R* is independently selected from the group consisting of H, $C_{1-6}$alkyl, $C_{1-6}$haloalkyl, $C_{2-6}$alkenyl, $C_{2-6}$alkynyl, 3-6 membered carbocyclyl, or 4-6 membered heterocyclyl;

m1 is 0, 1, 2, 3, or 4; and

n1 is 0, 1, 2, or 3.

In a fortieth embodiment, the present disclosure provides a compound of the thirty-ninth embodiment, or a pharmaceutically acceptable salt thereof, wherein

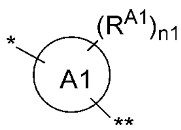

is

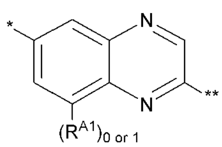 , 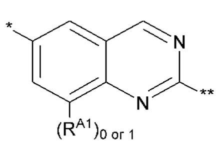 , 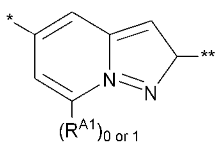 , or 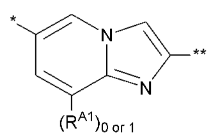 ,

wherein -* represents the point to which -$CH_2$- attaches; -** represents the point to which ring B1 attaches; and $R^{A1}$ is $C_{1-4}$alkyl or $C_{1-4}$alkoxy. The definitions of the remaining variables are provided in the thirty-ninth embodiment.

In a fourty-first embodiment, the present disclosure provides a compound of the thirty-ninth or fourtieth embodiments, or a pharmaceutically acceptable salt thereof, wherein:

ring B1 is

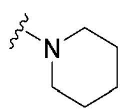 , 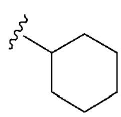 , or 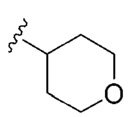 ;

$R^{B1}$ is $C_{1-4}$alkyl or $C_{1-4}$alkoxy; and

n1 is 0 or 1. The definitions of the remaining variables are provided in the thirty ninth or fourtieth embodiments.

In one embodiment, the present disclosure provides a compound selected from the compounds disclosed in

examples and Table 1, or a pharmaceutically acceptable salt thereof provided herein.

*2. Definitions*

The term "halogen," as used herein, refers to fluoride, chloride, bromide, or iodide.

The term "alkyl" used alone or as part of a larger moiety, such as "alkoxy" or "haloalkyl" and the like, means saturated aliphatic straight-chain or branched monovalent hydrocarbon radical of formula $-C_nH_{(2n+1)}$. Unless otherwise specified, an alkyl group typically has 1-6 carbon atoms, *i.e.* $C_{1-6}$alkyl. As used herein, a "$C_{1-6}$alkyl" group means a radical having from 1 to 6 carbon atoms in a linear or branched arrangement. Examples include methyl, ethyl, n-propyl, iso-propyl, n-butyl, iso-butyl, *tert*- butyl, n-pentyl, isopentyl, hexyl, and the like.

The terms "haloalkyl" means alkyl, as the case may be, substituted with one or more halogen atoms. In one embodiment, the alkyl can be substituted by one to three halogens. Examples of haloalkyl, include, but are not limited to, trifluoromethyl, trichloromethyl, pentafluoroethyl and the like.

The terms "hydroxylalkyl" means alkyl, as the case may be, substituted with one or more (e.g., one or two) hydroxy groups. In one embodiment, the alkyl can be substituted by one hydroxyl gropu.

The term "alkenyl" means an alkyl group in which one or more carbon/carbon single bond is replaced by a double bond.

The term "alkynyl" means an alkyl group in which one or more carbon/carbon single bond is replaced by a triple bond.

The term "alkoxy" means an alkyl radical attached through an oxygen linking atom, represented by -O-alkyl. For example, "$(C_1-C_4)$alkoxy" includes methoxy, ethoxy, propoxy, and butoxy.

The term "alkoxyalkyl" means alkyl *(e.g.,* $C_{1-6}$alkyl), as the case may be, substituted with one or more alkoxy groups (e.g., $(C_1-C_4)$alkoxy). In one embodiment, the alkyl can be substituted by one alkoxy group.

The term "carbocyclyl" refers to 3-12 membered non-aromatic hydrocarbon ring system. In one embodiment, carbocyclyl is 3-, 4-, 5-, 6-, 7-, or 8-membered monocyclic or bicyclic or 7-, 8-, 9-, 10-, 11-, or 12-membered bicyclic or tricyclic hydrocarbon ring, any of which may be saturated or partially unsaturated. Any substitutable ring atom can be substituted (e.g., by one or more substituents). Examples of such carbocycles include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, and cyclooctadienyl. In one embodiment, carbocyclyl is intended to include, bridged, fused, and spirocyclic rings. In a spirocyclic carbocyclyl, one atom is common to two different rings. An example of a spirocyclic carbocyclyl is spiro[3.3]heptanyl. In a bridged carbocyclyl, the rings share at least two common non-adjacent atoms. Examples of bridged carbocyclyls include bicyclo[2.2.1]heptanyl, bicyclo[2.2.1]hept-2-enyl, and adamantanyl. In a fused-ring carbocyclyl system, two or more rings may be fused together, such that two rings share one common bond.

The term "cycloalkyl" refers to a cyclic, bicyclic, tricyclic, or polycyclic saturated hydrocarbon groups having 3 to 12 ring carbons. In one embodiment, cycloalkyl may have 3 to 6 ring cabons. Any substitutable ring atom can be substituted (e.g., by one or more substituents). Examples of cycloalkyl groups include, without limitation, cyclopropyl, cyclobutyl, cyclopentyl, cyclohexyl, cycloheptyl, and cyclooctyl. Cycloalkyl may include multiple fused and/or bridged rings. Non-limiting examples of fused/bridged cycloalkyl include: bicyclo[1.1.0]butane, bicyclo[2.1.0]pentane, bicyclo[1.1.0]pentane, bicyclo[3.1.0]hexane, bicyclo[2.1.1]hexane, bicyclo[3.2.0]heptane, bicyclo[4.1.0]heptane, bicyclo[2.2.1]heptane, bicyclo [3.1.1]heptane, bicyclo[4.2.0]octane, bicyclo[3.2.1]octane, bicyclo[2.2.2]octane, and the like. Cycloalkyl also includes spirocyclic rings *(e.g.,* spirocyclic bicycle wherein two rings are connected through just one atom). Non-limiting examples of spirocyclic cycloalkyls include spiro[2.2]pentane, spiro[2.5]octane, spiro[3.5]nonane, spiro[3.5]nonane, spiro[3.5] nonane, spiro[4.4]nonane, spiro[2.6]nonane, spiro[4.5]decane, spiro[3.6]decane, spiro[5.5]undecane, and the like.

The term "heterocyclyl" or "heterocyclic" refers to a radical of a 3- to 12-membered non-aromatic ring system having ring carbon atoms and 1 to 4 ring heteroatoms, wherein each heteroatom is independently selected from nitrogen, quaternary nitrogen, oxidized nitrogen *(e.g.,* NO), oxygen, and sulfur, including sulfoxide and sulfone ("3-12 membered heterocyclyl"). In some embodiments, a heterocyclyl group is a 3-7 membered non-aromatic ring system having ring carbon atoms and 1-4 ring heteroatoms, wherein each heteroatom is independently selected from nitrogen, oxygen, and sulfur ("3-7 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or polycyclic (e.g., a bicyclic system ("bicyclic heterocyclyl") or tricyclic system ("tricyclic heterocyclyl"); polycyclic ring systems include fused, bridged, or spiro ring systems). Heterocyclyl polycyclic ring systems can include heteroatoms in one or more rings in the polycyclic ring system-including polycyclic ring systems having a non-aromatic ring fused to a phenyl or heteroaryl ring. When a heterocyclyl group is a polycyclic ring system, said ring system includes at least one non-aromatic ring. Exemplary monocyclic heterocyclyl groups include azetidinyl, oxetanyl, thietanyl, tetrahydrofuranyl, pyrrolidinyl, piperidinyl, tetrahydropyranyl, piperazinyl, morpholinyl, azepanyl, oxepanyl, thiepanyl, tetrahydropyridinyl, and the like. Heterocyclyl polycyclic ring systems can include heteroatoms in one or more rings in the polycyclic ring system. Substituents may be present on one or more rings in the polycyclic ring system.

Spiro heterocyclyl refers to 5 to 12 membered polycyclic heterocyclyl with rings connected through one common carbon atom (called as spiro atom), wherein said rings have one or more heteroatoms selected from the group consisting of nitrogen, quaternary nitrogen, oxidized nitrogen *(e.g.,* NO), oxygen, and sulfur, including sulfoxide and sulfone, the remaining ring atoms being C, wherein one or more rings may contain one or more double bonds, but none of the rings has a completely conjugated pi-electron system. Representive examples of spiro heterocyclyl include, but are not limited to the following groups:

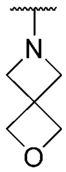, 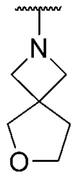, 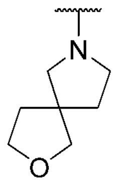, and 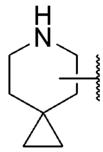.

Fused heterocyclyl refers to a 5 to 12 membered polycyclic heterocyclyl group, wherein each ring in the group shares an adjacent pair of ring atoms with another ring in the group, wherein one or more rings can contain one or more double bonds, but at least one of the rings does not have a completely conjugated π-electron system, and wherein said rings have one or more heteroatoms selected from the group consisting of nitrogen, quaternary nitrogen, oxidized nitrogen (e.g., NO), oxygen, and sulfur, including sulfoxide and sulfone, the remaining ring atoms being C. Representive examples of fused heterocyclyl include, but are not limited to the following groups:

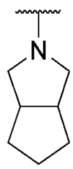, 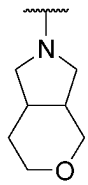, 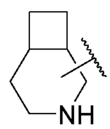, and 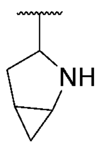.

Bridged heterocyclyl refers to a 5 to 12 membered polycyclic heterocyclyl group, wherein any two rings in the group share two disconnected atoms, the rings can have one or more double bonds but have no completely conjugated π-electron system, and the rings have one or more heteroatoms selected from the group consisting of nitrogen, quaternary nitrogen, oxidized nitrogen *(e.g.,* NO), oxygen, and sulfur, including sulfoxide and sulfone as ring atoms, the remaining ring atoms being C. Representive examples of bridged heterocyclyl include, but are not limited to the following groups:

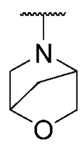, 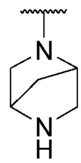, 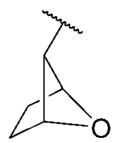, and 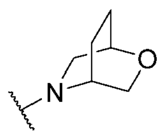.

Generally, the carbocyclyl, the cycloalkyl, or the heterocyclyl may be unsubstituted, or be substituted with one or more substituents as valency allows, wherein the substituents can be independently selected from a number of groups such as oxo, -CN, halogen, alkyl and alkoxyl, opotionally, the alkyl substitution may be further substituted.

The term "aryl" refers to a 6 to 10 membered all-carbon monocyclic ring or a polycyclic fused ring (a "fused" ring system means that each ring in the system shares an adjacent pair of carbon atoms with other ring in the system) group, and has a completely conjugated π-electron system. The term "aryl" may be used interchangeably with the terms "aryl ring" "carbocyclic aromatic ring", "aryl group" and "carbocyclic aromatic group". Representive examples of aryl are phenyl and naphthyl.

The term "heteroaryl" refers to a radical of a 5- to 10-membered aromatic ring system having ring carbon atoms and 1 to 4 ring heteroatoms, wherein each heteroatom is independently selected from nitrogen, oxygen, and sulfur. In some embodiments, a heteroaryl group is a 5 or 6 membered heteroaryl having ring carbon atoms and 1 to 4 ring heteroatoms (typically 1 to 2). A heteroaryl group may be attached through a ring carbon atom or, where valency permits, through a ring nitrogen atom. Generally, the heteroaryl may be unsubstituted, or be substituted with one or more substituents as valency allows with the substituents being independently selected from halogen, OH, alkyl, alkoxyl, and amino *(e.g.,* $NH_2$, NHalkyl, $N(alkyl)_2$), optionally, the alkyl may be further substituted.

Examples of monocyclic 5-6 membered heteroaryl groups include furanyl *(e.g.,* 2-furanyl, 3-furanyl), imidazolyl *(e.g.,* N-imidazolyl, 2-imidazolyl, 4-imidazolyl, 5-imidazolyl), isoxazolyl (e.g., 3-isoxazolyl, 4-isoxazolyl, 5-isoxazolyl), oxadiazolyl *(e.g.,* 2-oxadiazolyl, 5-oxadiazolyl), oxazolyl *(e.g.,* 2-oxazolyl, 4-oxazolyl, 5-oxazolyl), pyrazolyl *(e.g.,* 3-pyrazolyl, 4-pyrazolyl), pyrrolyl *(e.g.,* 1-pyrrolyl, 2-pyrrolyl, 3-pyrrolyl), pyridyl *(e.g.,* 2-pyridyl, 3-pyridyl, 4-pyridyl), pyrimidinyl *(e.g.,* 2-pyrimidinyl, 4-pyrimidinyl, 5-pyrimidinyl), pyridazinyl *(e.g.,* 3-pyridazinyl), thiazolyl *(e.g.,* 2-thiazolyl, 4-thiazolyl, 5-thiazolyl), triazolyl *(e.g.,* 2-triazolyl, 5-triazolyl), tetrazolyl *(e.g.,* tetrazolyl), thienyl *(e.g.,* 2-thienyl, 3-thienyl), pyrimidinyl, pyridinyl and pyridazinyl. Examples of polycyclic aromatic heteroaryl groups include carbazolyl, benzimidazolyl, benzothienyl, benzofuranyl, indolyl, quinolinyl, benzotriazolyl, benzothiazolyl, benzoxazolyl, benzimidazolyl, isoquinolinyl, indolyl, isoindolyl, acridinyl, or benzisoxazolyl. A "substituted heteroaryl group" is substituted at any one or more substitutable ring atom, which is a ring carbon or ring nitrogen atom bonded to a hydrogen.

As used herein, many moieties (e.g., alkyl, alkylene, cycloalkyl, aryl, heteroaryl, or heterocyclyl ) are referred to as being either "substituted" or "optionally substituted". When a moiety is modified by one of these terms, unless otherwise noted, it denotes that any portion of the moiety that is known to one skilled in the art as being available for substitution can be substituted, which includes one or more substituents. Where if more than one substituent is present, then each substituent may be independently selected. Such means for substitution are well-known in the art and/or taught by the instant disclosure. The optional substituents can be any substituents that are suitable to attach to the moiety.

Where suitable substituents are not specifically enumerated, exemplary substituents include, but are not limited to: $C_{1-5}$alkyl, $C_{1-5}$hydroxyalkyl, $C_{1-5}$haloalkyl, $C_{1-5}$alkoxy, $C_{1-5}$ haloalkoxy, halogen, hydroxyl, cyano, amino, -CN, $-NO_2$, $-OR^{c1}$, $-NR^{a1}R^{b1}$, $-S(O)_iR^{a1}$, $-NR^{a1}S(O)_iR^{b1}$, $-S(O)_iNR^{a1}R^{b1}$, $-C(=O)OR^{a1}$, $-OC(=O)OR^{a1}$, $-C(=S)OR^{a1}$, $-O(C=S)R^{a1}$, $-C(=O)NR^{a1}R^{b1}$, $-NR^{a1}C(=O)R^{b1}$, $-C(=S)NR^{a1}R^{b1}$, $-C(=O)R^{a1}$, $-C(=S)R^{a1}$, $NR^{a1}C(=S)R^{b1}$, $-O(C=O)NR^{a1}R^{b1}$, $-NR^{a1}(C=S)OR^{b1}$, $-O(C=S)NR^{a1}R^{b1}$, $-NR^{a1}(C=O)NR^{a1}R^{b1}$, $-NR^{a1}(C=S)NR^{a1}R^{b1}$, phenyl, or 5-6 membered heteroaryl. Each $R^{a1}$ and each $R^{b1}$ are independently selected from -H and $C_{1-5}$alkyl, optionally substituted with hydroxyl or $C_{1-3}$alkoxy; $R^{c1}$ is -H, $C_{1-5}$haloalkyl or $C_{1-5}$alkyl, wherein the $C_{1-5}$alkyl is optionally substituted with hydroxyl or $C_1$-$C_3$alkoxy.

The symbol

“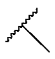”

as used herein, refers to the point where the moiety attaches.

***Pharmaceutically Acceptable Salts***

The term "pharmaceutically-acceptable salt" refers to a pharmaceutical salt that is, within the scope of sound medical judgment, suitable for use in contact with the tissues of humans and lower animals without undue toxicity, irritation, and allergic response, and is commensurate with a reasonable benefit/risk ratio. Pharmaceutically-acceptable salts are well known in the art. For example, S. M. Berge *et al*. describes pharmacologically acceptable salts in J. Pharm. Sci., 1977, 66, 1-19.

Pharmaceutically acceptable salts of the compounds of any one of the formulae described above include acid addition and base salts.

Included in the present teachings are pharmaceutically acceptable salts of the compounds disclosed herein. Compounds having basic groups can form pharmaceutically acceptable salts with pharmaceutically acceptable acid(s). Suitable pharmaceutically acceptable acid addition salts of the compounds described herein include salts of inorganic acids (such as hydrochloric, hydrobromic, phosphoric, metaphosphoric, nitric, and sulfuric acids) and of organic acids (such as acetic, benzene sulfonic, benzoic, ethanesulfonic, methanesulfonic, and succinic acids). Compounds of the present teachings with acidic groups such as carboxylic acids can form pharmaceutically acceptable salts with pharmaceutically acceptable base(s). Suitable pharmaceutically acceptable basic salts include ammonium salts, alkali metal salts (such as sodium and potassium salts) and alkaline earth metal salts (such as magnesium and calcium salts).

Pharmaceutically acceptable salts of compounds of any one of the formulae described above may be prepared by one or more of three methods:

(i) by reacting the compound of any one of the formulae described above with the desired acid or base;

(ii) by removing an acid- or base-labile protecting group from a suitable precursor of the compound of any one of the formulae described above or by ring-opening a suitable cyclic precursor, for example, a lactone or lactam, using the desired acid or base; or

(iii) by converting one salt of the compound of any one of the formulae described above to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of any one of the formulae described above, and pharmaceutically acceptable salts thereof, may

exist in unsolvated and solvated forms.

### *Stereoisomers and Other Variations*

The compounds of any one of the formulae described above may exhibit one or more kinds of isomerism (e.g. optical, geometric or tautomeric isomerism). Such variation is implicit to the compounds of any one of the formulae described above defined as they are by reference to their structural features and therefore within the scope of the present disclosure.

Compounds having one or more chiral centers can exist in various stereoisomeric forms, *i.e.,* each chiral center can have an R or S configuration, or can be a mixture of both. Stereoisomers are compounds that differ only in their spatial arrangement. Stereoisomers include all diastereomeric and enantiomeric forms of a compound. Enantiomers are stereoisomers that are mirror images of each other. Diastereomers are stereoisomers having two or more chiral centers that are not identifcal and are not mirror images of each other.

When a compound is designated by its chemical name (e.g., where the configuration is indicated in the chemical name by "R" or "S") or its structure *(e.g.,* the configuration is indicated by "wedge" bonds) that indicates a single enantiomer, unless indicated otherwise, the compound is at least 60%, 70%, 80%, 90%, 99% or 99.9% optically pure (also referred to as "enantiomerically pure"). Optical purity is the weight in the mixture of the named or depicted enantiomer divided by the total weight in the mixture of both enantiomers.

When the stereochemistry of a disclosed compound is named or depicted by structure, and the named or depicted structure encompasses more than one stereoisomer *(e.g.,* as in a diastereomeric pair), it is to be understood that one of the encompassed stereoisomers or any mixture of the encompassed stereoisomers is included. It is to be further understood that the stereoisomeric purity of the named or depicted stereoisomers at least 60%, 70%, 80%, 90%, 99% or 99.9% by weight. The stereoisomeric purity in this case is determined by dividing the total weight in the mixture of the stereoisomers encompassed by the name or structure by the total weight in the mixture of all of the stereoisomers.

When two stereoisomers are depicted by their chemical names or structures, and the chemical names or structures are connected by an "and", a mixture of the two stereoisomers is intended.

When two stereoisomers are depicted by their chemical names or structures, and the names or structures are connected by an "or", one or the other of the two stereoisomers is intended, but not both.

When a disclosed compound having a chiral center is depicted by a structure without showing a configuration at that chiral center, the structure is meant to encompass the compound with the S configuration at that chiral center, the compound with the R configuration at that chiral center, or the compound with a mixture of the R and S configuration at that chiral center. When a disclosed compound having a chiral center is depicted by its chemical name without indicating a configuration at that chiral center with "S" or "R", the name is meant to encompass the compound with the S configuration at that chiral center, the compound with the R configuration at that chiral center or the compound with a mixture of the R and S configuration at that chiral center.

Racemic mixture means 50% of one enantiomer and 50% of the corresponding enantiomer. When a compound with one chiral center is named or depicted without indicating the stereochemistry of the chiral center, it is understood that the name or structure encompasses both possible enantiomeric forms (e.g., both enantiomerically-pure, enantiomerically-enriched or racemic) of the compound. When a compound with two or more chiral centers is named or depicted without indicating the stereochemistry of the chiral centers, it is understood that the name or structure encompasses all possible diasteriomeric forms (e.g., diastereomerically pure, diastereomerically enriched and equimolar mixtures of one or more diastereomers (e.g., racemic mixtures) of the compound.

The term "geometric isomer" means isomers that differ in the orientation of substituent atoms in relationship to a carbon-carbon double bond, to a carbocyclic ring, or to a bridged bicyclic system. Substituent atoms (other than hydrogen) on each side of a carbon-carbon double bond may be in an E or Z configuration according to the Cahn-Ingold-Prelog priority rules. In the "E" configuration, the substituents having the highest priorities are on opposite sides in relationship to the carbon-carbon double bond. In the "Z" configuration, the substituents having the highest priorities are oriented on the same side in relationship to the carbon-carbon double bond.

Substituents around a carbon-carbon double bond can also be referred to as "cis" or "trans," where "cis" represents substituents on the same side of the double bond and "trans" represents substituents on opposite sides of the double bond. The arrangement of substituents around a carbocyclic ring can also be designated as "cis" or "trans." The term "cis" represents substituents on the same side of the plane of the ring, and the term "trans" represents substituents on opposite sides of the plane of the ring. Mixtures of compounds wherein the substituents are disposed on both the same and opposite sides of plane of the ring are designated "cis/trans."

The compounds of any one of the formulae described above may exhibit one or more kinds of tautomeric isomerism. Such variation is implicit to the compounds of any one of the formulae described above defined as they are by reference to their structural features and therefore within the scope of the present disclosure.

Where structural isomers are interconvertible via a low energy barrier, tautomeric isomerism ("tautomerism") can

occur. This can take the form of proton tautomerism in compounds of any one of the formulae described above containing, for example, an imino, keto, or oxime group, or so-called valence tautomerism in compounds which contain an aromatic moiety. It follows that a single compound may exhibit more than one type of isomerism.

In certain instances tautomeric forms of the disclosed compounds exist, such as the tautomeric structures shown below:

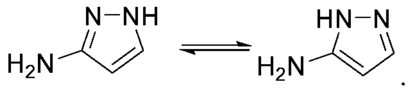

When a geometric isomer is depicted by name or structure, it is to be understood that the named or depicted isomer exists to a greater degree than another isomer, that is that the geometric isomeric purity of the named or depicted geometric isomer is greater than 50%, such as at least 60%, 70%, 80%, 90%, 99%, or 99.9% pure by weight. Geometric isomeric purity is determined by dividing the weight of the named or depicted geometric isomer in the mixture by the total weight of all of the geomeric isomers in the mixture.

It must be emphasized that the compounds of any one of the formulae described above have been drawn herein in a single tautomeric form, all possible tautomeric forms are included within the scope of the present disclosure.

*3. Administration and Dosing*

Typically, a compound of the present disclosure is administered in an amount effective to treat a condition as described herein. The compounds of the present disclosure can be administered as compound *per se,* or alternatively, as a pharmaceutically acceptable salt. For administration and dosing purposes, the compound per se or pharmaceutically acceptable salt thereof will simply be referred to as the compounds of the present disclosure.

The compounds of the present disclosure are administered by any suitable route in the form of a pharmaceutical composition adapted to such a route, and in a dose effective for the treatment intended. The compounds of the present disclosure may be administered orally, rectally, vaginally, parenterally, or topically.

The compounds of the present disclosure may be administered orally. Oral administration may involve swallowing, so that the compound enters the gastrointestinal tract, or buccal or sublingual administration may be employed by which the compound enters the bloodstream directly from the mouth.

In another embodiment, the compounds of the present disclosure may also be administered directly into the bloodstream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

In another embodiment, the compounds of the present disclosure may also be administered topically to the skin or mucosa, that is, dermally or transdermally. In another embodiment, the compounds of the present disclosure can also be administered intranasally or by inhalation. In another embodiment, the compounds of the present disclosure may be administered rectally or vaginally. In another embodiment, the compounds of the present disclosure may also be administered directly to the eye or ear.

The dosage regimen for the compounds of the present disclosure and/or compositions containing said compounds is based on a variety of factors, including the type, age, weight, sex and medical condition of the patient; the severity of the condition; the route of administration; and the activity of the particular compound employed. Thus the dosage regimen may vary widely. In one embodiment, the total daily dose of a compound of the present disclosure is typically from about 0.001 to about 100 mg/kg *(i.e.,* mg compound of the present disclosure per kg body weight) for the treatment of the indicated conditions discussed herein.

For oral administration, the compositions may be provided in the form of tablets containing 0.1- 500 milligrams of the active ingredient for the symptomatic adjustment of the dosage to the patient. A medicament typically contains from about 0.01 mg to about 500 mg of the active ingredient. Intravenously, doses may range from about 0.01 to about 10 mg/kg/minute during a constant rate infusion.

Suitable subjects according to the present disclosure include mammalian subjects, including non-human mammal such as primates, rodents (mice, rats, hamsters, rabbits *etc).* In one embodiment, humans are suitable subjects. Human subjects may be of either gender and at any stage of development.

*4. Pharmaceutical Compositions*

In another embodiment, the present disclosure comprises pharmaceutical compositions. Such pharmaceutical compositions comprise a compound of the present disclosure presented, or a pharmaceutically acceptable salt thereof

with a pharmaceutically acceptable carrier or excipient. Other pharmacologically active substances can also be present.

As used herein, "pharmaceutically acceptable carrier or excipient" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof, and may include isotonic agents, for example, sugars, sodium chloride, or polyalcohols such as mannitol, or sorbitol in the composition. Pharmaceutically acceptable substances such as wetting agents or minor amounts of auxiliary substances such as wetting or emulsifying agents, preservatives or buffers, which enhance the shelf life or effectiveness of the antibody or antibody portion.

The compositions of present disclosure may be in a variety of forms. These include, for example, liquid, semi-solid and solid dosage forms, such as liquid solutions *(e.g.,* injectable and infusible solutions), dispersions or suspensions, tablets, pills, powders, liposomes and suppositories. The form depends on the intended mode of administration and therapeutic application.

Typical compositions are in the form of injectable or infusible solutions, such as compositions similar to those used for passive immunization of humans with antibodies in general. One mode of administration is parenteral (e.g. intravenous, subcutaneous, intraperitoneal, intramuscular). In another embodiment, the antibody is administered by intravenous infusion or injection. In yet another embodiment, the antibody is administered by intramuscular or subcutaneous injection.

Oral administration of a solid dose form may be, for example, presented in discrete units, such as hard or soft capsules, pills, cachets, lozenges, or tablets, each containing a predetermined amount of at least one compound of the present disclosure. In another embodiment, the oral administration may be in a powder or granule form. In another embodiment, the oral dose form is sub-lingual, such as, for example, a lozenge. In such solid dosage forms, the compounds of any one of the formulae described above are ordinarily combined with one or more adjuvants. Such capsules or tablets may contain a controlled release formulation. In the case of capsules, tablets, and pills, the dosage forms also may comprise buffering agents or may be prepared with enteric coatings.

In another embodiment, oral administration may be in a liquid dose form. Liquid dosage forms for oral administration include, for example, pharmaceutically acceptable emulsions, solutions, suspensions, syrups, and elixirs containing inert diluents commonly used in the art (e.g., water). Such compositions also may comprise adjuvants, such as wetting, emulsifying, suspending, flavoring (e.g., sweetening), and/or perfuming agents.

In another embodiment, the present disclosure comprises a parenteral dose form.

"Parenteral administration" includes, for example, subcutaneous injections, intravenous injections, intraperitoneally, intramuscular injections, intrasternal injections, and infusion. Injectable preparations *(i.e.,* sterile injectable aqueous or oleaginous suspensions) may be formulated according to the known art using suitable dispersing, wetting agents, and/or suspending agents.

In another embodiment, the present disclosure comprises a topical dose form.

"Topical administration" includes, for example, transdermal administration, such as via transdermal patches or iontophoresis devices, intraocular administration, or intranasal or inhalation administration. Compositions for topical administration also include, for example, topical gels, sprays, ointments, and creams. A topical formulation may include a compound which enhances absorption or penetration of the active ingredient through the skin or other affected areas. When the compounds of present disclosure are administered by a transdermal device, administration will be accomplished using a patch either of the reservoir and porous membrane type or of a solid matrix variety. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, Finnin and Morgan, J. Pharm. Sci., 88:955-958, 1999.

Formulations suitable for topical administration to the eye include, for example, eye drops wherein the compound of present disclosure is dissolved or suspended in a suitable carrier. A typical formulation suitable for ocular or aural administration may be in the form of drops of a micronized suspension or solution in isotonic, pH-adjusted, sterile saline. Other formulations suitable for ocular and aural administration include ointments, biodegradable *(i.e.,* absorbable gel sponges, collagen) and non-biodegradable *(i.e.,* silicone) implants, wafers, lenses and particulate or vesicular systems, such as niosomes or liposomes. A polymer such as crossed linked polyacrylic acid, polyvinyl alcohol, hyaluronic acid, a cellulosic polymer, for example, hydroxypropylmethylcellulose, hydroxyethylcellulose, or methylcellulose, or a heteropolysaccharide polymer, for example, gelan gum, may be incorporated together with a preservative, such as benzalkonium chloride. Such formulations may also be delivered by iontophoresis.

For intranasal administration or administration by inhalation, the compounds of the present disclosure are conveniently delivered in the form of a solution or suspension from a pump spray container that is squeezed or pumped by the patient or as an aerosol spray presentation from a pressurized container or a nebulizer, with the use of a suitable propellant. Formulations suitable for intranasal administration are typically administered in the form of a dry powder (either

alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

In another embodiment, the present disclosure comprises a rectal dose form. Such rectal dose form may be in the form of, for example, a suppository. Cocoa butter is a traditional suppository base, but various alternatives may be used as appropriate.

Other carrier materials and modes of administration known in the pharmaceutical art may also be used. Pharmaceutical compositions of the present disclosure may be prepared by any of the well-known techniques of pharmacy, such as effective formulation and administration procedures.

The above considerations in regard to effective formulations and administration procedures are well known in the art and are described in standard textbooks. Formulation of drugs is discussed in, for example, Hoover, John E., Remington's Pharmaceutical Sciences, Mack Publishing Co., Easton, Pa., 1975; Liberman et al., Eds., Pharmaceutical Dosage Forms, Marcel Decker, New York, N.Y., 1980; and Kibbe et al., Eds., Handbook of Pharmaceutical Excipients (3rd Ed.), American Pharmaceutical Association, Washington, 1999.

*5. Method of Treatment*

Compounds of the present disclosure can inhibit CSF-1R and therefore are useful for treating diseases wherein the underlying pathology is, wholly or partially, mediated by CSF-1R. Such diseases include an autoimmune disease, an inflammatory disease, a neurodegenerative disease, cancer, a metabolic disease, obesity, or an obesity-related disease.

The term "autoimmune disease" refers to a disease or disorder arising from and/or directed against an individual's own tissues or organs, or a co-segregate or manifestation thereof, or resulting condition therefrom. Examples of autoimmune diseases include, but are not limited to: chronic obstructive pulmonary disease (COPD), allergic rhinitis, lupus erythematosus, myasthenia gravis, multiple sclerosis (MS), rheumatoid arthritis (RA), collagen-induced arthritis, psoriasis, inflammatory bowel disease (IBD), Crohn's disease, ulcerative colitis, asthma, autoimmune nephritis, idiopathic thrombocytopenic purpura (ITP) and myeloproliferative disease, such as myelofibrosis, post-polycythemia vera/essential thrombocytosis myelofibrosis (post-PV/ET myelofibrosis) .

The term "inflammatory disease" or "inflammatory disorder" refers to a pathological state that leads to inflammation, especially due to neutrophil chemotaxis. Non-limiting examples of inflammatory diseases include systemic inflammation and local inflammation, inflammation associated with immunosuppression, organ-graft refection, allergic disease, inflammatory skin disease (including psoriasis and atopic dermatitis); systemic scleroderma and sclerosis; reactions associated with inflammatory bowel diseases (IBD, such as Crohn's disease and ulcerative colitis); ischemia reperfusion injury, including reperfusion injury of tissue caused by surgery, myocardial ischemia, such as myocardial infarction, cardiac arrest, reperfusion after heart operation and abnormal contractile response of coronary vessel after percutaneous transluminal coronary angioplasty, surgical tissue reperfusion injury of stroke and abdominal aortic aneurysm; cerebral edema secondary to stroke; cranial trauma, and hemorrhagic shock; asphyxia; adult respiratory distress syndrome; acute lung injury; Behcet's disease; dermatomyositis; polymyositis; multiple sclerosis (MS); dermatitis; meningitis; encephalitis; uveitis; osteoarthritis; lupus nephritis; autoimmune disease such as rheumatoid arthritis (RA), Sjogren's syndrome, and vasculitis; diseases involving leukopedesis; central nervous system (CNS) inflammatory disease and multiple organ injury syndrome secondary to septicemia or trauma; alcoholic hepatitis; bacterial pneumonia; antigen-antibody complex mediated disease, including glomerulonephritis; pyaemia; sarcoidosis; immunopathologic responses to tissue/organ transplantation; lung inflammation, including pleurisy, alveolitis, vasculitis, pneumonia, chronic bronchitis, bronchiectasia, diffuse panbronchiolitis, hypersensitivity pneumonitis, idiopathic pulmonary fibrosis (IPF) , cystic fibrosis, *etc.* Preferably indications include, but are not limited to, chronic inflammation, autoimmune diabetes, rheumatoid arthritis (RA), rheumatoid spondylitis, gouty arthritis and other arthrosis conditions, multiple sclerosis (MS), asthma, systemic lupus erythematosus, adult respiratory distress syndrome, Behcet's disease, psoriasis, chronic pulmonary inflammatory disease, graft versus host reaction, Crohn's disease, ulcerative colitis, inflammatory bowel disease (IBD), Alzheimer's disease and pyresis, and any diseases associated with inflammation and related conditions.

In some embodiments, the autoimmune disease or inflammatory disease is chosen from rheumatoid arthritis, collagen-induced arthritis, osteoarthritis, pigmented villonodular synovitis (PVNS), systemic lupus erythematosus, multiple sclerosis, systemic scleroderma, autoimmune nephritis, inflammatory bowel disease, Crohn's disease, ulcerative colitis, psoriasis, atopic dermatitis, asthma, chronic obstructive pulmonary disease, Behcet's disease, idiopathic thrombocytopenic purpura, spinal arthritis, systemic juvenile idiopathic arthritis (SoJIA), pancreatitis, ischemia reperfusion injury of parenchymatous organs, organ-graft rejection, septicemia, systemic inflammatory response syndrome and chemotherapy drugs induced organ injury.

The compound of formula (I) or a pharmaceutically acceptable salt thereof described herein can be used to

achieve a beneficial therapeutic or prophylactic effect, for example, in subjects with a neurodegenerative disease.

The term "neurodegenerative diseases" refers to degenerative diseases or disorders of the nervous system caused by neuronal degeneration and apoptosis. Examples of neurodegenerative diseases include, but are not limited to: Parkinson's disease (PD), multiple system atrophy, Alzheimer's disease (AD), frontotemporal lobar dementia, Huntington's disease (HD), corticobasal degeneration, spinocerebellar ataxia, amyotrophic lateral sclerosis (ALS), spinal muscular atrophy (SMA), hereditary motor and sensory neuropathy (CMT), *etc.*

In some embodiments, the neurodegenerative disease is chosen from Parkinson's disease (PD), Alzheimer's disease (AD), Huntington's disease (HD), amyotrophic lateral sclerosis (ALS) and spinal muscular atrophy (SMA).

The compound of formula (I) or a pharmaceutically acceptable salt thereof described herein can be used to achieve a beneficial therapeutic or prophylactic effect, for example, in subjects with cancer.

As used herein, the term "cancer" refers to a cellular disorder characterized by uncontrolled or disregulated cell proliferation, decreased cellular differentiation, inappropriate ability to invade surrounding tissue, and/or ability to establish new growth at ectopic sites. The term "cancer" includes, but is not limited to, solid tumors and hematologic malignancies. The term "cancer" encompasses diseases of skin, tissues, organs, bone, cartilage, blood, and vessels. The term "cancer" encompasses primary cancer, and further metastatic cancer.

Non-limiting examples of solid tumors include pancreatic cancer; bladder cancer; colorectal cancer; colon cancer; breast cancer, including metastatic breast cancer; prostate cancer, including androgen-dependent and androgen-independent prostate cancer; testicular cancer; renal cancer, including, e.g., metastatic renal cell carcinoma; urothelial carcinoma; liver cancer; hepatocellular cancer; lung cancer, including, e.g., non-small cell lung cancer (NSCLC) , bronchioloalveolar carcinoma (BAC) , and adenocarcinoma of the lung; ovarian cancer, including, e.g., progressive epithelial or primary peritoneal cancer; cervical cancer; endometrial cancer; gastrointestinal stromal tumor (GIST); gastric cancer; esophageal cancer; head and neck cancer, including, e.g., squamous cell carcinoma of the head and neck; skin cancer, including, e.g., melanoma and basal carcinoma; neuroendocrine cancer, including metastatic neuroendocrine tumors; brain tumors, including, *e.g.,* glioma, anaplastic oligodendroglioma, adult glioblastoma multiforme, and adult anaplastic astrocytoma; bone cancer; sarcoma, including, e.g., Kaposi's sarcoma; adrenal carcinoma; mesothelioma; mesothelial carcinoma; choriocarcinoma; muscle carcinoma; connective tissue carcinoma; tenosynovial giant cell tumor; and thyroid carcinoma.

Non-limiting examples of hematologic malignancies include acute myelogenous leukemia (AML); chronic myelogenous leukemia (CML), including accelerated phase CML and CML blastic phase (CML-BP); acute lymphocytic leukemia (ALL); chronic lymphocytic leukemia (CLL); Hodgkin's lymphoma; non-Hodgkin's lymphoma (NHL); follicular lymphoma; mantle cell lymphoma (MCL); B-cell lymphoma; T cell lymphoma; diffuse large B-cell lymphoma (DLBCL); multiple myeloma (MM); Waldenstrom macroglobulinemia; myelodysplastic syndrome (MDS), including refractory anemia (RA) , refractory anemia with ring sideroblasts (RARS), refractory anemia with excess blasts (RAEB) and refractory anemia with excess blasts in transformation (RAEB-T); and myeloproliferative syndrome.

In some embodiments, the solid tumors include ovarian cancer, lung cancer (including non-small cell lung cancer) , glioblastoma (GBM), tenosynovial giant cell tumor, gastrointestinal stromal tumor (GIST), gastric cancer, esophageal cancer, colon cancer, colorectal cancer, pancreatic cancer, prostate cancer, breast cancer, cervical cancer, melanoma, mesothelioma, mesothelial carcinoma, renal cancer, liver cancer, thyroid carcinoma, head and neck cancer, urothelial carcinoma, bladder cancer, endometrial cancer, choriocarcinoma, adrenal carcinoma and sarcoma.

In some embodiments, typical hematologic malignancies include leukemia, for example acute lymphocytic leukemia (ALL), acute myelogenous leukemia (AML), chronic lymphocytic leukemia (CLL) and chronic myelogenous leukemia (CML); multiple myeloma (MM); and lymphoma, for example Hodgkin's lymphoma, non-Hodgkin's lymphoma (NHL), mantle cell lymphoma (MCL), follicular lymphoma, B-cell lymphoma, T-cell lymphoma and diffuse large B-cell lymphoma (DLBCL).

The term "metabolic diseases" refers to diseases or disorders caused by metabolic problems, including metabolic disorders and hypermetabolism. Examples of metabolic diseases include, but are not limited to: osteoporosis, diabetes, diabetic ketoacidosis, hyperglycemia and hyperosmolar syndrome, hypoglycemia, gout, protein-energy malnutrition, vitamin A deficiency disease, scurvy, vitamin D deficiency disease, *etc.*

The term "obesity-related diseases" refers to diseases or disorders related to, resulted from, or caused by obesity. Examples of obesity-related disease include, but are not limited to: diabetes, hypertension, insulin resistance syndrome, dyslipidemia, heart disease, cardiovascular disease (including atherosclerosis, abnormal heart rhythms, arrhythmias, myocardial infarction, congestive heart failure, coronary heart disease, and angina pectoris) , cerebral infarction, cerebral hemorrhage, osteoarthritis, metabolic syndrome, non-alcoholic fatty liver disease, non-alcoholic steatohepatitis, and the like.

The terms "subject", "individual" or "patient," used interchangeably, refer to any animal, including mammals, preferably mice, rats, other rodents, rabbits, dogs, cats, swine, cattle, sheep, horses, or primates, and most preferably humans.

The terms "treatment," "treat," and "treating" refer to reversing, alleviating, or inhibiting the progress of a disease

described herein. In some embodiments, treatment may be administered after one or more signs or symptoms of the disease have developed or have been observed *(i.e.,* therapeutic treatment). In other embodiments, treatment may be administered in the absence of signs or symptoms of the disease. For example, treatment may be administered to a susceptible subject prior to the onset of symptoms *(i.e.,* prophylactic treatment) (e.g., in light of a history of symptoms and/or in light of exposure to a pathogen). Treatment may also be continued after symptoms have resolved, for example, to delay or prevent recurrence.

The terms "condition," "disease," and "disorder" are used interchangeably.

The term "administer," "administering," or "administration" refers to methods introducing a compound disclosed herein, or a composition thereof, in or on a patient. These methods include, but are not limited to, intraarticular (in the joints), intravenous, intramuscular, intratumoral, intradermal, intraperitoneal, subcutaneous, orally, topically, intrathecally, inhalationally, transdermally, rectally, and the like. Administration techniques that can be employed with the agents and methods described herein are found in *e.g.,* Goodman and Gilman, The Pharmacological Basis of Therapeutics, current ed.; Pergamon; and Remington's, Pharmaceutical Sciences (current edition), Mack Publishing Co., Easton, Pa.

Generally, an effective amount of a compound taught herein varies depending upon various factors, such as the given drug or compound, the pharmaceutical formulation, the route of administration, the type of disease or disorder, the identity of the subject or host being treated, and the like, but can nevertheless be routinely determined by one skilled in the art. An effective amount of a compound of the present teachings may be readily determined by one of ordinary skill by routine methods known in the art.

The term "therapeutically effective amount" means an amount when administered to the subject which results in beneficial or desired results, including clinical results, e.g., inhibits, suppresses or reduces the symptoms of the condition being treated in the subject as compared to a control. For example, a therapeutically effective amount can be an amount effective for detectable killing or inhibition of the growth or spread of cancer cells; the size or number of tumors; or other measure of the level, stage, progression or severity of the cancer. The exact amount required will vary from subject to subject, depending on the species, age, and general condition of the subject, the severity of the disease, the particular anticancer agent, its mode of administration, combination treatment with other therapies, and the like.

*6. Preparation*

The compounds of any one of the formulae described above, may be prepared by the general and specific methods described below, using the common general knowledge of one skilled in the art of synthetic organic chemistry. Such common general knowledge can be found in standard reference books such as Comprehensive Organic Chemistry, Ed. Barton and Ollis, Elsevier; Comprehensive Organic Transformations: A Guide to Functional Group Preparations, Larock, John Wiley and Sons; and Compendium of Organic Synthetic Methods, Vol. I-XII (published by Wiley-Interscience). The starting materials used herein are commercially available or may be prepared by routine methods known in the art.

In the preparation of the compounds of any one of the formulae described above, it is noted that some of the preparation methods described herein may require protection of remote functionality *(e.g.,* primary amine, secondary amine, carboxyl in any one of the formulae described above precursors). The need for such protection will vary depending on the nature of the remote functionality and the conditions of the preparation methods. The need for such protection is readily determined by one skilled in the art. The use of such protection/deprotection methods is also within the skill in the art. For a general description of protecting groups and their use, see Greene, Protective Groups in Organic Synthesis, John Wiley & Sons, New York, 1991.

For example, certain compounds contain primary amines or carboxylic acid functionalities which may interfere with reactions at other sites of the molecule if left unprotected. Accordingly, such functionalities may be protected by an appropriate protecting group which may be removed in a subsequent step. Suitable protecting groups for amine and carboxylic acid protection include those protecting groups commonly used in peptide synthesis (such as N-t-butoxycarbonyl (Boc), benzyloxycarbonyl (Cbz), and 9-fluorenylmethylenoxycarbonyl (Fmoc) for amines, and lower alkyl or benzyl esters for carboxylic acids) which are generally not chemically reactive under the reaction conditions described and can typically be removed without chemically altering other functionality in the any one of the formulae described above compounds.

EXAMPLES

Compounds disclosed herein are prepared according to the exemplary procedures provided herein and modifications thereof known to those of skill in the art.

The Schemes described below are intended to provide a general description of the methodology employed in the preparation of the compounds of the present disclosure. The following abbreviations are used throughout the intermediates and examples: Ac means acetyl, ACN means Acetonitrile, AcOH means Acetic Acid, AIBN means 2,2'-Azobis(2-methylpropionitrile), Bn means benzyl, Bpin means 4,4,5,5-tetramethyl-1,3,2-dioxaborolan-2-yl, $(Bpin)_2$ means bis(pinacolato)diboron, DCM means Dichloromethane, DMF means *N,N*-Dimethylformamide, Et means ethyl, LG means leaving group, LAH means Lithium Aluminum Hydride, Me means methyl, NBS means N-Bromosuccinimide, $Pd(dppf)Cl_2$ means [1,1'-Bis(diphenylphosphino)ferrocene]dichloropalladium(II) , Pd(dtbpt)Cl2 means 1,1'-Bis(di-tert-butylphosphino)ferrocene palladium dichloride, PMB-Cl means 1-(chloromethyl)-4-methoxy-benzene, tBu means *tert-butyl,* THF means Tetrahydrofuran, hr means hours, min means minutes.

Methods for synthesizing critical intermediates

Intermediate 1: 2-[[3-methoxy-4-[(4-methoxyphenyl)methoxy]phenyl]methyl]-4,4,5,5-tetramethyl-1,3,2-dioxaborolane

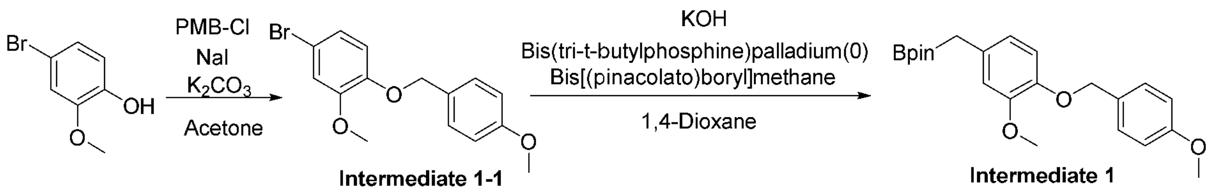


Intermediate 1-1

Intermediate 1

To a solution of 4-bromo-2-methoxy-phenol (3.00 g, 14.78 mmol) in Acetone (50 mL) was added Sodium iodide (1.11g, 7.388 mmol), Potassium carbonate (4.08 g, 29.55 mmol), 1-(chloromethyl)-4-methoxy-benzene (4.63 g, 29.55 mmol). The mixture was allowed to heat at 80 °C for 1 hr. The mixture was filtered and the filter cake was washed with ethyl acetate (3×50mL). The organic phases were combined and concentrated to afford 3.00 g of 4-bromo-2-methoxy-1-[(4-methoxyphenyl)methoxy]benzene as a white solid (yield=62.82%).

To a solution of 4-bromo-2-methoxy-1-[(4-methoxyphenyl)methoxy]benzene (3.000g, 9.283mmol) in 1,4-Dioxane (50 mL) was added 4,4,5,5-tetramethyl-2-[(4,4,5,5-tetramethyl-1,3,2-dioxaborolan-2-yl)methyl]-1,3,2-dioxaborolane (2.99 g, 11.14 mmol), Bis(tri-t-butylphosphine)palladium(0) (474 mg, 928 μmol) and Potassium hydroxide solution (1.7mL, 8 mol/L). The mixture was allowed to stir at 15 °C for 15 hr under nitrogen atmosphere. After evaporating the solvent, the residue was added 50 mL water and extracted with ethyl acetate (3×50mL). The organic phases were combined, dried over magnesium sulfate, filtered and concentrated. The residue was further purified by silica gel flash chromatography eluting with a gradient of 15% ethyl acetate in heptane to 35% ethyl acetate in heptane to afford 2.00 g of 2-[[3-methoxy-4-[(4-methoxyphenyl)methoxy]phenyl]methyl]-4,4,5,5-tetramethyl-1,3,2-dioxaborolane as an off-withe solid (yield=56.07%).

Intermediate 2: 2-methoxy-4-[(7-methoxy-4-quinolyl)methyl]phenol

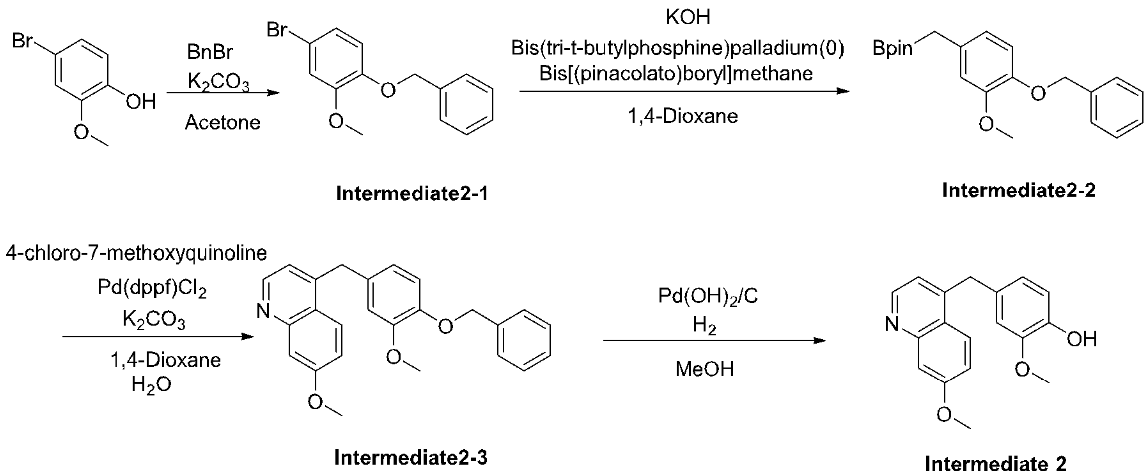


Intermediate2-1

Intermediate2-2

Intermediate2-3

Intermediate 2

To a solution of 4-bromo-2-methoxy-phenol (20.00 g, 98.51 mmol) in Acetone (150 mL) was added Potassium carbonate (20.421 g, 147.76 mmol), bromomethylbenzene (17.69 g, 103.43 mmol). The mixture was allowed to heat at 75 °C for 1 hr. The mixture was filtered and the filter cake was washed with ethyl acetate (3×50mL). The organic phases were

combined, concentrated and purified by silica gel flash chromatography eluting with a gradient of 5% ethyl acetate in heptane to 10% ethyl acetate in heptane to afford 24.02 g of 1-benzyloxy-4-bromo-2-methoxy-benzene as a white solid (yield=83.11%).

To a solution of 1-benzyloxy-4-bromo-2-methoxy-benzene (24.00 g, 81.868 mmol) in 1,4-Dioxane (500 mL) was added 4,4,5,5-tetramethyl-2-[(4,4,5,5-tetramethyl-1,3,2-dioxaborolan-2-yl)methyl]-1,3,2-dioxaborolane (28.52 g, 106.428 mmol), Bis(tri-t-butylphosphine)palladium(0) (1.26 g, 2.456 mmol) and Potassium hydroxide solution (18.42 mL, 8 mol/L). The mixture was allowed to stir at 15 °C for 15 hr under nitrogen atmosphere. After evaporating the solvent, the residue was added 500 mL water and extracted with ethyl acetate (3×250mL). The organic phases were combined, dried over magnesium sulfate, filtered and concentrated. The residue was further purified by silica gel flash chromatography eluting with a gradient of 5% ethyl acetate in heptane to 12% ethyl acetate in heptane to afford 17.10 g of 2-[(4-benzyloxy-3-methoxy-phenyl)methyl]-4,4,5,5-tetramethyl-1,3,2-dioxaborolane as a withe solid (yield=58.62%).

To a solution of 2-[(4-benzyloxy-3-methoxy-phenyl)methyl]-4,4,5,5-tetramethyl-1,3,2-dioxaborolane (12.08 g, 34.09 mmol) in 1,4-Dioxane (100 mL) was added 4-chloro-7-methoxy-quinoline (6.00 g, 30.99 mmol), [1,1'-Bis(diphenylphosphino)ferrocene]dichloropalladium(II) (2.27 g, 3.10 mmol), Potassium carbonate (8.57 g, 61.97 mmol) and water (20 mL). The mixture was heated at 100 °C for 15 hr under nitrogen atmosphere. After evaporating the solvent, the crude residue was purified by silica gel flash chromatography eluting with a gradient of 15% ethyl acetate in heptane to 100% ethyl acetate to afford 10.00 g of 4-[(4-benzyloxy-3-methoxy-phenyl)methyl]-7-methoxy-quinoline as a white solid (yield=83.72 %).

To a solution of 4-[(4-benzyloxy-3-methoxy-phenyl)methyl]-7-methoxy-quinoline (10.00 g, 25.94 mmol) in methanol (100 mL) was added Palladium hydroxide on carbon (1.50 g). The mixture was heated at 80 °C for 3 hr under hydrogen atmosphere. The mixture was filtered and the filter cake was washed with methanol (3×100mL). The organic phases were combined, concentrated and purified by silica gel flash chromatography eluting with a gradient of 35% ethyl acetate in heptane to 100% ethyl acetate to afford 5.50 g of 2-methoxy-4-[(7-methoxy-4-quinolyl)methyl]phenol as a grey solid (yield=71.78 %).

Intermediate 3: 5-(bromomethyl)-3-methoxy-2-((5-methoxypyridin-2-yl)methoxy)pyridine

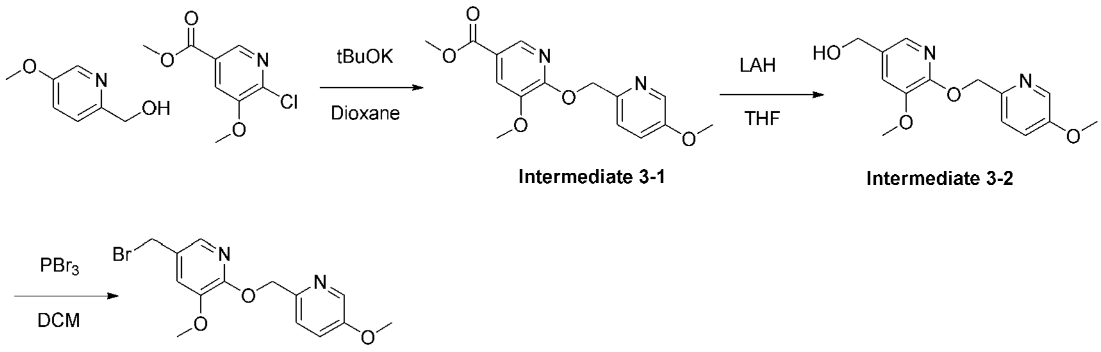


Intermediate 3-1

Intermediate 3-2

Intermediate 3

To a solution of (5-methoxypyridin-2-yl)methanol (24.90 g, 178.94 mmol) in 1,4-dioxane (200 mL) was added Potassium *tert*- butoxide (21.80 g, 194.28 mmol) at 0°C. After 10 min of stirring at 0 °C, the mixture was added methyl 6-chloro-5-methoxy-pyridine-3-carboxylate (30.00 g, 148.80 mmol). The mixture is heated to 100 °C for 12 hr and then cooled to room temperature. The mixture was diluted with water 1000 mL, extracted with ethyl acetate (3×1000 mL). The organic phases were combined, dried over magnesium sulfate, filtered and concentrated. The residue was further purified by silica gel flash chromatography eluting with a gradient of 100% heptane to 50% ethyl acetate in heptane to afford 12.00 g of methyl 5-methoxy-6-[(5-methoxy-2-pyridyl)methoxy]pyridine-3-carboxylate as an off-white solid (yield=26.5 %).

To a solution of 5-methoxy-6-[(5-methoxy-2-pyridyl)methoxy]pyridine-3-carboxylate (12.00 g, 39.44 mmol) in THF (200 mL) was added lithium aluminum hydride (1.80 g, 47.43 mmol) at 5°C under nitrogen atmosphere. After 1 hr stirring, the mixture was successively added water (1.8mL), 15% aqueous sodium hydroxide solution (1.8mL), and water (5.4 mL) to quench the reaction. The mixture was filtered and the filtrate was diluted with water 500 mL and extracted with ethyl acetate (3×500 mL). The organic phases were combined, dried over magnesium sulfate, filtered and concentrated. The residue was further purified by silica gel flash chromatography eluting with a gradient of 20% ethyl acetate in heptane to 100% ethyl acetate to afford 5.30 g of (5-methoxy-6-((5-methoxypyridin-2-yl)methoxy)pyridin-3-yl)methanol as a withe solid (yield=48.64%).

To a solution of (5-methoxy-6-((5-methoxypyridin-2-yl)methoxy)pyridin-3-yl)methanol (5.00 g, 18.10 mmol) in dichloromethane (200 mL) was added Phosphorus tribromide (1.97 g, 7.28 mmol) dropwise at 5°C. The mixture was allowed to stir at 10 °C for 10 min. The mixture was poured into saturated aqueous sodium bicarbonate solution and extracted with dichloromethane (300 mL). The organic layer was concentrated under 40 °C to give 6.00 g of 5-(bromomethyl)-3-methoxy-2-((5-methoxypyridin-2-yl)methoxy)pyridine as a colorless gum (yield=97.74%).

Intermediate 4: 8-(bromomethyl)-3-methoxy-1,5-naphthyridine

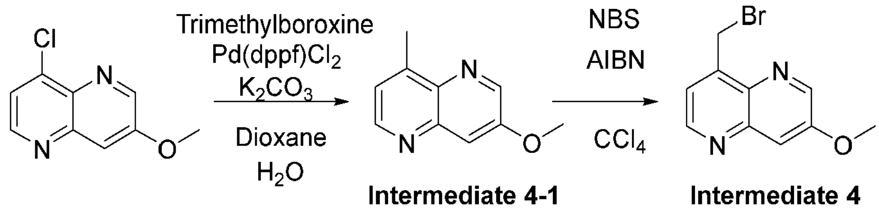


**Intermediate 4-1** **Intermediate 4**

To a solution of trimethylboroxine (1.17 g, 14.14 mmol) dissolved in THF (4 mL) was added 8-chloro-3-methoxy-1,5-naphthyridine (500 mg, 2.57 mmol), potassium carbonate (532 mg, 3.85 mmol), [1,1'-Bis(diphenylphosphino)ferrocene]dichloropalladium(II) (93 mg, 127 μmol) and water (0.8 mL). The mixture was heated in a microwave reactor at 100 °C for 1 hr under nitrogen atmosphere. After evaporating the solvent, the residue was diluted with brine (50mL) and extracted with ethyl acetate (3×50mL). The organic phases were combined, dried over magnesium sulfate, filtered and concentrated. The residue was further purified by silica gel flash chromatography eluting with a gradient of 100% heptane to 50% ethyl acetate in heptane to afford 300 mg of 3-methoxy-8-methyl-1,5-naphthyridine as a white solid (yield=67%).

To a solution of 3-methoxy-8-methyl-1,5-naphthyridine (300 mg, 1.73 mmol) in carbon tetrachloride (10 mL) was added N-Bromosuccinimide (306 mg, 1.73 mmol) and 2,2'-Azobis(2-methylpropionitrile) (28 mg, 173 μmol). The mixture was heated to 80 °C for 2 hr under nitrogen atmosphere. The mixture was filtered and the filter cake was washed with ethyl acetate (3×50mL). The filtrate were combined, concentrated and further purified by silica gel flash chromatography eluting with a gradient of 100% heptane to 30% ethyl acetate in heptane to afford 150 mg of 8-(bromomethyl)-3-methoxy-1,5-naphthyridine as a yellow solid (yield=25.8%).

Intermediate 5: 3-methoxy-2-[(5-methoxy-2-pyridyl)methoxy]-5-(4,4,5,5-tetramethyl-1,3,2-dioxaborolan-2-yl)pyridine

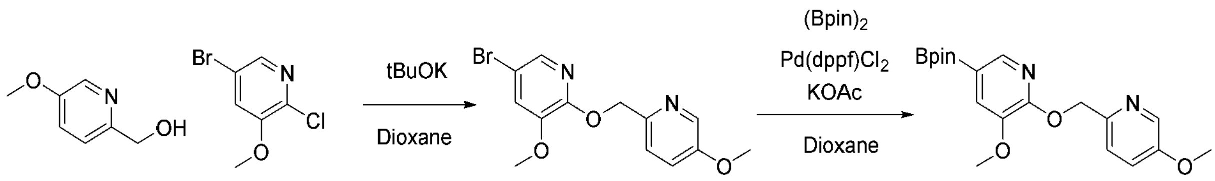


**Intermediate 5-1** **Intermediate 5**

To a solution of (5-methoxypyridin-2-yl)methanol (2.47 g, 17.72mmol) in 1,4-dioxane (30mL) was added potassium *tert*- butoxide (2.49 g, 22.15mmol) at 0°C. After 10 min of stirring at 0 °C, the mixture was added 5-bromo-2-chloro-3-methoxypyridine (3.942 g, 17.72mmol). The mixture is heated to 80 °C for 2 hr and then cooled to room temperature. A solution of saturated aqueous ammonia chloride (10mL) was added to quench the reaction. After evaporating the solvent, the residue was diluted with brine (50mL) and extracted with ethyl acetate (3×100mL). The organic phases were combined, dried over magnesium sulfate, filtered and concentrated. The residue was further purified by silica gel flash chromatography eluting with a gradient of 100% heptane to 50% ethyl acetate in heptane to afford 3.64g of 5-bromo-3-methoxy-2-((5-methoxypyridin-2-yl)methoxy)pyridine as a light-yellow solid (yield=79.5%).

To a solution of 5-bromo-3-methoxy-2-((5-methoxypyridin-2-yl)methoxy)pyridine (4.000g, 12.30 mmol) in 1,4-dioxane (50 mL) was added bis(pinacolato)diboron (4.69g, 18.45mmol), Bis(diphenylphosphino)ferrocene]dichloropalladium(II) (1.000 g, 1.367 mmol) and potassium acetate (2.415g, 12.302 mmol). The mixture was heated to 80 °C for 12 hr under nitrogen atmosphere. After evaporating the solvent, the residue was diluted with brine (50mL) and extracted with ethyl acetate (3×50mL). The organic phases were combined, dried over magnesium sulfate, filtered and concentrated.

The residue was further purified by silica gel flash chromatography eluting with a gradient of 100% heptane to 50% ethyl acetate in heptane to afford 3.64 g of 3-methoxy-2-((5-methoxypyridin-2-yl)methoxy)-5-(4,4,5,5-tetramethyl-1,3,2-dioxaborolan-2-yl)pyridine as a light yellow solid (yield=79.5%).

Intermediate 6: 5-methoxy-6-((5-methoxypyridin-2-yl)methoxy)pyridin-3-ol

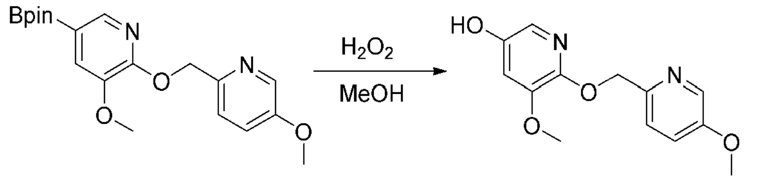


To a solution of 3-methoxy-2-[(5-methoxy-2-pyridyl)methoxy]-5-(4,4,5,5-tetramethyl-1,3,2-dioxaborolan-2-yl) pyridine (400 mg, 1.08 mmol) in methanol (20 mL) was added 30% aqueous hydrogen peroxide solution (146 mg, 1.29 mmol). The mixture was allowed to stir at 25 °C for 15 hr. The mixture was added 10 ml saturated aqueous Sodium Thiosulfate solution and extracted with ethyl acetate (3×50mL).The combined organic layers were dried over magnesium sulfate, filtered and concentrated. The residue was further purified by silica gel flash chromatography eluting with a gradient of 15% ethyl acetate in heptane to 100% ethyl acetate to afford 140 mg of 5-methoxy-6-[(5-methoxy-2-pyridyl) methoxy]pyridin-3-ol as a white solid (yield=49.68%).

General methods for synthesizing representative examples

Method A:

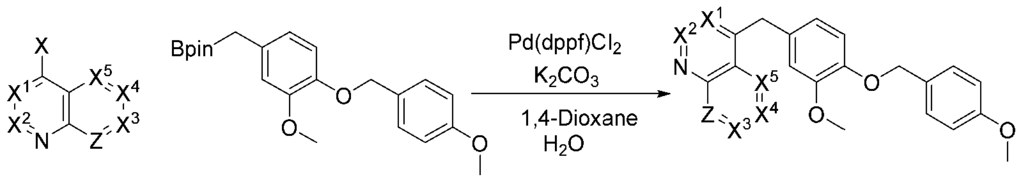


**Intermediate 1** **Example**

Example 1: 7-methoxy-4-[[3-methoxy-4-[(4-methoxyphenyl)methoxy]phenyl]methyl] quinolone

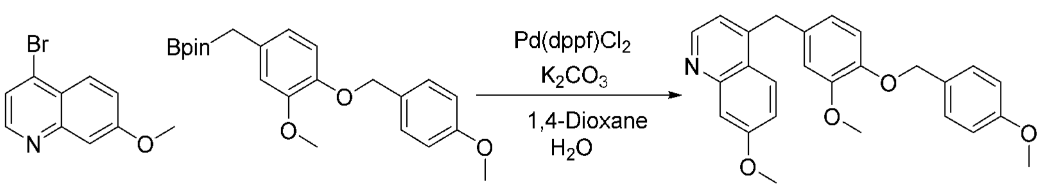


**Intermediate 1** **Example 1**

To a solution of 2-[[3-methoxy-4-[(4-methoxyphenyl)methoxy]phenyl]methyl]-4,4,5,5-tetramethyl-1,3,2-dioxaborolane (242 mg, 630 µmol) in 1,4-Dioxane (15 mL) was added 4-bromo-7-methoxy-quinoline (100 mg,420 µmol), [1,1'-Bis(diphenylphosphino)ferrocene]dichloropalladium(II) (30 mg, 41 µmol), Potassium carbonate (174 mg, 1.26 mmol) and water (3 mL). The mixture was heated at 100 °C for 1 hr under nitrogen atmosphere. After evaporating the solvent, the crude residue was purified by silica gel flash chromatography eluting with a gradient of 15% ethyl acetate in heptane to 100% ethyl acetate to afford 160 mg of 7-methoxy-4-[[3-methoxy-4-[(4-methoxyphenyl)methoxy]phenyl]methyl]quinolone as a light yellow solid (yield=91.68%). $^1$H NMR (400 MHz, DMSO) δ 8.73 (d, *J* = 4.5 Hz, 1H), 8.11 (d, *J* = 9.2 Hz, 1H), 7.40 (d, *J* = 2.6 Hz, 1H), 7.36 - 7.31 (m, 2H), 7.24 (dd, *J* = 9.2, 2.7 Hz, 1H), 7.16 (d, *J* = 4.5 Hz, 1H), 6.98 - 6.89 (m, 4H), 6.68 (dd, J = 8.2, 1.9 Hz, 1H), 4.92 (s, 2H), 4.35 (s, 2H), 3.91 (s, 3H), 3.75 (s, 3H), 3.70 (s, 3H). MS (m/z): 416.1 $(M+H)^+$.

Example 2: 4-(3-methoxy-4-((4-methoxybenzyl)oxy)benzyl)-1,7-naphthyridine

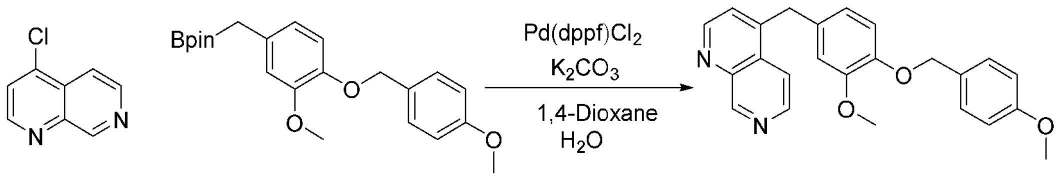


Intermediate 1 Example 2

To a solution of 2-[[3-methoxy-4-[(4-methoxyphenyl)methoxy]phenyl]methyl]-4,4,5,5-tetramethyl-1,3,2-dioxaborolane (350 mg, 911 μmol) in 1,4-Dioxane (15 mL) was added 4-chloro-1,7-naphthyridine (100 mg, 608 μmol), [1,1'-Bis(diphenylphosphino)ferrocene]dichloropalladium(II) (44 mg, 60 μmol), Potassium carbonate (252 mg, 1.82 mmol) and water (3 mL). The mixture was heated at 100 °C for 1 hr under nitrogen atmosphere. After evaporating the solvent, the crude residue was purified by silica gel flash chromatography eluting with a gradient of 15% ethyl acetate in heptane to 100% ethyl acetate to afford 120 mg of 7-methoxy-4-[[3-methoxy-4-[(4-methoxyphenyl)methoxy]phenyl]methyl]quinolone as a white solid (yield=51.11%). $^1$H NMR (400 MHz, DMSO) δ 9.39 (s, 1H), 8.97 (d, *J* = 4.3 Hz, 1H), 8.62 (d, *J* = 5.8 Hz, 1H), 8.11 (d, *J* = 5.8 Hz, 1H), 7.55 (d, *J* = 4.3 Hz, 1H), 7.34 (d, *J* = 8.5 Hz, 2H), 7.03 - 6.89 (m, 4H), 6.73 (d, *J* = 8.1 Hz, 1H), 4.93 (s, 2H), 4.41 (s, 2H), 3.75 (s, 3H), 3.72 (s, 3H). MS (m/z): 387.1 (M+H)$^+$.

Example 3: 4-(3-methoxy-4-((4-methoxybenzyl)oxy)benzyl)quinolone

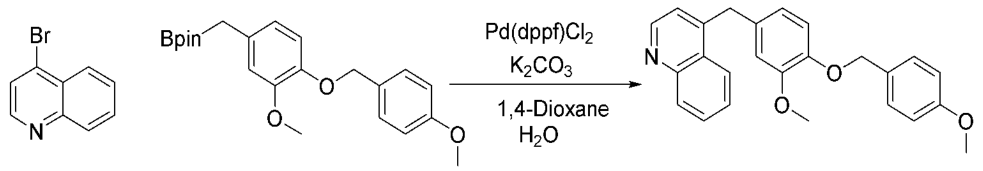


Intermediate 1 Example 3

To a solution of 2-[[3-methoxy-4-[(4-methoxyphenyl)methoxy]phenyl]methyl]-4,4,5,5-tetramethyl-1,3,2-dioxaborolane (277 mg, 721 μmol) in 1,4-Dioxane (15 mL) was added 4-bromoquinoline (100 mg, 481 μmol), [1,1'-Bis(diphenylphosphino)ferrocene] dichloropalladium(II) (35 mg, 48 μmol), Potassium carbonate (199 mg, 1.44 mmol) and water (3 mL). The mixture was heated at 100 °C for 2 hr under nitrogen atmosphere. After evaporating the solvent, the crude residue was purified by silica gel flash chromatography eluting with a gradient of 15% ethyl acetate in heptane to 50% ethyl acetate in heptane to afford 120 mg of 4-(3-methoxy-4-((4-methoxybenzyl)oxy)benzyl)quinoline as a white solid (yield=64.77%). $^1$H NMR (400 MHz, DMSO) δ 8.82 (d, *J* = 4.4 Hz, 1H), 8.22 (d, *J* = 8.2 Hz, 1H), 8.03 (d, *J* = 8.2 Hz, 1H), 7.78 - 7.70 (m, 1H), 7.61 (dd, *J* = 11.2, 4.0 Hz, 1H), 7.33 (dd, *J* = 9.8, 6.5 Hz, 3H), 7.00 - 6.89 (m, 4H), 6.70 (dd, *J* = 8.2, 1.6 Hz, 1H), 4.93 (s, 2H), 4.40 (s, 2H), 3.75 (s, 3H), 3.71 (s, 3H). MS (m/z): 386.1 (M+H)$^+$.

<u>Method B:</u>

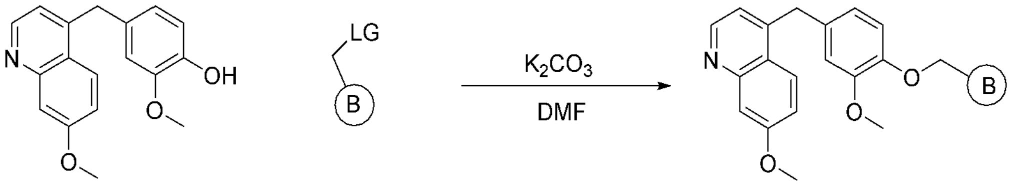


Intermediate 2 Examples

Example 6: 4-(4-(imidazo[1,2-a]pyridin-7-ylmethoxy)-3-methoxybenzyl)-7-methoxy quinoline

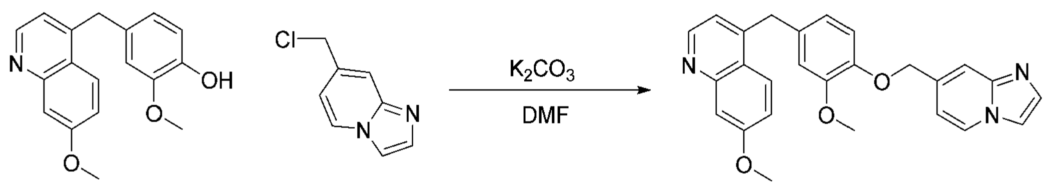


Intermediate 2

Example 6

To a solution of 2-methoxy-4-[(7-methoxy-4-quinolyl)methyl]phenol (100 mg, 339 μmol) in DMF (10 mL) was added 7-(chloromethyl)imidazo[1,2-a]pyridine (85 mg, 510 μmol), Potassium carbonate (71 mg, 514 μmol). The mixture was heated at 80 °C for 12 hr. The mixture was diluted with water 50 mL, extracted with ethyl acetate (50 mL). The organic layer was concentrated and purified by silica gel flash chromatography eluting with a gradient of 20% ethyl acetate in heptane to 10% methanol in dichloromethane to afford 50 mg of 4-(4-(imidazo[1,2-a]pyridin-7-ylmethoxy)-3-methoxybenzyl)-7-methoxyquinoline as a light yellow solid (yield=34.71%). $^1$H NMR(400 MHz, DMSO) δ8.72 (d, *J* = 4.4 Hz, 1H), 8.53 (dd, *J* = 7.2, 1.2 Hz, 1H), 8.11 (d, *J* = 9.2 Hz, 1H), 7.94-7.93 (m, 1H), 7.64-7.49 (m, 2H), 7.39 (d, *J* = 2.8 Hz, 1H), 7.24 (dd, J = 9.2, 2.8 Hz, 1H), 7.16 (d, *J* = 4.4 Hz, 1H), 7.02-6.88 (m, 3H), 6.69 (dd, *J* = 8.0, 2.0 Hz, 1H), 5.09 (s, 2H), 4.36 (s, 2H), 3.91 (s, 3H), 3.75 (s, 3H). MS (m/z): 426.1 $(M+H)^+$.

Example 8: 7-methoxy-4-(3-methoxy-4-(pyridin-3-ylmethoxy)benzyl)quinolone

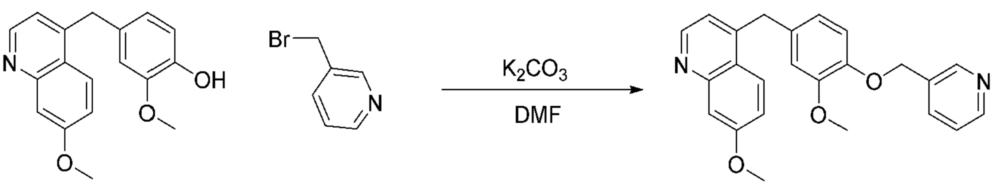


Intermediate 2

Example 8

To a solution of 2-methoxy-4-[(7-methoxy-4-quinolyl)methyl]phenol (160 mg, 542 μmol) in DMF (5 mL) was added 3-(bromomethyl)pyridine hydrobromide (150 mg, 593 μmol), Potassium carbonate (190 mg, 1.38 mmol). The mixture was allowed to stir at 20 °C for 16 hr. The mixture was diluted with water 15 mL, extracted with ethyl acetate (25 mL). The organic layer was concentrated and purified by high performance liquid chromatography eluting with a gradient of 10% acetonitrile in water to 65% acetonitrile in water to afford 46 mg of 7-methoxy-4-(3-methoxy-4-(pyridin-3-ylmethoxy)benzyl)quinoline as an off-white solid (yield=22.08 %). $^1$H NMR(400 MHz, DMSO) δ8.73 (d, *J* = 4.5 Hz, 1H), 8.63 (d, *J* = 1.6 Hz, 1H), 8.54 (dd, *J*= 4.8, 1.5 Hz, 1H), 8.11 (d, J= 9.2 Hz, 1H), 7.83 (dt, *J*= 7.8, 1.9 Hz, 1H), 7.47 - 7.34 (m, 2H), 7.24 (dt, *J* = 13.0, 6.5 Hz, 1H), 7.17 (d, *J* = 4.5 Hz, 1H), 6.98 (dd, *J* = 7.1, 5.1 Hz, 2H), 6.70 (dd, *J* = 8.2, 1.9 Hz, 1H), 5.07 (s, 2H), 4.36 (s, 2H), 3.91 (s, 3H), 3.72 (s, 3H) MS (m/z): 387.1 $(M+H)^+$.

Example 18: 4-(4-(imidazo[1,2-a]pyridin-2-ylmethoxy)-3-methoxybenzyl)-7-methoxy quinoline

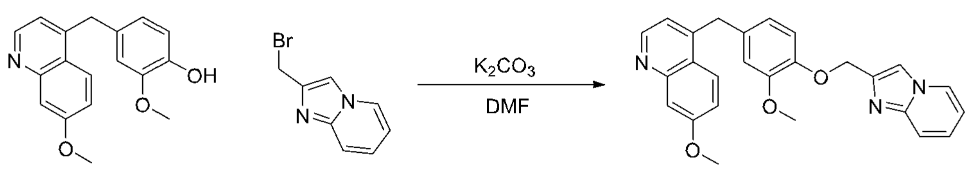


Intermediate 2

Example 18

To a solution of 2-methoxy-4-[(7-methoxy-4-quinolyl)methyl]phenol (100 mg, 339 μmol) in DMF (10 mL) was added 2-(bromomethyl)imidazo[1,2-a]pyridine (72 mg, 341 μmol), Potassium carbonate (57 mg, 412 μmol). The mixture was heated at 80 °C for 12 hr. The mixture was diluted with water 50 mL, extracted with ethyl acetate (50 mL). The organic

layer was concentrated and purified by silica gel flash chromatography eluting with a gradient of 20% ethyl acetate in heptane to 6% methanol in dichloromethane to afford 25 mg of 4-(4-(imidazo[1,2-a]pyridin-2-ylmethoxy)-3-methoxybenzyl)-7-methoxyquinoline as a light yellow solid (yield=17.35%). $^1$H NMR(400 MHz, DMSO) δ8.73 (d, *J* = 4.4 Hz, 1H), 8.54-8.51 (m, 1H), 8.12 (d, *J* = 9.2 Hz, 1H), 7.95 (s, 1H), 7.52 (d, *J* = 8.8 Hz, 1H), 7.40 (d, *J* = 2.8 Hz, 1H), 7.30 - 7.20 (m, 2H), 7.17 (d, *J* = 4.4 Hz, 1H), 7.04 (d, *J* = 8.4 Hz, 1H), 6.97 (d, *J* = *2.0* Hz, 1H), 6.93 - 6.84 (m, 1H), 6.69 (dd, *J* = 8.0, 2.0 Hz, 1H), 5.12 (s, 2H), 4.35 (s, 2H), 3.91 (s, 3H), 3.71 (s, 3H).MS (m/z): 426.1 (M+H)$^+$.

Method C:

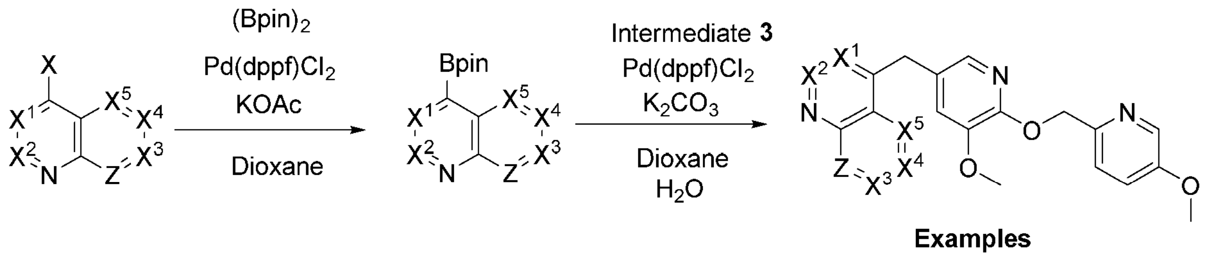


Example **13:** 7-methoxy-4-((5-methoxy-6-((5-methoxypyridin-2-yl)methoxy)pyridin-3-yl)methyl)quinolone

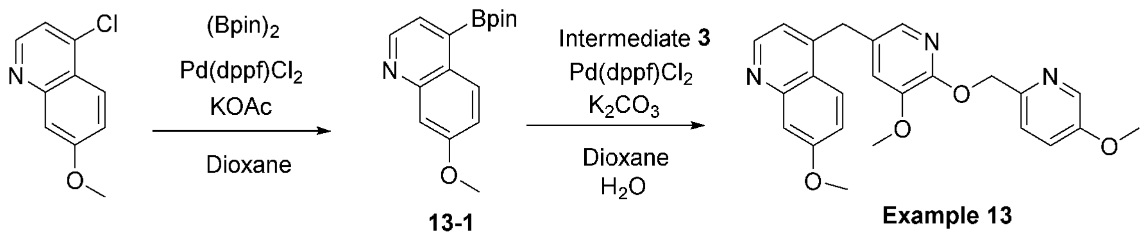


To a solution of 4-chloro-7-methoxy-quinoline (5.00 g, 25.823 mmol) dissolved in 1,4-dioxane (100 mL) was added 4,4,5,5-tetramethyl-2-(4,4,5,5-tetramethyl-1,3,2-dioxaborolan-2-yl)-1,3,2-dioxaborolane (7.22 g, 28.432 mmol), Potassium Acetate (3.80 g, 38.72 mmol) and [1,1'-Bis(diphenylphosphino)ferrocene]dichloropalladium(II) (1.89 g, 2.583 mmol). The mixture was heated at 100°C for 3 hr under nitrogen atmosphere. The mixture was diluted with water (300 mL) and extracted with ethyl acetate (400 mL). The organic phases were combined, dried over magnesium sulfate, filtered and concentrated. The residue was further purified by silica gel flash chromatography eluting with a gradient of 20% ethyl acetate in heptane to 100% ethyl acetate to afford 3.00 g of 7-methoxy-4-(4,4,5,5-tetramethyl-1,3,2-dioxaborolan-2-yl) quinoline as a brown solid (yield=40.74%).

To a solution of 7-methoxy-4-(4,4,5,5-tetramethyl-1,3,2-dioxaborolan-2-yl)quinoline (50 mg, 175 μmol) dissolved in 1,4-dioxane (10 mL) was added 5-(bromomethyl)-3-methoxy-2-[(5-methoxy-2-pyridyl)methoxy]pyridine (60 mg, 177 μmol), potassium carbonate (37 mg, 268 μmol), [1,1'-Bis(diphenylphosphino)ferrocene]dichloropalladium (II) (13 mg, 18 μmol) and water (2 mL). The mixture was heated at 100 °C for 2 hr under nitrogen atmosphere. The mixture was diluted with water (50 mL) and extracted with ethyl acetate (50 mL).The organic phases were combined, dried over magnesium sulfate, filtered and concentrated. The residue was further purified by silica gel flash chromatography eluting with a gradient of 20% ethyl acetate in heptane to 50% ethyl acetate in heptane to afford 5 mg of 7-methoxy-4-((5-methoxy-6-((5-methoxypyridin-2-yl)methoxy)pyridin-3-yl)methyl)quinoline as a light yellow solid (yield=6.83 %). $^1$H NMR(400 MHz, DMSO) δ8.73 (d, *J* = 4.4 Hz, 1H), 8.25 (t, *J* = 2.0 Hz, 1H), 8.17 (d, *J* = 9.2 Hz, 1H), 7.60 (d, *J* = 2.0 Hz, 1H), 7.41-7.38 (m, 3H), 7.31-7.26 (m, 2H), 7.29-7.15 (m, 1H), 5.30 (s, 2H), 4.38 (s, 2H), 3.92 (s, 3H), 3.82 (s, 3H), 3.74 (s, 3H). MS (m/z): 418.1 (M+H)$^+$.

Example **15:** 4-((5-methoxy-6-((5-methoxypyridin-2-yl)methoxy)pyridin-3-yl)methyl)-1,5-naphthyridine

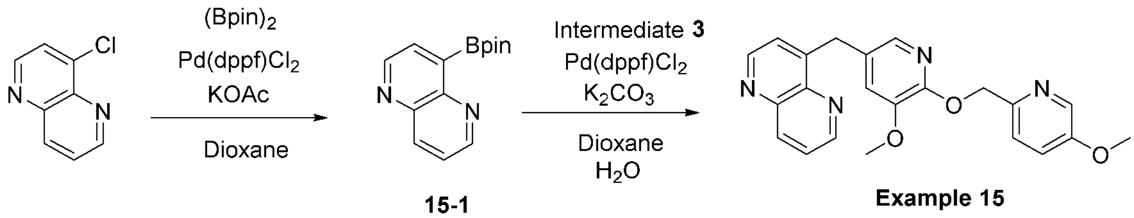


**15-1**

**Example 15**

To a solution of 4-chloro-1,5-naphthyridine (2.000 g, 12.151 mmol) dissolved in 1,4-dioxane (100 mL) was added 4,4,5,5-tetramethyl-2-(4,4,5,5-tetramethyl-1,3,2-dioxaborolan-2-yl)-1,3,2-dioxaborolane (3.39 g, 13.35 mmol), Potassium Acetate (1.79 g, 18.22 mmol) and [1,1'-Bis(diphenylphosphino)ferrocene]dichloropalladium(II) (889 mg, 1.22 mmol). The mixture was heated at 100 °C for 18 hr under nitrogen atmosphere. The mixture was filtered and the filtrate was washed by ethyl acetate (100 mL) The organic phases were combined, dried over magnesium sulfate, filtered and concentrated to afford 3.00 g of 4-(4,4,5,5-tetramethyl-1,3,2-dioxaborolan-2-yl)-1,5-naphthyridine as a brown solid (yield=40.74%).

To a solution of crude 4-(4,4,5,5-tetramethyl-1,3,2-dioxaborolan-2-yl)-1,5-naphthyridine (113 mg, 441 μmol) dissolved in 1,4-dioxane (10 mL) was added 5-(bromomethyl)-3-methoxy-2-[(5-methoxy-2-pyridyl)methoxy]pyridine (100 mg, 295 μmol), potassium carbonate (122 mg, 883 μmol), [1,1'-Bis(diphenylphosphino)ferrocene]dichloropalladium(II) (22 mg, 30 μmol) and water (2 mL). The mixture was heated at 100 °C for 16 hr under nitrogen atmosphere. The mixture was filtered and the filter cake was washed with ethyl acetate (3×50mL). The filtrate were combined, concentrated and further purified by silica gel flash chromatography eluting with a gradient of 50% ethyl acetate in heptane to 100% ethyl acetate to afford 27 mg of 4-[[5-methoxy-6-[(5-methoxy-2-pyridyl)methoxy]-3-pyridyl]methyl]-1,5-naphthyridine as an off-white solid (yield=23.58%). $^1$H NMR(400 MHz, DMSO) δ9.09 (dd, *J* = 4.1, 1.7 Hz, 1H), 8.91 (d, *J* = 4.4 Hz, 1H), 8.44 (dd, *J* = 8.5, 1.7 Hz, 1H), 8.26 (t, *J* = 1.8 Hz, 1H), 7.83 (dd, *J* = 8.5, 4.1 Hz, 1H), 7.68 (d, *J* = 1.8 Hz, 1H), 7.58 (d, *J* = 4.4 Hz, 1H), 7.39 (d, *J* = 1.7 Hz, 3H), 5.31 (s, 2H), 4.57 (s, 2H), 3.82 (s, 3H), 3.75 (s, 3H). MS (m/z): 389.1 $(M+H)^+$.

Method D:

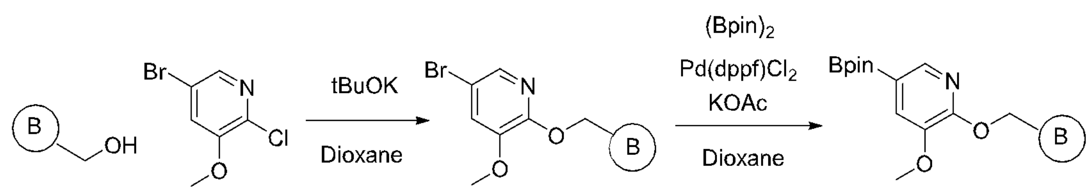


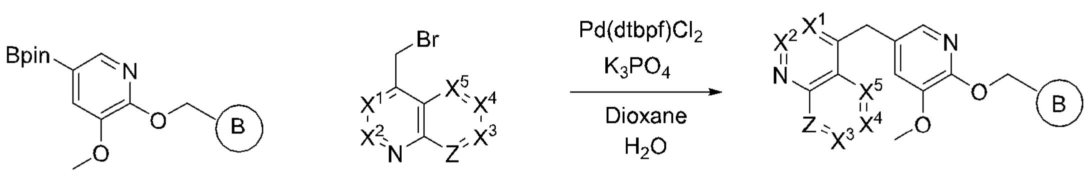


**Examples**

Example 16: 3-methoxy-8-((5-methoxy-6-((5-methoxypyridin-2-yl)methoxy)pyridin-3-yl)methyl)-1,5-naphthyridine

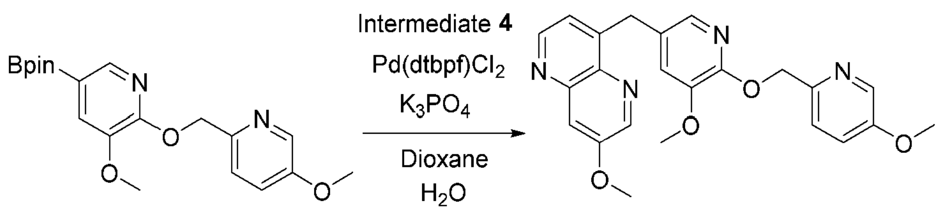


**Intermediate 5**

**Example 16**

To a solution of 8-(bromomethyl)-3-methoxy-1,5-naphthyridine (50mg, 198 μmol) in 1,4-dioxane (25 mL) was added 3-methoxy-2-((5-methoxypyridin-2-yl)methoxy)-5-(4,4,5,5-tetramethyl-1,3,2-dioxaborolan-2-yl)pyridine (147 mg, 395 μmol), tripotassium orthophosphate (126 mg, 593μmol), 1,1'-Bis(di-tert-butylphosphino)ferrocene palladium dichloride (10 mg, 19.8 μmol)) and water (5 mL). The mixture was heated to 90 °C for 12 hr under nitrogen atmosphere. After evaporating the solvent, the residue was diluted with brine (50mL) and extracted with ethyl acetate (3×50mL). The organic phases were combined, dried over magnesium sulfate, filtered and concentrated. The residue was further purified by silica gel flash chromatography eluting with a gradient of 50% ethyl acetate in heptane to 5% ethanol in ethyl acetate to afford 19 mg of 3-methoxy-8-((5-methoxy-6-((5-methoxypyridin-2-yl)methoxy)pyridin-3-yl)methyl)-1,5-naphthyridine as an off-white solid (yield=31%). $^1$H NMR(400 MHz, DMSO) δ8.82 (dd, *J* = 6.4, 3.7 Hz, 2H), 8.28 - 8.22 (m, 1H), 7.79 (d, *J* = 2.8 Hz, 1H), 7.65 (d, *J* = 1.6 Hz, 1H), 7.38 (td, *J* = 12.0, 3.1 Hz, 4H), 5.31 (s, 2H), 4.52 (s, 2H), 4.00 (s, 3H), 3.82 (s, 3H), 3.74 (s, 3H). MS (m/z): 419.1(M+H)$^+$.

Example **32:** 3-methoxy-8-((5-methoxy-6-((6-methoxypyridazin-3-yl)methoxy)pyridin-3-yl)methyl)-1,5-naphthyridine

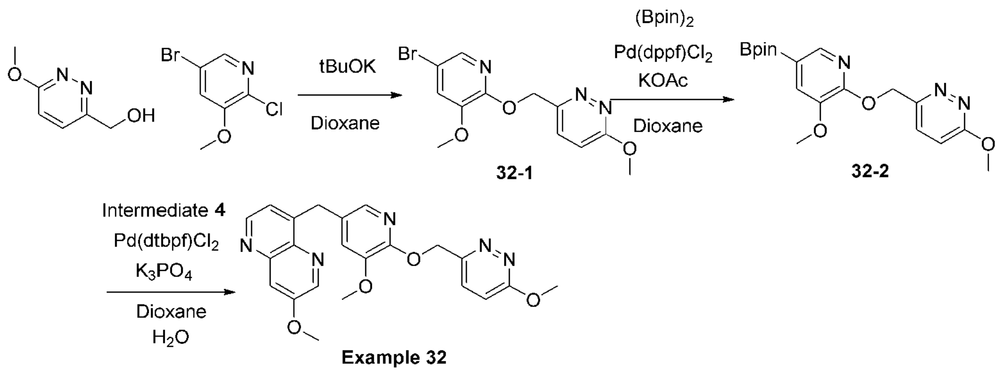

**Example 32**

To a solution of (6-methoxypyridazin-3-yl)methanol (0.63 g, 4.50 mmol) in 1,4-dioxane (13mL) was added Potassium *tert*- butoxide (0.66 g, 5.85 mmol) at 0°C. After 10 min of stirring at 0 °C, the mixture was added 5-bromo-2-chloro-3-methoxypyridine (1.00 g, 4.50 mmol). The mixture is heated to 100 °C for 2 hr and then cooled to room temperature. The mixture was poured into water (50 mL) and extracted with ethyl acetate (50 mL×2). The organic layer was concentrated to give 1.00 g of 3-[(5-bromo-3-methoxy-2-pyridyl)oxymethyl]-6-methoxy-pyridazine as a white solid (yield = 68.21%).

To a solution of 3-[(5-bromo-3-methoxy-2-pyridyl)oxymethyl]-6-methoxy-pyridazine (1.00 g, 3.07 mmol) in 1,4-dioxane (10 mL) was added bis(pinacolato)diboron (934 mg, 3.68 mmol), Bis(diphenylphosphino)ferrocene]dichloropalladium(II) (224 mg, 306 μmol) and potassium acetate (602 mg, 6.13 mmol). The mixture was heated to 100 °C for 16 hr under nitrogen atmosphere. The mixture was diluted with water (50 mL) and extracted with ethyl acetate (50 mL×2).The organic phases were combined, dried over magnesium sulfate, filtered, concentrated and purified by silica gel flash chromatography eluting with a gradient of 20% ethyl acetate in heptane to 50% ethyl acetate to afford 1.00 g of 3-methoxy-6-[[3-methoxy-5-(4,4,5,5-tetramethyl-1,3,2-dioxaborolan-2-yl)-2-pyridyl]oxymethyl]pyridazine as a white solid (yield=87.39 %).

To a solution of 4-(bromomethyl)-7-methoxy-quinoline (50 mg, 198 μmol) in 1,4-dioxane (10 mL) was added 3-methoxy-6-[[3-methoxy-5-(4,4,5,5-tetramethyl-1,3,2-dioxaborolan-2-yl)-2-pyridyl]oxymethyl]pyridazine (88 mg, 236 μmol), Tripotassium Orthophosphate (126 mg, 594 μmol), 1,1'-Bis(di-tert-butylphosphino)ferrocene palladium dichloride (13 mg, 20 μmol)) and water (2mL). The mixture was heated to 90 °C for 2 hr under nitrogen atmosphere. The mixture was diluted with water (50 mL) and extracted with ethyl acetate (50 mL×2).The organic phases were combined, dried over magnesium sulfate, filtered, concentrated and purified by silica gel flash chromatography eluting with a gradient of 50% ethyl acetate in heptane to 5% methanol in dichloromethane to afford 6 mg of 3-methoxy-8-[[5-methoxy-6-[(6-methoxypyridazin-3-yl)methoxy]-3-pyridyl]methyl]-1,5-naphthyridine as a white solid (yield=7.24%).$^1$H NMR(400 MHz, DMSO) δ8.83 (d, *J* =2.88Hz,1H), 8.81 (d, *J* =4.48Hz,1H), 7.79 (d, *J* =2.88Hz,1H), 7.66 (d, *J* =2.48Hz,1H), 7.64 (s, 1H), 7.41 (d, *J* =4.44Hz,lH), 7.38 (d, *J* =1.8Hz,1H), 7.23 (d, *J* =9.08Hz,1H), 5.47 (s,2H), 4.52 (s,2H), 4.02 (s,3H), 4.00(s,3H), 3.73 (s,3H) MS (m/z): 420.1 (M+H)$^+$.

Example **46:** 5-(((3-methoxy-5-((7-methoxy-1,5-naphthyridin-4-yl)methyl)pyridin-2-yl)oxy)methyl)-3-methylisoxazole

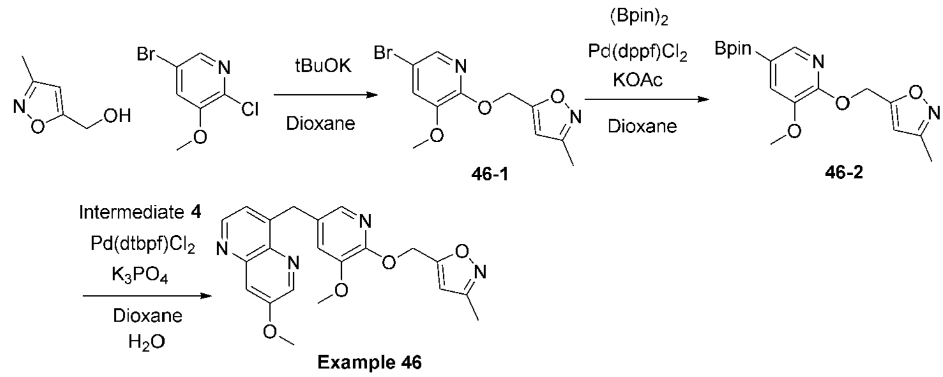


46-1

46-2

Example 46

To a solution of (3-methylisoxazol-5-yl)methanol (1.00 g, 8.84 mmol) in 1,4-dioxane (30mL) was added Potassium *tert*- butoxide (1.09 g, 9.73 mmol) at 0°C. After 10 min of stirring at 0 °C, the mixture was added 5-bromo-2-chloro-3-methoxypyridine (1.97 g, 8.84 mmol). The mixture is heated to 80 °C for 3 hr and then cooled to room temperature. The mixture was poured into water (50 mL) and extracted with ethyl acetate (50 mL×2). The organic layer was concentrated and purified by silica gel flash chromatography eluting with a gradient of 100% heptane to 25% ethyl acetate in heptane to give 1.69 g of 5-[(5-bromo-3-methoxy-2-pyridyl)oxymethyl]-3-methyl-isoxazole as a white solid (yield = 63.83%).

To a solution of 5-[(5-bromo-3-methoxy-2-pyridyl)oxymethyl]-3-methyl-isoxazole (800 mg, 2.68 mmol) in 1,4-dioxane (30 mL) was added bis(pinacolato)diboron (700 mg, 2.74mmol), Bis(diphenylphosphino)ferrocene]dichloropalladium(II) (200 mg, 273 μmol) and potassium acetate (550 mg, 5.60 mmol). The mixture was heated to 90 °C for 5 hr under nitrogen atmosphere. The mixture was diluted with water (50 mL) and extracted with ethyl acetate (50 mL×2).The organic phases were combined, dried over magnesium sulfate, filtered, concentrated and purified by silica gel flash chromatography eluting with a gradient of 5% ethyl acetate in heptane to 35% ethyl acetate in heptane to afford 850 mg of 5-[[3-methoxy-5-(4,4,5,5-tetramethyl-1,3,2-dioxaborolan-2-yl)-2-pyridyl]oxymethyl]-3-methyl-isoxazole as an off-white solid (yield=91.81%).

To a solution of 4-(bromomethyl)-7-methoxy-quinoline (35mg, 138 μmol) in 1,4-dioxane (25 mL) was added 5-[[3-methoxy-5-(4,4,5,5-tetramethyl-1,3,2-dioxaborolan-2-yl)-2-pyridyl]oxymethyl]-3-methyl-isoxazole (126 mg, 364 μmol), Tripotassium Orthophosphate (105 mg, 495 μmol), 1,1'-Bis(di-tert-butylphosphino)ferrocene palladium dichloride (98 mg, 150 μmol)) and water (5 mL). The mixture was heated to 90 °C for 12 hr under nitrogen atmosphere. The mixture was diluted with water (50 mL) and extracted with ethyl acetate (50 mL×2).The organic phases were combined, dried over magnesium sulfate, filtered, concentrated and purified by silica gel flash chromatography eluting with a gradient of 5% ethyl acetate in heptane to 100% ethyl acetate to afford 30 mg of 5-[[3-methoxy-5-[(7-methoxy-1,5-naphthyridin-4-yl)methyl]-2-pyridyl]oxymethyl]-3-methyl-isoxazole (yield=55.28%).$^1$H NMR(400 MHz, DMSO)δ8.82 (dd, J = 6.5, 3.7 Hz, 2H), 7.79 (d, *J* = 2.9 Hz, 1H), 7.67 (d, *J* = 1.8 Hz, 1H), 7.39 (dd, J = 8.8, 3.1 Hz, 2H), 6.39 (s, 1H), 5.39 (s, 2H), 4.53 (s, 2H), 3.99 (s, 3H), 3.73 (s, 3H), 2.21 (s, 3H). MS (m/z): 393.1(M+H)$^+$.

Example 73: 2-(((3-methoxy-5-((7-methoxy-1,5-naphthyridin-4-yl)methyl)pyridin-2-yl)oxy)methyl)-6,7-dihydro-4H-pyrazolo[5,1-c] [1,4] oxazine

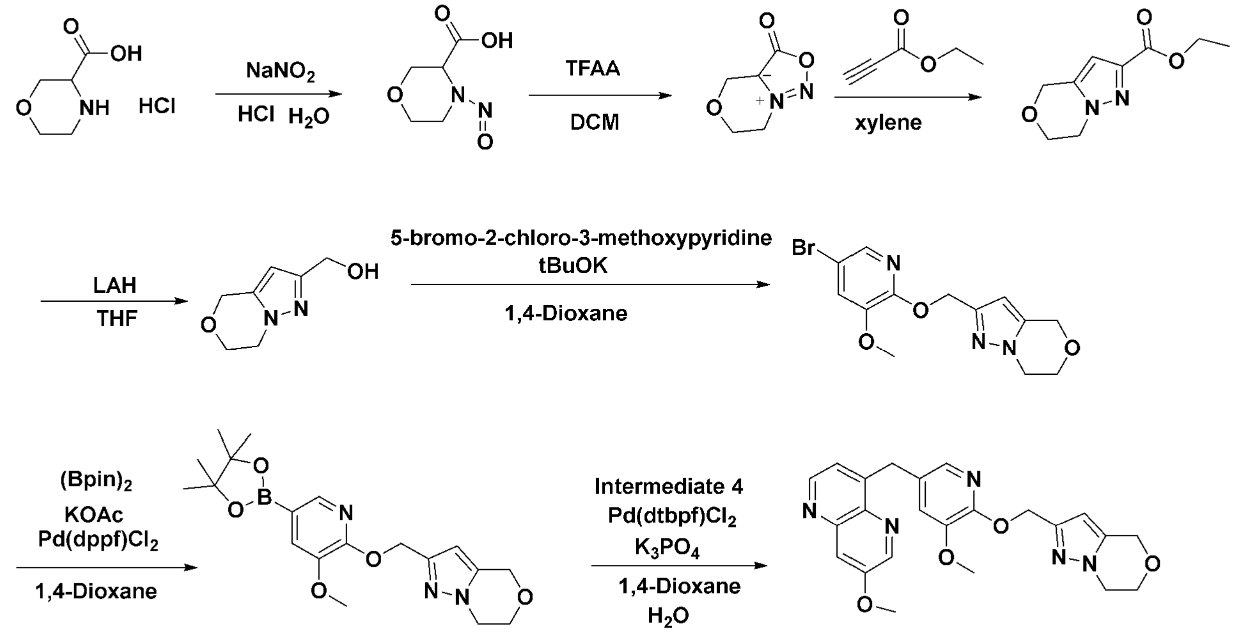


Example 73

To a solution of morpholine-3-carboxylic acid (3.929 g, 23.44 mmol) in concentrated hydrochloric acid (3.5 mL) and $H_2O$ (3 mL) was added the solution of sodium nitrite (2.588 g, 37.51 mmol) in $H_2O$ (3 mL) at 0°C. The mixture is allowed to stir at 0 °C for 2 hr. The mixture was extracted with dichloromethane (50 mL×20). The organic layer was dried by magnesium sulfate and concentrated to obtain 2.68 g of 4-nitrosomorpholine-3-carboxylic acid as a light yellow oil (yield = 71.39%).

To a solution of 4-nitrosomorpholine-3-carboxylic acid (2.68 g, 16.73 mmol) in dichloromethane (30 mL) was added trifluoroacetic anhydride (3.515 g, 16.73 mmol) at 5°C. The mixture is allowed to stir at 5 °C for 2 hr. After evaporating the solvent, the reside was purified by silica gel flash chromatography eluting with a gradient of 65% ethyl acetate in heptane to 100% ethyl acetate to afford 1.83 g of 3-oxo-6,7-dihydro-3H-[1,2,3]oxadiazolo[4,3-c][1,4]oxazin-8(4H)-ium-3a-ide as a light yellow solid (yield = 76.94%).

To a suspension of 3-oxo-6,7-dihydro-3H-[1,2,3]oxadiazolo[4,3-c][1,4]oxazin-8(4H)-ium-3a-ide (1.83 g, 12.88 mmol) in Xylene (30 mL) was added ethyl prop-2-ynoate (2.32 g, 11.83 mmol) at 20°C. The mixture is heated at 135 °C for 12 hr. After evaporating the solvent, the reside was purified by silica gel flash chromatography eluting with a gradient of 5% ethyl acetate in heptane to 50% ethyl acetate in heptane to afford 2.32 g of ethyl 6,7-dihydro-4H-pyrazolo[5,1-c][1,4] oxazine-2-carboxylate as a light yellow solid (yield = 91.83%).

To a suspension of lithium aluminium hydride (540 mg, 14.23 mmol) in anhydrous THF (30 mL) was added the suspension of 6,7-dihydro-4H-pyrazolo[5,1-c][1,4]oxazine-2-carboxylate (2.30 g, 11.72 mmol) in THF (20 mL) at -20°C. The mixture is stired at 20 °C for 3 hr. After quench the reaction by using $H_2O$ and 15% NaOH aqueous solution, the mixture was filtrated and concentrated to afford 1.42 g of 6,7-dihydro-4H-pyrazolo[5,1-c][1,4]oxazin-2-ylmethanol as a light yellow oil (yield = 78.57%).

To a solution of 6,7-dihydro-4H-pyrazolo[5,1-c][1,4]oxazin-2-ylmethanol 700 mg, 4.54 mmol) in 1,4-dioxane (30mL) was added potassium *tert*- butoxide (580 mg, 5.17 mmol) at 0°C. After 10 min of stirring at 0 °C, the mixture was added 5-bromo-2-chloro-3-methoxypyridine (1.01 g, 4.54 mmol). The mixture is heated to 80 °C for 3 hr and then cooled to room temperature. The mixture was poured into water (50 mL) and extracted with ethyl acetate (50 mL×2). The organic layer was concentrated and purified by silica gel flash chromatography eluting with a gradient of 100% heptane to 100% ethyl acetate to give 1.12 g of 2-[(5-bromo-3-methoxy-2-pyridyl)oxymethyl]-6,7-dihydro-4H-pyrazolo[5,1-c][1,4]oxazine as a light yellow oil (yield = 72.51%).

To a solution of 2-[(5-bromo-3-methoxy-2-pyridyl)oxymethyl]-6,7-dihydro-4H-pyrazolo[5,1-c][1,4]oxazine (1.12 g, 3.29 mmol) in 1,4-dioxane (30 mL) was added bis(pinacolato)diboron (1.732 g, 6.82mmol), Bis(diphenylphosphino) ferrocene]dichloropalladium(II) (250 mg, 342 μmol) and potassium acetate (670 mg, 6.83 mmol). The mixture was heated to 80 °C for 12 hr under nitrogen atmosphere. The mixture was purified by silica gel flash chromatography eluting with a gradient of 5% ethyl acetate in heptane to 100% ethyl acetate to afford 1.10 g of 2-[[3-methoxy-5-(4,4,5,5-tetramethyl-1,3,2-dioxaborolan-2-yl)-2-pyridyl]oxymethyl]-6,7-dihydro-4H-pyrazolo[5,1-c][1,4]oxazine as a light brown solid (yield=86.32%).

To a solution of 4-(bromomethyl)-7-methoxy-quinoline (30mg, 119 μmol) in 1,4-dioxane (10 mL) was added 2-[[3-methoxy-5-(4,4,5,5-tetramethyl-1,3,2-dioxaborolan-2-yl)-2-pyridyl]oxymethyl]-6,7-dihydro-4H-pyrazolo[5,1-c][1,4]oxa-

zine (47 mg, 121 μmol), Tripotassium Orthophosphate (75 mg, 353 μmol), 1,1'-Bis(di-tert-butylphosphino)ferrocene palladium dichloride (8 mg, 12.4 μmol)) and water (2 mL). The mixture was heated to 90 °C for 3 hr under nitrogen atmosphere. The mixture was purified by silica gel flash chromatography eluting with a gradient of 100% ethyl acetate to 60% tetrahydrofuran in ethyl acetate to afford 12 mg of 2-[[3-methoxy-5-[(7-methoxy-1,5-naphthyridin-4-yl)methyl]-2-pyridyl]oxymethyl]-6,7-dihydro-4H-pyrazolo[5,1-c][1,4]oxazine (yield=23.36%).$^1$H NMR(400 MHz, DMSO) δ8.82 (dd, *J* = 7.3, 3.7 Hz, 2H), 7.79 (d, *J* = 2.9 Hz, 1H), 7.67 (d, *J* = 1.9 Hz, 1H), 7.41 (d, *J* = 4.5 Hz, 1H), 7.32 (d, *J* = 1.8 Hz, 1H), 6.07 (s, 1H), 5.18 (s, 2H), 4.75 (s, 2H), 4.52 (s, 2H), 4.09 - 4.01 (m, 4H), 3.99 (s, 3H), 3.69 (s, 3H).. MS (m/z): 434.1 $(M+H)^+$. Example 136: (R)-3-methoxy-8-((5-methoxy-6-((5-methoxy-5,6-dihydro-4H-pyrrolo[1,2-b]pyrazol-2-yl)methoxy)pyridin-3-yl)methyl)-1,5-naphthyridine

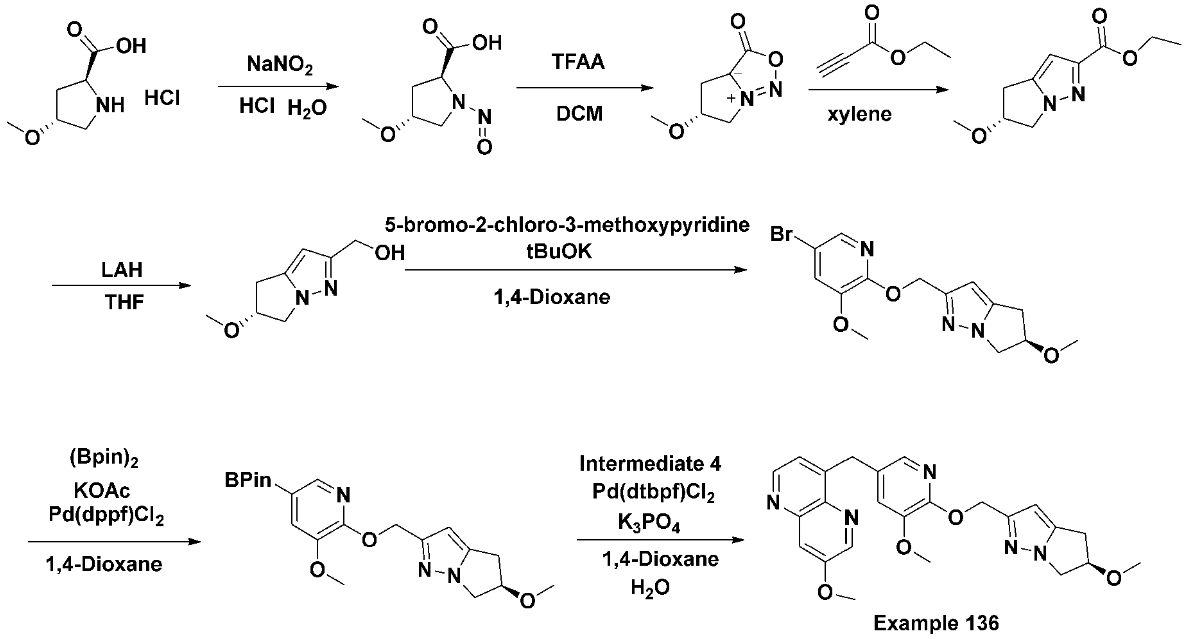

Example 136

To a solution of (2S,4R)-4-methoxypyrrolidine-2-carboxylic acid (7.000 g, 38.54 mmol) in concentrated hydrochloric acid (10 mL) was added the solution of sodium nitrite (4.255 g, 61.67 mmol) in $H_2O$ (10 mL) at 0°C. The mixture is allowed to stir at 25 °C for 2 hr. The mixture was extracted with dichloromethane (50 mL×20). The organic layer was dried by sodium sulfate and concentrated to obtain 6.00 g of (2S,4R)-4-methoxy-1-nitroso-pyrrolidine-2-carboxylic acid as a light yellow solid.

To a solution of (2S,4R)-4-methoxy-1-nitroso-pyrrolidine-2-carboxylic acid (5.50 g, 31.58 mmol) in dichloromethane (40 mL) was added trifluoroacetic anhydride (10.613 g, 50.53 mmol) at 0°C. The mixture is allowed to stir at 25 °C for 1 hr. After evaporating the solvent, the reside was purified by silica gel flash chromatography eluting with a gradient of 65% ethyl acetate in heptane to 100% ethyl acetate to afford 3.00 g of (R)-5-methoxy-3-oxo-5,6-dihydro-3H-pyrrolo[1,2-c][1,2,3]oxadiazol-7(4H)-ium-3a-ide as a brown solid (yield = 60.84%).

To a suspension of (R)-5-methoxy-3-oxo-5,6-dihydro-3H-pyrrolo[1,2-c][1,2,3]oxadiazol-7(4H)-ium-3a-ide (3.00 g, 19.21 mmol) in xylene (40 mL) was added ethyl prop-2-ynoate (7.539 g, 76.86 mmol) at 20°C. The mixture is heated at 135 °C for 16 hr. After evaporating the solvent, the reside was purified by silica gel flash chromatography eluting with a gradient of 5% ethyl acetate in heptane to 100% ethyl acetate to afford 1.80 g of ethyl (5R)-5-methoxy-5,6-dihydro-4H-pyrrolo[1,2-b]pyrazole-2-carboxylate as a yellow oil (yield =44.56%).

To a suspension of lithium aluminium hydride (1.00 g, 26.35 mmol) in anhydrous THF (20 mL) was added the suspension of ethyl (5R)-5-methoxy-5,6-dihydro-4H-pyrrolo[1,2-b]pyrazole-2-carboxylate (1.80g, 8.56 mmol) in THF (20 mL) at -20°C. The mixture is stired at 25 °C for 1 hr. After quench the reaction by using $H_2O$ and 15% NaOH aqueous solution, the mixture was filtrated and concentrated to afford 1.30 g of [(5R)-5-methoxy-5,6-dihydro-4H-pyrrolo[1,2-b]pyrazol-2-yl]methanol as a brown oil.

To a solution of 6,7-dihydro-4H-pyrazolo[5,1-c][1,4]oxazin-2-ylmethanol (756 mg, 4.50 mmol) in 1,4-dioxane (20mL) was added potassium *tert*- butoxide (900 mg, 8.02 mmol) at 0°C. After 10 min of stirring at 0 °C, the mixture was added 5-bromo-2-chloro-3-methoxypyridine (1.00 g, 4.50 mmol). The mixture is heated to 95 °C for 1 hr and then cooled to room temperature. The mixture was poured into water (50 mL) and extracted with ethyl acetate (50 mL×2). The organic layer was concentrated and purified by silica gel flash chromatography eluting with a gradient of 50% ethyl acetate in heptane to 100% ethyl acetate to give 400 mg of (5R)-2-[(5-bromo-3-methoxy-2-pyridyl)oxymethyl]-5-methoxy-5,6-dihydro-4H-pyrrolo[1,2-b]pyrazole as a light yellow oil (yield =25.12%).

To a solution of (5R)-2-[(5-bromo-3-methoxy-2-pyridyl)oxymethyl]-5-methoxy-5,6-dihydro-4H-pyrrolo[1,2-b]pyrazole (400 mg, 1.13 mmol) in 1,4-dioxane (20 mL) was added bis(pinacolato)diboron (430 mg, 1.69 mmol), Bis(diphenylphosphino)ferrocene]dichloropalladium(II) (124 mg, 169 μmol) and potassium acetate (332 mg, 3.38 mmol). The mixture was heated to 95 °C for 1 hr under nitrogen atmosphere. The mixture purified by silica gel flash chromatography eluting with a gradient of 5% ethyl acetate in heptane to 100% ethyl acetate to afford 400 mg of (5R)-5-methoxy-2-[[3-methoxy-5-(4,4,5,5-tetramethyl-1,3,2-dioxaborolan-2-yl)-2-pyridyl]oxymethyl]-5,6-dihydro-4H-pyrrolo[1,2-b]pyrazole as a brown oil (yield=88.27%).

To a solution of 4-(bromomethyl)-7-methoxy-quinoline (50mg, 198 μmol) in 1,4-dioxane (5 mL) was added (5R)-5-methoxy-2-[[3-methoxy-5-(4,4,5,5-tetramethyl-1,3,2-dioxaborolan-2-yl)-2-pyridyl]oxymethyl]-5,6-dihydro-4H-pyrrolo[1,2-b]pyrazole (110 mg, 274 μmol), Tripotassium Orthophosphate (126 mg, 594 μmol), 1,1'-Bis(di-tert-butylphosphino)ferrocene palladium dichloride (19 mg, 29.4 μmol) and water (1 mL). The mixture was heated to 95 °C for 2 hr under nitrogen atmosphere. The mixture was purified by silica gel flash chromatography eluting with a gradient of 100% ethyl acetate to 20% ethanol in ethyl acetate to afford 10 mg of (R)-3-methoxy-8-((5-methoxy-6-((5-methoxy-5,6-dihydro-4H-pyrrolo[1,2-b]pyrazol-2-yl)methoxy)pyridin-3-yl)methyl)-1,5-naphthyridine (yield=11.31%).$^{1}$H NMR(400 MHz, DMSO) δ8.82 (dd, J = 6.8, 3.7 Hz, 2H), 7.79 (d, J = 2.9 Hz, 1H), 7.67 (d, J = 1.7 Hz, 1H), 7.40 (d, J = 4.5 Hz, 1H), 7.31 (d, J = 1.7 Hz, 1H), 6.02 (s, 1H), 5.16 (s, 2H), 4.58 (ddd, J = 8.5, 6.0, 2.4 Hz, 1H), 4.52 (s, 2H), 4.25 (dd, J = 11.8, 5.7 Hz, 1H), 4.02 (d, J = 2.4 Hz, 1H), 4.00 (s, 3H), 3.69 (s, 3H), 3.30 (s, 3H), 3.11 (dd, J = 16.7, 6.4 Hz, 1H), 2.79 (dd, J = 16.6, 2.2 Hz, 1H). MS (m/z): 448.1 (M+H)$^{+}$.

Method E:

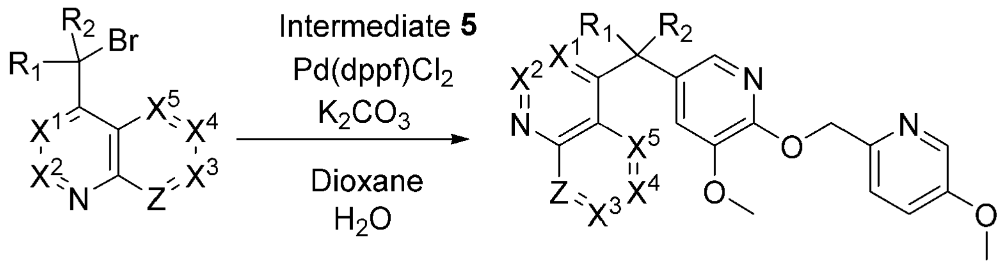


**Examples**

Example 19: 4-((5-methoxy-6-((5-methoxypyridin-2-yl)methoxy)pyridin-3-yl)methyl)-1,7-naphthyridine

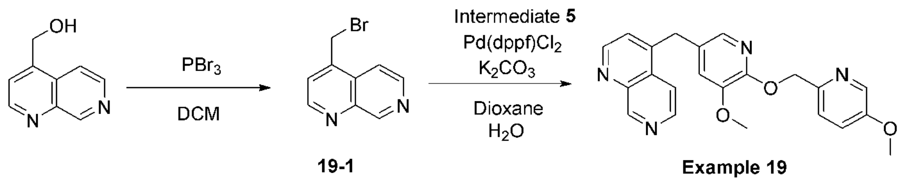


19-1

Example 19

To a solution of 1,7-naphthyridin-4-ylmethanol (80 mg, 499 μmmol) dissolved in dichloromethane (10 mL) was added Phosphorus tribromide (405mg, 1.50 mmol) dropwise at 0 °C. The mixture was allowed to stir at 15 °C for 15 hr. The mixture was added saturated aqueous NaHCO3 solution (15 mL) and extracted with DCM (20 mL × 3). The combined organic layers were dried over anhydrous $Mg_2SO_4$, filtered and concentrated to afford 50 mg of 4-(bromomethyl)-1,7-naphthyridine as white solid (yield=50%).

To a solution of 3-methoxy-2-[(5-methoxy-2-pyridyl)methoxy]-5-(4,4,5,5-tetramethyl-1,3,2-dioxaborolan-2-yl)pyridine (125 mg, 334 μmol) dissolved in 1,4-dioxane (10 mL) was added 4-(bromomethyl)-1,7-naphthyridine (50 mg, 225.15 μmol), potassium carbonate (93 mg, 673 μmol), [1,1'-Bis(diphenylphosphino)ferrocene]dichloropalladium (II) (16 mg, 22 μmol) and water (2 mL). The mixture was heated at 95 °C for 3 hr under nitrogen atmosphere. After evaporating the solvent, the reside was purified by silica gel flash chromatography eluting with a gradient of 15% ethyl acetate in heptane to 100% ethyl acetate to afford 2.5 mg of 4-[[5-methoxy-6-[(5-methoxy-2-pyridyl)methoxy]-3-pyridyl]methyl]-1,7-naphthyridine as a white solid (yield=2.87%).$^{11}$H NMR (400 MHz, DMSO) δ 9.41 (s, 1H), 8.98 (d, *J* = 4.4 Hz, 1H), 8.65 (d, *J* = 5.8 Hz,

1H), 8.26 (s, 1H), 8.17 (d, *J* = 5.8 Hz, 1H), 7.66 (s, 1H), 7.57 (d, *J* = 4.4 Hz, 1H), 7.40 (d, *J* = 1.6 Hz, 2H), 7.32 (s, 1H), 5.31 (s, 2H), 4.44 (s, 2H), 3.82 (s, 3H), 3.75 (s, 3H). MS (m/z): 389.1 (M+H)+.

Example 22: 8-((5-methoxy-6-((5-methoxypyridin-2-yl)methoxy)pyridin-3-yl)methyl)pyrido[3,2-d]pyrimidine

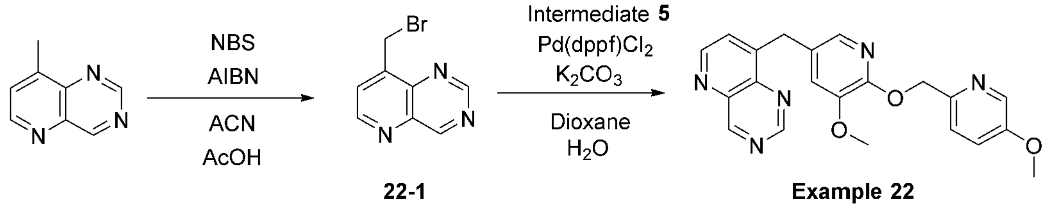


**22-1** **Example 22**

To a solution of 8-methylpyrido[3,2-d]pyrimidine (50 mg, 344 μmol) dissolved in Acetonitrile (10 mL) was added N-Bromosuccinimide (183 mg, 1.028 mmol), 2,2'-Azobis(2-methylpropionitrile) (113 mg, 688 μmol) and acetic acid (0.02mL). The mixture was heated to 80 °C for 15 hr under nitrogen atmosphere. After evaporating the solvent, the residue was purified by silica gel flash chromatography eluting with a gradient of 15% ethyl acetate in heptane to 100% ethyl acetate to afford 20 mg of 8-(bromomethyl)pyrido[3,2-d]pyrimidine as a white solid (yield=25.92%).

To a solution of 3-methoxy-2-[(5-methoxy-2-pyridyl)methoxy]-5-(4,4,5,5-tetramethyl-1,3,2-dioxaborolan-2-yl) pyridine ( 66 mg, 177 μmol) dissolved in 1,4-dioxane (10 mL) was added 8-(bromomethyl)pyrido[3,2-d]pyrimidine (20 mg, 89 μmol), potassium carbonate (37 mg, 268 μmol), [1,1'-Bis(diphenylphosphino)ferrocene]dichloropalladium (II) (6 mg, 8 μmol) and water (2 mL). The mixture was heated at 90 °C for 3 hr under nitrogen atmosphere. After evaporating the solvent, the reside was purified by silica gel flash chromatography eluting with a gradient of 15% ethyl acetate in heptane to 100% ethyl acetate to afford 3.0 mg of 8-[[5-methoxy-6-[(5-methoxy-2-pyridyl)methoxy]-3-pyridyl]methyl]pyrido[3,2-d] pyrimidine as a white solid (yield=8.63%).$^1$H NMR (400 MHz, DMSO) δ 9.68 (s, 1H), 9.51 (s, 1H), 9.05 (d, *J* = 4.4 Hz, 1H), 8.26 (t, *J* = 1.8 Hz, 1H), 7.81 (d, *J* = 4.4 Hz, 1H), 7.68 (d, *J* = 1.8 Hz, 1H), 7.38 (t, *J* = 2.5 Hz, 3H), 5.31 (s, 2H), 4.53 (s, 2H), 3.82 (s, 3H), 3.75 (s, 3H). MS (m/z): 390.1 (M+H)+.

Example **62:** 3-chloro-8-((5-methoxy-6-((5-methoxypyridin-2-yl)methoxy)pyridin-3-yl)methyl)-1,5-naphthyridine

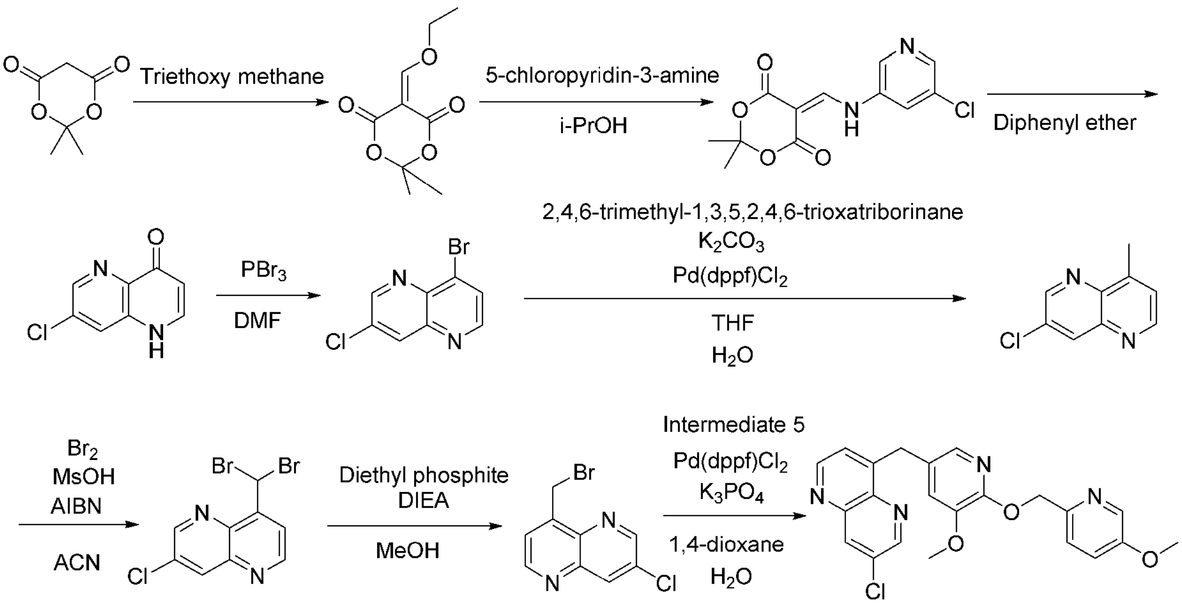


To the diethoxymethoxyethane (905 g, 6.11 mol) was added 2,2-dimethyl-1,3-dioxane-4,6-dione (200 g, 1.39 mmol). The mixture was heated to 90 °C for 1 hr. The mixture was concentrated to afford 281.6 g of 5-(ethoxymethylene)-2,2-dimethyl-1,3-dioxane-4,6-dione as an red oil. (Crude product).

To a solution of 5-chloropyridin-3-amine (150 g, 1.17 mol) dissolved in isopropanol (1500 mL) was added

5-(ethoxymethylene)-2,2-dimethyl-1,3-dioxane-4,6-dione (278 g, 1.39 mol). The mixture was heated at 80 °C for 2 hr and then cooled down. A large number of solids were precipitated and the mixture was filtered. The filter cake was washed three times with 150ml isopropyl alcohol and dried to afford 325 g of 5-[[(5-chloro-3-pyridyl)amino]methylene]-2,2-dimethyl-1,3-dioxane-4,6-dione as a yellow solid. (Crude product).

To the phenyl ether (3000 mL) was added 5-[[(5-chloro-3-pyridyl)amino]methylene]-2,2-dimethyl-1,3-dioxane-4,6-dione (325 g, 1.15 mol) in portions at 200 °C. The mixture was heated at 200 °C for 0.5 hr and then cooled down to 60 °C. A large number of solids were precipitated and the mixture was filtered. The filter cake was washed three times with 1000ml heptane and dried to afford 138 g of 7-chloro-1H-1,5-naphthyridin-4-one as a brown solid. (Crude product).

To a solution of 7-chloro-1H-1,5-naphthyridin-4-one (133.9 g, 550 mmol) dissolved in N,N-dimethylformamide (2070 mL) was slowly added phosphorus tribromide (256.5 g, 948 mmol) in dropwise at 0 °C. The mixture was stired at 20 °C for 3.5 hr. The mixture was poured into ice water (10 L) and added saturated $Na_2CO_3$ aqueous solution to adjust the pH value of the mixture to 7-8. A large number of solids were precipitated and the mixture was filtered. The filter cake was washed five times with 200ml water and dried to afford 133.9 g of 8-bromo-3-chloro-1,5-naphthyridine as a yellow solid. (Crude product)

To a solution of 8-bromo-3-chloro-1,5-naphthyridine (110.9 g, 455 mmol) dissolved in THF (440 mL) was added the solution of 2,4,6-trimethyl-1,3,5,2,4,6-trioxatriborinane (57.17 g, 455 mmol) dissolved in THF(440 mL), potassium carbonate (94.38 g, 683 mmol), [1,1'-bis(diphenylphosphino)ferrocene]dichloro palladium(II) (16.67 g, 22.78 mmol) and water (176 mL). The mixture was heated at 60 °C for 36 hr under nitrogen atmosphere. The mixture was filtrated and the filter cake was washed two times with 200 mL ethyl acetate. After partition, the organic phases were combined, dried over magnesium sulfate, filtered, concentrated and purified by silica gel flash chromatography eluting with a gradient of 15% ethyl acetate in heptane to 50% ethyl acetate in heptane to afford 75 g of 3-chloro-8-methyl-1,5-naphthyridine as a light yellow solid (yield=92.19 %).

To a solution of 3-chloro-8-methyl-1,5-naphthyridine (75.0 g, 420 mmol) dissolved in acetonitrile (1875 mL) was added methanesulfonic acid (80.71 g, 840 mmol). After stiring at 20 °C for 1 h, the mixture was added Azo-bis-isobutryonitrile (10.34 g, 62.97 mmol) and bromine (268.4 g, 1.68 mol). The mixture was heated at 78 °C for 20 hr. After evaporating the solvent, the mixture was added water (1.5L) and saturated NaOH aqueous soluetion to adjust the pH value of the mixture to 7.5. A large number of solids were precipitated and the mixture was filtered. The filter cake was washed five times with 100ml water and dried to afford 141.3 g of 3-chloro-8-(dibromomethyl)-1,5-naphthyridine as a yellow solid. (Crude product).

To a solution of 3-chloro-8-(dibromomethyl)-1,5-naphthyridine (141.3 g, 420 mmol) dissolved in methanol (420 mL) was added diethyl phosphite (87.01 g, 630 mmol), N,N-diisopropylethylamine (81.43 g, 630 mmol). The mixture was heated at 20 °C for 5 hr. After evaporating the solvent, the mixture was added water (3.0 L) and saturated NaOH aqueous soluetion to adjust the pH value of the mixture to 8.5. A large number of solids were precipitated and the mixture was filtered. The filter cake was washed five times with 100ml methyl tert-butyl ether and dried to afford 90.00 g of 8-(bromomethyl)-3-chloro-1,5-naphthyridine as a off-white solid. (yield=83.21%).

To a solution of 8-(bromomethyl)-3-chloro-1,5-naphthyridine (25.00 g, 97.08 mmol) dissolved in 1,4-dioxane (1250 mL) was added 3-methoxy-2-[(5-methoxy-2-pyridyl)methoxy]-5-(4,4,5,5-tetramethyl-1,3,2-dioxaborolan-2-yl)pyridine (36.25 mg, 97.39 mmol), Tripotassium phosphate (61.75 g, 291 mmol), [1,1'-bis(diphenylphosphino)ferrocene] dichloro palladium(II) (355 mg, 4.85 mmol) and water (250 mL). The mixture was heated at 78 °C for 16 hr under nitrogen atmosphere. After evaporating the solvent, the reside was purified by silica gel flash chromatography eluting with a gradient of 50% ethyl acetate in heptane to 100% ethyl acetate to afford 11.00 g of 3-chloro-8-[[5-methoxy-6-[(5-methoxy-2-pyridyl)methoxy]-3-pyridyl]methyl]-1,5-naphthyridine as a off-white solid (yield=26.80%). $^1H$ NMR(400 MHz, DMSO) δ9.11 (d, *J* = 2.4 Hz, 1H), 8.94 (d, *J* = 4.4 Hz, 1H), 8.61 (d, *J* = 2.4 Hz, 1H), 8.28 - 8.22 (m, 1H), 7.64 (dd, *J* = 17.7, 3.1 Hz, 2H), 7.41 - 7.33 (m, 3H), 5.31 (s, 2H), 4.55 (s, 2H), 3.82 (s, 3H), 3.75 (s, 3H). MS (m/z): 423.0 $(M+H)^+$.

Example 65: 8-((5-methoxy-6-((5-methoxypyridin-2-yl)methoxy)pyridin-3-yl)methyl)-1,5-naphthyridine-3-carbonitrile

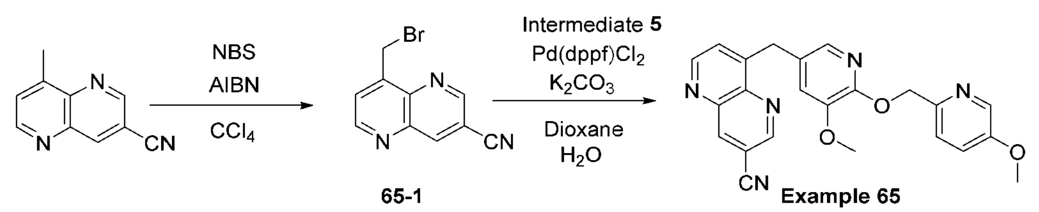


65-1

Example 65

To a solution of 8-methyl-1,5-naphthyridine-3-carbonitrile (70 mg, 414 μmol) dissolved in tetrachloromethane (7 mL) was added N-Bromosuccinimide (74 mg, 416 μmol) and 2,2'-Azobis(2-methylpropionitrile) (14 mg, 85 μmol). The mixture was heated to 80 °C for 16 hr under nitrogen atmosphere. After evaporating the solvent, the residue was purified by silica gel flash chromatography eluting with a gradient of 100% heptane to 35% ethyl acetate in heptane to afford 35 mg of 8-(bromomethyl)-1,5-naphthyridine-3-carbonitrile as an off-white solid (yield=34.10%).

To a solution of 3-methoxy-2-[(5-methoxy-2-pyridyl)methoxy]-5-(4,4,5,5-tetramethyl-1,3,2-dioxaborolan-2-yl) pyridine (16 mg, 43 μmol) dissolved in 1,4-dioxane (10 mL) was added 8-(bromomethyl)-1,5-naphthyridine-3-carbonitrile (35 mg, 35 μmol), potassium carbonate (147 mg, 105 μmol), [1,1'-bis(diphenylphosphino)ferrocene]dichloro palladium(II) (3 mg, 4 μmol) and water (2 mL). The mixture was heated at 90 °C for 2 hr under nitrogen atmosphere. After evaporating the solvent, the reside was purified by silica gel flash chromatography eluting with a gradient of 50% ethyl acetate in heptane to 100% ethyl acetate to afford 4.0 mg of 8-[[5-methoxy-6-[(5-methoxy-2-pyridyl)methoxy]-3-pyridyl]methyl]-1,5-naphthyridine-3-carbonitrile as a white solid (yield=27.43%). $^1$H NMR(400 MHz, DMSO) δ9.37 (d, *J* = 2.0 Hz, 1H), 9.12 (d, *J* = 2.0 Hz, 1H), 9.05 (d, *J* = 4.4 Hz, 1H), 8.28 - 8.23 (m, 1H), 7.75 (d, *J* = 4.4 Hz, 1H), 7.65 (d, *J* = 1.8 Hz, 1H), 7.41 - 7.34 (m, 3H), 5.30 (s, 2H), 4.56 (s, 2H), 3.83 (s, 3H), 3.75 (s, 3H). MS (m/z): 414.1 $(M+H)^+$.

Method F:

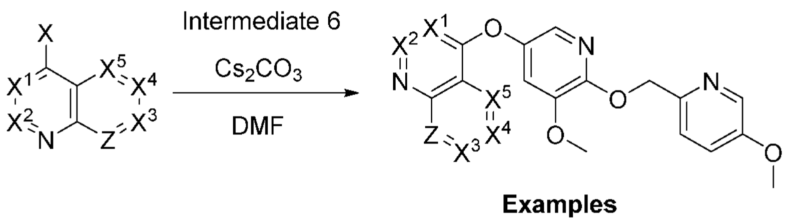


**Examples**

Example 54: 3-methoxy-8-((5-methoxy-6-((5-methoxypyridin-2-yl)methoxy)pyridin-3-yl)oxy)-1,5-naphthyridine

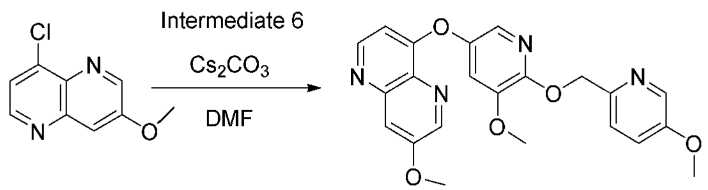


**Example 54**

To a solution of 5-methoxy-6-[(5-methoxy-2-pyridyl)methoxy]pyridin-3-ol ( 50 mg, 191 μmol) dissolved in N,N-Dimethylformamide (3 mL) was added Caesium carbonate (100 mg, 307 μmol) and 8-chloro-3-methoxy-1,5-naphthyridine (30 mg, 154 μmol). The mixture was heated at 120 °C for 1 hr under nitrogen atmosphere. After evaporating the solvent, the reside was purified by silica gel flash chromatography eluting with a gradient of 15% ethyl acetate in heptane to 100% ethyl acetate to afford 40 mg of 3-methoxy-8-((5-methoxy-6-((5-methoxypyridin-2-yl)methoxy)pyridin-3-yl)oxy)-1,5-naphthyridine as a white solid (yield=61.72%). $^1$H NMR (400 MHz, DMSO) δ 8.75 (d, *J* = 2.8 Hz, 1H), 8.70 (d, *J* = 5.2 Hz, 1H), 8.30 (d, *J* = 2.2 Hz, 1H), 7.80 (d, *J* = 2.8 Hz, 1H), 7.72 (d, *J* = 2.4 Hz, 1H), 7.51 - 7.40 (m, 3H), 6.79 (d, *J* = 5.2 Hz, 1H), 5.39 (s, 2H), 4.01 (s, 3H), 3.85 (s, 3H), 3.81 (s, 3H).MS (m/z): 421.0 $(M+H)^+$.

Method G:

General route of synthesizing the compound formula (I) when Q = N, X1 is C=O

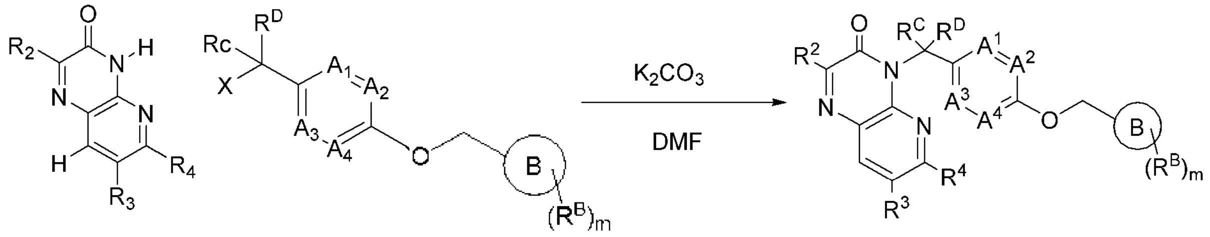


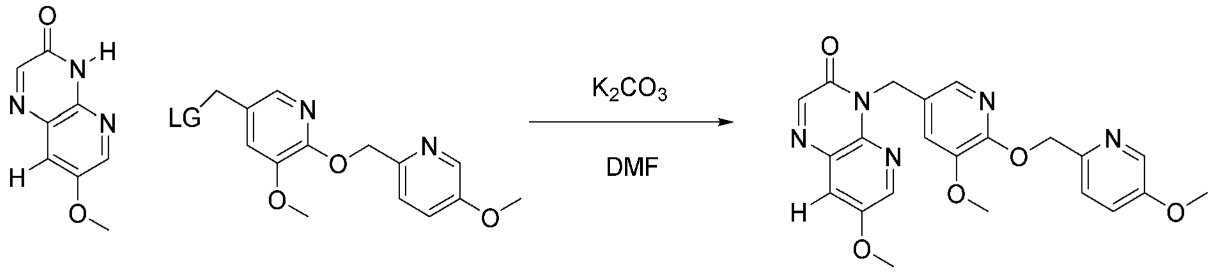


Method H:

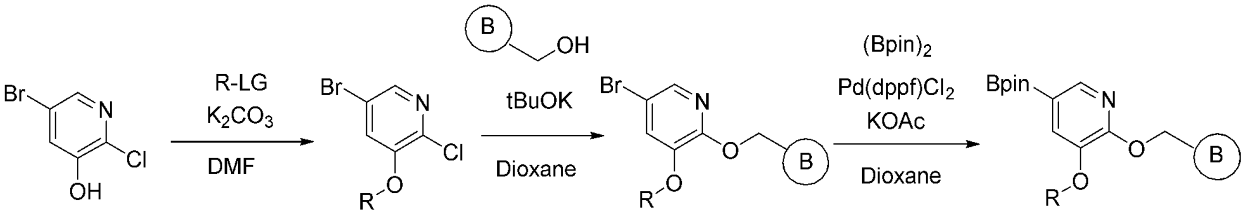


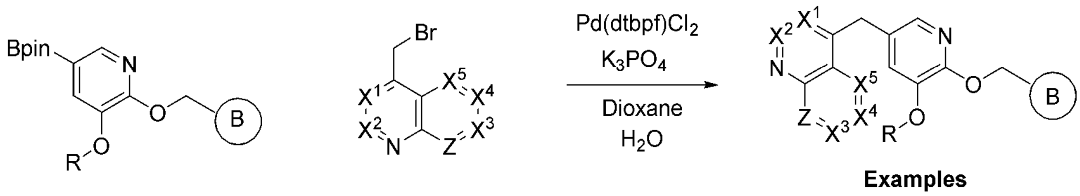


Example 68: 8-((5-(difluoromethoxy)-6-((5-methoxypyridin-2-yl)methoxy)pyridin-3-yl)methyl)-3-methoxy-1,5-naphthyridine

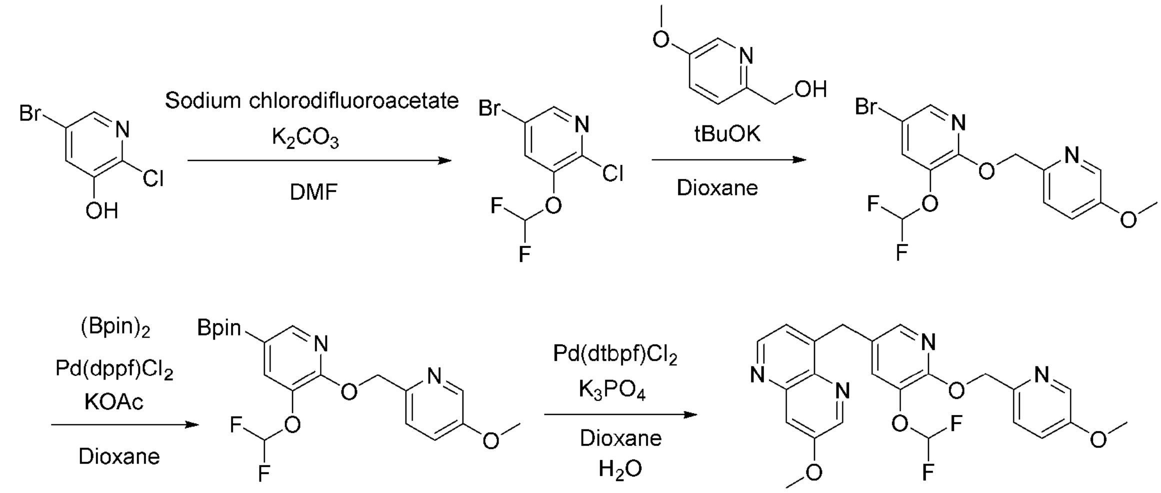


**Example 68**

To a solution of 5-bromo-2-chloro-pyridin-3-ol (5.00 g, 23.988 mmol) dissolved in *N,N*-Dimethylformamide (120 mL) was added Sodium chlorodifluoroacetate (7.363 g, 47.975 mmol) and $K_2CO_3$ (4.973 g, 35.982 mmol). The mixture was heated at 100 °C for 1 hr. After evaporating the solvent, the reside was purified by silica gel flash chromatography eluting with a gradient of to 100% heptane to 5% ethyl acetate in heptane to afford 5.10 g of 5-bromo-2-chloro-3-(difluoromethoxy)pyridine as a colorless oil (yield=82.26%).

To a solution of (5-methoxy-2-pyridyl)methanol ( 269 mg, 1.933 mmol) dissolved in 1,4-dioxane (5 mL) was added potassium t-butoxide (282 mg, 2.513 mmol) and 5-bromo-2-chloro-3-(difluoromethoxy)pyridine (500 mg, 1.935 mmol). The mixture was heated at 100 °C for 1 hr. After evaporating the solvent, the reside was purified by silica gel flash chromatography eluting with a gradient of to 100% heptane to 10% ethyl acetate in heptane to afford 540 mg of 5-bromo-3-(difluoromethoxy)-2-[(5-methoxy-2-pyridyl)methoxy]pyridine as a light yellow solid (yield=77.29%).

To a solution of 5-bromo-3-(difluoromethoxy)-2-[(5-methoxy-2-pyridyl)methoxy]pyridine ( 500 mg, 1.385 mmol) dissolved in 1,4-dioxane (10 mL) was added bis(pinacolato)diboron (527 mg, 2.075mmol), Bis(diphenylphosphino) ferrocene]dichloropalladium(II) (152 mg, 208 μmol) and potassium acetate (408 mg, 4.157 mmol). The mixture was heated to 100 °C for 2 hr under nitrogen atmosphere. After evaporating the solvent, the reside was purified by silica gel flash chromatography eluting with a gradient of 5% ethyl acetate in heptane to 20% ethyl acetate in heptane to afford 500 mg of 3-(difluoromethoxy)-2-[(5-methoxy-2-pyridyl)methoxy]-5-(4,4,5,5-tetramethyl-1,3,2-dioxaborolan-2-yl)pyridine as a light yellow solid (yield=88.47%).

To a solution of 4-(bromomethyl)-7-methoxy-quinoline (50 mg, 198 μmol) in 1,4-dioxane (1 mL) was added 3-(difluoromethoxy)-2-[(5-methoxy-2-pyridyl)methoxy]-5-(4,4,5,5-tetramethyl-1,3,2-dioxaborolan-2-yl)pyridine (121 mg, 296 μmol), Tripotassium Orthophosphate (126 mg, 594 μmol), 1,1'-Bis(di-tert-butylphosphino)ferrocene palladium dichloride (13 mg, 20 μmol)) and water (0.2mL). The mixture was heated to 90 °C for 1.5 hr under nitrogen atmosphere. After evaporating the solvent, the residue was purified by silica gel flash chromatography eluting with a gradient of 50% ethyl acetate in heptane to 100% ethyl acetate to afford 30 mg of 8-[[5-(difluoromethoxy)-6-[(5-methoxy-2-pyridyl) methoxy]-3-pyridyl]methyl]-3-methoxy-1,5-naphthyridine as a white solid (yield=33.42%). $^1$H NMR(400 MHz, DMSO) δ8.82 (dd, J = 5.6, 3.7 Hz, 2H), 8.25 (t, J = 1.8 Hz, 1H), 8.05 (d, J = 2.0 Hz, 1H), 7.79 (d, J = 2.9 Hz, 1H), 7.65 (d, J = 1.9 Hz, 1H), 7.46 (d, J = 4.5 Hz, 1H), 7.40 (d, J = 1.8 Hz, 2H), 7.16 (t, J = 73.8 Hz, 1H), 5.37 (s, 2H), 4.54 (s, 2H), 3.99 (s, 3H), 3.82 (s, 3H). MS (m/z): 455.1 $(M+H)^+$.

Method I:

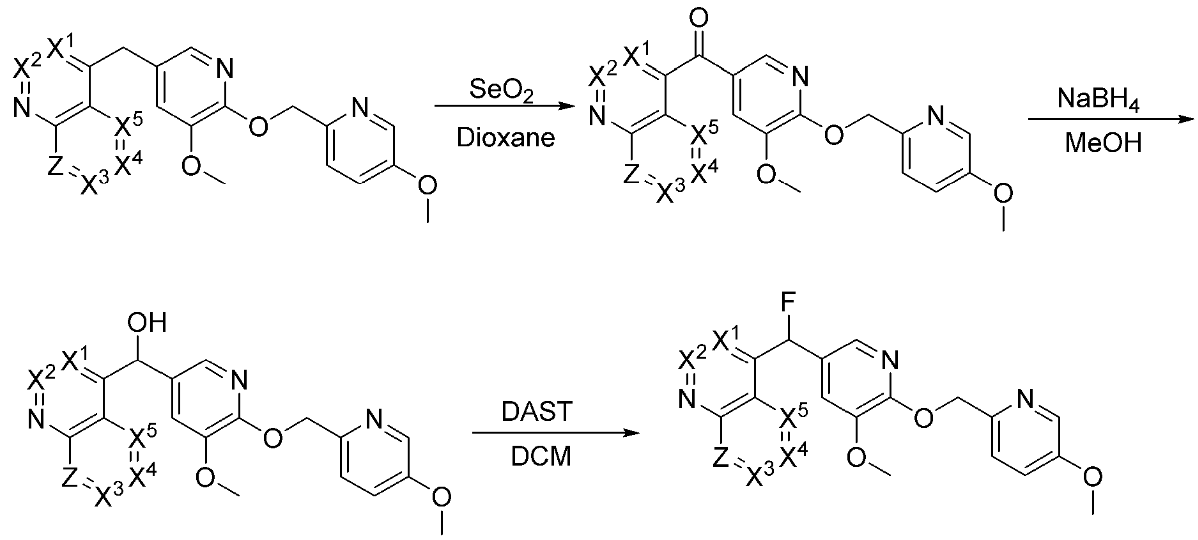


Examples

Example 84: 8-(fluoro(5-methoxy-6-((5-methoxypyridin-2-yl)methoxy)pyridin-3-yl)methyl)-3-methoxy-1,5-naphthyridine

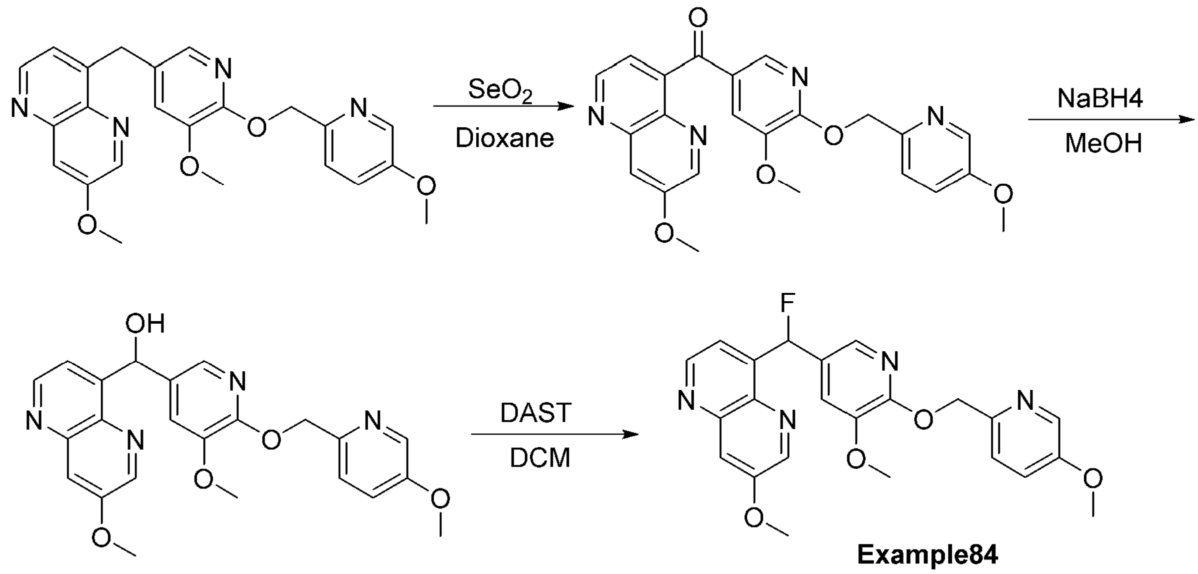


**Example84**

To a solution of 3-methoxy-8-((5-methoxy-6-((5-methoxypyridin-2-yl)methoxy)pyridin-3-yl)methyl)-1,5-naphthyridine (220 mg, 526 μmol) dissolved in 1,4-Dioxane (20 mL) was added $SeO_2$ (1.167 g, 10.515 mmol). The mixture was heated at 60 °C for 15 hr. The mixture was filtered and the solvent was evaporated. The reside was purified by silica gel flash chromatography eluting with a gradient of to 20% ethyl acetate in heptane to 100% ethyl acetate to afford 160 mg of [5-methoxy-6-[(5-methoxy-2-pyridyl)methoxy]-3-pyridyl]-(7-methoxy-1,5-naphthyridin-4-yl)methanone as a yellow solid (yield=70.38%).

To a solution of [5-methoxy-6-[(5-methoxy-2-pyridyl)methoxy]-3-pyridyl]-(7-methoxy-1,5-naphthyridin-4-yl) methanone (100 mg, 231 μmol) dissolved in MeOH (20 mL) was added $NaBH_4$ (18 mg, 476 μmol). The mixture was stirred at 25 °C for 1 hr. The mixture was added 20 ml water and then extracted with EtOAc (20 mL x 3).The combined organic layers were dried over anhydrous $Mg_2SO_4$, filtered and concentrated in vacuo. The reside was purified by silica gel flash chromatography eluting with a gradient of to 20% ethyl acetate in heptane to 100% ethyl acetate to afford 60 mg of [5-methoxy-6-[(5-methoxy-2-pyridyl)methoxy]-3-pyridyl]-(7-methoxy-1,5-naphthyridin-4-yl)methanol as a yellow oil (yield=59.72%).

To a solution of [5-methoxy-6-[(5-methoxy-2-pyridyl)methoxy]-3-pyridyl]-(7-methoxy-1,5-naphthyridin-4-yl)methanol (60 mg, 138 μmol) dissolved in DCM (10 mL) was added diethylaminosulphur trifluoride (DAST) (67 mg, 416 μmol). The mixture was stired at 25 °C for 1 hr. The mixture was added 20 ml $NaHCO_3$ and extracted with DCM (20 mL x 3).The combined organic layers were dried over anhydrous $Mg_2SO_4$, filtered and concentrated in vacuo. The reside was purified by silica gel flash chromatography eluting with a gradient of to 20% ethyl acetate in heptane to 100% ethyl acetate to afford 20 mg of 8-[fluoro-[5-methoxy-6-[(5-methoxy-2-pyridyl)methoxy]-3-pyridyl]methyl]-3-methoxy-1,5-naphthyridine as a white solid (yield=33.18%).$^1$H NMR (400 MHz, DMSO) δ 9.04(s, 1H), 8.74(s, 1H), 8.24(s, 1H), 7.83-7.87(m, 2H), 7.73(s, 1H), 7.61(d, 1H), 7.42(s, 1H), 7.37(s, 2H), 5.31(s, 2H), 3.98(s, 3H), 3.81(s, 3H), 3.77(s, 3H). MS (m/z): 437.1 $(M+H)^+$.

Method J:

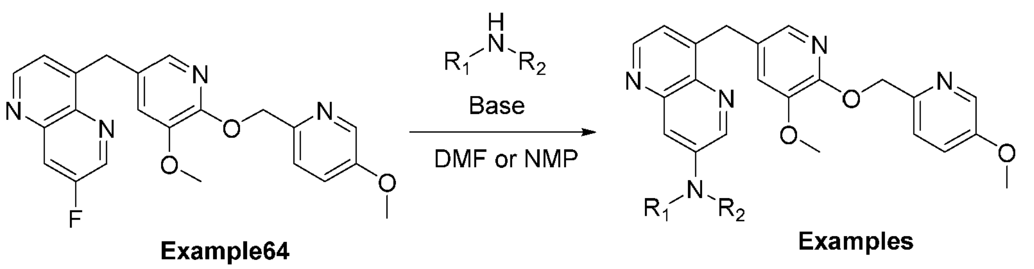


Example64

Examples

Example 100: 8-((5-methoxy-6-((5-methoxypyridin-2-yl)methoxy)pyridin-3-yl)methyl)-N,N-dimethyl-1,5-naphthyridin-3-amine

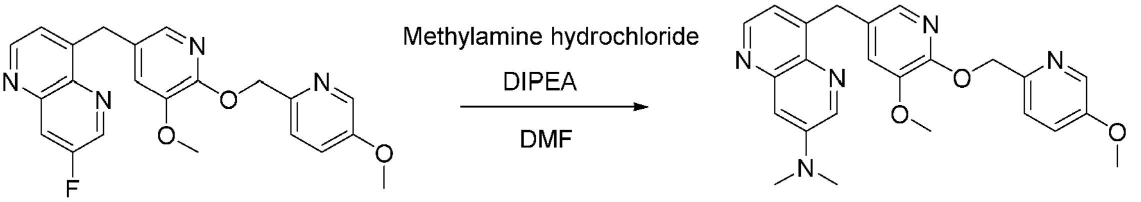


Example64

Example100

To a solution of 3-fluoro-8-((5-methoxy-6-((5-methoxypyridin-2-yl)methoxy)pyridin-3-yl)methyl)-1,5-naphthyridine ( 200 mg, 492 μmol) dissolved in N,N-Dimethylformamide (3 mL) was added Methylamine hydrochloride (80 mg, 981 μmol) and DIPEA (320 mg, 2.476 mmol). The mixture was heated in a microwave reactor at 180 °C for 1 hr under nitrogen atmosphere. After evaporating the solvent, the reside was purified by silica gel flash chromatography eluting with a gradient of 15% ethyl acetate in heptane to 100% ethyl acetate to afford 90 mg of 8-((5-methoxy-6-((5-methoxypyridin-2-yl)methoxy)pyridin-3-yl)methyl)-N,N-dimethyl-1,5-naphthyridin-3-amine as a light yellow solid (yield=42.39%). $^1$H NMR (400 MHz, DMSO) δ8.87(d, 1H), 8.67(d, 1H), 8.25(s, 1H), 7.64(s, 1H), 7.33-7.37(m, 3H), 7.27(s, 1H), 7.20(s, 1H), 5.30(s, 2H), 4.46(s, 2H), 3.82(s, 3H), 3.74(s, 3H), 3.12(s, 6H).MS (m/z): 432.1 $(M+H)^+$.

Example 119: 4-(8-((5-methoxy-6-((5-methoxypyridin-2-yl)methoxy)pyridin-3-yl)methyl)-1,5-naphthyridin-3-yl)morpholine

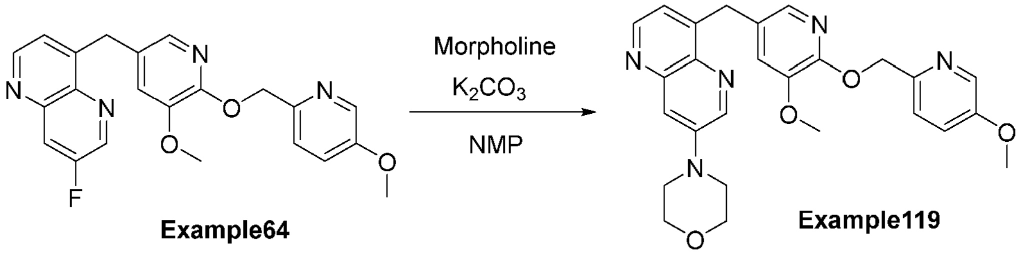


Example64 Example119

To a solution of 3-fluoro-8-((5-methoxy-6-((5-methoxypyridin-2-yl)methoxy)pyridin-3-yl)methyl)-1,5-naphthyridine (220 mg, 541 μmol) dissolved in N-Methyl pyrrolidone (2 mL) was added Morpholine (176 mg, 2.02 mmol) and $K_2CO_3$ (320 mg, 2.069 mmol). The mixture was heated in a microwave reactor at 200 °C for 1 hr under nitrogen atmosphere. After evaporating the solvent, the reside was purified by reversed phase column chromatography eluting with a gradient of 10% acetonitrile in water to 100% acetonitrile to afford 40 mg of 4-(8-((5-methoxy-6-((5-methoxypyridin-2-yl)methoxy)pyridin-3-yl)methyl)-1,5-naphthyridin-3-yl)morpholine as a white solid (yield=15.61%). $^1$H NMR (400 MHz, DMSO) δ9.04(s, 1H),8.03(d, 1H), 8.25(s, 1H), 7.63(s, 1H),7.53(s, 1H), 7.29-7.37(m, 4H),5.30(s, 2H),4.48(s, 2H),3.82(s, 7H),3.7(s, 3H),3.40(m,4H).MS (m/z): 475.1 $(M+H)^+$.

<u>Method K:</u>

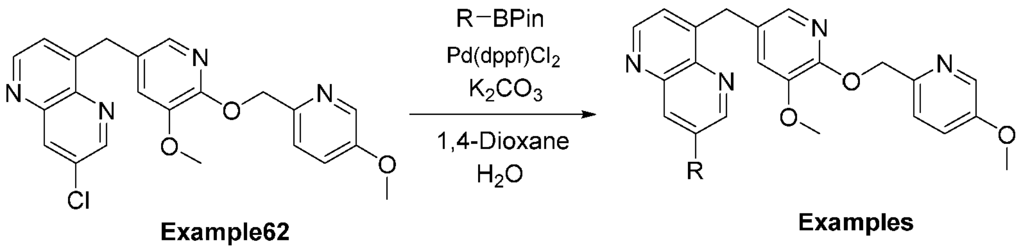


Example62 Examples

Example 134: 8-((5-methoxy-6-((5-methoxypyridin-2-yl)methoxy)pyridin-3-yl)methyl)-3-(1-methyl-1H-pyrazol-3-yl)-1,5-naphthyridine

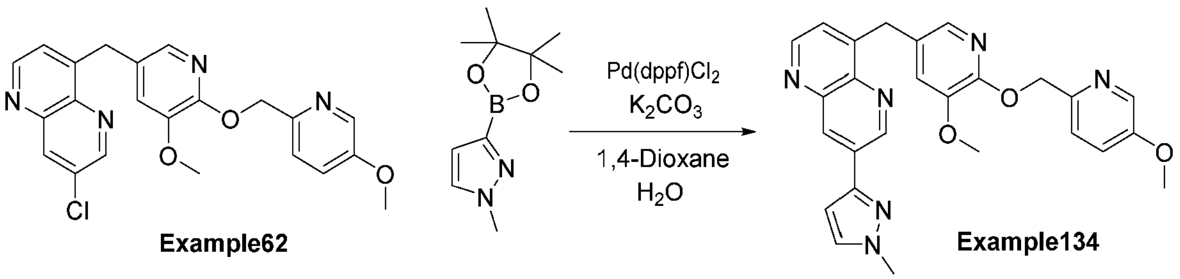


Example62 Example134

To a solution of 3-chloro-8-((5-methoxy-6-((5-methoxypyridin-2-yl)methoxy)pyridin-3-yl)methyl)-1,5-naphthyridine (850 mg, 2.01 mmol) dissolved in 1,4-Dioxane (15.0 mL) and $H_2O$ (3.0 mL) was added 1-methyl-3-(4,4,5,5-tetramethyl-1,3,2-dioxaborolan-2-yl)pyrazole (627 mg, 3.013 mmol), $K_2CO_3$ (834mg, 6.034 mmol) and bis(diphenylphosphino)ferrocene]dichloropalladium(II) (147 mg, 201 μmol). The mixture was heated at 80 °C for 16 hr under nitrogen atmosphere. After evaporating the solvent, the reside was purified by reversed phase column chromatography eluting with a gradient of 100% ethyl acetate to 70% tetrahydrofuran in ethyl acetate to afford 700 mg of 8-((5-methoxy-6-((5-methoxypyridin-2-yl)methoxy)pyridin-3-yl)methyl)-3-(1-methyl-1H-pyrazol-3-yl)-1,5-naphthyridine as a white solid (yield=74.33%). $^1$H NMR (400 MHz, DMSO) δ 9.55 (d, J = 2.1 Hz, 1H), 8.89 (d, J = 4.4 Hz, 1H), 8.68 (d, J = 2.1 Hz, 1H), 8.25 (t, J = 1.7 Hz, 1H), 7.88 (d, J = 2.2 Hz, 1H), 7.69 (d, J = 1.7 Hz, 1H), 7.51 (d, J = 4.4 Hz, 1H), 7.39 (dd, J = 4.9, 1.8 Hz, 3H), 7.10 (d, J = 2.3 Hz, 1H), 5.30 (s, 2H), 4.56 (s, 2H), 3.98 (s, 3H), 3.81 (s, 3H), 3.74 (s, 3H).MS (m/z): 469.1 (M+H)+.

Example 138: 5-(8-((5-methoxy-6-((5-methoxypyridin-2-yl)methoxy)pyridin-3-yl)methyl)-1,5-naphthyridin-3-yl)-2-methylthiazole

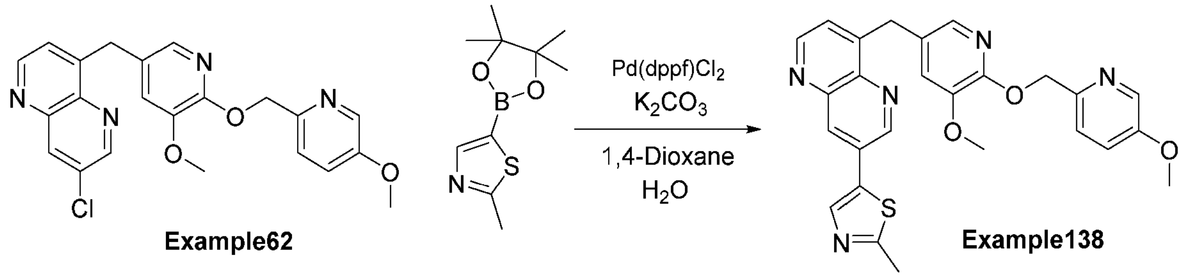


To a solution of 3-chloro-8-((5-methoxy-6-((5-methoxypyridin-2-yl)methoxy)pyridin-3-yl)methyl)-1,5-naphthyridine (70 mg, 166 μmol) dissolved in 1,4-Dioxane (2.5 mL) and $H_2O$ (0.5 mL) was added 2-methyl-5-(4,4,5,5-tetramethyl-1,3,2-dioxaborolan-2-yl)thiazole (56 mg, 249 μmol) , $K_2CO_3$ (69 mg, 499 μmol) and bis(diphenylphosphino) ferrocene]dichloropalladium(II) (13 mg, 17.8 μmol). The mixture was heated in a microwave reactor at 100 °C for 1 hr under nitrogen atmosphere. After evaporating the solvent, the reside was purified by reversed phase column chromatography eluting with a gradient of 10% ethyl acetate in heptane to 100% ethyl acetate e to afford 8 mg of 5-(8-((5-methoxy-6-((5-methoxypyridin-2-yl)methoxy)pyridin-3-yl)methyl)-1,5-naphthyridin-3-yl)-2-methylthiazole as an off-white solid (yield=16.48%). $^1H$ NMR (400 MHz, DMSO) δ9.41(d, 1H), 8.92(d, 1H), 8.54(d, 1H), 8.46(d, 1H), 8.25(d, 1H), 7.67(d, 1H), 7.58(d, 1H), 7.38(d, 3H), 5.30(s, 2H), 4.56(s,2H), 3.82(s, 3H), 3.75(s,3H), 2.76(s,3H).MS (m/z): 486.1 $(M+H)^+$.

Example 140: 8-((5-methoxy-6-((5-methoxypyridin-2-yl)methoxy)pyridin-3-yl)methyl)-3-(pyridin-3-yl)-1,5-naphthyridine

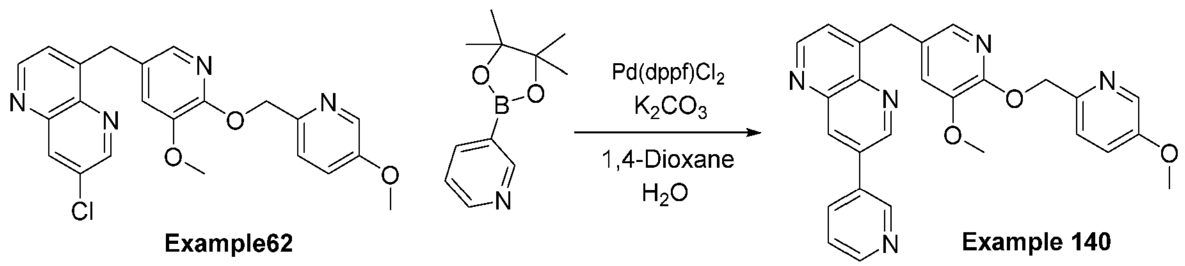


To a solution of 3-chloro-8-((5-methoxy-6-((5-methoxypyridin-2-yl)methoxy)pyridin-3-yl)methyl)-1,5-naphthyridine (60 mg, 142 μmol) dissolved in 1,4-Dioxane (3.0 mL) and $H_2O$ (0.6 mL) was added 3-(4,4,5,5-tetramethyl-1,3,2-dioxaborolan-2-yl)pyridine (58 mg, 283 μmol), $K_2CO_3$ (60 mg, 434 μmol) and bis(diphenylphosphino)ferrocene]dichloropalladium(II) (10 mg, 14.2 μmol).. The mixture was heated in a microwave reactor at 90 °C for 1 hr under nitrogen atmosphere. After evaporating the solvent, the reside was purified by reversed phase column chromatography eluting with a gradient of 10% ethyl acetate in heptane to 100% ethyl acetate to afford 20 mg of 8-((5-methoxy-6-((5-methoxypyridin-2-yl)methoxy)pyridin-3-yl)methyl)-3-(pyridin-3-yl)-1,5-naphthyridine as a white solid (yield=30.28%). $^1H$ NMR (400 MHz, DMSO) δ9.49(s, 1H), 9.20(s, 1H), 8.96(s, 1H), 8.78(s, 1H), 8.71(d, 1H), 8.39-8.42(m, 1H), 8.25(s, 1H), 7.68(s, 1H), 7.59-7.62(m, 2H), 7.38-7.41(m, 3H), 5.31(s, 2H), 4.60(s, 2H), 3.82(s, 3H), 3.76(s, 3H)..MS (m/z): 466.1 $(M+H)^+$.

Method L:

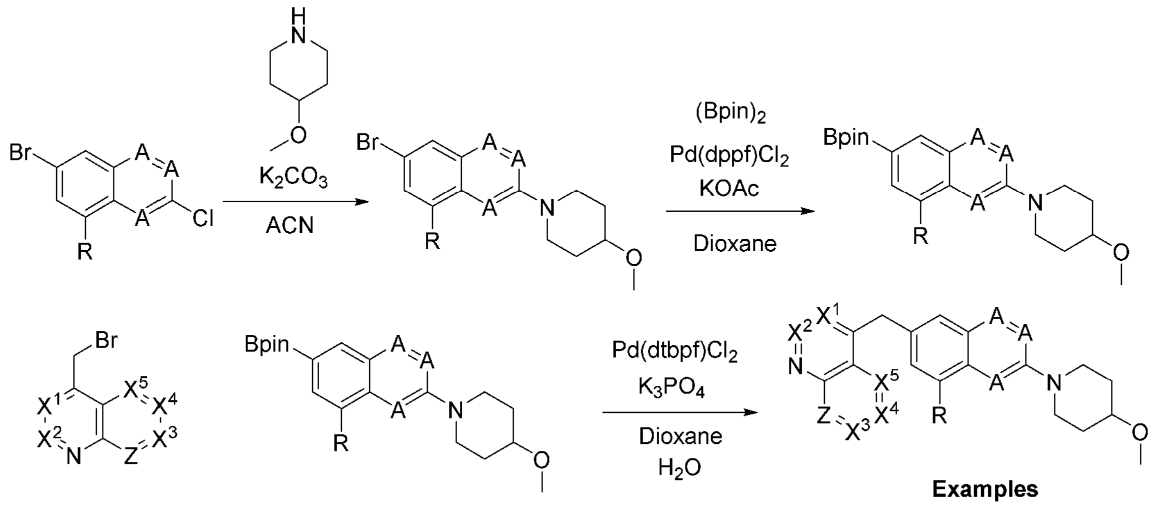


**Examples**

Example 153: 3-methoxy-8-[[2-(4-methoxy-1-piperidyl)quinoxalin-6-yl]methyl]-1,5-naphthyridine

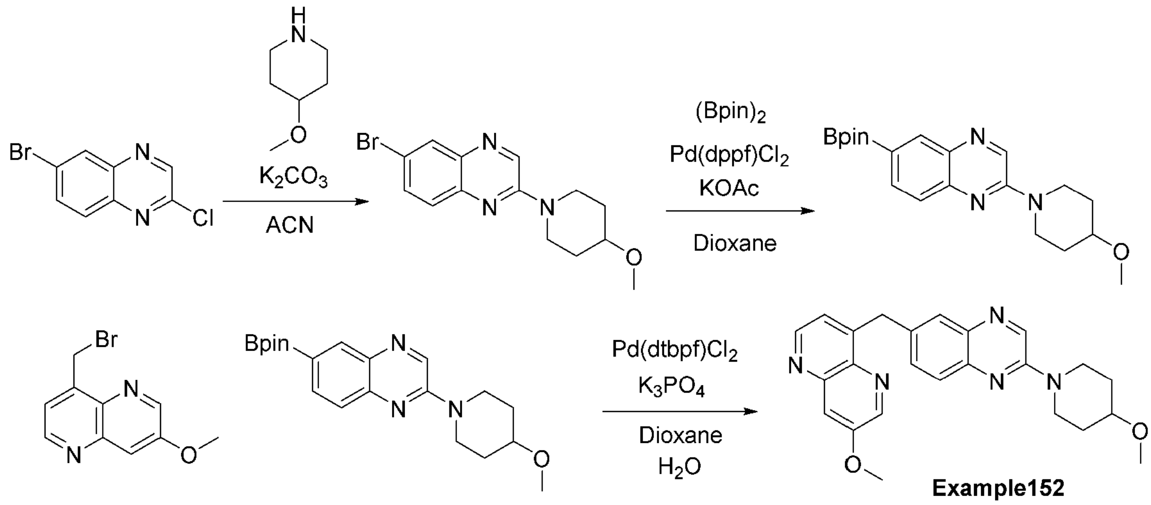


**Example152**

To a solution of 6-bromo-2-chloro-quinoxaline (0.50 g, 2.053 mmol) dissolved in acetonitrile (10 mL) was added 4-methoxypiperidine (0.473 g, 4.107 mmol) and $K_2CO_3$ (0.567g, 4.103 mmol). The mixture was stired at 90 °C for 15 hr. After evaporating the solvent, the reside was purified by silica gel flash chromatography eluting with a gradient of to 15% ethyl acetate in heptane to 50% ethyl acetate in heptane to afford 0.45 g of 6-bromo-2-(4-methoxy-1-piperidyl)quinoxaline as a yellow solid (yield=68.01%).

To a solution of 6-bromo-2-(4-methoxy-1-piperidyl)quinoxaline (450 mg, 1.397 mmol) dissolved in 1,4-dioxane (15 mL) was added bis(pinacolato)diboron (532 mg, 2.095mmol), Bis(diphenylphosphino)ferrocene]dichloropalladium(II) (100 mg, 140 μmol) and potassium acetate (411 mg, 4.188 mmol). The mixture was heated to 100 °C for 2 hr under nitrogen atmosphere. After evaporating the solvent, the reside was purified by silica gel flash chromatography eluting with a gradient of 15% ethyl acetate in heptane to 100% ethyl acetate to afford 300 mg of 2-(4-methoxy-1-piperidyl)-6-(4,4,5,5-tetramethyl-1,3,2-dioxaborolan-2-yl)quinoxaline as a yellow oil (yield=58.17%).

To a solution of 4-(bromomethyl)-7-methoxy-quinoline (70 mg, 277 μmol) in 1,4-dioxane (10 mL) was added 2-[(5-methoxy-2-pyridyl)methoxy]-5-(4,4,5,5-tetramethyl-1,3,2-dioxaborolan-2-yl)pyridine (100 mg, 271 μmol), Tripotassium Orthophosphate (176 mg, 829 μmol), 1,1'-Bis(di-tert-butylphosphino)ferrocene palladium dichloride (20 mg, 28 μmol) and water (2mL). The mixture was heated to 90 °C for 2 hr under nitrogen atmosphere. After evaporating the solvent, the residue was purified by silica gel flash chromatography eluting with a gradient of 15% ethyl acetate in heptane to 100% ethyl acetate to afford 30 mg of 3-methoxy-8-[[2-(4-methoxy-1-piperidyl)quinoxalin-6-yl]methyl]-1,5-naphthyridine as a yellow solid (yield=26.11%).$^1$H NMR(400 MHz, DMSO) δ 8.78-8.83(m, 3H), 7.70(s, 1H), 7.78(s, 1H), 7.45-7.57(m, 3H), 4.72(s, 2H), 4.09-4.15(m, 2H), 4.00(s, 3H), 3.37-3.49( m, 3H), 3.29(s, 3H), 1.91-1.95(m, 2H), 1.44-1.52(m, 2H).MS (m/z):

417.1 (M+H)+.

Method M:

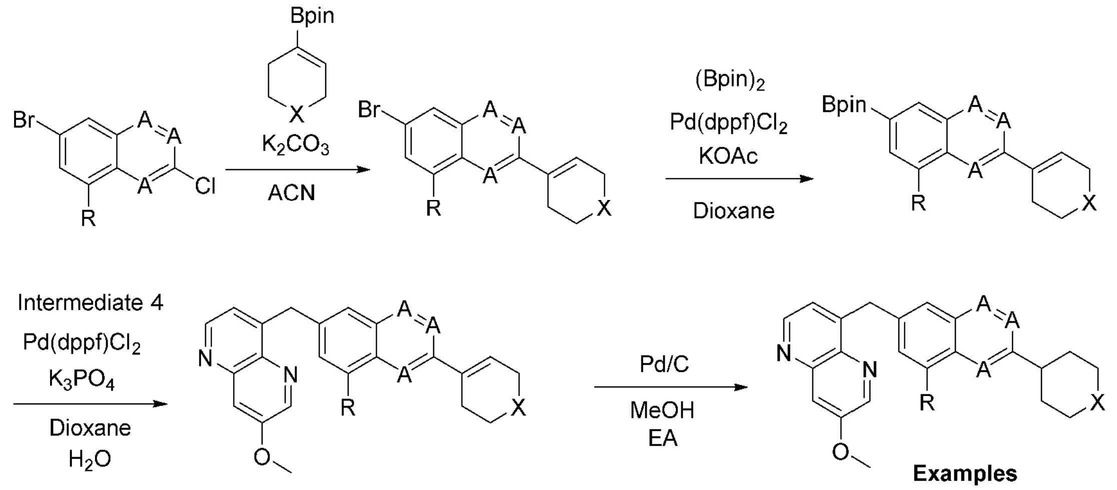


Example 154: 6-((7-methoxy-1,5-naphthyridin-4-yl)methyl)-2--4-methoxycyclohexyl)quinoxaline

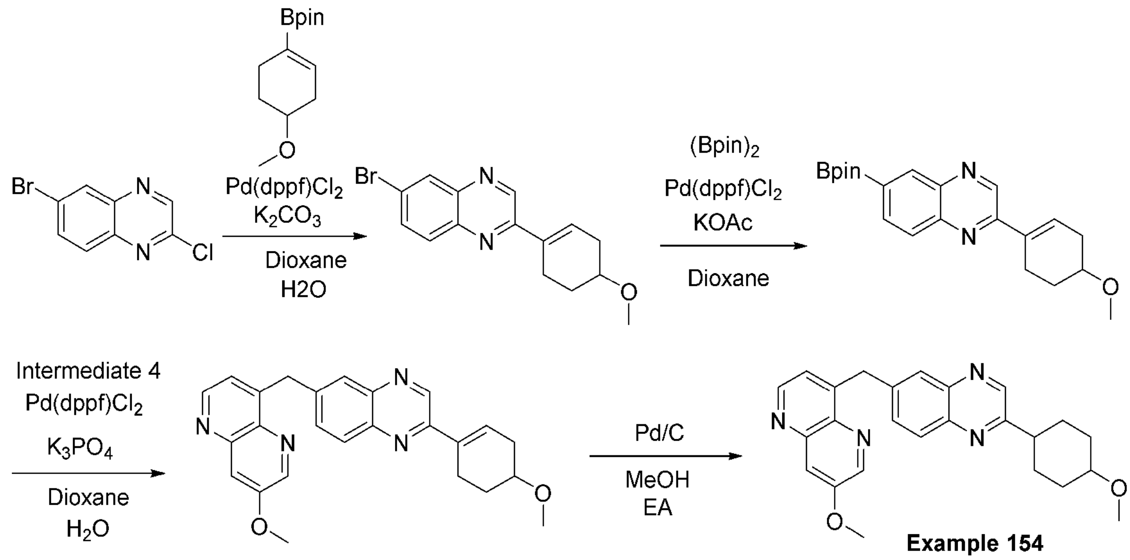


To a solution of 6-bromo-2-chloro-quinoxaline (0.50 g, 2.053 mmol) dissolved in 1,4-dioxane (10 mL) was added 2-(4-methoxycyclohexen-1-yl)-4,4,5,5-tetramethyl-1,3,2-dioxaborolane (0.489 g, 2.054 mmol), Bis(diphenylphosphino) ferrocene]dichloropalladium(II) (150 mg, 205 μmol), $K_2CO_3$ (0.568g, 4.11 mmol) and $H_2O$ (2 mL). The mixture was stired at 75 °C for 2 hr. After evaporating the solvent, the reside was purified by silica gel flash chromatography eluting with a gradient of to 15% ethyl acetate in heptane to 40% ethyl acetate in heptane to afford 0.35 g of 6-bromo-2-(4-methoxycyclohexen-1-yl)quinoxaline as a white solid (yield=68.01%).

To a solution of 6-bromo-2-(4-methoxycyclohexen-1-yl)quinoxaline (350 mg, 1.097 mmol) dissolved in 1,4-dioxane (20 mL) was added bis(pinacolato)diboron (417 mg, 1.642mmol), Bis(diphenylphosphino)ferrocene]dichloropalladium(II) (80 mg, 110 μmol) and potassium acetate (322 mg, 3.281 mmol). The mixture was heated to 100 °C for 2 hr under nitrogen atmosphere. After evaporating the solvent, the reside was purified by silica gel flash chromatography eluting with a gradient of 15% ethyl acetate in heptane to 40% ethyl acetate in heptane to afford 350 mg of 2-(4-methoxycyclohexen-1-yl)-6-(4,4,5,5-tetramethyl-1,3,2-dioxaborolan-2-yl)quinoxaline as a white solid (yield=87.15%).

To a solution of 4-(bromomethyl)-7-methoxy-quinoline (100 mg, 395 μmol) in 1,4-dioxane (10 mL) was added 2-(4-methoxycyclohexen-1-yl)-6-(4,4,5,5-tetramethyl-1,3,2-dioxaborolan-2-yl)quinoxaline (144 mg, 393 μmol), Tripotassium Orthophosphate (251 mg, 1.182 mmol), Bis(diphenylphosphino)ferrocene]dichloropalladium(II) (30 mg, 40 μmol) and water (2mL). The mixture was heated to 90 °C for 2 hr under nitrogen atmosphere. After evaporating the solvent, the residue was purified by silica gel flash chromatography eluting with a gradient of 15% ethyl acetate in heptane to 100% ethyl acetate to afford 100 mg of 2-(4-methoxycyclohexen-1-yl)-6-[(7-methoxy-1,5-naphthyridin-4-yl)methyl]quinoxaline as a white solid (yield=61.36%).

To a solution of 2-(4-methoxycyclohexen-1-yl)-6-[(7-methoxy-1,5-naphthyridin-4-yl)methyl]quinoxaline (100 mg, 242 μmol) in ethyl acetate (10 mL) and methanol (5mL) was added 50mg of Pd/C. The mixture was stured to 20 °C for 1 hr under hydrogen atmosphere. The mixture was filtered and concentrated in vacuo. The residue was purified by reversed phase column chromatography eluting with a gradient of 5% acetonitrile in $H_2O$ to 100% acetonitrile to afford 10 mg of 6-((7-methoxy-1,5-naphthyridin-4-yl)methyl)-2--4-methoxycyclohexyl)quinoxaline as a white solid (yield=9.95%).$^1$H NMR(400 MHz, DMSO) δ8.83-8.84(m, 3H), 7.93-7.95(m, 2H), 7.77-7.80(m, 2H), 7.49(d, 1H), 4.83(s, 2H), 4.00(s, 3H), 3.50(s, 1H), 3.25(s, 3H), 2.99-3.05( m, 1H), 1.86-1.99(m, 4H), 1.66-1.70(m, 2H), 1.53-1.60(m, 2H) .MS (m/z): 415.2 $(M+H)^+$.

<u>Method N:</u>

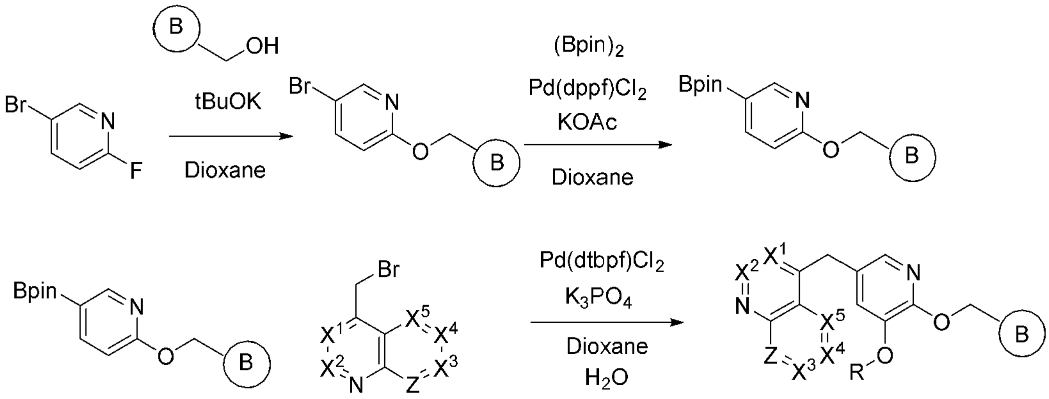


**Examples**

Example 151: 3-methoxy-8-((6-((5-methoxypyridin-2-yl)methoxy)pyridin-3-yl)methyl)-1,5-naphthyridine

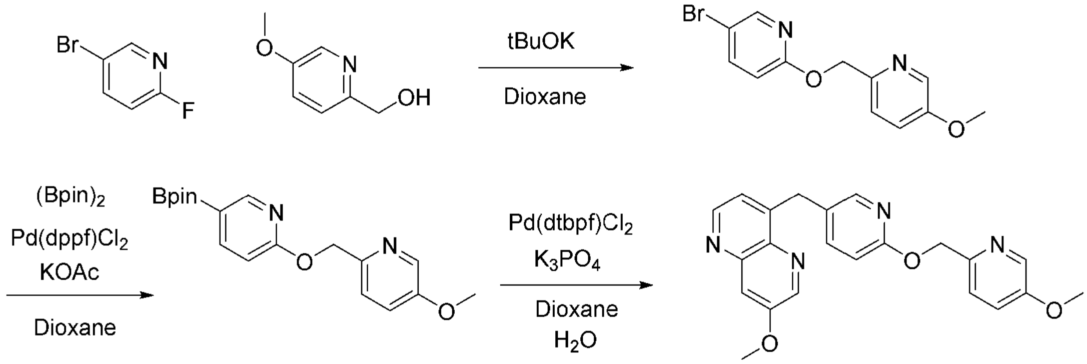


**Example 151**

To a solution of (5-methoxy-2-pyridyl)methanol (1.00 g, 7.186 mmol) dissolved in 1,4-dioxane (15 mL) was added potassium t-butoxide (1.00 g, 8.912 mmol) and 5-bromo-2-fluoro-pyridine (1.00 g, 5.682 mmol). The mixture was stired at 20 oC for 1 hr. The mixture was added saturated $NH_4Cl$ solution to quench the reaction. After evaporating the solvent, the reside was purified by silica gel flash chromatography eluting with a gradient of to 100% heptane to 20% ethyl acetate in heptane to afford 1.67 g of 2-[(5-bromo-2-pyridyl)oxymethyl]-5-methoxy-pyridine as a white solid (yield=99.58%).

To a solution of 2-[(5-bromo-2-pyridyl)oxymethyl]-5-methoxy-pyridine (400 mg, 1.355 mmol) dissolved in 1,4-dioxane (15 mL) was added bis(pinacolato)diboron (516 mg, 2.032mmol), Bis(diphenylphosphino)ferrocene]dichloropalladium(II) (99 mg, 136 μmol) and potassium acetate (266 mg, 2.71 mmol). The mixture was heated to 100 °C for 12 hr under nitrogen atmosphere. After evaporating the solvent, the reside was purified by silica gel flash chromatography eluting with a gradient of 5% ethyl acetate in heptane to 50% ethyl acetate in heptane to afford 200 mg of 2-[(5-methoxy-2-pyridyl)methoxy]-5-(4,4,5,5-tetramethyl-1,3,2-dioxaborolan-2-yl)pyridine as a white solid (yield=43.12%).

To a solution of 4-(bromomethyl)-7-methoxy-quinoline (81 mg, 320 μmol) in 1,4-dioxane (5 mL) was added 2-[(5-methoxy-2-pyridyl)methoxy]-5-(4,4,5,5-tetramethyl-1,3,2-dioxaborolan-2-yl)pyridine (100 mg, 292 μmol), Tripotassium Orthophosphate (155 mg, 730 μmol), 1,1'-Bis(di-tert-butylphosphino)ferrocene palladium dichloride (19 mg, 29 μmol) and water (1mL). The mixture was heated to 90 °C for 12 hr under nitrogen atmosphere. After evaporating the solvent, the residue was purified by silica gel flash chromatography eluting with a gradient of 5% ethyl acetate in heptane to 100% ethyl acetate to afford 35 mg of 3-methoxy-8-[[6-[(5-methoxy-2-pyridyl)methoxy]-3-pyridyl]methyl]-1,5-naphthyridine as a white solid (yield=30.84%).$^1$H NMR(400 MHz, $CDCl_3$)δ 8.78 (t, J = 3.7 Hz, 2H), 8.33 (d, J = 2.7 Hz, 1H), 8.16 (d, J = 2.0 Hz, 1H), 7.67 (d, J = 2.8 Hz, 1H), 7.55 (dd, J = 8.5, 2.4 Hz, 1H), 7.40 (d, J = 8.6 Hz, 1H), 7.21 (dd, J = 8.5, 2.9 Hz, 2H), 6.79 (d, J = 8.5 Hz, 1H), 5.44 (s, 2H), 4.57 (s, 2H), 4.02 (s, 3H), 3.88 (s, 3H) MS (m/z): 489.1 $(M+H)^+$.

Method O:

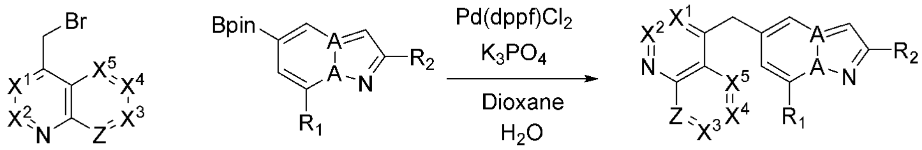


**Examples**

Example **158:** 3-methoxy-8-((2-(tetrahydro-2H-pyran-4-yl)pyrazolo[1,5-a]pyridin-5-yl)methyl)-1,5-naphthyridine

To a solution of 4-(bromomethyl)-7-methoxy-quinoline (150 mg, 593 μmol) in 1,4-dioxane (10 mL) was added 2-tetrahydropyran-4-yl-5-(4,4,5,5-tetramethyl-1,3,2-dioxaborolan-2-yl)pyrazolo[1,5-a]pyridine (200 mg, 609 μmol), Tripotassium Orthophosphate (388 mg, 1.828 mmol), Bis(diphenylphosphino)ferrocene]dichloropalladium(II) (45 mg, 61 μmol) and water (2mL). The mixture was heated to 90 °C for 2 hr under nitrogen atmosphere. After evaporating the solvent, the residue was purified by silica gel flash chromatography eluting with a gradient of 15% ethyl acetate in heptane to 100% ethyl acetate to afford 100 mg of 3-methoxy-8-[(2-tetrahydropyran-4-ylpyrazolo[1,5-a]pyridin-5-yl)methyl]-1,5-naphthyridine as a white solid (yield=43.83%).1H NMR(400 MHz, DMSO)δ 8.80-8.83(m, 2H), 8.46(d, 1H), 7.79(d, 1H), 7.41-7.47(m, 2H), 6.73(d, 1H), 6.32(s, 1H), 4.59(s, 2H), 3.99(s, 3H), 3.89-3.92(m, 2H), 3.42-3.48(m,2H), 2.95-3.00( m, 1H), 1.84-1.87(m, 2H), 1.65-1.75(m, 2H) . MS (m/z): 375.2 $(M+H)^+$.

Additional compounds were prepared by modifications of the methods exemplified herein. All of the compounds and their corresponding characterization data are presented in Table 1 below.

Table 1

| Example No (Method) | Structure; IUPAC name | LC-MS (M+H) + | $^1$H NMR (ppm) |
|---|---|---|---|
| 1 (A) | 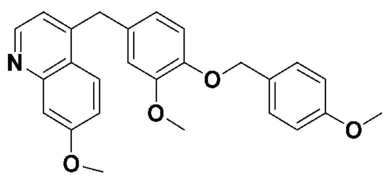 7-methoxy-4-(3-methoxy-4-((4-methoxybenzyl)oxy) benzyl)qu inoline | 416.1 | $^1$H NMR (400 MHz, DMSO) δ 8.73 (d, *J* = 4.5 Hz, 1H), 8.11 (d, *J* = 9.2 Hz, 1H), 7.40 (d, *J* = 2.6 Hz, 1H), 7.36 - 7.31 (m, 2H), 7.24 (dd, *J* = 9.2, 2.7 Hz, 1H), 7.16 (d, *J* = 4.5 Hz, 1H), 6.98 - 6.89 (m, 4H), 6.68 (dd, *J* = 8.2, 1.9 Hz, 1H), 4.92 (s, 2H), 4.35 (s, 2H), 3.91 (s, 3H), 3.75 (s, 3H), 3.70 (s, 3H). |

(continued)

| Example No (Method) | Structure; IUPAC name | LC-MS (M+H) + | $^1$H NMR (ppm) |
|---|---|---|---|
| 2 (A) | 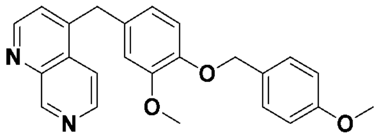 4-(3-methoxy-4-((4-methoxybenzyl)oxy)benzyl)-1,7-naphthyridine | 387.1 | $^1$H NMR (400 MHz, DMSO) δ 9.39 (s, 1H), 8.97 (d, *J* = 4.3 Hz, 1H), 8.62 (d, *J* = 5.8 Hz, 1H), 8.11 (d, *J* = 5.8 Hz, 1H), 7.55 (d, *J* = 4.3 Hz, 1H), 7.34 (d, *J* = 8.5 Hz, 2H), 7.03 - 6.89 (m, 4H), 6.73 (d, *J* = 8.1 Hz, 1H), 4.93 (s, 2H), 4.41 (s, 2H), 3.75 (s, 3H), 3.72 (s, 3H). |
| 3 (A) | 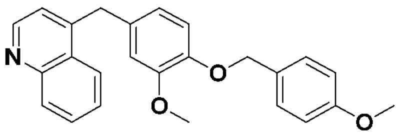 4-(3-methoxy-4-((4-methoxybenzyl)oxy)benzyl)quinoline | 386.1 | $^1$H NMR (400 MHz, DMSO) δ 8.82 (d, *J* = 4.4 Hz, 1H), 8.22 (d, *J* = 8.2 Hz, 1H), 8.03 (d, *J* = 8.2 Hz, 1H), 7.78 - 7.70 (m, 1H), 7.61 (dd, *J* = 11.2, 4.0 Hz, 1H), 7.33 (dd, *J* = 9.8, 6.5 Hz, 3H), 7.00 - 6.89 (m, 4H), 6.70 (dd, *J* = 8.2, 1.6 Hz, 1H), 4.93 (s, 2H), 4.40 (s, 2H), 3.75 (s, 3H), 3.71 (s, 3H). |
| 4 (A) | 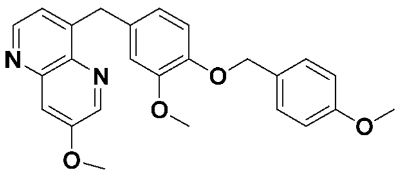 3-methoxy-8-(3-methoxy-4-((4-methoxybenzyl)oxy) benzyl)-1,5-naphthyridine | 417.2 | $^1$H NMR (400 MHz, DMSO) δ 8.80 (dd, *J* = 9.4, 3.7 Hz, 2H), 7.78 (d, *J* = 2.9 Hz, 1H), 7.34 (t, *J* = 6.2 Hz, 3H), 7.01 (d, *J* = 1.9 Hz, 1H), 6.95 - 6.89 (m, 3H), 6.77 (dd, *J* = 8.2, 1.9 Hz, 1H), 4.93 (s, 2H), 4.50 (s, 2H), 3.99 (s, 3H), 3.75 (s, 3H), 3.70 (s, 3H). |
| 5 (A) | 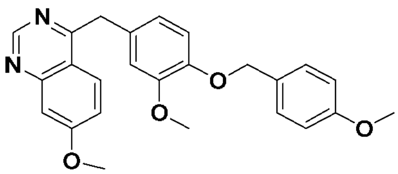 7-methoxy-4-(3-methoxy-4-((4-methoxybenzyl)oxy) benzyl)qu inazoline | 417.2 | $^1$H NMR (400 MHz, DMSO) δ 9.09 (s, 1H), 8.34 (d, *J* = 8.9 Hz, 1H), 7.33 (dd, *J* = 8.7, 3.9 Hz, 4H), 7.01 (d, *J* = 1.6 Hz, 1H), 6.91 (dd, *J* = 8.3, 6.0 Hz, 3H), 6.76 (d, *J* = 8.1 Hz, 1H), 4.91 (s, 2H), 4.49 (s, 2H), 3.95 (s, 3H), 3.74 (s, 3H), 3.70 (s, 3H). |
| 6 (B) | 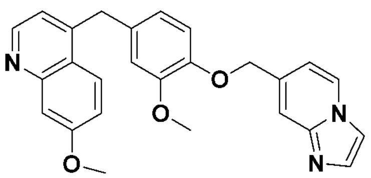 4-(4-(imidazo[1,2-a]pyridin-7-ylmethoxy)-3-methoxybenzyl)-7-methoxyquinoline | 426.1 | $^1$H NMR(400 MHz, DMSO) δ8.72 (d, *J* = 4.4 Hz, 1H), 8.53 (dd, *J* = 7.2, 1.2 Hz, 1H), 8.11 (d, *J*= 9.2 Hz, 1H), 7.94-7.93 (m, 1H), 7.64-7.49 (m, 2H), 7.39 (d, *J* = 2.8 Hz, 1H), 7.24 (dd, *J* = 9.2, 2.8 Hz, 1H), 7.16 (d, *J* = 4.4 Hz, 1H), 7.02-6.88 (m, 3H), 6.69 (dd, *J* = 8.0, 2.0 Hz, 1H), 5.09 (s, 2H), 4.36 (s, 2H), 3.91 (s, 3H), 3.75 (s, 3H). |

(continued)

| Example No (Method) | Structure; IUPAC name | LC-MS (M+H) + | $^1$H NMR (ppm) |
|---|---|---|---|
| 7 (B) | 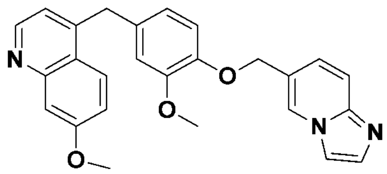 4-(4-(imidazo[1,2-a]pyridin-6-ylmethoxy)-3-methoxybenzyl)-7-methoxyquinoline | 426.1 | $^1$H NMR(400 MHz, DMSO) δ8.73 (d, *J* = 4.4 Hz, 1H), 8.63-8.62 (m, 1H), 8.11 (d, *J* = 7.8 Hz, 1H), 7.97-7.96 (m, 1H), 7.66-7.49 (m, 2H), 7.40 (d, *J* = 2.7 Hz, 1H), 7.29-7.23 (m, 2H), 7.16 (d, *J* = 4.4 Hz, 1H), 7.03-6.96 (m, 2H), 6.72-6.69 (m, 1H), 5.02 (s, 2H), 4.36 (s, 2H), 3.91 (s, 3H), 3.72 (s, 3H). |
| 8 (B) | 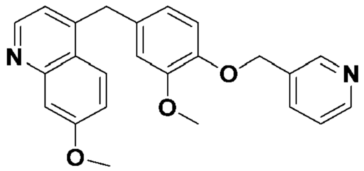 7-methoxy-4-(3-methoxy-4-(pyridin-3-ylmethoxy) benzyl)quinoline | 387.1 | $^1$H NMR(400 MHz, DMSO) δ8.73 (d, *J* = 4.5 Hz, 1H), 8.63 (d, *J* = 1.6 Hz, 1H), 8.54 (dd, *J* = 4.8, 1.5 Hz, 1H), 8.11 (d, *J* = 9.2 Hz, 1H), 7.83 (dt, *J* = 7.8, 1.9 Hz, 1H), 7.47 - 7.34 (m, 2H), 7.24 (dt, *J* = 13.0, 6.5 Hz, 1H), 7.17 (d, *J* = 4.5 Hz, 1H), 6.98 (dd, *J* = 7.1, 5.1 Hz, 2H), 6.70 (dd, *J* = 8.2, 1.9 Hz, 1H), 5.07 (s, 2H), 4.36 (s, 2H), 3.91 (s, 3H), 3.72 (s, 3H) |
| 9 (B) | 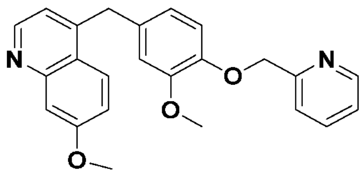 7-methoxy-4-(3-methoxy-4-(pyridin-2-ylmethoxy) benzyl)quinoline | 387.1 | $^1$H NMR(400 MHz, DMSO) δ8.73 (d, *J* = 4.4 Hz, 1H), 8.55 (d, *J* = 4.4 Hz, 1H), 8.12 (d, *J* = 9.2 Hz, 1H), 7.83 (td, *J* = 7.7, 1.5 Hz, 1H), 7.50 (d, *J* = 7.8 Hz, 1H), 7.40 (d, *J* = 2.5 Hz, 1H), 7.33 (dd, *J* = 7.0, 5.2 Hz, 1H), 7.23 (dt, *J* = 16.2, 8.1 Hz, 1H), 7.17 (d, *J* = 4.4 Hz, 1H), 7.00 (d, *J* = 1.4 Hz, 1H), 6.91 (t, *J* = 9.4 Hz, 1H), 6.75 - 6.62 (m, 1H), 5.10 (s, 2H), 4.36 (s, 2H), 3.91 (s, 3H), 3.75 (s, 3H) |
| 10 (B) | 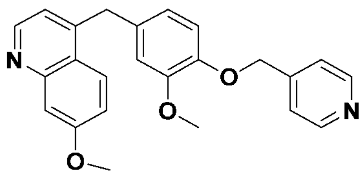 7-methoxy-4-(3-methoxy-4-(pyridin-4-ylmethoxy) benzyl)quinoline | 387.1 | $^1$H NMR(400 MHz, DMSO) δ8.76 (d, *J* = 4.5 Hz, 1H), 8.62 (d, *J* = 5.9 Hz, 2H), 7.93 (d, *J* = 9.2 Hz, 1H), 7.45 (t, *J* = 10.2 Hz, 1H), 7.37 (d, *J* = 5.9 Hz, 2H), 7.20 (dd, *J* = 9.2, 2.6 Hz, 1H), 7.03 (d, *J* = 4.5 Hz, 1H), 6.77 (t, *J* = 5.0 Hz, 2H), 6.66 (dd, *J* = 8.2, 2.0 Hz, 1H), 5.16 (s, 2H), 4.36 (s, 2H), 3.98 (s, 3H), 3.85 (s, 3H). |

(continued)

| Example No (Method) | Structure; IUPAC name | LC-MS (M+H) + | $^1$H NMR (ppm) |
|---|---|---|---|
| 11 (B) | 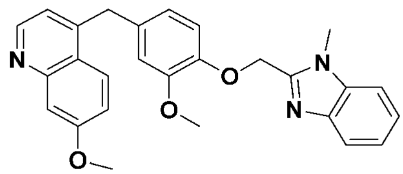 7-methoxy-4-(3-methoxy-4-((1-methyl-1H-benzo[d] imidazol-2-yl)methoxy)benzyl)quinoline | 440.1 | $^1$H NMR(400 MHz, DMSO) δ8.81 (d, *J* = 4.8 Hz, 1H), 8.22 (d, *J* = 9.2 Hz, 1H), 7.66-7.60 (m, 3H), 7.44 (d, *J* = 2.4 Hz, 1H), 7.36-7.20 (m, 3H), 7.11 (d, *J* = 8.4 Hz, 1H), 7.02 (d, *J* = 2.0 Hz, 1H), 6.76-6.73 (m, 1H), 5.34 (s, 2H), 4.43 (s, 2H), 3.93 (s, 3H), 3.87 (s, 3H), 3.72 (s, 3H). |
| 12 (B) | 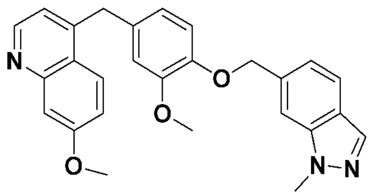 7-methoxy-4-(3-methoxy-4-((1-methyl-1H-indazol-6-yl)methoxy)benzyl)quinoline | 440.1 | $^1$H NMR(400 MHz, DMSO) δ8.72 (d, *J* = 4.5 Hz, 1H), 8.11 (d, *J* = 9.2 Hz, 2H), 7.76-7.69 (m, 3H), 7.40 (s, 1H), 7.28-7.23(m, 2H), 7.21-7.19(m, 1H), 7.17 (d, *J* = 4.8 Hz, 1H), 7.00-6.98 (m, 1H), 5.15 (s, 2H), 4.35 (s, 2H), 4.03 (s, 3H), 3.92 (s, 3H), 3.73 (s, 3H). |
| 13 (C) | 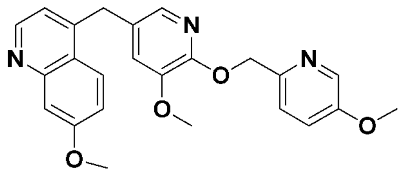 7-methoxy-4-((5-methoxy-6-((5-methoxypyridin-2-yl) methoxy)pyridin-3-yl)methyl)quinoline | 418.1 | $^1$H NMR(400 MHz, DMSO) δ8.73 (d, *J* = 4.4 Hz, 1H), 8.25 (t, *J* = 2.0 Hz, 1H), 8.17 (d, *J* = 9.2 Hz, 1H), 7.60 (d, *J* = 2.0 Hz, 1H), 7.41-7.38 (m, 3H), 7.31-7.26 (m, 2H), 7.29-7.15 (m, 1H), 5.30 (s, 2H), 4.38 (s, 2H), 3.92 (s, 3H), 3.82 (s, 3H), 3.74 (s, 3H). |
| 14 (B) | 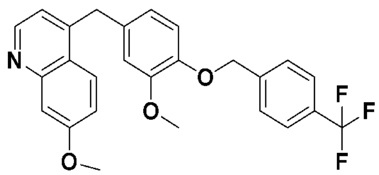 7-methoxy-4-(3-methoxy-4-((4-(trifluoromethyl)benzyl)oxy)be nzyl)quinoline | 454.1 | $^1$H NMR(400 MHz, DMSO) δ8.73 (d, *J* = 4.4 Hz, 1H), 8.11 (d, J = 9.2 Hz, 1H), 7.75 (d, *J* = 8.0 Hz, 2H), 7.64 (d, *J* = 8.0 Hz, 2H), 7.40 (d, *J* = 2.4 Hz, 1H), 7.24 (dd, J = 9.2, 2.8 Hz, 1H), 7.16 (d, *J* = 4.4 Hz, 1H), 7.00 (d, *J* = 2.0 Hz, 1H), 6.92 (d, *J* = 8.0 Hz, 1H), 6.69 (dd, *J* = 8.4, 2.0 Hz, 1H), 5.14 (s, 2H), 4.36 (s, 2H), 3.91 (s, 3H), 3.74 (s, 3H) |
| 15 (C) | 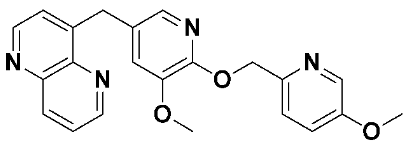 4-((5-methoxy-6-((5-methoxypyridin-2-yl)methoxy) pyridin-3-yl)methyl)-1,5-naphthyridine | 389.1 | $^1$H NMR(400 MHz, DMSO) δ9.09 (dd, *J* = 4.1, 1.7 Hz, 1H), 8.91 (d, *J* = 4.4 Hz, 1H), 8.44 (dd, *J* = 8.5, 1.7 Hz, 1H), 8.26 (t, *J* = 1.8 Hz, 1H), 7.83 (dd, J= 8.5, 4.1 Hz, 1H), 7.68 (d, *J* = 1.8 Hz, 1H), 7.58 (d, *J* = 4.4 Hz, 1H), 7.39 (d, *J* = 1.7 Hz, 3H), 5.31 (s, 2H), 4.57 (s, 2H), 3.82 (s, 3H), 3.75 (s, 3H). |

(continued)

| Example No (Method) | Structure; IUPAC name | LC-MS (M+H) + | $^1$H NMR (ppm) |
|---|---|---|---|
| 16 (D) | 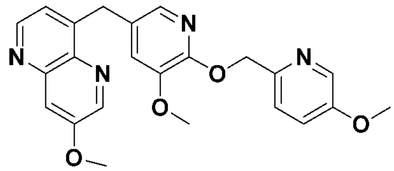 3-methoxy-8-((5-methoxy-6-((5-methoxypyridin-2-yl) methoxy)pyridin-3-yl)methyl)-1,5-naphthyridine | 419.1 | $^1$H NMR(400 MHz, DMSO) δ8.82 (dd, *J* = 6.4, 3.7 Hz, 2H), 8.28 - 8.22 (m, 1H), 7.79 (d, *J* = 2.8 Hz, 1H), 7.65 (d, *J* = 1.6 Hz, 1H), 7.38 (td, *J* = 12.0, 3.1 Hz, 4H), 5.31 (s, 2H), 4.52 (s, 2H), 4.00 (s, 3H), 3.82 (s, 3H), 3.74 (s, 3H). |
| 17 (B) | 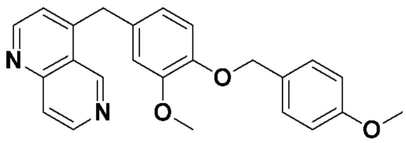 4-(3-methoxy-4-((4-methoxybenzyl)oxy)benzyl)-1,6-naphthyridine | 387.1 | $^1$H NMR(400 MHz, DMSO) δ 9.68(d, J =0.7 Hz, 1H), 9.03 (d, *J* =4.4Hz, 1H), 8.73(d, J =5.8Hz, 1H), 7.90-7.91(m,1H), 7.46(d, *J* =4.5Hz, 1H), 7.31-7.36 (m, 2H), 7.01(d, *J* =2.0Hz, 1H), 6.92-6.97 (m, 3H), 6.74-6.76 (m, 1H), 4.93 (s,2H), 4.54 (s, 2H), 3.75 (s,3H), 3.72 (s,3H) |
| 18 (B) | 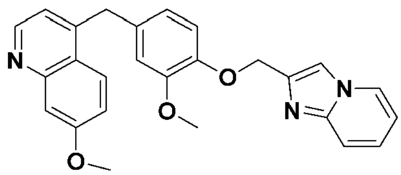 4-(4-(imidazo[1,2-a]pyridin-2-ylmethoxy)-3-methoxybenzyl)-7-methoxyquinoline | 426.1 | $^1$H NMR(400 MHz, DMSO) δ8.73 (d, *J* = 4.4 Hz, 1H), 8.54-8.51 (m, 1H), 8.12 (d, *J* = 9.2 Hz, 1H), 7.95 (s, 1H), 7.52 (d, *J* = 8.8 Hz, 1H), 7.40 (d, *J* = 2.8 Hz, 1H), 7.30 - 7.20 (m, 2H), 7.17 (d, *J* = 4.4 Hz, 1H), 7.04 (d, *J* = 8.4 Hz, 1H), 6.97 (d, *J* = 2.0 Hz, 1H), 6.93 - 6.84 (m, 1H), 6.69 (dd, *J* = 8.0, 2.0 Hz, 1H), 5.12 (s, 2H), 4.35 (s, 2H), 3.91 (s, 3H), 3.71 (s, 3H). |
| 19 (E) | 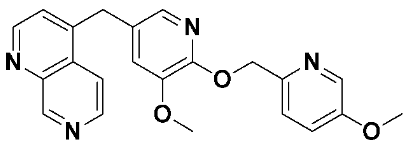 4-((5-methoxy-6-((5-methoxypyridin-2-yl)methoxy) pyridin-3-yl)methyl)-1,7-naphthyridine | 389.1 | $^1$H NMR (400 MHz, DMSO) δ 9.41 (s, 1H), 8.98 (d, *J* = 4.4 Hz, 1H), 8.65 (d, *J* = 5.8 Hz, 1H), 8.26 (s, 1H), 8.17 (d, *J* = 5.8 Hz, 1H), 7.66 (s, 1H), 7.57 (d, *J* = 4.4 Hz, 1H), 7.40 (d, *J* = 1.6 Hz, 2H), 7.32 (s, 1H), 5.31 (s, 2H), 4.44 (s, 2H), 3.82 (s, 3H), 3.75 (s, 3H). |
| 20 (B) | 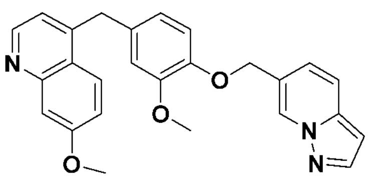 7-methoxy-4-(3-methoxy-4-(pyrazolo[1,5-a]pyridin-6-ylmethoxy)benzyl)quinoline | 426.1 | $^1$H NMR(400 MHz, DMSO) δ8.78 (s, 1H), 8.73 (s, 1H), 8.11 (d, *J* = 7.9 Hz, 1H), 8.00 (s, 1H), 7.71 (d, *J* = 7.6 Hz, 1H), 7.39 (s, 1H), 7.25 (s, 2H), 7.16 (s, 1H), 6.99 (d, *J* = 6.5 Hz, 2H), 6.68 (d, *J* = 24.8 Hz, 1H), 6.62 (s, 1H), 5.05 (s, 2H), 4.36 (s, 2H), 3.91 (s, 3H), 3.72 (s, 3H). |

(continued)

| Example No (Method) | Structure; IUPAC name | LC-MS (M+H) + | $^1$H NMR (ppm) |
|---|---|---|---|
| 21 (B) | 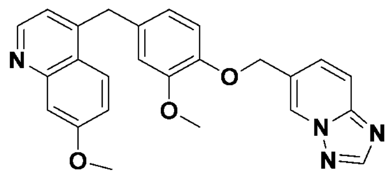 4-(4-([1,2,4]triazolo[1,5-a]pyridin-6-ylmethoxy)-3-methoxybenzyl)-7-methoxyquinoline | 427.1 | $^1$H NMR(400 MHz, DMSO) δ9.05 (s, 1H), 8.73 (d, *J* = 4.4 Hz, 1H), 8.52 (s, 1H), 8.11 (d, *J*= 9.2 Hz, 1H), 7.88 (d, *J* = 9.1 Hz, 1H), 7.73 (dd, *J* = 9.2, 1.2 Hz, 1H), 7.39 (d, *J* = 2.5 Hz, 1H), 7.24 (dd, *J* = 9.2, 2.6 Hz, 1H), 7.16 (d, *J* = 4.4 Hz, 1H), 7.01 (dd,*J* = 9.0, 4.8 Hz, 2H), 6.71 (d, *J* = 8.1 Hz, 1H), 5.16 (d, *J* = 18.9 Hz, 2H), 4.36 (s, 2H), 3.91 (s, 3H), 3.73 (s, 3H) |
| 22 (E) | 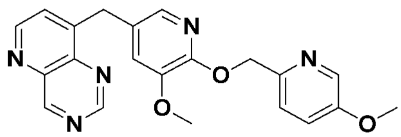 8-((5-methoxy-6-((5-methoxypyridin-2-yl)methoxy) pyridin-3-yl)methyl)pyrido[3,2-d]pyrimidine | 390.1 | $^1$H NMR (400 MHz, DMSO) δ 9.68 (s, 1H), 9.51 (s, 1H), 9.05 (d, *J* = 4.4 Hz, 1H), 8.26 (t, *J* = 1.8 Hz, 1H), 7.81 (d, *J* = 4.4 Hz, 1H), 7.68 (d, *J* = 1.8 Hz, 1H), 7.38 (t, *J* = 2.5 Hz, 3H), 5.31 (s, 2H), 4.53 (s, 2H), 3.82 (s, 3H), 3.75 (s, 3H). |
| 23 (B) | 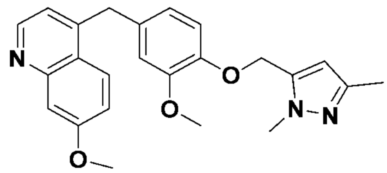 4-(4-((1,3-dimethyl-1H-pyrazol-5-yl)methoxy)-3-methoxybenzyl)-7-methoxyquinoline | 404.1 | $^1$H NMR(400 MHz, DMSO) δ8.73 (d, *J* = 4.4 Hz, 1H), 8.12 (d, *J* = 9.2 Hz, 1H), 7.40 (d, *J* = 2.8 Hz, 1H), 7.25 (d, *J* = 4.4 Hz, 1H), 7.18 (d, *J* = 4.4 Hz, 1H), 7.00-6.98 (m, 2H), 6.70 (dd, *J* = 8.0, 2.0 Hz, 1H), 6.09 (s, 1H), 5.00 (s, 2H), 4.36 (s, 2H), 3.91 (s, 3H), 3.72 (s, 3H), 3.71 (s, 3H), 2.10 (s, 3H). |
| 24 (A) | 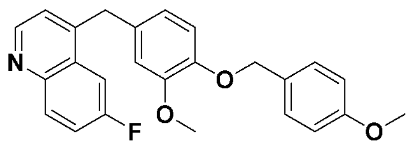 6-fluoro-4-(3-methoxy-4-((4-methoxybenzyl)oxy) benzyl)qu inoline | 404.1 | $^1$H NMR(400 MHz, DMSO) δ8.80 (d, *J* = 4.4 Hz, 1H), 8.09 (dd, *J* = 9.2, 5.8 Hz, 1H), 7.96 (dd, *J* = 10.6, 2.8 Hz, 1H), 7.66 (td, *J* = 8.8, 2.8 Hz, 1H), 7.40 - 7.29 (m, 3H), 7.01 - 6.89 (m, 4H), 6.72 (dd, *J* = 8.2, 1.9 Hz, 1H), 4.93 (s, 2H), 4.36 (s, 2H), 3.75 (s, 3H), 3.71 (s, 3H) |
| 25 (B) | 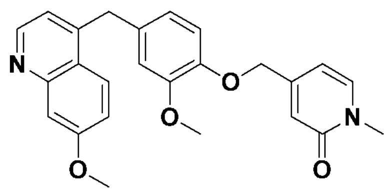 4-((2-methoxy-4-((7-methoxyquinolin-4-yl)methyl) phenoxy)methyl)-1-methylpyridin-2(1H)-one | 417.1 | $^1$H NMR (400 MHz, DMSO) δ 8.72 (d, *J* = 4.4 Hz, 1H), 8.11 (d, *J* = 9.2 Hz, 1H), 7.66 (d, *J* = 7.0 Hz, 1H), 7.39 (d, *J* = 2.5 Hz, 1H), 7.24 (dd, *J* = 9.2, 2.6 Hz, 1H), 7.17 (d, *J* = 4.5 Hz, 1H), 7.00 (d, *J* = 1.6 Hz, 1H), 6.86 (d, *J* = 8.3 Hz, 1H), 6.68 (d, *J* = 8.2 Hz, 1H), 6.39 (s, 1H), 6.19 (dd, *J* = 6.9, 1.7 Hz, 1H), 4.91 (s, 2H), 4.35 (s, 2H), 3.91 (s, 3H), 3.75 (s, 3H), 3.39 (s, 3H). |

(continued)

| Example No (Method) | Structure; IUPAC name | LC-MS (M+H) + | $^1$H NMR (ppm) |
|---|---|---|---|
| 26 (B) | 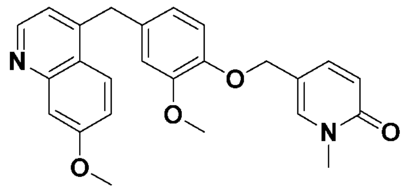 5-((2-methoxy-4-((7-methoxyquinolin-4-yl)methyl) phenoxy)methyl)-1-methylpyridin-2(1H)-one | 417.1 | $^1$H NMR (400 MHz, DMSO) δ 8.73 (d, *J* = 4.4 Hz, 1H), 8.11 (d, *J* = 9.2 Hz, 1H), 7.82 (d, *J* = 2.2 Hz, 1H), 7.48 (dd, *J* = 9.3, 2.5 Hz, 1H), 7.40 (d, *J* = 2.6 Hz, 1H), 7.25 (dd, *J* = 9.2, 2.6 Hz, 1H), 7.16 (d, *J* = 4.5 Hz, 1H), 6.96 (dd, *J* = 7.2, 5.0 Hz, 2H), 6.70 (d, *J* = 8.2 Hz, 1H), 6.40 (d, *J* = 9.3 Hz, 1H), 4.71 (s, 2H), 4.36 (s, 2H), 3.91 (s, 3H), 3.70 (s, 3H), 3.41 (s, 3H). |
| 27 (B) | 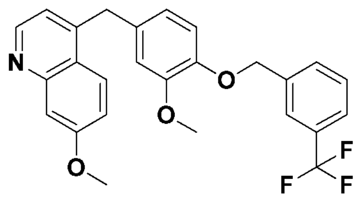 7-methoxy-4-(3-methoxy-4-((3-(trifluoromethyl)benzyl)oxy)be nzyl)quinoline | 454.1 | $^1$H NMR (400 MHz, DMSO) δ 8.73 (d, *J* = 4.5 Hz, 1H), 8.12 (d, *J* = 9.2 Hz, 1H), 7.79 (s, 1H), 7.72 (dd, *J* = 13.1, 7.6 Hz, 2H), 7.63 (t, *J* = 7.6 Hz, 1H), 7.40 (d, *J* = 2.6 Hz, 1H), 7.24 (dd, *J* = 9.2, 2.6 Hz, 1H), 7.16 (d, *J* = 4.5 Hz, 1H), 6.98 (dd, *J* = 17.7, 4.9 Hz, 2H), 6.70 (d, *J* = 8.3 Hz, 1H), 5.13 (s, 2H), 4.36 (s, 2H), 3.91 (s, 3H), 3.73 (s, 3H). |
| 28 (B) | 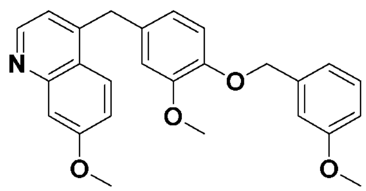 7-methoxy-4-(3-methoxy-4-((3-methoxybenzyl)oxy) benzyl)qu inoline | 416.1 | $^1$H NMR(400 MHz, DMSO) δ8.75 (d, *J* = 4.5 Hz, 1H), 7.94 (d, *J* = 9.2 Hz, 1H), 7.47 (d, *J* = 2.5 Hz, 1H), 7.34 - 7.25 (m, 1H), 7.25 - 7.14 (m, 1H), 7.06 - 6.96 (m, 3H), 6.88 - 6.78 (m, 2H), 6.74 (t, *J* = 9.3 Hz, 1H), 6.70 - 6.61 (m, 1H), 5.25 - 5.03 (m, 2H), 4.32 (d, J = 25.9 Hz, 2H), 3.99 (d, *J* = 7.4 Hz, 3H), 3.83 (s, 3H), 3.82 (s, 3H). |
| 29 (B) | 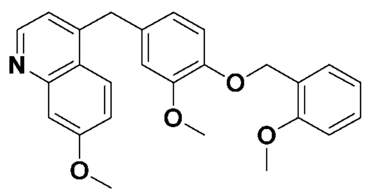 7-methoxy-4-(3-methoxy-4-((2-methoxybenzyl)oxy) benzyl)qu inoline | 416.1 | $^1$H NMR(400 MHz, DMSO) δ8.97 (s, 1H), 8.42 (d, *J* = 9.2 Hz, 1H), 7.55 (d, *J* = 2.5 Hz, 1H), 7.51 - 7.48 (m, 2H), 7.37 - 7.30 (m, 2H), 7.04 - 7.01 (m, 2H), 6.97 - 6.92 (m, 2H), 6.75 - 6.72 (m, 1H), 4.98 (s, 2H), 4.54 (s, 2H), 3.98 (s, 3H), 3.79 (s, 3H), 3.72 (s, 3H). |

(continued)

| Example No (Method) | Structure; IUPAC name | LC-MS (M+H) + | $^1$H NMR (ppm) |
|---|---|---|---|
| 30 (B) | 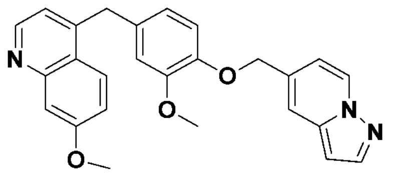 7-methoxy-4-(3-methoxy-4-(pyrazolo[1,5-a]pyridin-5-ylmethoxy)benzyl)quinoline | 426.1 | $^1$H NMR(400 MHz, DMSO) δ8.72 (d, *J* = 4.5 Hz, 1H), 8.67 (d, *J* = 7.2 Hz, 1H), 8.12 (d, *J* = 9.2 Hz, 1H), 7.99 (d, *J* = 2.2 Hz, 1H), 7.70 (s, 1H), 7.39 (d, *J* = 2.6 Hz, 1H), 7.24 (dd, J = 9.2, 2.7 Hz, 1H), 7.17 (d, *J* = 4.5 Hz, 1H), 7.00 (d, *J* = 1.9 Hz, 1H), 6.96 (d, *J* = 8.2 Hz, 1H), 6.89 (dd, *J* = 7.2, 1.8 Hz, 1H), 6.70 (dd, *J* = 8.2, 1.9 Hz, 1H), 6.61 (d, *J* = 1.6 Hz, 1H), 5.08 (s, 2H), 4.36 (s, 2H), 3.91 (s, 3H), 3.75 (s, 3H). |
| 31 (C) | 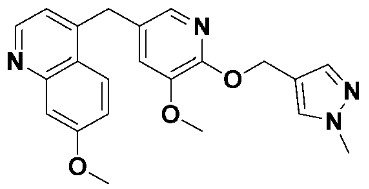 7-methoxy-4-((5-methoxy-6-((1-methyl-1H-pyrazol-4-yl)methoxy)pyridin-3-yl)methyl)quinoline | 391.1 | $^1$H NMR(400 MHz, DMSO) δ8.73 (d, *J* = 4.5 Hz, 1H), 8.16 (d, *J* = 9.2 Hz, 1H), 7.74 (s, 1H), 7.63 (d, *J* = 1.9 Hz, 1H), 7.46 (s, 1H), 7.41 (d, *J* = 2.7 Hz, 1H), 7.27 (dd, *J* = 9.3, 2.7 Hz, 1H), 7.22 (d, *J* = 2.0 Hz, 1H), 7.17 (d, *J* = 4.5 Hz, 1H), 5.15 (s, 2H), 4.37 (s, 2H), 3.92 (s, 3H), 3.80 (s, 3H), 3.69 (s, 3H). |
| 32 (D) | 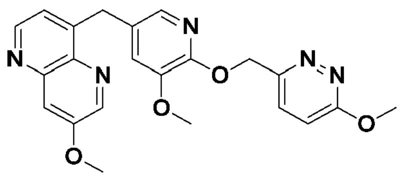 3-methoxy-8-((5-methoxy-6-((6-methoxypyridazin-3-yl)methoxy)pyridin-3-yl)methyl)-1,5-naphthyridine | 420.1 | $^1$H NMR(400 MHz, DMSO) δ8.83 (d, J =2.88Hz,1H), 8.81 (d, J =4.48Hz,1H), 7.79 (d, *J* =2.88Hz, 1H), 7.66 (d, *J* =2.48Hz,1H), 7.64 (s,1H), 7.41 (d, *J* =4.44Hz,1H), 7.38 (d, *J* =1.8Hz,1H), 7.23 (d, *J* =9.08Hz, 1H), 5.47 (s,2H), 4.52 (s,2H), 4.02 (s,3H), 4.00(s,3H), 3.73 (s,3H) |
| 33 (B) | 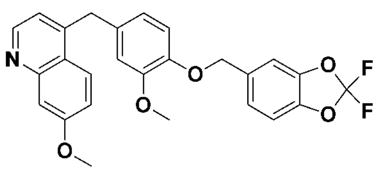 4-(4-((2,2-difluorobenzo[d][1,3]dioxol-5-yl)methoxy)-3-methoxybenzyl)-7-methoxyquinoline | 466.1 | $^1$H NMR(400 MHz, DMSO) δ8.73 (d, *J* = 4.4 Hz, 1H), 8.11 (d, *J* = 9.2 Hz, 1H), 7.47 (d, *J* = 1.4 Hz, 1H), 7.40 (dd, *J* = 6.6, 5.6 Hz, 2H), 7.26 (ddd, *J* = 11.9, 8.8, 2.1 Hz, 2H), 7.16 (d, *J* = 4.5 Hz, 1H), 6.98 (d, *J* = 1.9 Hz, 1H), 6.94 (d, *J* = 8.3 Hz, 1H), 6.69 (dd, *J* = 8.2, 1.9 Hz, 1H), 5.02 (s, 2H), 4.36 (s, 2H), 3.89 (d, *J* = 14.1 Hz, 3H), 3.72 (s, 3H) |

(continued)

| Example No (Method) | Structure; IUPAC name | LC-MS (M+H) + | $^1$H NMR (ppm) |
|---|---|---|---|
| 34 (B) | 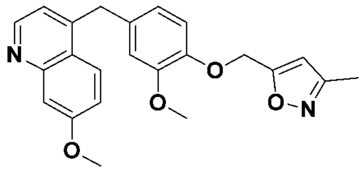 5-((2-methoxy-4-((7-methoxyquinolin-4-yl)methyl) phenoxy)methyl)-3-methylisoxazole | 391.1 | $^1$H NMR(400 MHz, DMSO) δ8.73 (d, *J* = 4.4 Hz, 1H), 8.11 (d, *J* = 9.2 Hz, 1H), 7.39 (d, *J* = 2.5 Hz, 1H), 7.23 (dt, *J* = 15.8, 7.9 Hz, 1H), 7.17 (d, *J* = 4.4 Hz, 1H), 7.00 (d, *J* = 1.2 Hz, 1H), 6.96 (d, *J* = 8.2 Hz, 1H), 6.70 (d, *J* = 8.1 Hz, 1H), 6.43 (s, 1H), 5.13 (s, 2H), 4.36 (s, 2H), 3.91 (s, 3H), 3.72 (s, 3H), 2.22 (s, 3H) |
| 35 (B) | 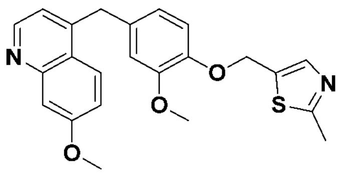 5-((2-methoxy-4-((7-methoxyquinolin-4-yl)methyl) phenoxy)methyl)-2-methylthiazole | 407.1 | $^1$H NMR(400 MHz, DMSO) δ8.76 (d, *J* = 4.3 Hz, 1H), 7.93 (d, *J* = 9.2 Hz, 1H), 7.57 (d, *J* = 7.3 Hz, 1H), 7.47 (d, *J* = 2.6 Hz, 1H), 7.20 (dd, *J* = 9.2, 2.6 Hz, 1H), 7.02 (d, *J* = 4.5 Hz, 1H), 6.92 - 6.83 (m, 1H), 6.73 (t, *J* = 6.9 Hz, 1H), 6.69 (dd, *J* = 8.1, 2.0 Hz, 1H), 5.23 (s, 2H), 4.36 (s, 2H), 3.97 (s, 3H), 3.80 (s, 3H), 2.70 (s, 3H) |
| 36 (B) | 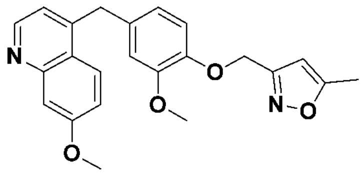 3-((2-methoxy-4-((7-methoxyquinolin-4-yl)methyl) phenoxy)methyl)-5-methylisoxazole | 391.1 | $^1$H NMR(400 MHz, DMSO) δ8.76 (d, *J* = 4.5 Hz, 1H), 7.93 (d, *J* = 9.2 Hz, 1H), 7.47 (d, *J* = 2.6 Hz, 1H), 7.20 (dd, *J* = 9.2, 2.6 Hz, 1H), 7.02 (d, *J* = 4.5 Hz, 1H), 6.92 (d, *J* = 8.2 Hz, 1H), 6.77 - 6.71 (m, 1H), 6.71 - 6.65 (m, 1H), 6.17 (d, *J* = 13.6 Hz, 1H), 5.18 (s, 2H), 4.36 (s, 2H), 3.98 (s, 3H), 3.81 (s, 3H), 2.43 (s, 3H) |
| 37 (D) | 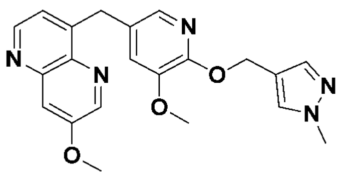 3-methoxy-8-((5-methoxy-6-((1-methyl-1H-pyrazol-4-yl)methoxy)pyridin-3-yl)methyl)-1,5-naphthyridine | 392.8 | $^1$H NMR (400 MHz, DMSO) δ 8.82 (dd, *J* = 7.6, 3.7 Hz, 2H), 7.79 (d, *J* = 2.9 Hz, 1H), 7.74 (s, 1H), 7.68 (d, *J* = 1.7 Hz, 1H), 7.45 (s, 1H), 7.39 (d, *J* = 4.4 Hz, 1H), 7.29 (d, *J* = 1.7 Hz, 1H), 5.14 (s, 2H), 4.51 (s, 2H), 3.99 (s, 3H), 3.79 (s, 3H), 3.68 (s, 3H). |
| 38 (D) | 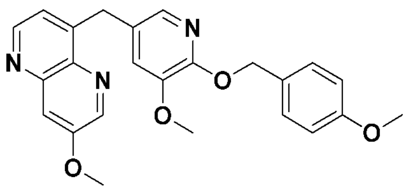 3-methoxy-8-((5-methoxy-6-((4-methoxybenzyl)oxy) pyridin-3-yl)methyl)-1,5-naphthyridine | 418.1 | $^1$H NMR (400 MHz, DMSO) δ 8.82 (dd, *J* = 7.5, 3.7 Hz, 2H), 7.79 (d, *J* = 2.9 Hz, 1H), 7.66 (d, *J* = 1.7 Hz, 1H), 7.40 (d, *J* = 4.5 Hz, 1H), 7.33 (dd, *J* = 12.6, 5.2 Hz, 3H), 6.92 (d, *J* = 8.7 Hz, 2H), 5.22 (s, 2H), 4.52 (s, 2H), 3.99 (s, 3H), 3.74 (s, 3H), 3.70 (s, 3H). |

(continued)

| Example No (Method) | Structure; IUPAC name | LC-MS (M+H) + | $^1$H NMR (ppm) |
|---|---|---|---|
| 39 (D) | 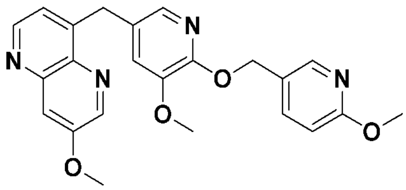 3-methoxy-8-((5-methoxy-6-((6-methoxypyridin-3-yl) methoxy)pyridin-3-yl)methyl)-1,5-naphthyridine | 419.1 | $^1$H NMR (400 MHz, DMSO) δ 8.82 (dd, *J* = 7.4, 3.7 Hz, 2H), 8.24 (d, *J* = 2.0 Hz, 1H), 7.81 - 7.74 (m, 2H), 7.68 (d, *J* = 1.5 Hz, 1H), 7.40 (d, *J* = 4.5 Hz, 1H), 7.33 (d, *J* = 1.6 Hz, 1H), 6.82 (d, *J* = 8.5 Hz, 1H), 5.25 (s, 2H), 4.52 (s, 2H), 3.99 (s, 3H), 3.84 (s, 3H), 3.70 (s, 3H). |
| 40 (D) | 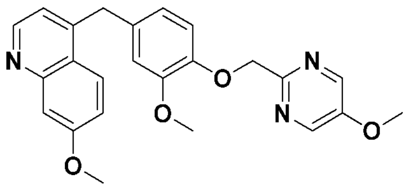 7-methoxy-4-(3-methoxy-4-((5-methoxypyrimidin-2-yl)methoxy)benzyl)quinoline | 418.1 | $^1$H NMR(400 MHz, DMSO) δ8.72 (d, *J* = 4.4 Hz, 1H), 8.56 (s, 1H), 8.11 (d, *J* = 9.1 Hz, 1H), 7.39 (d, *J* = 2.7 Hz, 1H), 7.25 (d, *J* = 2.6 Hz, 1H), 7.23 (d, *J* = 2.7 Hz, 1H), 7.17 (d, *J* = 4.5 Hz, 1H), 6.96 (d, *J* = 2.0 Hz, 1H), 6.86 (d, *J* = 8.2 Hz, 1H), 6.63 (s, 1H), 5.11 (s, 2H), 4.34 (s, 2H), 3.91 (s, 6H), 3.71 (s, 3H) |
| 41 (D) | 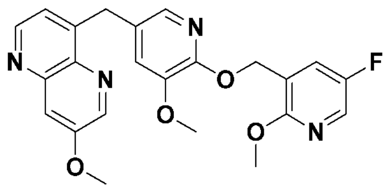 8-((6-((5-fluoro-2-methoxypyridin-3-yl)methoxy)-5-methoxypyridin-3-yl)methyl)-3-methoxy-1,5-naphthyridine | 437.1 | $^1$H NMR(400 MHz, DMSO) δ8.82 (dd, *J* = 6.5, 3.7 Hz, 2H), 8.13 (d, *J* = 3.0 Hz, 1H), 7.79 (d, *J* = 2.9 Hz, 1H), 7.68 - 7.59 (m, 2H), 7.39 (dd, *J* = 10.5, 3.1 Hz, 2H), 5.25 (s, 2H), 4.53 (s, 2H), 4.06 - 3.93 (m, 3H), 3.89 (s, 3H), 3.73 (d, *J* = 19.0 Hz, 3H) |
| 42 (D) | 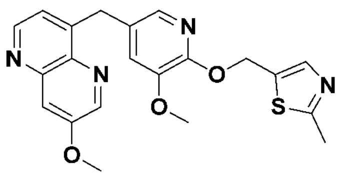 5-(((3-methoxy-5-((7-methoxy-1,5-naphthyridin-4-yl) methyl)pyridin-2-yl)oxy)methyl)-2-methylthiazole | 409.0 | $^1$H NMR (400 MHz, DMSO) δ 8.82 (dd, *J* = 6.0, 3.7 Hz, 2H), 7.79 (d, *J* = 2.8 Hz, 1H), 7.69 (d, *J* = 5.8 Hz, 2H), 7.41 (d, *J* = 4.4 Hz, 1H), 7.34 (d, *J* = 1.4 Hz, 1H), 5.48 (s, 2H), 4.53 (s, 2H), 4.00 (s, 3H), 3.70 (s, 3H), 2.59 (s, 3H). |
| 43 (D) | 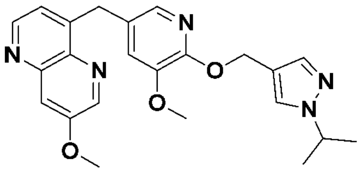 8-((6-((1-isopropyl-1H-pyrazol-4-yl)methoxy)-5-methoxypyridin-3-yl)methyl)-3-methoxy-1,5-naphthyridine | 298.1 (Fragmentation Peak+ H)$^+$ | $^1$H NMR(400 MHz, DMSO) δ8.83 (dd, *J* = 7.7, 3.7 Hz, 2H), 7.85 - 7.77 (m, 2H), 7.69 (d, *J* = 1.8 Hz, 1H), 7.47 (s, 1H), 7.40 (d, *J* = 4.5 Hz, 1H), 7.30 (d, *J* = 1.8 Hz, 1H), 5.14 (s, 2H), 4.51 (d, *J* = 10.0 Hz, 2H), 4.47 - 4.41 (m, 1H), 4.00 (s, 3H), 3.69 (s, 3H), 1.39 (d, *J* = 6.7 Hz, 6H) |

(continued)

| Example No (Method) | Structure; IUPAC name | LC-MS (M+H) + | $^1$H NMR (ppm) |
|---|---|---|---|
| 44 (D) | 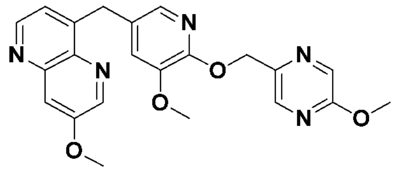 3-methoxy-8-((5-methoxy-6-((5-methoxypyrazin-2-yl)methoxy)pyridin-3-yl)methyl)-1,5-naphthyridine | 420.1 | $^1$H NMR(400 MHz, DMSO) δ8.82 (dd, *J* = 6.9, 3.7 Hz, 2H), 8.33 - 8.28 (m, 2H), 7.79 (d, *J* = 2.9 Hz, 1H), 7.67 (d, *J* = 1.8 Hz, 1H), 7.41 (d, *J* = 4.5 Hz, 1H), 7.36 (d, *J* = 1.8 Hz, 1H), 5.34 (s, 2H), 4.53 (s, 2H), 4.00 (s, 3H), 3.91 (s, 3H), 3.72 (s, 3H). |
| 45 (D) | 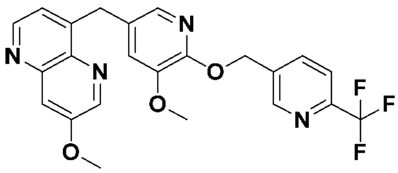 3-methoxy-8-((5-methoxy-6-((6-(trifluoromethyl)pyridin-3-yl)methoxy)pyridin-3-yl)methyl)-1,5-naphthyridine | 457.1 | $^1$H NMR(400 MHz, DMSO) δ8.82 (dd, *J* = 6.6, 3.5 Hz, 3H), 8.11 (d, *J* = 7.7 Hz, 1H), 7.92 (d, *J* = 8.1 Hz, 1H), 7.79 (d, *J* = 2.8 Hz, 1H), 7.67 (d, *J* = 1.2 Hz, 1H), 7.45 - 7.34 (m, 2H), 5.47 (s, 2H), 4.52 (s, 2H), 3.99 (s, 3H), 3.75 (s, 3H) |
| 46 (D) | 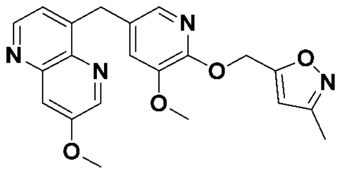 5-(((3-methoxy-5-((7-methoxy-1,5-naphthyridin-4-yl)methyl)pyridin-2-yl)oxy)methyl)-3-methylisoxazole | 393.1 | $^1$H NMR(400 MHz, DMSO) δ8.82 (dd, *J* = 6.5, 3.7 Hz, 2H), 7.79 (d, *J* = 2.9 Hz, 1H), 7.67 (d, *J* = 1.8 Hz, 1H), 7.39 (dd, *J* = 8.8, 3.1 Hz, 2H), 6.39 (s, 1H), 5.39 (s, 2H), 4.53 (s, 2H), 3.99 (s, 3H), 3.73 (s, 3H), 2.21 (s, 3H) |
| 47 (D) | 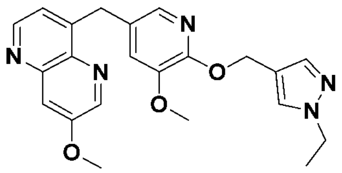 8-((6-((1 -ethyl-1H-pyrazol-4-yl)methoxy)-5-methoxypyridin-3-yl)methyl)-3-methoxy-1,5-naphthyridine | 298.1 (Fragmentation Peak+ H)+ | $^1$H NMR(400 MHz, DMSO) δ8.82 (dd, *J* = 7.2, 3.7 Hz, 2H), 7.79 (d, *J* = 2.9 Hz, 2H), 7.69 (d, *J* = 1.8 Hz, 1H), 7.47 (s, 1H), 7.40 (d, *J* = 4.4 Hz, 1H), 7.30 (d, *J* = 1.8 Hz, 1H), 5.15 (s, 2H), 4.52 (s, 2H), 4.09 (q, *J* = 7.3 Hz, 2H), 4.00 (s, 3H), 3.68 (s, 3H), 1.34 (t, *J* = 7.3 Hz, 3H) |
| 48 (D) | 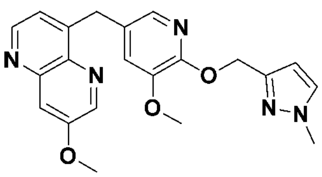 3-methoxy-8-((5-methoxy-6-((1-methyl-1H-pyrazol-3-yl)methoxy)pyridin-3-yl)methyl)-1,5-naphthyridine | 392.1 | $^1$H NMR (400 MHz, DMSO) δ 8.82 (dd, *J* = 7.0, 3.6 Hz, 2H), 7.79 (d, *J* = 2.7 Hz, 1H), 7.68 (s, 1H), 7.62 (s, 1H), 7.40 (d, *J* = 4.4 Hz, 1H), 7.31 (s, 1H), 6.24 (d, *J* = 1.9 Hz, 1H), 5.18 (s, 2H), 4.52 (s, 2H), 4.00 (s, 3H), 3.81 (s, 3H), 3.69 (s, 3H). |
| 49 (D) | 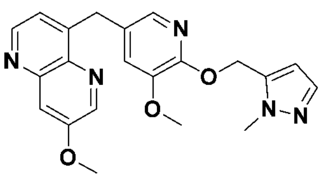 3-methoxy-8-((5-methoxy-6-((1-methyl-1H-pyrazol-5-yl)methoxy)pyridin-3-yl)methyl)-1,5-naphthyridine | 392.1 | $^1$H NMR (400 MHz, DMSO) δ 8.82 (dd, *J* = 6.3, 3.7 Hz, 2H), 7.79 (d, *J* = 2.7 Hz, 1H), 7.70 (s, 1H), 7.41 (d, *J* = 4.4 Hz, 1H), 7.35 (s, 2H), 6.33 (s, 1H), 5.34 (s, 2H), 4.53 (s, 2H), 4.00 (s, 3H), 3.81 (s, 3H), 3.71 (s, 3H). |

(continued)

| Example No (Method) | Structure; IUPAC name | LC-MS (M+H) + | $^1$H NMR (ppm) |
|---|---|---|---|
| 50 (D) | 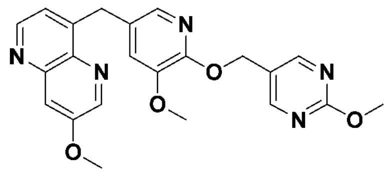 3-methoxy-8-((5-methoxy-6-((2-methoxypyrimidin-5-yl)methoxy)pyridin-3-yl)methyl)-1,5-naphthyridine | 420.1 | $^1$H NMR(400 MHz, DMSO) $\delta$8.82 (dd, $J$ = 7.2, 3.7 Hz, 2H), 8.70 (s, 2H), 7.79 (d, $J$ = 2.9 Hz, 1H), 7.69 (d, $J$ = 1.8 Hz, 1H), 7.40 (d, $J$ = 4.5 Hz, 1H), 7.35 (d, $J$ = 1.8 Hz, 1H), 5.28 (s, 2H), 4.52 (s, 2H), 4.00 (s, 3H), 3.91 (s, 3H), 3.71 (s, 3H) |
| 51 (D) | 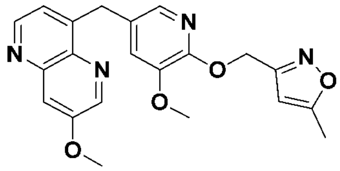 3-(((3-methoxy-5-((7-methoxy-1,5-naphthyridin-4-yl) methyl)pyridin-2-yl)oxy)methyl)-5-methylisoxazole | 393.1 | $^1$H NMR(400 MHz, DMSO) $\delta$8.82 (dd, $J$ = 6.3, 3.7 Hz, 2H), 7.79 (d, $J$ = 2.9 Hz, 1H), 7.67 (d, $J$ = 1.7 Hz, 1H), 7.41 (d, $J$ = 4.5 Hz, 1H), 7.37 (d, $J$ = 1.8 Hz, 1H), 6.24 (d, $J$ = 0.6 Hz, 1H), 5.31 (s, 2H), 4.53 (s, 2H), 4.00 (s, 3H), 3.73 (s, 3H), 2.38 (s, 3H) |
| 52 (D) | 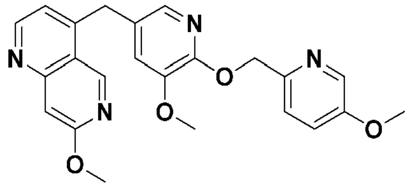 7-methoxy-4-((5-methoxy-6-((5-methoxypyridin-2-yl) methoxy)pyridin-3-yl)methyl)-1,6-naphthyridine | 419.1 | $^1$H NMR (400 MHz, DMSO) $\delta$ 9.46 (s, 1H), 8.90 (d, $J$ = 4.4 Hz, 1H), 8.26 (t, $J$ = 1.7 Hz, 1H), 7.67 (d, $J$ = 1.6 Hz, 1H), 7.40 (d, $J$ = 1.7 Hz, 2H), 7.32 (d, $J$ = 1.7 Hz, 1H), 7.23 (d, $J$ = 3.9 Hz, 2H), 5.31 (s, 2H), 4.50 (s, 2H), 3.99 (s, 3H), 3.82 (s, 3H), 3.75 (s, 3H). |
| 53 (D) | 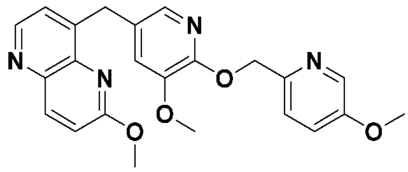 2-methoxy-8-((5-methoxy-6-((5-methoxypyridin-2-yl) methoxy)pyridin-3-yl)methyl)-1,5-naphthyridine | 419.1 | $^1$H NMR (400 MHz, DMSO) $\delta$ 8.71 (d, $J$ = 4.5 Hz, 1H), 8.26 (t, $J$ = 5.8 Hz, 2H), 7.74 (d, $J$ = 1.8 Hz, 1H), 7.58 (d, $J$ = 4.5 Hz, 1H), 7.37 (dd, $J$ = 9.5, 1.7 Hz, 3H), 7.28 (d, $J$ = 9.0 Hz, 1H), 5.30 (s, 2H), 4.44 (s, 2H), 4.08 (s, 3H), 3.82 (s, 3H), 3.74 (s, 3H). |
| 54 (F) | 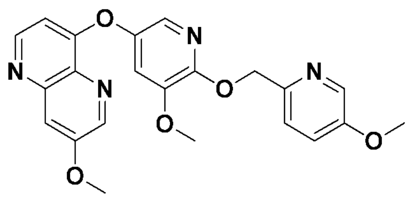 3-methoxy-8-((5-methoxy-6-((5-methoxypyridin-2-yl) methoxy)pyridin-3-yl)oxy)-1,5-naphthyridine | 421.0 | $^1$H NMR (400 MHz, DMSO) $\delta$ 8.75 (d, $J$ = 2.8 Hz, 1H), 8.70 (d, $J$ = 5.2 Hz, 1H), 8.30 (d, $J$ = 2.2 Hz, 1H), 7.80 (d, $J$ = 2.8 Hz, 1H), 7.72 (d, $J$ = 2.4 Hz, 1H), 7.51 - 7.40 (m, 3H), 6.79 (d, $J$ = 5.2 Hz, 1H), 5.39 (s, 2H), 4.01 (s, 3H), 3.85 (s, 3H), 3.81 (s, 3H). |

(continued)

| Example No (Method) | Structure; IUPAC name | LC-MS (M+H) + | $^1H$ NMR (ppm) |
|---|---|---|---|
| 55 (D) | 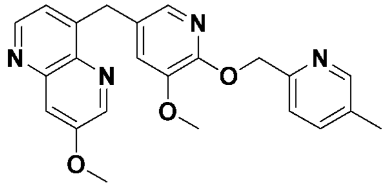 3-methoxy-8-((5-methoxy-6-((5-methylpyridin-2-yl) methoxy)pyridin-3-yl)methyl)-1,5-naphthyridine | 403.1 | $^1H$ NMR(400 MHz, DMSO) δ 8.82 (dd, *J* = 6.6, 3.7 Hz, 2H), 8.38 (s, 1H), 7.79 (d, *J* = 2.9 Hz, 1H), 7.63 (d, *J* = 1.8 Hz, 1H), 7.60 (dd, *J* = 7.9, 1.7 Hz, 1H), 7.41 (d, *J* = 4.5 Hz, 1H), 7.36 (d, *J* = 1.8 Hz, 1H), 7.29 (d, *J* = 7.9 Hz, 1H), 5.33 (s, 2H), 4.52 (s, 2H), 4.00 (s, 3H), 3.75 (s, 3H), 2.28 (s, 3H) |
| 56 (D) | 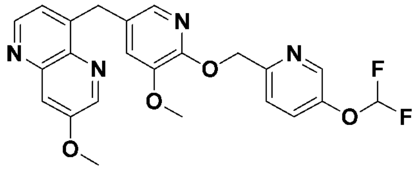 8-((6-((5-(difluoromethoxy)pyridin-2-yl)methoxy)-5-methoxypyridin-3-yl)methyl)-3-methoxy-1,5-naphthyridine | 455.1 | $^1H$ NMR(400 MHz, DMSO) δ8.82 (dd, *J* = 6.3, 3.7 Hz, 2H), 8.46 (d, *J* = 2.8 Hz, 1H), 7.79 (d, *J* = 2.9 Hz, 1H), 7.71 - 7.61 (m, 2H), 7.51 - 7.44 (m, 1H), 7.39 (dd, *J* = 11.3, 3.1 Hz, 2H), 7.30 (s, 1H), 5.38 (s, 2H), 4.52 (s, 2H), 4.00 (s, 3H), 3.76 (s, 3H). |
| 57 (D) | 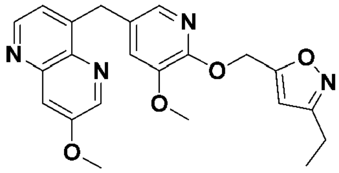 3-ethyl-5-(((3-methoxy-5-((7-methoxy-1,5-naphthyridin-4-yl)methyl)pyridin-2-yl)oxy)methyl)isoxazole | 407.1 | $^1H$ NMR(400 MHz, DMSO) δ8.82 (dd, *J* = 6.0, 3.7 Hz, 2H), 7.79 (d, *J* = 2.9 Hz, 1H), 7.68 (d, *J* = 1.4 Hz, 1H), 7.40 (dd, *J* = 10.4, 3.0 Hz, 2H), 6.46 (s, 1H), 5.39 (s, 2H), 4.53 (s, 2H), 4.00 (s, 3H), 3.73 (s, 3H), 2.61 (q, *J* = 7.6 Hz, 2H), 1.17 (t, *J* = 7.6 Hz, 3H) |
| 58 (D) | 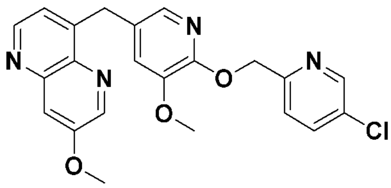 8-((6-((5-chloropyridin-2-yl)methoxy)-5-methoxypyridin-3-yl)methyl)-3-methoxy-1,5-naphthyridine | 420.3 | $^1H$ NMR(400 MHz, DMSO) δ8.82 (dd, *J* = 6.2, 3.7 Hz, 2H), 8.60 (d, *J* = 2.2 Hz, 1H), 7.93 (dd, *J* = 8.4, 2.4 Hz, 1H), 7.79 (d, *J* = 2.8 Hz, 1H), 7.63 (s, 1H), 7.46 - 7.35 (m, 3H), 5.39 (s, 2H), 4.52 (s, 2H), 4.00 (s, 3H), 3.76 (s, 3H). |
| 59 (D) | 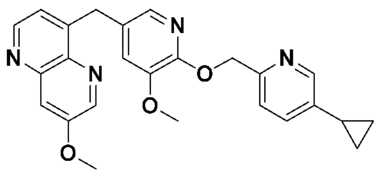 8-((6-((5-cyclopropylpyridin-2-yl)methoxy)-5-methoxypyridin-3-yl)methyl)-3-methoxy-1,5-naphthyridine | 429.1 | $^1H$ NMR (400 MHz, DMSO) δ 8.84 (dd, *J* = 5.1, 3.8 Hz, 2H), 8.53 (s, 1H), 7.89 (d, *J* = 7.7 Hz, 1H), 7.80 (d, *J* = 2.9 Hz, 1H), 7.67 (d, *J* = 1.6 Hz, 1H), 7.43 (d, *J* = 4.6 Hz, 1H), 7.39 (d, *J* = 8.1 Hz, 1H), 7.36 (d, *J* = 1.7 Hz, 1H), 5.31 (s, 2H), 4.53 (s, 2H), 4.00 (s, 3H), 3.72 (s, 3H), 2.16 (ddd, *J* = 13.0, 8.2, 4.9 Hz, 1H), 1.08 - 0.93 (m, 4H). |

(continued)

| Example No (Method) | Structure; IUPAC name | LC-MS (M+H) + | $^1$H NMR (ppm) |
|---|---|---|---|
| 60 (D) | 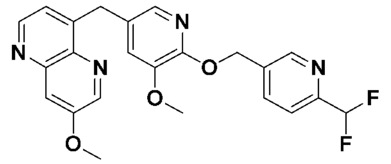 8-((6-((6-(difluoromethyl)pyridin-3-yl)methoxy)-5-methoxypyridin-3-yl)methyl)-3-methoxy-1,5-naphthyridine | 439.1 | $^1$H NMR (400 MHz, Acetone) δ 8.85 (t, *J* = 4.0 Hz, 2H), 8.73 (s, 1H), 8.02 (dd, *J* = 8.0, 1.7 Hz, 1H), 7.79 (d, *J* = 2.8 Hz, 1H), 7.68 (dd, *J* = 16.4, 4.8 Hz, 2H), 7.45 (d, *J* = 4.6 Hz, 1H), 7.37 (d, *J* = 1.7 Hz, 1H), 6.95 (t, *J* = 55.0 Hz, 1H), 5.42 (s, 2H), 4.54 (s, 2H), 4.00 (s, 3H), 3.73 (s, 3H). |
| 61 (D) | 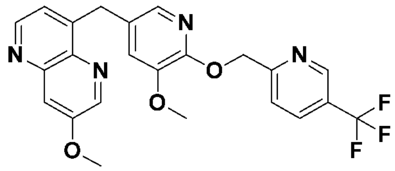 3-methoxy-8-((5-methoxy-6-((5-(trifluoromethyl)pyridin-2-yl)methoxy)pyridin-3-yl)methyl)-1,5-naphthyridine | 457.1 | $^1$H NMR (400 MHz, $CDCl_3$) δ 8.93 (s, 1H), 8.90 - 8.82 (m, 2H), 8.20 (dd, *J* = 8.3, 2.0 Hz, 1H), 7.79 (d, *J* = 2.9 Hz, 1H), 7.59 (dd, *J* = 14.8, 4.9 Hz, 2H), 7.48 (d, *J* = 4.6 Hz, 1H), 7.41 (d, *J* = 1.7 Hz, 1H), 5.49 (s, 2H), 4.54 (s, 2H), 4.00 (s, 3H), 3.78 (s, 3H). |
| 62 (E) | 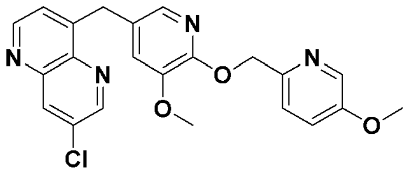 3-chloro-8-((5-methoxy-6-((5-methoxypyridin-2-yl)methoxy)pyridin-3-yl)methyl)-1,5-naphthyridine | 423.0 | $^1$H NMR(400 MHz, DMSO) δ9.11 (d, *J* = 2.4 Hz, 1H), 8.94 (d, *J* = 4.4 Hz, 1H), 8.61 (d, *J* = 2.4 Hz, 1H), 8.28 - 8.22 (m, 1H), 7.64 (dd, *J* = 17.7, 3.1 Hz, 2H), 7.41 - 7.33 (m, 3H), 5.31 (s, 2H), 4.55 (s, 2H), 3.82 (s, 3H), 3.75 (s, 3H) |
| 63 (E) | 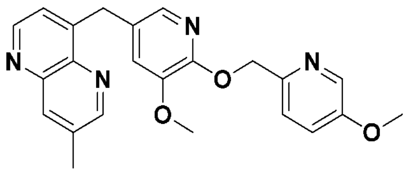 8-((5-methoxy-6-((5-methoxypyridin-2-yl)methoxy)pyridin-3-yl)methyl)-3-methyl-1,5-naphthyridine | 403.1 | $^1$H NMR (400 MHz, DMSO) δ 8.97 (d, *J* = 2.1 Hz, 1H), 8.87 (d, *J* = 4.5 Hz, 1H), 8.28 (d, *J* = 1.4 Hz, 1H), 8.24 - 8.19 (m, 1H), 7.66 (d, *J* = 1.8 Hz, 1H), 7.54 (d, *J* = 4.5 Hz, 1H), 7.46 - 7.40 (m, 2H), 7.38 (d, *J* = 1.8 Hz, 1H), 5.32 (s, 2H), 4.56 (s, 2H), 3.83 (s, 3H), 3.74 (s, 3H), 2.58 (s, 3H). |
| 64 (E) | 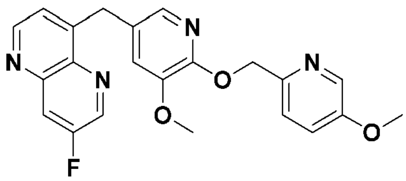 3-fluoro-8-((5-methoxy-6-((5-methoxypyridin-2-yl)methoxy)pyridin-3-yl)methyl)-1,5-naphthyridine | 407.1 | $^1$H NMR(400 MHz, DMSO) δ9.16 (d, *J* = 2.8 Hz, 1H), 8.93 (d, *J* = 4.4 Hz, 1H), 8.31 (dt, *J* = 8.7, 4.3 Hz, 1H), 8.27 - 8.21 (m, 1H), 7.66 (d, *J* = 1.9 Hz, 1H), 7.58 (d, *J* = 4.4 Hz, 1H), 7.41 - 7.32 (m, 3H), 5.28 (d, *J* = 17.6 Hz, 2H), 4.56 (s, 2H), 3.83 (s, 3H), 3.74 (s, 3H |

(continued)

| Example No (Method) | Structure; IUPAC name | LC-MS (M+H) + | $^1$H NMR (ppm) |
|---|---|---|---|
| 65 (E) | 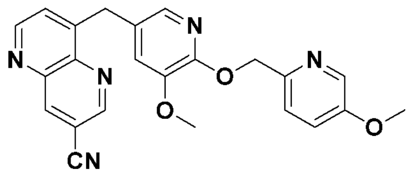 8-((5-methoxy-6-((5-methoxypyridin-2-yl)methoxy) pyridin-3-yl)methyl)-1,5-naphthyridine-3-carbonitrile | 414.1 | $^1$H NMR(400 MHz, DMSO) δ9.37 (d, *J* = 2.0 Hz, 1H), 9.12 (d, *J* = 2.0 Hz, 1H), 9.05 (d, *J* = 4.4 Hz, 1H), 8.28 - 8.23 (m, 1H), 7.75 (d, *J* = 4.4 Hz, 1H), 7.65 (d, *J* = 1.8 Hz, 1H), 7.41 - 7.34 (m, 3H), 5.30 (s, 2H), 4.56 (s, 2H), 3.83 (s, 3H), 3.75 (s, 3H). |
| 66 (D) | 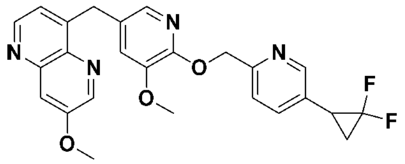 8-((6-((5-(2,2-difluorocyclopropyl)pyridin-2-yl)methoxy)-5-methoxypyridin-3-yl)methyl)-3-methoxy-1,5-naphthyridine | 465.1 | $^1$H NMR(400 MHz, DMSO) δ 8.80-8.83(m, 2H), 8.50(s, 1H), 7.78(s, 1H), 7.62-7.68(m, 2H), 7.36-7.41 (m, 3H), 5.37(s, 2H), 4.52(s, 2H), 3.99(s, 3H), 3.75(s, 3H), 3.12-3.10(m, 1H), 2.00-2.08(m, 2H). |
| 67 (D) | 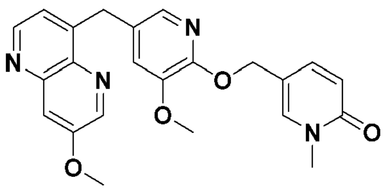 5-(((3-methoxy-5-((7-methoxy-1,5-naphthyridin-4-yl) methyl)pyridin-2-yl)oxy)methyl)-1-methylpyridin-2(1H)-one | 419.0 | $^1$H NMR(400 MHz, DMSO) δ 8.81-8.83(m, 2H), 7.84(s, 1H), 7.79(s, 1H), 7.68(s, 1H), 7.49-7.52(m, 1H), 7.40(s, 1H), 7.32(s, 1H), 6.37-6.40(m, 1H), 5.02(s, 2H), 4.52(s, 2H), 3.99(s, 3H), 3.70(s, 3H), 3.41(s, 3H). |
| 68 (H) | 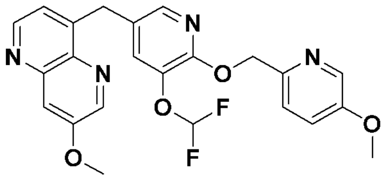 8-((5-(difluoromethoxy)-6-((5-methoxypyridin-2-yl) methoxy)pyridin-3-yl)methyl)-3-methoxy-1,5-naphthyridine | 455.1 | $^1$H NMR(400 MHz, DMSO) δ 8.82 (dd, J = 5.6, 3.7 Hz, 2H), 8.25 (t, J = 1.8 Hz, 1H), 8.05 (d, J = 2.0 Hz, 1H), 7.79 (d, J = 2.9 Hz, 1H), 7.65 (d, J = 1.9 Hz, 1H), 7.46 (d, J = 4.5 Hz, 1H), 7.40 (d, J = 1.8 Hz, 2H), 7.16 (t, J = 73.8 Hz, 1H), 5.37 (s, 2H), 4.54 (s, 2H), 3.99 (s, 3H), 3.82 (s, 3H). |
| 69 (H) | 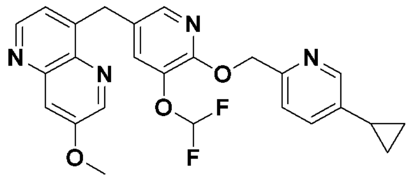 8-((6-((5-cyclopropylpyridin-2-yl)methoxy)-5-(difluoromethoxy)pyridin-3-yl)methyl)-3-methoxy-1,5-naphthyridine | 465.1 | $^1$H NMR(400 MHz, DMSO) δ 8.82 (dd, J = 5.6, 3.7 Hz, 2H), 8.37 (d, J = 2.0 Hz, 1H), 8.04 (d, J = 2.0 Hz, 1H), 7.79 (d, J = 2.9 Hz, 1H), 7.66 (d, J = 1.7 Hz, 1H), 7.46 (d, J = 4.5 Hz, 1H), 7.42 (dd, J = 8.1, 2.3 Hz, 1H), 7.30 (d, J = 8.1 Hz, 1H), 7.18 (t, J = 73.8 Hz, 1H), 5.39 (s, 2H), 4.54 (s, 2H), 3.99 (s, 3H), 2.04 - 1.84 (m, 1H), 1.06 - 0.86 (m, 2H), 0.81 - 0.63 (m, 2H). |

(continued)

| Example No (Method) | Structure; IUPAC name | LC-MS (M+H) + | $^1H$ NMR (ppm) |
|---|---|---|---|
| 70 (D) | 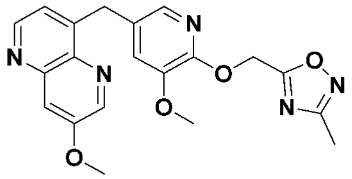 5-(((3-methoxy-5-((7-methoxy-1,5-naphthyridin-4-yl)methyl)pyridin-2-yl)oxy)methyl)-3-methyl-1,2,4-oxadiazole | 394.1 | $^1H$ NMR(400 MHz, DMSO) δ 8.85 - 8.77 (m, 2H), 7.79 (d, *J* = 2.9 Hz, 1H), 7.59 (d, *J* = 1.8 Hz, 1H), 7.41 (dd, *J* = 6.0, 3.1 Hz, 2H), 5.58 (s, 2H), 4.52 (s, 2H), 3.99 (s, 3H), 3.77 (s, 3H), 2.32 (s, 3H). |
| 71 (D) | 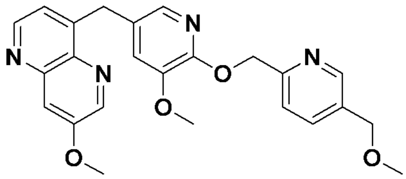 3-methoxy-8-((5-methoxy-6-((5-(methoxymethyl)pyridin-2-yl)methoxy)pyridin-3-yl)methyl)-1,5-naphthyridine | 433.1 | $^1H$ NMR(400 MHz, DMSO) δ 8.82 (dd, *J* = 6.7, 3.7 Hz, 2H), 8.49 (d, *J* = 1.6 Hz, 1H), 7.79 (d, *J* = 2.9 Hz, 1H), 7.73 (dd, *J* = 8.0, 2.2 Hz, 1H), 7.63 (d, *J* = 1.8 Hz, 1H), 7.45 - 7.34 (m, 3H), 5.38 (s, 2H), 4.52 (s, 2H), 4.43 (s, 2H), 3.99 (s, 3H), 3.76 (s, 3H), 3.30 (s, 3H). |
| 72 (D) | 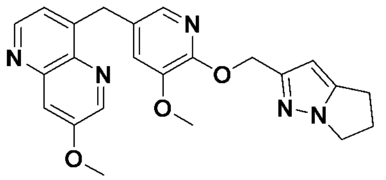 8-((6-((5,6-dihydro-4H-pyrrolo[1,2-b]pyrazol-2-yl)methoxy)-5-methoxypyridin-3-yl)methyl)-3-methoxy-1,5-naphthyridine | 418.1 | $^1H$ NMR(400 MHz, DMSO) δ 8.82 (dd, J = 7.1, 3.7 Hz, 2H), 7.79 (d, J = 2.9 Hz, 1H), 7.67 (d, J = 1.8 Hz, 1H), 7.41 (d, J = 4.5 Hz, 1H), 7.31 (d, J = 1.8 Hz, 1H), 5.99 (s, 1H), 5.15 (s, 2H), 4.52 (s, 2H), 4.02 (t, J = 7.2 Hz, 2H), 4.00 (s, 3H), 3.69 (s, 3H), 2.79 (t, J = 7.3 Hz, 2H), 2.48 (t, J = 7.0 Hz, 2H). |
| 73 (D) | 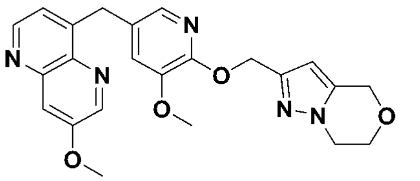 2-(((3-methoxy-5-((7-methoxy-1,5-naphthyridin-4-yl)methyl)pyridin-2-yl)oxy)methyl)-6,7-dihydro-4H-pyrazolo[5,1-c][1,4]oxazine | 434.1 | $^1H$ NMR(400 MHz, DMSO) δ8.82 (dd, *J* = 7.3, 3.7 Hz, 2H), 7.79 (d, *J* = 2.9 Hz, 1H), 7.67 (d, *J* = 1.9 Hz, 1H), 7.41 (d, *J* = 4.5 Hz, 1H), 7.32 (d, *J* = 1.8 Hz, 1H), 6.07 (s, 1H), 5.18 (s, 2H), 4.75 (s, 2H), 4.52 (s, 2H), 4.09 - 4.01 (m, 4H), 3.99 (s, 3H), 3.69 (s, 3H). |
| 74 (H) | 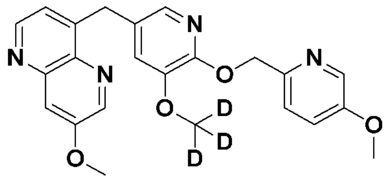 3-methoxy-8-((5-(methoxy-d3)-6-((5-methoxypyridin-2-yl)methoxy)pyridin-3-yl)methyl)-1,5-naphthyridine | 422.1 | $^1H$ NMR(400 MHz, DMSO) δ 8.82 (dd, *J* = 7.0, 3.7 Hz, 2H), 8.30 - 8.22 (m, 1H), 7.79 (d, *J* = 2.9 Hz, 1H), 7.64 (d, *J* = 1.9 Hz, 1H), 7.38 (ddd, *J* = 13.1, 8.5, 3.2 Hz, 4H), 5.29 (s, 2H), 4.52 (s, 2H), 3.99 (s, 3H), 3.82 (s, 3H). |

(continued)

| Example No (Method) | Structure; IUPAC name | LC-MS (M+H) + | $^1$H NMR (ppm) |
|---|---|---|---|
| 75 (D) | 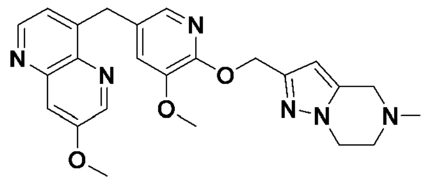 3-methoxy-8-((5-methoxy-6-((5-methyl-4,5,6,7-tetrahydropyrazolo[1,5-a]pyrazin-2-yl)methoxy)pyridin-3-yl)methyl)-1,5-naphthyridine | 447.1 | $^1$H NMR(400 MHz, DMSO) δ 8.82 (dd, J = 7.4, 3.7 Hz, 2H), 7.79 (d, J = 2.9 Hz, 1H), 7.67 (d, J = 1.8 Hz, 1H), 7.40 (d, J = 4.5 Hz, 1H), 7.31 (d, J = 1.8 Hz, 1H), 6.02 (s, 1H), 5.16 (s, 2H), 4.52 (s, 2H), 4.04 (t, J = 5.6 Hz, 2H), 3.99 (s, 3H), 3.69 (s, 3H), 3.53 (s, 2H), 2.81 (t, J = 5.6 Hz, 2H), 2.36 (s, 3H). |
| 76 (D) | 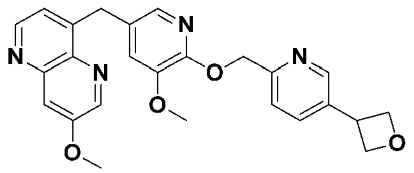 3-methoxy-8-((5-methoxy-6-((5-(oxetan-3-yl)pyridin-2-yl)methoxy)pyridin-3-yl)methyl)-1,5-naphthyridine | 445.1 | $^1$H NMR(400 MHz, DMSO) δ 8.82 (dd, *J* = 6.5, 3.7 Hz, 2H), 8.52 (d, *J* = 2.2 Hz, 1H), 7.91 (dd, *J* = 8.1, 2.3 Hz, 1H), 7.79 (d, *J* = 2.9 Hz, 1H), 7.63 (d, *J* = 1.8 Hz, 1H), 7.45 - 7.34 (m, 3H), 5.37 (s, 2H), 4.94 (dd, *J* = 8.4, 6.0 Hz, 2H), 4.66 - 4.57 (m, 2H), 4.52 (s, 2H), 4.34 - 4.24 (m, 1H), 3.99 (s, 3H), 3.76 (s, 3H). |
| 77 (E) | 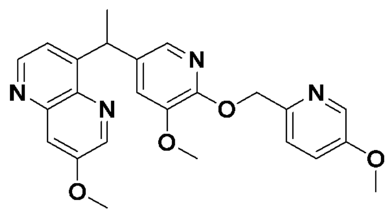 3-methoxy-8-(1-(5-methoxy-6-((5-methoxypyridin-2-yl)methoxy)pyridin-3-yl)ethyl)-1,5-naphthyridine | 433.1 | $^1$H NMR(400 MHz, DMSO) δ 8.86(s, 1H), 8.80(s, 1H), 8.25(s, 1H), 7.78(s, 1H), 7.65(s, 1H), 7.52(s, 1H), 7.32-7.40(m, 3H), 5.45-5.50(m, 1H), 5.28(s, 2H), 3.99(s, 3H),3.82(s, 3H), 3.76(s, 3H), 1.70(d, 3H). |
| 78 (D) | 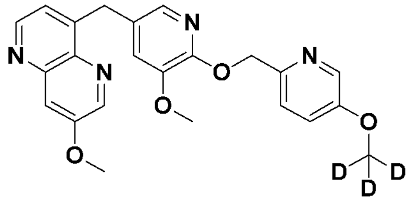 3-methoxy-8-((5-methoxy-6-((5-(methoxy-d3)pyridin-2-yl)methoxy)pyridin-3-yl)methyl)-1,5-naphthyridine | 422.1 | $^1$H NMR(400 MHz, DMSO) δ 8.82 (dd, *J* = 6.9, 3.7 Hz, 2H), 8.28 - 8.23 (m, 1H), 7.79 (d, *J* = 2.9 Hz, 1H), 7.65 (d, *J* = 1.9 Hz, 1H), 7.43 - 7.30 (m, 4H), 5.30 (s, 2H), 4.52 (s, 2H), 4.00 (s, 3H), 3.74 (s, 3H). |
| 79 (E) | 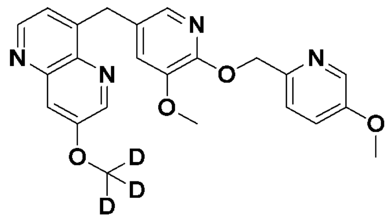 3-(methoxy-d3)-8-((5-methoxy-6-((5-methoxypyridin-2-yl)methoxy)pyridin-3-yl)methyl)-1,5-naphthyridine | 422.1 | $^1$H NMR(400 MHz, DMSO) δ 8.82 (dd, *J* = 6.8, 3.7 Hz, 2H), 8.28 - 8.21 (m, 1H), 7.79 (d, *J* = 2.9 Hz, 1H), 7.65 (d, *J* = 1.8 Hz, 1H), 7.46 - 7.30 (m, 4H), 5.30 (s, 2H), 4.52 (s, 2H), 3.82 (s, 3H), 3.74 (s, 3H). |

(continued)

| Example No (Method) | Structure; IUPAC name | LC-MS (M+H) + | $^1$H NMR (ppm) |
|---|---|---|---|
| 80 (D) | 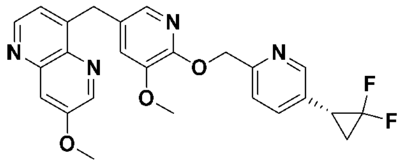 (R)-8-((6-((5-(2,2-difluorocyclopropyl)pyridin-2-yl) methoxy)-5-methoxypyridin-3-yl)methyl)-3-methoxy-1,5-naphthyridine Or 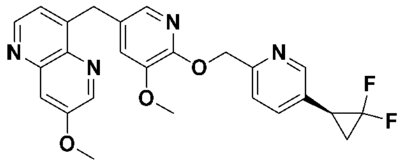 (S)-8-((6-((5-(2,2-difluorocyclopropyl)pyridin-2-yl) methoxy)-5-methoxypyridin-3-yl)methyl)-3-methoxy-1,5-naphthyridine | 465.1 | $^1$H NMR(400 MHz, DMSO) δ 8.80-8.83(m, 2H), 8.50(s, 1H), 7.78(s, 1H), 7.62-7.68(m, 2H), 7.36-7.41 (m, 3H), 5.37(s, 2H), 4.52(s, 2H), 3.99(s, 3H), 3.75(s, 3H), 3.12-3.10(m, 1H), 2.00-2.08(m, 3H). |
| 81 (D) | 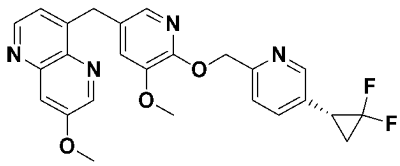 (R)-8-((6-((5-(2,2-difluorocyclopropyl)pyridin-2-yl) methoxy)-5-methoxypyridin-3-yl)methyl)-3-methoxy-1,5-naphthyridine Or 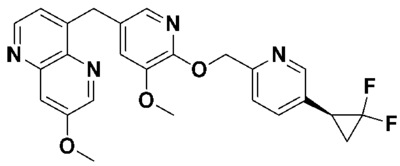 (S)-8-((6-((5-(2,2-difluorocyclopropyl)pyridin-2-yl) methoxy)-5-methoxypyridin-3-yl)methyl)-3-methoxy-1,5-naphthyridine | 465.1 | $^1$H NMR(400 MHz, DMSO) δ 8.80-8.83(m, 2H), 8.50(s, 1H), 7.78(s, 1H), 7.62-7.68(m, 2H), 7.36-7.41 (m, 3H), 5.37(s, 2H), 4.52(s, 2H), 3.99(s, 3H), 3.75(s, 3H), 3.12-3.10(m, 1H), 2.00-2.08(m, 3H). |
| 82 (D) | 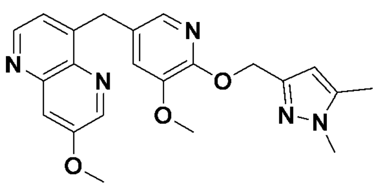 8-((6-((1,5-dimethyl-1H-pyrazol-3-yl)methoxy)-5-methoxypyridin-3-yl)methyl)-3-methoxy-1,5-naphthyridine | 406.1 | $^1$H NMR(400 MHz, DMSO) δ 8.82 (dd, J = 7.0, 3.7 Hz, 2H), 7.79 (d, J = 2.9 Hz, 1H), 7.66 (d, J = 1.8 Hz, 1H), 7.40 (d, J = 4.5 Hz, 1H), 7.30 (d, J = 1.8 Hz, 1H), 6.03 (s, 1H), 5.11 (s, 2H), 4.51 (s, 2H), 4.00 (s, 3H), 3.68 (d, J = 5.9 Hz, 6H), 2.21 (s, 3H). |

(continued)

| Example No (Method) | Structure; IUPAC name | LC-MS (M+H) + | $^1$H NMR (ppm) |
|---|---|---|---|
| 83 (D) | 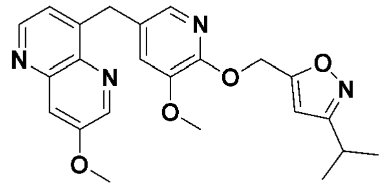 3-isopropyl-5-(((3-methoxy-5-((7-methoxy-1,5-naphthyridin-4-yl)methyl)pyridin-2-yl)oxy)methyl)isoxazole | 421.1 | $^1$H NMR(400 MHz, DMSO) δ 8.82 (dd, J = 5.8, 3.7 Hz, 2H), 7.79 (d, J = 2.8 Hz, 1H), 7.68 (d, J = 1.5 Hz, 1H), 7.41 (d, J = 4.4 Hz, 1H), 7.38 (d, J = 1.6 Hz, 1H), 6.51 (s, 1H), 5.38 (s, 2H), 4.53 (s, 2H), 3.99 (s, 3H), 3.73 (s, 3H), 2.98 (dt, J = 13.8, 6.9 Hz, 1H), 1.21 (s, 3H), 1.19 (s, 3H). |
| 84 (**I**) | 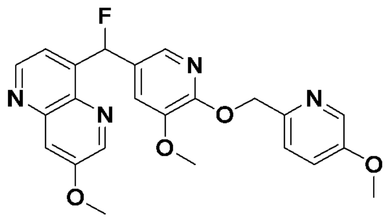 8-(fluoro(5-methoxy-6-((5-methoxypyridin-2-yl)methoxy)pyridin-3-yl)methyl)-3-methoxy-1,5-naphthyridine | 437.1 | $^1$H NMR(400 MHz, DMSO) δ 9.04(s, 1H), 8.74(s, 1H), 8.24(s, 1H), 7.83-7.87(m, 2H), 7.73(s, 1H), 7.61(d, 1H), 7.42(s, 1H), 7.37(s, 2H), 5.31(s, 2H), 3.98(s, 3H), 3.81 (s, 3H), 3.77(s, 3H). |
| 85 (D) | 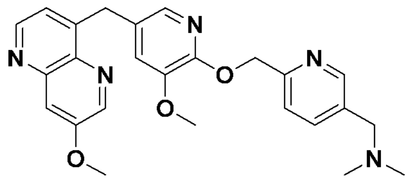 1-(6-(((3-methoxy-5-((7-methoxy-1,5-naphthyridin-4-yl)methyl)pyridin-2-yl)oxy)methyl)pyridin-3-yl)-N,N-dimethylmethanamine | 446.1 | $^1$H NMR(400 MHz, DMSO) δ 8.91 - 8.73 (m, 2H), 8.42 (s, 1H), 7.78 (d, J = 2.5 Hz, 1H), 7.68 (d, J = 7.8 Hz, 1H), 7.63 (s, 1H), 7.40 (d, J = 4.2 Hz, 1H), 7.35 (d, J = 7.7 Hz, 2H), 5.35 (s, 2H), 4.51 (s, 2H), 3.98 (s, 3H), 3.75 (s, 3H), 3.33 (s, 2H), 2.12 (s, 6H) |
| 86 (D) | 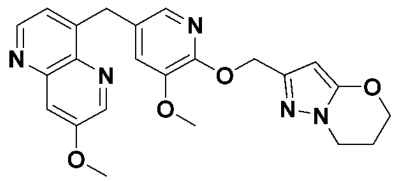 2-(((3-methoxy-5-((7-methoxy-1,5-naphthyridin-4-yl)methyl)pyridin-2-yl)oxy)methyl)-6,7-dihydro-5H-pyrazolo[5,1-b][1,3]oxazine | 434.1 | $^1$H NMR(400 MHz, DMSO) δ 8.82 (dd, J = 7.1, 3.7 Hz, 2H), 7.79 (d, J = 2.9 Hz, 1H), 7.66 (d, J = 1.8 Hz, 1H), 7.40 (d, J = 4.5 Hz, 1H), 7.31 (d, J = 1.8 Hz, 1H), 5.49 (s, 1H), 5.06 (s, 2H), 4.52 (s, 2H), 4.29 - 4.19 (m, 2H), 4.04 (t, J = 6.2 Hz, 2H), 4.00 (s, 3H), 3.70 (s, 3H), 2.20 - 2.09 (m, 2H) |
| 87 (D) | 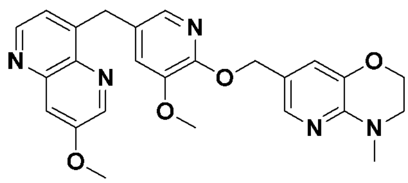 7-(((3-methoxy-5-((7-methoxy-1,5-naphthyridin-4-yl)methyl)pyridin-2-yl)oxy)methyl)-4-methyl-3,4-dihydro-2H-pyrido[3,2-b][1,4]oxazine | 460.1 | $^1$H NMR(400 MHz, DMSO) δ 8.82 (dd, *J* = 7.1, 3.7 Hz, 2H), 7.79 (d, *J* = 2.9 Hz, 1H), 7.64 (d, *J* = 1.9 Hz, 1H), 7.40 (d, *J* = 4.5 Hz, 1H), 7.34 (d, *J* = 1.9 Hz, 1H), 6.88 (d, *J* = 7.7 Hz, 1H), 6.52 (d, *J* = 7.8 Hz, 1H), 5.13 (s, 2H), 4.52 (s, 2H), 4.25 - 4.14 (m, 2H), 3.99 (s, 3H), 3.73 (s, 3H), 3.42 (d, *J* = 4.6 Hz, 2H), 2.99 (s, 3H) |

(continued)

| Example No (Method) | Structure; IUPAC name | LC-MS (M+H) + | $^1$H NMR (ppm) |
|---|---|---|---|
| 88 (D) | 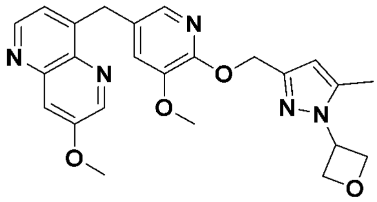 3-methoxy-8-((5-methoxy-6-((5-methyl-1-(oxetan-3-yl)-1H-pyrazol-3-yl)methoxy)pyridin-3-yl) methyl)-1,5-naphthyridine | 448.1 | $^1$H NMR(400 MHz, DMSO) δ 8.82 (dd, *J* = 6.8, 3.7 Hz, 2H), 7.79 (d, *J* = 2.9 Hz, 1H), 7.68 (d, *J* = 1.8 Hz, 1H), 7.41 (d, *J* = 4.5 Hz, 1H), 7.32 (d, *J* = 1.8 Hz, 1H), 6.09 (s, 1H), 5.52 (ddd, *J* = 14.1, 7.6, 6.5 Hz, 1H), 5.21 (s, 2H), 4.89 (dt, *J* = 14.1, 6.4 Hz, 4H), 4.53 (s, 2H), 4.00 (s, 3H), 3.70 (s, 3H), 2.18 (s, 3H). |
| 89 (D) | 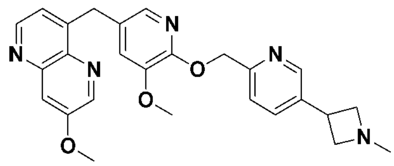 3-methoxy-8-((5-methoxy-6-((5-(1-methylazetidin-3-yl)pyridin-2-yl)methoxy)pyridin-3-yl)methyl)-1,5-naphthyridine | 458.1 | $^1$H NMR(400 MHz, DMSO) δ 8.82 (dd, *J* = 6.1, 3.7 Hz, 2H), 8.48 (d, *J* = 2.0 Hz, 1H), 7.88 - 7.75 (m, 2H), 7.63 (d, *J* = 1.7 Hz, 1H), 7.47 - 7.32 (m, 3H), 5.35 (s, 2H), 4.52 (s, 2H), 3.99 (s, 3H), 3.75 (s, 3H), 3.64 - 3.48 (m, 4H), 3.09 (s, 1H), 2.26 (s, 3H). |
| 90 (D) | 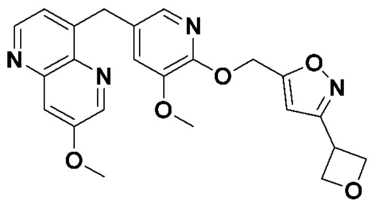 5-(((3-methoxy-5-((7-methoxy-1,5-naphthyridin-4-yl) methyl)pyridin-2-yl)oxy)methyl)-3-(oxetan-3-yl)isoxazole | 435.1 | $^1$H NMR(400 MHz, DMSO) δ 8.82 (dd, J = 5.4, 3.8 Hz, 2H), 7.79 (d, J = 2.8 Hz, 1H), 7.68 (s, 1H), 7.45 - 7.34 (m, 2H), 6.71 (s, 1H), 5.44 (s, 2H), 4.89 (dd, J = 8.3, 6.0 Hz, 2H), 4.64 (t, J = 6.3 Hz, 2H), 4.53 (s, 2H), 4.40 - 4.28 (m, 1H), 4.00 (s, 3H), 3.74 (s, 3H) |
| 91 (D) | 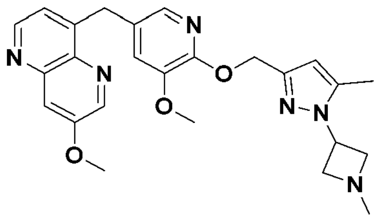 3-methoxy-8-((5-methoxy-6-((5-methyl-1-(1-methylazetidin-3-yl)-1H-pyrazol-3-yl)methoxy)pyridin-3-yl) methyl)-1,5-naphthyridine | 461.2 | $^1$H NMR(400 MHz, DMSO) δ 8.82 (dd, *J* = 6.9, 3.7 Hz, 2H), 7.79 (d, *J* = 2.9 Hz, 1H), 7.68 (d, *J* = 1.7 Hz, 1H), 7.41 (d, *J* = 4.5 Hz, 1H), 7.32 (d, *J* = 1.7 Hz, 1H), 6.05 (s, 1H), 5.16 (s, 2H), 4.89 - 4.79 (m, 1H), 4.52 (s, 2H), 4.00 (s, 3H), 3.70 (s, 3H), 2.30 (s, 3H), 2.16 (s, 3H). |
| 92 (D) | 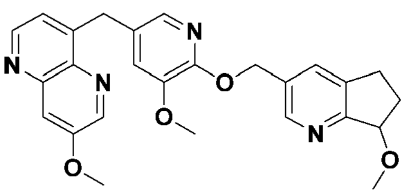 3-methoxy-8-((5-methoxy-6-((7-methoxy-6,7-dihydro-5H-cyclopenta[b]pyridin-3-yl)methoxy)pyridin-3-yl)methyl)-1,5-naphthyridine | 459.1 | $^1$H NMR(400 MHz, DMSO) δ 8.80-8.82 (m, 2H), 7.78 (d, 1H), 7.66 (t, 2H), 7.36-7.40 (m,2H), 7.26 (d, 1H), 5.36 (s, 2H), 4.63 (q, 1H), 4.51 (s, 2H), 3.99 (s, 3H), 3.74 (s,3H), 3.38(d,3H), 2.90-2.97(m, 1H), 2.72-2.80(m, 1H), 2.23-2.26(m,1H), 1.98-2.02(m,1H) |

(continued)

| Example No (Method) | Structure; IUPAC name | LC-MS (M+H) + | $^1$H NMR (ppm) |
|---|---|---|---|
| 93 (D) | 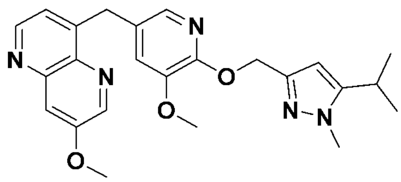 8-((6-((5-isopropyl-1-methyl-1H-pyrazol-3-yl)methoxy)-5-methoxypyridin-3-yl)methyl)-3-methoxy-1,5-naphthyridine | 434.2 | $^1$H NMR(400 MHz, DMSO) δ 8.82 (dd, J = 6.6, 3.7 Hz, 2H), 7.79 (d, J = 2.8 Hz, 1H), 7.68 (d, J = 1.1 Hz, 1H), 7.41 (d, J = 4.4 Hz, 1H), 7.31 (d, J = 1.3 Hz, 1H), 6.07 (s, 1H), 5.11 (s, 2H), 4.52 (s, 2H), 4.00 (s, 3H), 3.73 (s, 3H), 3.69 (s, 3H), 2.99 (dt, J = 13.6, 6.8 Hz, 1H), 1.17 (d, J = 6.8 Hz, 6H) |
| 94 (D) | 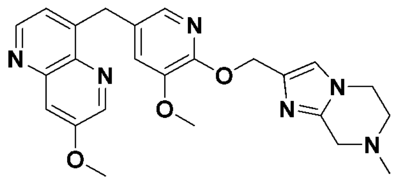 3-methoxy-8-((5-methoxy-6-((7-methyl-5,6,7,8-tetrahydroimidazo[1,2-a]pyrazin-2-yl)methoxy)pyridin-3-yl)methyl)-1,5-naphthyridine | 447.1 | $^1$H NMR(400 MHz, DMSO) δ 8.82 (dd, J = 7.4, 3.7 Hz, 2H), 7.79 (d, J = 2.9 Hz, 1H), 7.66 (d, J = 1.8 Hz, 1H), 7.40 (d, J = 4.5 Hz, 1H), 7.29 (d, J = 1.8 Hz, 1H), 7.07 (s, 1H), 5.08 (s, 2H), 4.52 (s, 2H), 4.00 (s, 3H), 3.93 (t, J = 5.5 Hz, 2H), 3.69 (s, 3H), 3.48 (s, 2H), 2.74 (t, J = 5.5 Hz, 2H), 2.37 (s, 3H) |
| 95 (E) | 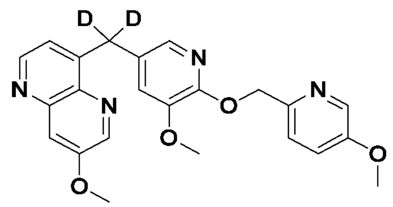 8-(difluoro(5-methoxy-6-((5-methoxypyridin-2-yl)methoxy)pyridin-3-yl)methyl)-3-methoxy-1,5-naphthyridine | 421.0 | $^1$H NMR(400 MHz, DMSO) δ 8.83-8.81(m,2H), 8.25(t,1H), 7.78(d,J=2.88,1H), 7.65(d,J=1.76,1H), 7.35-7.43(m,4H), 5.30(s,2H), 4.00(s,3H), 3.82(s,3H), 3.74(s,3H), |
| 96 (D) | 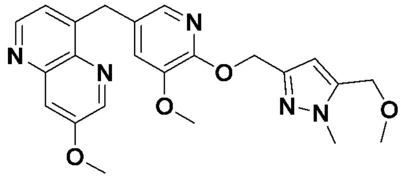 3-methoxy-8-((5-methoxy-6-((5-(methoxymethyl)-1-methyl-1H-pyrazol-3-yl)methoxy)pyridin-3-yl)methyl)-1,5-naphthyridine | 436.1 | $^1$H NMR(400 MHz, DMSO) δ 8.82 (dd, J = 6.9, 3.7 Hz, 2H), 7.79 (d, J = 2.9 Hz, 1H), 7.67 (d, J = 1.6 Hz, 1H), 7.40 (d, J = 4.5 Hz, 1H), 7.31 (d, J = 1.7 Hz, 1H), 6.26 (s, 1H), 5.16 (s, 2H), 4.52 (s, 2H), 4.42 (s, 2H), 4.00 (s, 3H), 3.75 (s, 3H), 3.70 (s, 3H), 3.25 (s, 3H). |
| 97 (D) | 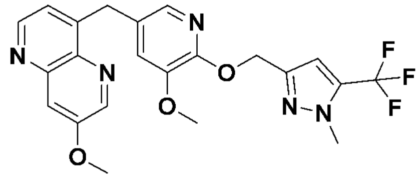 3-methoxy-8-((5-methoxy-6-((1-methyl-5-(trifluoromethyl)-1H-pyrazol-3-yl)methoxy)pyridin-3-yl)methyl)-1,5-naphthyridine | 460.1 | $^1$H NMR (400 MHz, CDCl3) δ 8.78 (t,2H), 7.66-7.72 (m, 2H), 7.24(d,1H), 7.08 (d, 1H), 6.72 (s, 1H), 5.43 (s, 2H), 4.58 (s, 2H), 3.99 (d, 6H),3.79(s,3H) |

(continued)

| Example No (Method) | Structure; IUPAC name | LC-MS (M+H) + | $^1$H NMR (ppm) |
|---|---|---|---|
| 98 (D) | 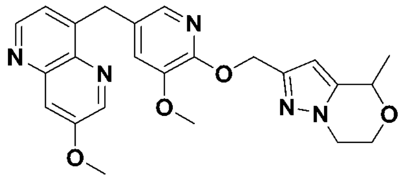 2-(((3-methoxy-5-((7-methoxy-1,5-naphthyridin-4-yl) methyl)pyridin-2-yl)oxy)methyl)-4-methyl-6,7-dihydro-4H-pyrazolo [5,1-c][1,4]oxazine | 448.1 | $^1$H NMR(400 MHz, DMSO) δ 8.80-8.83(m, 2H), 7.79(s, 1H), 7.68(s, 1H), 7.41(s, 1H), 7.31(s, 1H), 6.13(s, 1H), 5.18(s, 2H), 4.76-4.81(m, 1H), 4.52(s, 2H), 3.87-4.25(m, 4H), 4.00(s,3H),3.70(s, 3H), 1.41(d, 3H). |
| 99 (D) | 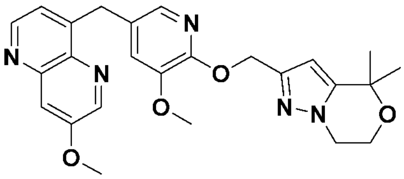 2-(((3-methoxy-5-((7-methoxy-1,5-naphthyridin-4-yl) methyl)pyridin-2-yl)oxy)methyl)-4,4-dimethyl-6,7-dihydro-4H-pyrazolo[5,1-c][1,4]oxazine | 462.1 | $^1$H NMR(400 MHz, DMSO) δ 8.80-8.83(m, 2H), 7.79(s, 1H), 7.68(s, 1H), 7.41(s, 1H), 7.31(s, 1H), 6.16(s, 1H), 5.15(s, 2H), 4.53(s, 2H), 3.99-4.03(m, 7H), 3.70(s, 3H), 1.46(s, 6H). |
| 100 (J) | 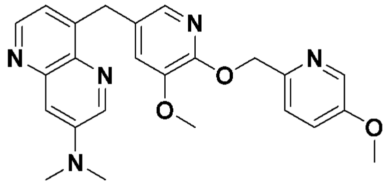 8-((5-methoxy-6-((5-methoxypyridin-2-yl)methoxy) pyridin-3-yl)methyl)-N,N-dimethyl-1,5-naphthyridin-3-amine | 432.1 | $^1$H NMR(400 MHz, DMSO) δ 8.87(d, 1H), 8.67(d, 1H), 8.25(s, 1H), 7.64(s, 1H), 7.33-7.37(m, 3H), 7.27(s, 1H), 7.20(s, 1H), 5.30(s, 2H), 4.46(s, 2H), 3.82(s, 3H), 3.74(s, 3H), 3.12(s, 6H). |
| 101 (D) | 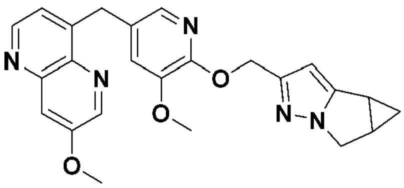 2-(((3-methoxy-5-((7-methoxy-1,5-naphthyridin-4-yl) methyl)pyridin-2-yl)oxy)methyl)-3b,4,4a,5-tetrahydrocyclopropa[3,4]pyrr olo[1,2-b]pyrazole | 430.1 | $^1$H NMR(400 MHz, DMSO) δ 8.83 (dd, J = 6.5, 3.5 Hz, 2H), 7.80 (s, 1H), 7.68 (s, 1H), 7.42 (d, J = 4.2 Hz, 1H), 7.32 (s, 1H), 6.04 (s, 1H), 5.12 (s, 2H), 4.53 (s, 2H), 4.21 (d, J = 5.8 Hz, 1H), 4.08 - 3.92 (m, 4H), 3.70 (s, 3H), 2.33 (d, J = 12.7 Hz, 2H), 1.23 (dd, J = 12.5, 7.5 Hz, 1H), 0.49 (d, J = 4.1 Hz, 1H) |
| 102 (D) | 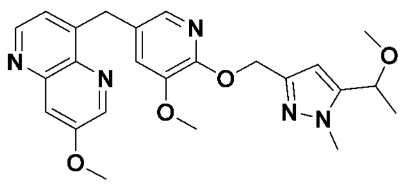 3-methoxy-8-((5-methoxy-6-((5-(1-methoxyethyl)-1-methyl-1H-pyrazol-3-yl)methoxy)pyridin-3-yl) methyl)-1,5-naphthyridine | 450.1 | $^1$H NMR(400 MHz, DMSO) δ 8.81-8.83(m,2H), 7.79(d,J=2.88,1H), 7.68(d,J=1.8,1H), 7.41(d,J=4.44,1H), 7.32(d,J=1.8,1H), 6.22(s,1H), 5.15(s,2H), 4.58(d,J=6.56,1H), 4.54(t,2H), 4.00(s,3H), 3.78(s,3H), 3.70(s,3H), 3.18(s,3H), 1.40(d,J=6.52,3H), |

(continued)

| Example No (Method) | Structure; IUPAC name | LC-MS (M+H) + | $^1$H NMR (ppm) |
|---|---|---|---|
| 103 (D) | 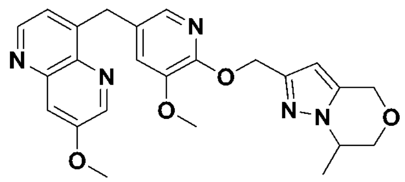 2-(((3-methoxy-5-((7-methoxy-1,5-naphthyridin-4-yl) methyl)pyridin-2-yl)oxy)methyl)-7-methyl-6,7-dihydro-4H-pyrazolo [5,1-c][1,4]oxazine | 448.1 | $^1$H NMR(400 MHz, DMSO) δ 8.82 (dd, *J* = 6.7, 3.7 Hz, 2H), 7.79 (d, *J* = 2.9 Hz, 1H), 7.67 (s, 1H), 7.41 (d, *J* = 4.4 Hz, 1H), 7.32 (s, 1H), 6.05 (s, 1H), 5.19 (d, *J* = 2.8 Hz, 2H), 4.75 (d, *J* = 3.8 Hz, 2H), 4.52 (s, 2H), 4.28 - 4.23 (m, 1H), 4.09 (dd, *J* = 11.8, 4.2 Hz, 1H), 4.00 (s, 3H), 3.70 (s, 3H), 3.66 (s, 1H), 1.39 (d, *J* = 6.5 Hz, 3H). |
| 104 (D) | 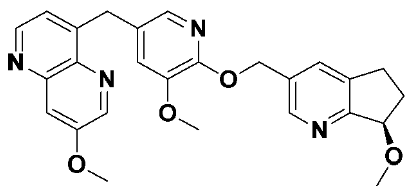 (R)-3-methoxy-8-((5-methoxy-6-((7-methoxy-6,7-dihydro-SH-cyclopenta[b]pyridin-3-yl)methoxy)pyridin-3-yl)methyl)-1,5-naphthyridine or 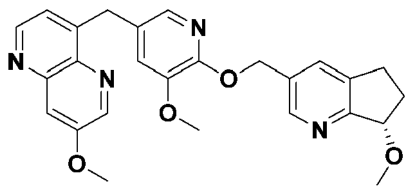 (S)-3-methoxy-8-((5-methoxy-6-((7-methoxy-6,7-dihydro-SH-cyclopenta[b]pyridin-3-yl)methoxy)pyridin-3-yl)methyl)-1,5-naphthyridine | 459.1 | $^1$H NMR(400 MHz, DMSO) δ 8.80-8.83(m, 2H),7.78(s, 1H), 7.66(t, 2H), 7.36-7.40 (m, 2H), 7.26 (s, 1H), 5.36 (s, 2H), 4.62-4.64 (m, 1H), 4.51(s, 2H), 3.99(s, 3H), 3.75(s, 3H),3.38(s, 3H), 2.90-2.97 (m, 1H), 2.72-2.80(m, 1H), 2.26-2.33(m, 1H), 1.97-2.01(m, 1H). |
| 105 (D) | 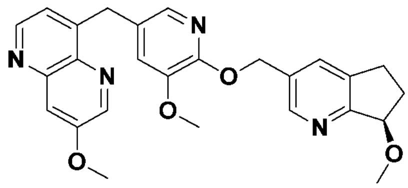 (R)-3-methoxy-8-((5-methoxy-6-((7-methoxy-6,7-dihydro-5H-cyclopenta[b]pyridin-3-yl)methoxy)pyridin-3-yl)methyl)-1,5-naphthyridine or 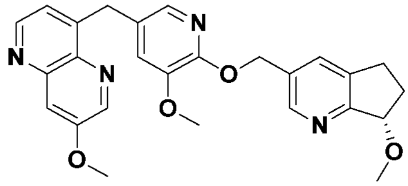 (S)-3-methoxy-8-((5-methoxy-6-((7-methoxy-6,7-dihydro-5H-cyclopenta[b]pyridin-3-yl)methoxy)pyridin-3-yl)methyl)-1,5-naphthyridine | 459.1 | $^1$H NMR(400 MHz, DMSO) δ 8.80-8.83 (m, 2H),7.78 (s, 1H), 7.66(t, 2H), 7.36-7.40(m, 2H), 7.26(s, 1H), 5.36(s, 2H), 4.62-4.64(m, 1H), 4.51(s, 2H), 3.99(s, 3H), 3.75(s, 3H), 3.38(s, 3H), 2.90-2.97 (m, 1H), 2.72-2.80(m, 1H), 2.26-2.33(m, 1H), 1.97-2.01(m, 1H). |

(continued)

| Example No (Method) | Structure; IUPAC name | LC-MS (M+H) + | $^1$H NMR (ppm) |
|---|---|---|---|
| 106 (D) | 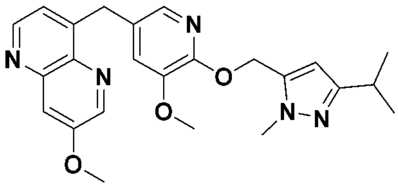 8-((6-((3-isopropyl-1-methyl-1H-pyrazol-5-yl)methoxy)-5-methoxypyridin-3-yl)methyl)-3-methoxy-1,5-naphthyridine | 434.2 | $^1$H NMR(400 MHz, DMSO) δ 8.80-8.83(m, 2H), 7.78(d, 1H), 7.69(d, 1H), 7.41(d, 1H), 7.34(d, 1H), 6.14(s, 1H), 5.26(s, 2H), 4.52(s, 2H), 3.99(s, 3H), 3.71(d, 6H), 2.76-2.85(m, 1H), 1.15(d, 6H). |
| 107 (D) | 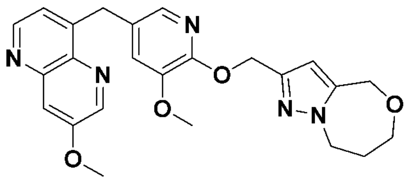 2-(((3-methoxy-5-((7-methoxy-1,5-naphthyridin-4-yl)methyl)pyridin-2-yl)oxy)methyl)-7,8-dihydro-4H,6H-pyrazolo[5,1-c][1,4]oxazepine | 448.1 | $^1$H NMR(400 MHz, DMSO) δ 8.82 (dd, J = 6.8, 3.7 Hz, 2H), 7.79 (d, J = 2.9 Hz, 1H), 7.67 (d, J = 1.6 Hz, 1H), 7.41 (d, J = 4.5 Hz, 1H), 7.31 (d, J = 1.6 Hz, 1H), 6.21 (s, 1H), 5.14 (s, 2H), 4.57 (s, 2H), 4.52 (s, 2H), 4.40 - 4.32 (m, 2H), 4.00 (s, 3H), 3.97 - 3.90 (m, 2H), 3.70 (s, 3H), 1.87 - 1.77 (m, 2H) |
| 108 (D) | 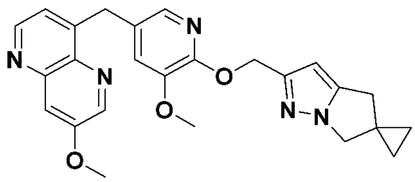 2'-(((3-methoxy-5-((7-methoxy-1,5-naphthyridin-4-yl)methyl)pyridin-2-yl)oxy)methyl)-4'H,6'H-spiro[cyclopropane-1,5'-pyrrolo[1,2-b]pyrazole] | 444.2 | $^1$H NMR(400 MHz, DMSO) δ 8.82 (dd, J = 6.9, 3.7 Hz, 2H), 7.79 (d, J = 2.9 Hz, 1H), 7.68 (d, J = 1.5 Hz, 1H), 7.41 (d, J = 4.5 Hz, 1H), 7.31 (d, J = 1.6 Hz, 1H), 6.03 (s, 1H), 5.18 (s, 2H), 4.52 (s, 2H), 3.99 (d, J = 5.3 Hz, 5H), 3.70 (s, 3H), 2.81 (s, 2H), 0.83 - 0.66 (m, 4H) |
| 109 (D) | 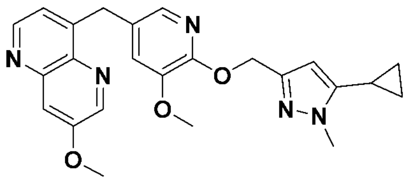 8-((6-((5-cyclopropyl-1-methyl-1H-pyrazol-3-yl)methoxy)-5-methoxypyridin-3-yl)methyl)-3-methoxy-1,5-naphthyridine | 432.1 | $^1$H NMR(400 MHz, DMSO) δ 8.81 (dd, J = 6.0, 3.7 Hz, 2H), 7.78 (d, J = 2.7 Hz, 1H), 7.66 (s, 1H), 7.40 (d, J = 4.4 Hz, 1H), 7.30 (s, 1H), 5.89 (s, 1H), 5.08 (s, 2H), 4.51 (s, 2H), 3.99 (s, 3H), 3.79 (s, 3H), 3.69 (s, 3H), 1.90 - 1.78 (m, 1H), 0.96 - 0.87 (m, 2H), 0.58 (q, J = 5.4 Hz, 2H). |
| 110 (D) | 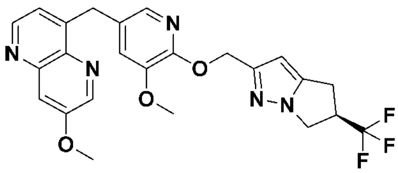 (R)-3-methoxy-8-((5-methoxy-6-((5-(trifluoromethyl)-5,6-dihydro-4H-pyrrolo[1,2-b]pyrazol-2-yl)methoxy)pyridin-3-yl)methyl)-1,5-naphthyridine | 486.1 | $^1$H NMR(400 MHz, DMSO) δ 8.82 (dd, *J* = 6.3, 3.7 Hz, 2H), 7.79 (d, *J* = 2.8 Hz, 1H), 7.67 (s, 1H), 7.41 (d, *J* = 4.4 Hz, 1H), 7.32 (s, 1H), 6.07 (s, 1H), 5.17 (s, 2H), 4.52 (s, 2H), 4.42 (dd, *J* = 11.3, 9.3 Hz, 1H), 4.16 (dd, *J* = 11.5, 5.4 Hz, 1H), 4.11 - 4.02 (m, 1H), 4.00 (s, 3H), 3.70 (s, 3H), 3.23 (dd, *J* = 16.5, 9.5 Hz, 1H), 2.94 (dd, *J* = 16.5, 5.1 Hz, 1H). |

(continued)

| Example No (Method) | Structure; IUPAC name | LC-MS (M+H) + | $^1$H NMR (ppm) |
|---|---|---|---|
| 111 (D) | 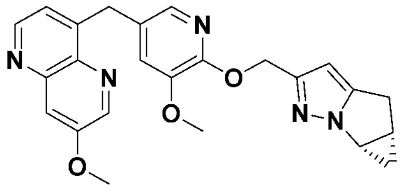 (4aS,5aS)-2-(((3-methoxy-5-((7-methoxy-1,5-naphthyridin-4-yl)methyl)pyridin-2-yl)oxy)methyl)-4,4a,5,5a-tetrahydrocyclopropa[4,5]pyrr olo[1,2-b] pyrazole | 430.2 | $^1$H NMR(400 MHz, DMSO) δ 8.82 (dd, *J* = 6.7, 3.6 Hz, 2H), 7.79 (d, *J* = 2.7 Hz, 1H), 7.67 (s, 1H), 7.40 (d, *J* = 4.4 Hz, 1H), 7.31 (s, 1H), 5.95 (s, 1H), 5.13 (s, 2H), 4.52 (s, 2H), 4.02 (d, *J* = 6.2 Hz, 1H), 4.00 (s, 3H), 3.69 (s, 3H), 3.05 (dd, *J* = 16.9, 6.6 Hz, 1H), 2.84 (d, *J* = 16.8 Hz, 1H), 2.18 (dt, *J* = 12.3, 6.1 Hz, 1H), 1.08 (dt, *J* = 8.4, 5.6 Hz, 1H), 0.30 (t, *J* = 4.2 Hz, 1H). |
| 112 (D) | 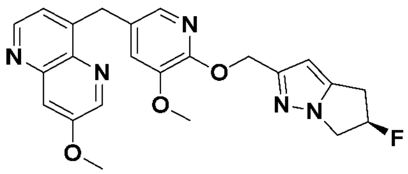 (R)-8-((6-((5-fluoro-5,6-dihydro-4H-pyrrolo[1,2-b]pyrazol-2-yl)methoxy)-5-methoxypyridin-3-yl)methyl)-3-methoxy-1,5-naphthyridine | 436.1 | $^1$H NMR(400 MHz, DMSO) δ 8.81 (dd, *J* = 6.7, 3.6 Hz, 2H), 7.78 (d, *J* = 2.7 Hz, 1H), 7.67 (s, 1H), 7.40 (d, *J* = 4.4 Hz, 1H), 7.31 (s, 1H), 6.10 (s, 1H), 5.82 (dt, *J* = 52.5, 4.6 Hz, 1H), 5.17 (s, 2H), 4.51 (s, 2H), 4.37 (ddd, *J* = 34.0, 13.2, 4.3 Hz, 1H), 4.24 (dd, *J* = 26.2, 13.2 Hz, 1H), 3.99 (s, 3H), 3.69 (s, 3H), 3.29 - 3.18 (m, 1H), 3.02 (dd, *J* = 26.8, 17.6 Hz, 1H) |
| 113 (D) | 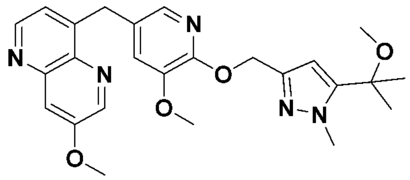 3-methoxy-8-((5-methoxy-6-((5-(2-methoxypropan-2-yl)-1-methyl-1H-pyrazol-3-yl)methoxy)pyridin-3-yl)methyl)-1,5-naphthyridine | 464.1 | $^1$H NMR(400 MHz, DMSO) δ 8.81-8.83(m,2H), 7.79(d,J=2.76,1H), 7.68(s,1H), 7.41(d,J=4.44,1H), 7.32(s,1H), 6.20(s,1H), 5.13(s,2H), 4.52(s,2H), 4.00(s,3H), 3.88(s,3H), 3.70(s,3H), 2.97(s,3H), 1.48(s,6H), |
| 114 (D) | 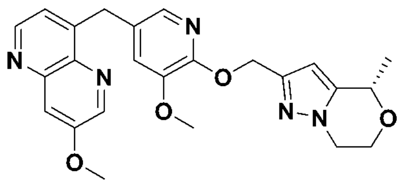 (S)-2-(((3-methoxy-5-((7-methoxy-1,5-naphthyridin-4-yl)methyl)pyridin-2-yl)oxy)methyl)-4-methyl-6,7-dihydro-4H-pyrazolo [5,1-c][1,4]oxazine Or 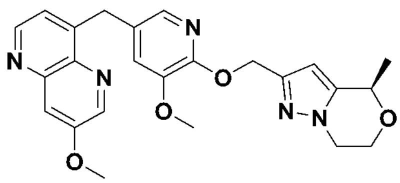 (R)-2-(((3-methoxy-5-((7-methoxy-1,5-naphthyridin-4-yl)methyl)pyridin-2-yl)oxy)methyl)-4-methyl-6,7-dihydro-4H-pyrazolo[5,1-c][1,4]oxazine | 448.1 | $^1$H NMR(400 MHz, DMSO) δ 8.81-8.83(m, 2H), 7.79(d, 1H), 7.67(d, 1H), 7.41(d, 1H), 7.31(d, 1H), 6.13(s, 1H), 5.18(d, 2H), 4.76-4.81(m, 1H), 4.52(s, 2H), 4.18-4.23(m, 1H), 4.03-4.05(m, 2H), 4.00(s, 3H), 3.87-3.93(m, 1H), 3.7(s, 3H), 1.42(d,3H) |

(continued)

| Example No (Method) | Structure; IUPAC name | LC-MS (M+H) + | $^1$H NMR (ppm) |
|---|---|---|---|
| 115 (D) | 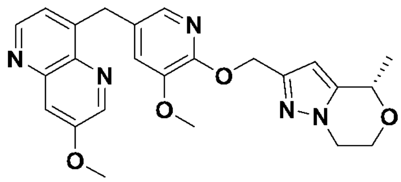 (S)-2-(((3-methoxy-5-((7-methoxy-1,5-naphthyridin-4-yl)methyl)pyridin-2-yl)oxy)methyl)-4-methyl-6,7-dihydro-4H-pyrazolo[5,1-c][1,4]oxazine Or 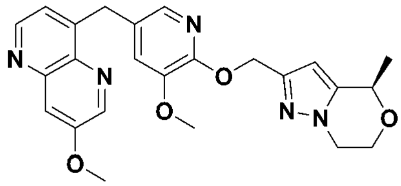 (R)-2-(((3-methoxy-5-((7-methoxy-1,5-naphthyridin-4-yl)methyl)pyridin-2-yl)oxy)methyl)-4-methyl-6,7-dihydro-4H-pyrazolo[5,1-c][1,4]oxazine | 448.1 | $^1$H NMR(400 MHz, DMSO) δ 8.81-8.83(m, 2H), 7.79(d, 1H), 7.67(d, 1H), 7.41(d, 1H), 7.31(d, 1H), 6.13(s, 1H), 5.18(d, 2H), 4.76-4.81(m, 1H), 4.52(s, 2H), 4.18-4.23(m, 1H), 4.03-4.05(m, 2H), 4.00(s, 3H), 3.87-3.93(m, 1H), 3.7(s, 3H), 1.42(d,3H). |
| 116 (J) | 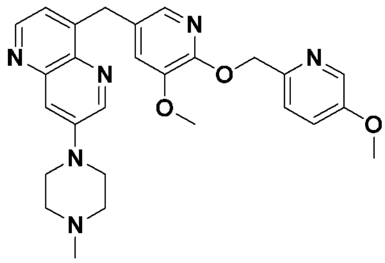 8-((5-methoxy-6-((5-methoxypyridin-2-yl)methoxy)pyridin-3-yl)methyl)-3-(4-methylpiperazin-1-yl)-1,5-naphthyridine | 487.1 | $^1$H NMR(400 MHz, DMSO) δ 9.03(d, 1H),8.7(d, 1H), 8.25(s, 1H), 7.63(s, 1H),7.51(s, 1H), 7.37(s, 2H),7.34(s, 1H), 7.28(d, 1H), 5.29(s, 2H), 4.47(s, 2H),3.81(s, 3H),3.73(s, 3H),3.41(s, 4H),2.6(s, 4H),2.27(s,3H). |
| 117 (D) | 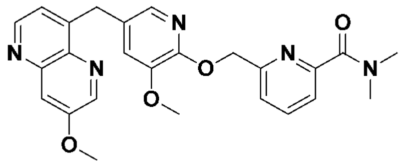 6-(((3-methoxy-5-((7-methoxy-1,5-naphthyridin-4-yl)methyl)pyridin-2-yl)oxy)methyl)-N,N-dimethylpicolinamide | 460.2 | $^1$H NMR(400 MHz, DMSO) δ8.82(t,2H), 7.90(t,1H), 7.78(d,J=2.64,1H), 7.62(s,1H), 7.45(d,J=7.76,2H), 7.40(t,2H), 5.41(s,2H), 4.51 (s,2H), 3.99(s,3H), 3.76(s,3H), 2.98(s,3H), 2.84(s,3H), |
| 118 (D) | 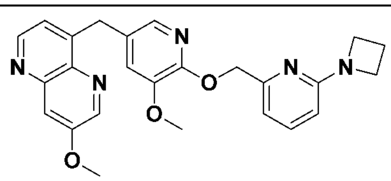 8-((6-((6-(azetidin-1-yl)pyridin-2-yl)methoxy)-5-methoxypyridin-3-yl)methyl)-3-methoxy-1,5-naphthyridine | 444.1 | $^1$H NMR(400 MHz, DMSO) δ 8.80-8.82(m, 2H), 7.79(s, 1H), 7.63(s, 1H), 7.36-7.4747(m, 3H), 6.59(d, 1H), 6.23(d, 1H), 5.19(s, 2H), 4.52(s, 2H),3.99(s, 3H), 3.90(t, 4H), 3.76(s, 3H), 3.26-3.33(m, 2H). |

(continued)

| Example No (Method) | Structure; IUPAC name | LC-MS (M+H) + | $^1$H NMR (ppm) |
|---|---|---|---|
| 119 (J) | 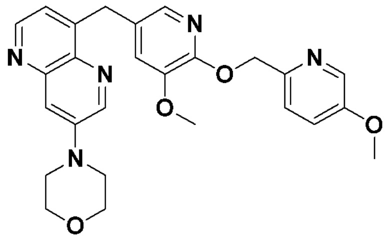 4-(8-((5-methoxy-6-((5-methoxypyridin-2-yl)methoxy)pyridin-3-yl)methyl)-1,5-naphthyridin-3-yl)morpholine | 475.1 | $^1$H NMR(400 MHz, DMSO) δ 9.04(s, 1H),8.03(d, 1H), 8.25(s, 1H), 7.63(s, 1H),7.53(s, 1H), 7.29-7.37(m, 4H),5.30(s, 2H),4.48(s, 2H),3.82(s, 7H),3.7(s, 3H),3.40(m,4H). |
| 120 (D) | 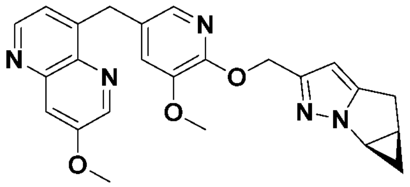 (4aR,5aR)-2-(((3-methoxy-5-((7-methoxy-1,5-naphthyridin-4-yl)methyl)pyridin-2-yl)oxy)methyl)-4,4a,5,5a-tetrahydrocyclopropa[4,5]pyrr olo[1,2-b] pyrazole | 430.1 | $^1$H NMR(400 MHz, DMSO) δ 8.82 (dd, *J* = 6.9, 3.6 Hz, 2H), 7.79 (d, *J* = 2.7 Hz, 1H), 7.67 (s, 1H), 7.40 (d, *J* = 4.4 Hz, 1H), 7.31 (s, 1H), 5.95 (s, 1H), 5.13 (s, 2H), 4.52 (s, 2H), 4.02 (d, *J* = 5.9 Hz, 1H), 4.00 (s, 3H), 3.69 (s, 3H), 3.05 (dd, *J* = 16.9, 6.5 Hz, 1H), 2.84 (d, *J* = 16.9 Hz, 1H), 2.18 (dt, *J* = 12.0, 6.0 Hz, 1H), 1.08 (dt, *J* = 8.6, 5.6 Hz, 1H), 0.30 (t, *J* = 4.2 Hz, 1H) |
| 121 (D) | 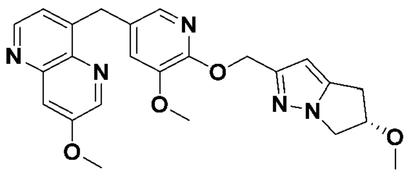 (S)-3-methoxy-8-((5-methoxy-6-((5-methoxy-5,6-dihydro-4H-pyrrolo[1,2-b]pyrazol-2-yl)methoxy)pyridin-3-yl)methyl)-1,5-naphthyridine | 448.1 | $^1$H NMR(400 MHz, DMSO) δ 8.82 (m, 2H), 7.79 (d, 1H), 7.67 (d, 1H), 7.40 (d, 1H), 7.30 (d, 1H), 6.02 (s, 1H), 5.16(s, 2H), 4.58 (m, 1H), 4.52(s, 2H), 4.25 (m, 1H), 4.01(m, 4H), 3.69 (s, 3H), 3.30 (s, 3H), 3.10 (m, 1H), 2.80 (m, 1H) |
| 122 (D) | 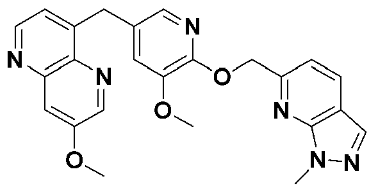 3-methoxy-8-((5-methoxy-6-((1-methyl-1H-pyrazolo [3,4-b]pyridin-6-yl)methoxy)pyridin-3-yl)methyl)-1,5-naphthyridine | 443.1 | $^1$H NMR(400 MHz, DMSO) δ 8.91 - 8.78 (m, 2H), 8.22 (d, J = 8.2 Hz, 1H), 8.12 (s, 1H), 7.78 (s, 1H), 7.63 (s, 1H), 7.40 (d, J = 7.2 Hz, 2H), 7.25 (d, J = 8.2 Hz, 1H), 5.51 (s, 2H), 4.52 (s, 2H), 4.04 (s, 3H), 3.99 (s, 3H), 3.77 (s, 3H). |
| 123 (D) | 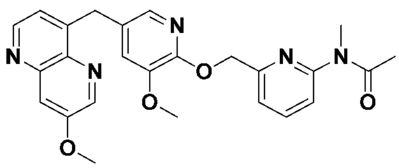 N-(6-(((3-methoxy-5-((7-methoxy-1,5-naphthyridin-4-yl)methyl)pyridin-2-yl)oxy)methyl)pyridin-2-yl)-N-methylacetamide | 460.1 | $^1$H NMR(400 MHz, DMSO) δ 8.80-8.83(m, 2H), 7.84(t, 1H), 7.79(s, 1H), 7.62(s, 1H), 7.37-7.41(m, 3H), 7.26(d, 1H), 5.37( s, 2H), 4.52(s, 2H),3.99(s, 3H), 3.76(s, 3H), 3.25(s, 3H), 1.99(s, 3H) . |

(continued)

| Example No (Method) | Structure; IUPAC name | LC-MS (M+H) + | $^1H$ NMR (ppm) |
|---|---|---|---|
| 124 (D) | 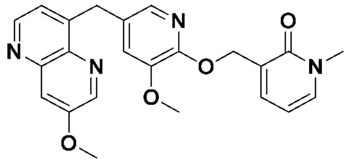 3-(((3-methoxy-5-((7-methoxy-1,5-naphthyridin-4-yl) methyl)pyridin-2-yl)oxy)methyl)-1-methylpyridin-2(1H)-one | 419.1 | $^1H$ NMR(400 MHz, DMSO) δ 8.82 (dd, J = 7.1, 3.7 Hz, 2H), 7.79 (d, J = 2.9 Hz, 1H), 7.68 (dd, J = 6.8, 2.0 Hz, 1H), 7.65 (d, J = 1.8 Hz, 1H), 7.46 - 7.38 (m, 2H), 7.34 (d, J = 1.8 Hz, 1H), 6.22 (t, J = 6.8 Hz, 1H), 5.12 (s, 2H), 4.52 (s, 2H), 4.00 (s, 3H), 3.74 (s, 3H), 3.46 (s, 3H). |
| 125 (D) | 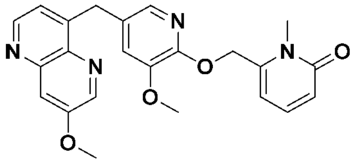 6-(((3-methoxy-5-((7-methoxy-1,5-naphthyridin-4-yl) methyl)pyridin-2-yl)oxy)methyl)-1-methylpyridin-2(1H)-one | 419.1 | $^1H$ NMR(400 MHz, DMSO) δ 8.82 (dd, J = 5.5, 3.7 Hz, 2H), 7.79 (d, J = 2.9 Hz, 1H), 7.70 (d, J = 1.7 Hz, 1H), 7.41 (dd, J = 6.0, 3.2 Hz, 2H), 7.39 - 7.35 (m, 1H), 6.38 (dd, J = 16.2, 8.0 Hz, 2H), 5.30 (s, 2H), 4.54 (s, 2H), 4.00 (s, 3H), 3.75 (s, 3H), 3.43 (s, 3H). |
| 126 (J) | 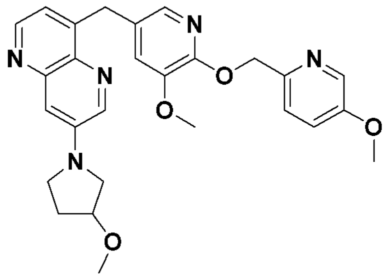 8-((5-methoxy-6-((5-methoxypyridin-2-yl)methoxy) pyridin-3-yl)methyl)-3-(3-methoxypyrrolidin-1-yl)-1,5-naphthyridine | 488.2 | $^1H$ NMR(400 MHz, DMSO) δ 8.71(d, 1H), 8.66(d, 1H), 8.34(d, 1H), 7.73 (s, 1H), 7.44 (d, 1H), 7.27 (d, 1H), 7.23(d, 1H), 7.06-7.10(m, 2H), 5.56(s, 2H), 4.55(s, 2H), 4.25(s,1H), 3.89(d,3H), 3.84(d,3H), 3.66-3.72(m,2H), 3.60-3.64(m,2H), 3.46(s,3H), 2.32-2.33(m, 1H), 2.16-2.27(m,1H) |
| 127 (J) | 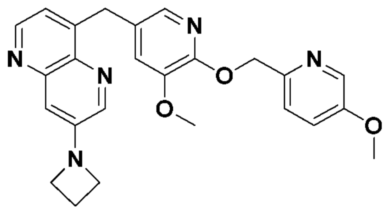 3-(azetidin-1-yl)-8-((5-methoxy-6-((5-methoxypyridin-2-yl)methoxy)pyridin-3-yl)methyl)-1,5-naphthyridine | 444.1 | $^1H$ NMR(400 MHz, DMSO) δ 8.66(s, 1H),8.47(s, 1H), 8.24(s, 1H), 7.62(s, 1H),7.37(s, 3H), 7.21(s, 1H),7.02(s, 1H),5.29(s, 2H),4.45(s, 2H),4.06(s, 4H),3.77(d,6H),2.40(s,2H) |

(continued)

| Example No (Method) | Structure; IUPAC name | LC-MS (M+H) + | $^1$H NMR (ppm) |
|---|---|---|---|
| 128 (D) | 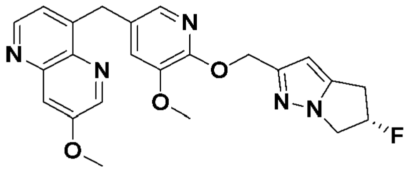 (S)-8-((6-((5-fluoro-5,6-dihydro-4H-pyrrolo[1,2-b]pyrazol-2-yl)methoxy)-5-methoxypyridin-3-yl)methyl)-3-methoxy-1,5-naphthyridine | 436.1 | $^1$H NMR(400 MHz, DMSO) δ 8.82 (dd, *J* = 6.7, 3.7 Hz, 2H), 7.79 (d, *J* = 2.9 Hz, 1H), 7.67 (d, *J* = 1.8 Hz, 1H), 7.41 (d, *J* = 4.5 Hz, 1H), 7.31 (d, *J* = 1.8 Hz, 1H), 6.10 (s, 1H), 5.83 (dt, *J* = 52.5, 4.8 Hz, 1H), 5.18 (s, 2H), 4.52 (s, 2H), 4.37 (ddd, *J* = 34.0, 13.2, 4.4 Hz, 1H), 4.25 (dd, *J* = 26.1, 13.2 Hz, 1H), 4.00 (s, 3H), 3.70 (s, 3H), 3.31 - 3.17 (m, 1H), 3.02 (dd, *J* = 26.8, 17.6 Hz, 1H) |
| 129 (D) | 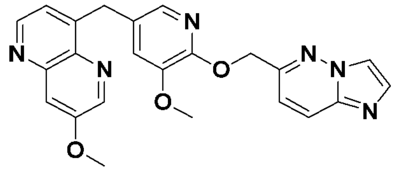 8-((6-(imidazo[1,2-b]pyridazin-6-ylmethoxy)-5-methoxypyridin-3-yl)methyl)-3-methoxy-1,5-naphthyridine | 429.1 | $^1$H NMR(400 MHz, DMSO) δ 8.81 (dd, *J* = 6.0, 3.7 Hz, 2H), 8.28 (s, 1H), 8.12 (d, *J* = 9.4 Hz, 1H), 7.78 (d, *J* = 1.4 Hz, 2H), 7.65 (d, *J* = 1.7 Hz, 1H), 7.40 (dd, *J* = 5.5, 3.1 Hz, 2H), 7.28 (d, *J* = 9.4 Hz, 1H), 5.45 (s, 2H), 4.52 (s, 2H), 3.99 (s, 3H), 3.75 (s, 3H). |
| 130 (E) | 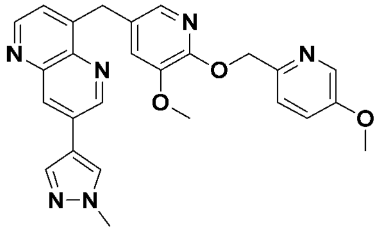 8-((5-methoxy-6-((5-methoxypyridin-2-yl)methoxy) pyridin-3-yl)methyl)-3-(1-methyl-1H-pyrazol-4-yl)-1,5-naphthyridine | 469.1 | $^1$H NMR(400 MHz, DMSO) δ 9.36 (d, J = 2.2 Hz, 1H), 8.86 (d, J = 4.4 Hz, 1H), 8.54 (d, J = 2.2 Hz, 1H), 8.52 (s, 1H), 8.25 (t, J = 1.7 Hz, 1H), 8.23 (s, 1H), 7.68 (d, J = 1.7 Hz, 1H), 7.48 (d, J = 4.4 Hz, 1H), 7.38 (d, J = 1.7 Hz, 3H), 5.31 (s, 2H), 4.54 (s, 2H), 3.94 (s, 3H), 3.82 (s, 3H), 3.75 (s, 3H). |
| 131 (J) | 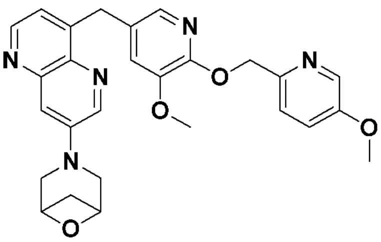 3-(8-((5-methoxy-6-((5-methoxypyridin-2-yl)methoxy)pyridin-3-yl)methyl)-1,5-naphthyridin-3-yl)-6-oxa-3-azabicyclo[3.1.1]heptane | 487.1 | $^1$H NMR(400 MHz, DMSO) δ 8.90(d, 1H), 8.70(d, 1H), 8.25(s, 1H), 7.64(d, 1H), 7.34-7.37(m, 4H), 7.23(d, 1H), 5.30(s, 2H), 4.80(d, 2H), 4.48(s, 2H), 3.79-3.83(m,5H), 3.74(s, 3H), 3.64-3.67(d,2H), 3.20-3.22(m,1H), 1.97-1.99(s,1H) |

(continued)

| Example No (Method) | Structure; IUPAC name | LC-MS (M+H) + | $^1H$ NMR (ppm) |
|---|---|---|---|
| 132 (D) | 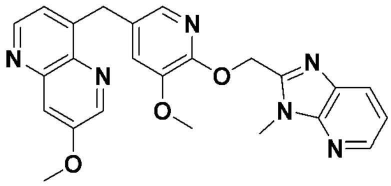 3-methoxy-8-((5-methoxy-6-((3-methyl-3H-imidazo [4,5-b]pyridin-2-yl)methoxy)pyridin-3-yl)methyl)-1,5-naphthyridine | 443.2 | $^1H$ NMR(400 MHz, DMSO) δ 8.81-8.83(m,2H), 8.41(d,J=3.4,1H), 8.04(d,J=6.88,1H), 7.79(d,J=2.8,1H), 7.69(s,1H), 7.41(t,2H), 7.28-7.31(m,1H), 5.59(s,2H), 4.54(s,2H), 4.00(s,3H), 3.87(s,3H), 3.74(s,3H), |
| 133 (E) | 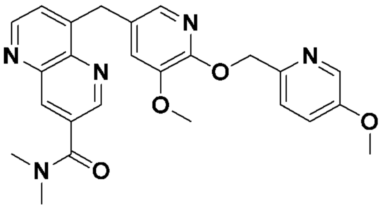 8-((5-methoxy-6-((5-methoxypyridin-2-yl)methoxy) pyridin-3-yl)methyl)-N,N-dimethyl-1,5-naphthyridine-3-carboxamide | 460.1 | $^1H$ NMR(400 MHz, DMSO) δ 9.09(s,1H), 8.95(d,J=4.2,1H), 8.45(s,1H), 8.25(s, 1H), 7.63(t,2H), 7.39(s,3H), 5.31(s,2H), 4.57(s,2H), 3.82(s,3H), 3.75(s,3H), 3.09(s,3H), 3.03(s,3H), |
| 134 (K) | 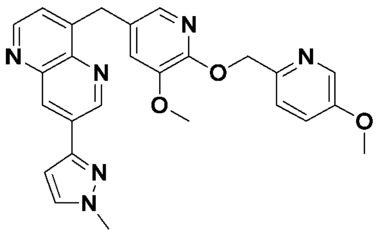 8-((5-methoxy-6-((5-methoxypyridin-2-yl)methoxy) pyridin-3-yl)methyl)-3-(1-methyl-1H-pyrazol-3-yl)-1,5-naphthyridine | 469.1 | $^1H$ NMR(400 MHz, DMSO) δ 9.55 (d, J = 2.1 Hz, 1H), 8.89 (d, J = 4.4 Hz, 1H), 8.68 (d, J = 2.1 Hz, 1H), 8.25 (t, J = 1.7 Hz, 1H), 7.88 (d, J = 2.2 Hz, 1H), 7.69 (d, J = 1.7 Hz, 1H), 7.51 (d, J = 4.4 Hz, 1H), 7.39 (dd, J = 4.9, 1.8 Hz, 3H), 7.10 (d, J = 2.3 Hz, 1H), 5.30 (s, 2H), 4.56 (s, 2H), 3.98 (s, 3H), 3.81 (s, 3H), 3.74 (s, 3H) |
| 135 (J) | 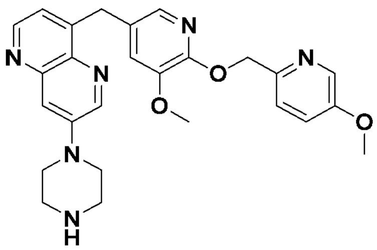 8-((5-methoxy-6-((5-methoxypyridin-2-yl)methoxy) pyridin-3-yl)methyl)-3-(piperazin-1-yl)-1,5-naphthyridine | 473.2 | $^1H$ NMR(400 MHz, DMSO) δ 9.04(d, 1H), 8.71(d, 1H), 8.26(s, 1H), 7.65(s, 1H), 7.49(d, 1H), 7.39(s, 2H), 7.36(s, 1H), 7.28(s, 1H), 5.31(s, 2H), 4.48(s, 2H), 3.83(s, 3H), 3.75(s,3H), 3.30(s,4H), 2.91(m,4H) |

(continued)

| Example No (Method) | Structure; IUPAC name | LC-MS (M+H) + | $^1H$ NMR (ppm) |
|---|---|---|---|
| 136 (D) | 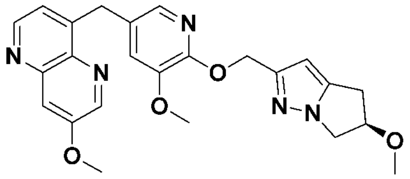 (R)-3-methoxy-8-((5-methoxy-6-((5-methoxy-5,6-dihydro-4H-pyrrolo[1,2-b]pyrazol-2-yl)methoxy)pyridin-3-yl)methyl)-1,5-naphthyridine | 448.1 | $^1H$ NMR(400 MHz, DMSO) δ 8.82 (dd, J = 6.8, 3.7 Hz, 2H), 7.79 (d, J = 2.9 Hz, 1H), 7.67 (d, J = 1.7 Hz, 1H), 7.40 (d, J = 4.5 Hz, 1H), 7.31 (d, J = 1.7 Hz, 1H), 6.02 (s, 1H), 5.16 (s, 2H), 4.58 (ddd, J = 8.5, 6.0, 2.4 Hz, 1H), 4.52 (s, 2H), 4.25 (dd, J = 11.8, 5.7 Hz, 1H), 4.02 (d, J = 2.4 Hz, 1H), 4.00 (s, 3H), 3.69 (s, 3H), 3.30 (s, 3H), 3.11 (dd, J = 16.7, 6.4 Hz, 1H), 2.79 (dd, J = 16.6, 2.2 Hz, 1H). |
| 137 (K) | 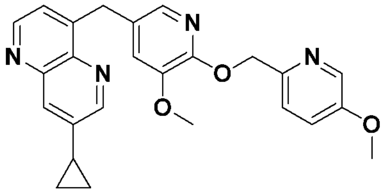 3-cyclopropyl-8-((5-methoxy-6-((5-methoxypyridin-2-yl)methoxy)pyridin-3-yl)methyl)-1,5-naphthyridine | 430.1 | $^1H$ NMR(400 MHz, DMSO) δ 8.91(d, 1H), 8.83(d, 1H), 8.25(d, 1H), 8.00(d, 1H), 7.65(d, 1H), 7.47(d, 1H), 7.37(m, 3H), 5.30(s, 2H), 4.52(s , 2H), 3.82(s, 3H), 3.74(s,3H), 2.24(m,1H), 1.24(m,2H), 0.98(m,2H) |
| 138 (K) | 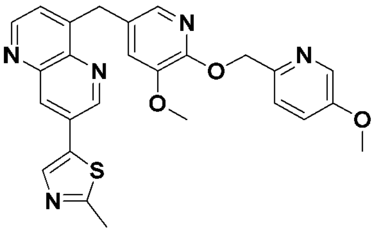 5-(8-((5-methoxy-6-((5-methoxypyridin-2-yl)methoxy)pyridin-3-yl)methyl)-1,5-naphthyridin-3-yl)-2-methylthiazole | 486.1 | $^1H$ NMR(400 MHz, DMSO) δ 9.41(d, 1H), 8.92(d, 1H), 8.54(d, 1H), 8.46(d, 1H), 8.25(d, 1H), 7.67(d, 1H), 7.58(d, 1H), 7.38(d, 3H), 5.30(s, 2H), 4.56(s,2H), 3.82(s, 3H), 3.75(s,3H), 2.76(s,3H) |
| 139 (K) | 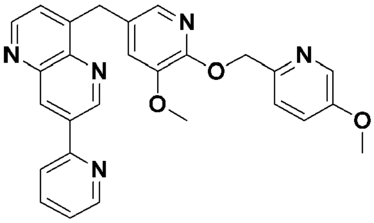 8-((5-methoxy-6-((5-methoxypyridin-2-yl)methoxy) pyridin-3-yl)methyl)-3-(pyridin-2-yl)-1,5-naphthyridine | 466.1 | $^1H$ NMR(400 MHz, DMSO) δ 9.80(s, 1H), 9.01(s, 1H), 8.95(s, 1H), 8.81(s, 1H), 8.33(d, 1H), 8.25(s, 1H), 7.99-8.03(m, 1H), 7.70(s, 1H), 7.60(s, 1H), 7.49-7.52(m, 1H), 7.38-7.41(m, 3H), 5.31(s, 2H), 4.60(s, 2H), 3.82(s, 3H), 3.75(s, 3H). |

(continued)

| Example No (Method) | Structure; IUPAC name | LC-MS (M+H) + | $^1$H NMR (ppm) |
|---|---|---|---|
| 140 (K) | 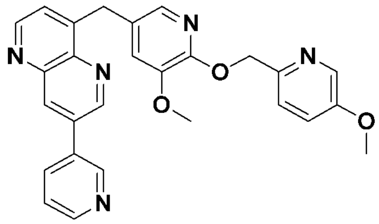 8-((5-methoxy-6-((5-methoxypyridin-2-yl)methoxy) pyridin-3-yl)methyl)-3-(pyridin-3-yl)-1,5-naphthyridine | 466.1 | $^1$H NMR(400 MHz, DMSO) δ 9.49(s, 1H), 9.20(s, 1H), 8.96(s, 1H), 8.78(s, 1H), 8.71(d, 1H), 8.39-8.42(m, 1H), 8.25(s, 1H), 7.68(s, 1H), 7.59-7.62(m, 2H), 7.38-7.41(m, 3H), 5.31(s, 2H), 4.60(s, 2H), 3.82(s, 3H), 3.76(s, 3H). |
| 141 (J) | 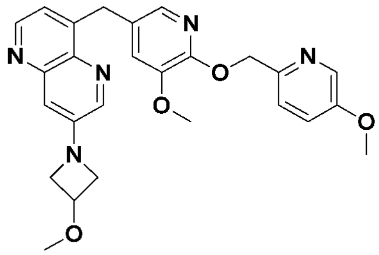 8-((5-methoxy-6-((5-methoxypyridin-2-yl)methoxy) pyridin-3-yl)methyl)-3-(3-methoxyazetidin-1-yl)-1,5-naphthyridine | 474.1 | $^1$H NMR(400 MHz, DMSO) δ 8.68(s, 1H), 8.51(d, 1H), 8.25(s, 1H), 7.63(s, 1H), 7.33-7.37(m, 3H), 7.22(s, 1H), 7.09(s, 1H), 5.30(s, 2H), 4.40-4.45(m, 3H), 4.27-4.31(m, 2H), 3.82-.3.90(m, 2H), 3.82(s, 3H), 3.74(s, 3H), 3.29(s, 3H) . |
| 142 (J) | 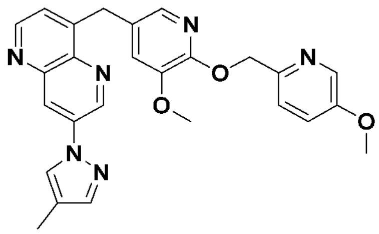 8-((5-methoxy-6-((5-methoxypyridin-2-yl)methoxy) pyridin-3-yl)methyl)-3-(4-methyl-1H-pyrazol-1-yl)-1,5-naphthyridine | 469.1 | $^1$H NMR(400 MHz, DMSO) δ 9.65(s, 1H), 8.92(s, 1H), 8.65-8.67(m, 2H), 8.25(d, 1H), 7.76(s, 1H), 7.68(s, 1H), 7.54(s, 1H), 7.38(s, 3H), 5.31(s, 2H), 4.57(s, 2H), 3.82(s, 3H), 3.75(s, 3H), 2.17(s, 3H) . |
| 143 (J) | 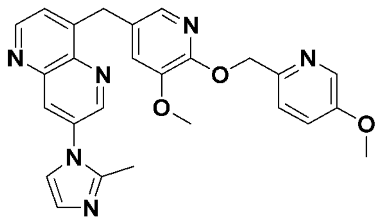 8-((5-methoxy-6-((5-methoxypyridin-2-yl)methoxy) pyridin-3-yl)methyl)-3-(2-methyl-1H-imidazol-1-yl)-1,5-naphthyridine | 469.1 | $^1$H NMR(400 MHz, DMSO) δ 9.21(s, 1H), 8.99(s, 1H), 8.57(s, 1H), 8.25(d, 1H), 7.59-7.68(m, 3H), 7.38-7.40(m, 3H), 7.03(s, 1H), 5.31(s, 2H), 4.60(s, 2H), 3.82(s, 3H), 3.76(s, 3H), 2.43(s, 3H) . |

(continued)

| Example No (Method) | Structure; IUPAC name | LC-MS (M+H) + | $^1$H NMR (ppm) |
|---|---|---|---|
| 144 (J) | 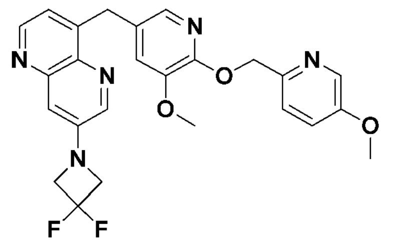 3-(3,3-difluoroazetidin-1-yl)-8-((5-methoxy-6-((5-methoxypyridin-2-yl)methoxy)pyridin-3-yl) methyl)-1,5-naphthyridine | 480.1 | $^1$H NMR(400 MHz, DMSO) δ 8.74(s, 1H), 8.60(d, 1H), 8.25(s, 1H), 7.63(s, 1H), 7.32-7.37(m, 5H), 5.30(s, 2H), 4.54-4.60(m, 4H), 4.48(s, 2H), 3.82(s, 3H), 3.74(s, 3H) . |
| 145 (D) | 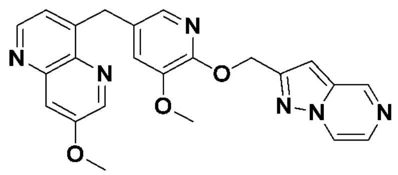 3-methoxy-8-((5-methoxy-6-(pyrazolo[1,5-a]pyrazin-2-ylmethoxy)pyridin-3-yl)methyl)-1,5-naphthyridine | 429.1 | $^1$H NMR(400 MHz, DMSO) δ 9.00 (d, J = 0.8 Hz, 1H), 8.85 - 8.74 (m, 2H), 8.36 (d, J = 4.7 Hz, 1H), 7.85 (d, J = 4.8 Hz, 1H), 7.73 (s, 1H), 7.67 (d, J = 2.8 Hz, 1H), 7.24 (d, J = 4.5 Hz, 1H), 7.11 (d, J = 1.4 Hz, 1H), 6.89 (s, 1H), 5.73 (s, 2H), 4.58 (s, 2H), 4.02 (s, 3H), 3.81 (s, 3H). |
| 146 (E) | 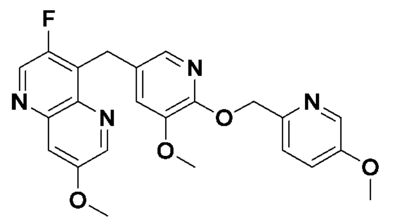 3-fluoro-7-methoxy-4-((5-methoxy-6-((5-methoxypyridin-2-yl)methoxy)pyridin-3-yl)methyl)-1,5-naphthyridine | 437.1 | $^1$H NMR(400 MHz, DMSO) δ8.99(s, 1H), 7.89(d, 1H), 8.23(d, 1H), 7.86(d, 1H), 7.53(s, 1H), 7.28-7.38(m, 3H), 5.27(s, 2H), 4.52(s, 2H), 3.99(s, 3H), 3.81(s, 3H), 3.71(s, 3H). |
| 147 (D) | 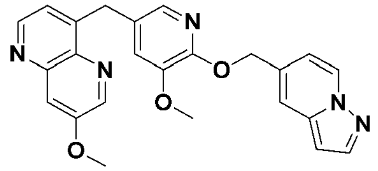 3-methoxy-8-((5-methoxy-6-(pyrazolo[1,5-a]pyridin-5-ylmethoxy)pyridin-3-yl)methyl)-1,5-naphthyridine | 428.1 | $^1$H NMR(400 MHz, DMSO) δ 8.80-8.83(m,2H), 8.64(d, 1H), 7.98 (d 1H), 7.78 (d,1H), 7.67 (s, 2H), 7.37-7.41(m, 2H), 6.88-6.90 (m, 1H), 6.59 (d, 1H), 5.36 (s, 2H), 4.53(s,2H), 4.00(s,3H), 3.76(s,3H) |
| 148 (D) | 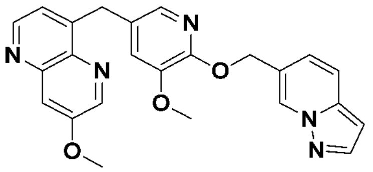 3-methoxy-8-((5-methoxy-6-(pyrazolo[1,5-a]pyridin-6-ylmethoxy)pyridin-3-yl)methyl)-1,5-naphthyridine | 428.1 | $^1$H NMR(400 MHz, DMSO) δ 8.82 (dd, J = 6.9, 3.7 Hz, 2H), 8.77 (s, 1H), 8.00 (d, J = 2.1 Hz, 1H), 7.79 (d, J = 2.8 Hz, 1H), 7.69 (d, J = 9.0 Hz, 2H), 7.40 (d, J = 4.4 Hz, 1H), 7.35 (d, J = 1.3 Hz, 1H), 7.28 (d, J = 9.1 Hz, 1H), 6.61 (d, J = 1.5 Hz, 1H), 5.34 (s, 2H), 4.53 (s, 2H), 4.00 (s, 3H), 3.72 (s, 3H). |

(continued)

| Example No (Method) | Structure; IUPAC name | LC-MS (M+H) + | $^1$H NMR (ppm) |
|---|---|---|---|
| 149 (D) | 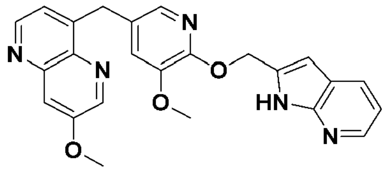 8-((6-((1H-pyrrolo[2,3-b]pyridin-2-yl)methoxy)-5-methoxypyridin-3-yl)methyl)-3-methoxy-1,5-naphthyridine | 428.1 | $^1$H NMR(400 MHz, DMSO) δ 11.78(s, 1H), 8.81-8.84(m, 2H), 8.18-8.20(m, 1H), 7.91(d, 1H), 7.79(d, 1H), 7.71(d, 1H), 7.35-7.41(m, 2H),7.02-7.06(m, 1H), 6.48(d, 1H), 5.40(s, 2H), 4.54(s, 2H), 4.00(s, 3H), 3.71(s, 3H). |
| 150 (L) | 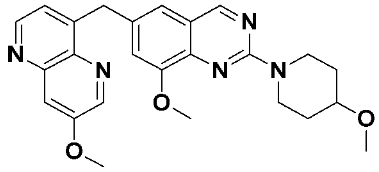 8-methoxy-6-((7-methoxy-1,5-naphthyridin-4-yl) methyl)-2-(4-methoxypiperidin-1-yl)quinazoline | 446.1 | $^1$H NMR(400 MHz, DMSO) δ 9.03 (s, 1H), 8.82 (t, J = 4.0 Hz, 2H), 7.79 (d, J = 2.9 Hz, 1H), 7.42 (d, J = 4.5 Hz, 1H), 7.26 (d, J = 1.5 Hz, 1H), 7.19 (d, J = 1.4 Hz, 1H), 4.66 (s, 2H), 4.31 (dt, J = 9.7, 4.4 Hz, 2H), 4.00 (s, 3H), 3.86 (s, 3H), 3.50 - 3.37 (m, 3H), 3.29 (s, 3H), 1.94 - 1.84 (m, 2H), 1.46 - 1.35 (m, 2H). |
| 151 (N) | 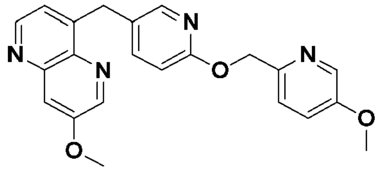 3-methoxy-8-((6-((5-methoxypyridin-2-yl)methoxy) pyridin-3-yl)methyl)-1,5-naphthyridine | 389.1 | $^1$H NMR(400 MHz, $CDCl_3$) δ 8.78 (t, J = 3.7 Hz, 2H), 8.33 (d, J = 2.7 Hz, 1H), 8.16 (d, J = 2.0 Hz, 1H), 7.67 (d, J = 2.8 Hz, 1H), 7.55 (dd, J = 8.5, 2.4 Hz, 1H), 7.40 (d, J = 8.6 Hz, 1H), 7.21 (dd, J = 8.5, 2.9 Hz, 2H), 6.79 (d, J = 8.5 Hz, 1H), 5.44 (s, 2H), 4.57 (s, 2H), 4.02 (s, 3H), 3.88 (s, 3H) |
| 152 (L) | 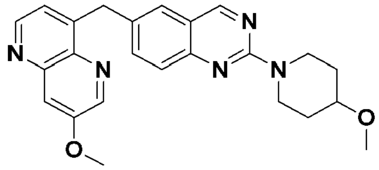 6-((7-methoxy-1,5-naphthyridin-4-yl)methyl)-2-(4-methoxypiperidin-1-yl)quinazoline | 416.1 | $^1$H NMR(400 MHz, $CDCl_3$) δ 8.93 (s, 1H), 8.79 (t, J = 3.0 Hz, 2H), 7.68 (d, J = 2.8 Hz, 1H), 7.63 (dd, J = 8.7, 1.9 Hz, 1H), 7.53 (d, J = 10.0 Hz, 2H), 7.22 (d, J = 4.5 Hz, 1H), 4.74 (s, 2H), 4.54 - 4.37 (m, 2H), 4.02 (s, 3H), 3.51 (dt, J = 8.3, 5.7 Hz, 3H), 3.42 (s, 3H), 2.06 - 1.93 (m, 2H), 1.72 - 1.63 (m, 2H) |
| 153 (L) | 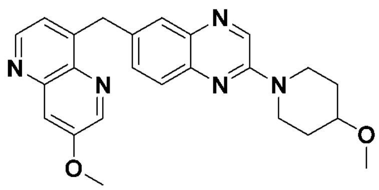 6-((7-methoxy-1,5-naphthyridin-4-yl)methyl)-2-(4-methoxypiperidin-1-yl)quinoxaline | 417.1 | $^1$H NMR(400 MHz, DMSO) δ 8.78-8.83(m, 3H), 7.70(s, 1H), 7.78(s, 1H), 7.45-7.57(m, 3H), 4.72(s, 2H), 4.09-4.15(m, 2H), 4.00(s, 3H), 3.37-3.49( m, 3H), 3.29(s, 3H), 1.91-1.95(m, 2H), 1.44-1.52(m, 2H). |

(continued)

| Example No (Method) | Structure; IUPAC name | LC-MS (M+H) + | $^1H$ NMR (ppm) |
|---|---|---|---|
| 154 (M) | 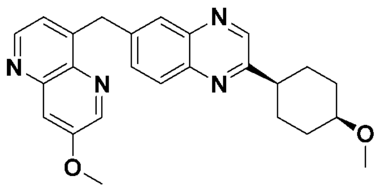 6-((7-methoxy-1,5-naphthyridin-4-yl) methyl)-2-((1s,4s)-4-methoxycyclohexyl)quinoxalin e Or 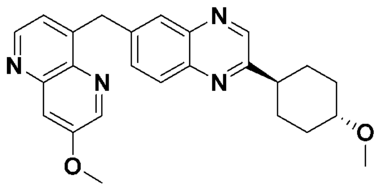 6-((7-methoxy-1,5-naphthyridin-4-yl) methyl)-2-((1r,4r)-4-methoxycyclohexyl)quinoxalin e | 415.2 | $^1H$ NMR(400 MHz, DMSO) δ 8.83-8.84(m, 3H), 7.93-7.95(m, 2H), 7.77-7.80(m, 2H), 7.49(d, 1H), 4.83(s, 2H), 4.00(s, 3H), 3.50(s, 1H), 3.25(s, 3H), 2.99-3.05( m, 1H), 1.86-1.99(m, 4H), 1.66-1.70(m, 2H), 1.53-1.60(m, 2H) . |
| 155 (M) | 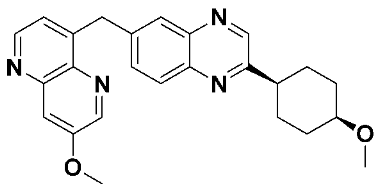 6-((7-methoxy-1,5-naphthyridin-4-yl) methyl)-2-((1s,4s)-4-methoxycyclohexyl)quinoxalin e Or 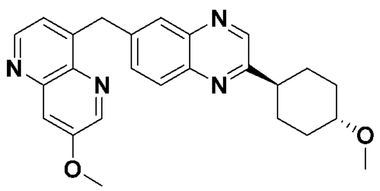 6-((7-methoxy-1,5-naphthyridin-4-yl) methyl)-2-((1r,4r)-4-methoxycyclohexyl)quinoxalin e | 415.2 | $^1H$ NMR(400 MHz, DMSO) δ8.82-8.87(m, 3H), 7.91-7.94(m, 2H), 7.77-7.81(m, 2H), 7.49(d, 1H), 4.83(s, 2H), 4.00(s, 3H), 3.28(s, 3H), 3.19-3.25(m,1H), 2.92-2.98( m, 1H), 2.13-2.16(m, 2H), 1.97-2.01(m, 2H), 1.67-1.71(m, 2H), 1.24-1.28(m, 2H) . |
| 156 (O) | 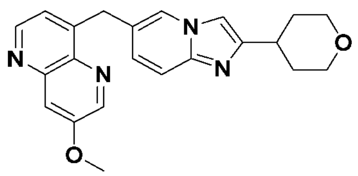 3-methoxy-8-((2-(tetrahydro-2H-pyran-4-yl)imidazo [1,2-a]pyridin-6-yl)methyl)-1,5-naphthyridine | 375.1 | $^1H$ NMR(400 MHz, DMSO) δ 8.82 (s, 2H), 8.38 (s, 1H), 7.80 (s, 1H), 7.65 (s, 1H), 7.44 (d, *J* = 4.2 Hz, 1H), 7.39 (d, *J* = 9.0 Hz, 1H), 7.15 (d, *J* = 9.2 Hz, 1H), 4.56 (s, 2H), 4.00 (s, 3H), 3.91 (d, *J* = 10.6 Hz, 2H), 3.45 (s, 2H), 2.90 (s, 1H), 1.89 (d, *J* = 13.1 Hz, 2H), 1.66 (dd, *J* = 20.8, 11.2 Hz, 2H). |

(continued)

| Example No (Method) | Structure; IUPAC name | LC-MS (M+H) + | $^1H$ NMR (ppm) |
|---|---|---|---|
| 157 (M) | 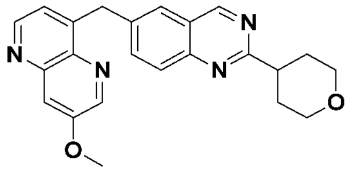 6-((7-methoxy-1,5-naphthyridin-4-yl)methyl)-2-(tetrahydro-2H-pyran-4-yl)quinazoline | 387.1 | $^1H$ NMR(400 MHz, DMSO) δ 9.47 (s, 1H), 8.82 (dd, J = 9.0, 3.6 Hz, 2H), 7.96 (d, J = 7.0 Hz, 2H), 7.87 (d, J = 9.2 Hz, 1H), 7.80 (d, J = 2.8 Hz, 1H), 7.46 (d, J = 4.4 Hz, 1H), 4.81 (s, 2H), 3.99 (s, 4H), 3.95 (s, 1H), 3.49 (td, J = 11.2, 3.0 Hz, 2H), 3.19 (dt, J = 9.8, 5.4 Hz, 1H), 1.99 - 1.84 (m, 4H). |
| 158 (O) | 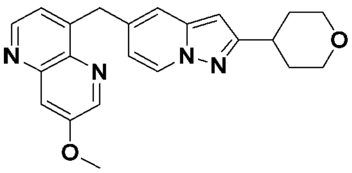 3-methoxy-8-((2-(tetrahydro-2H-pyran-4-yl)pyrazolo [1,5-a]pyridin-5-yl)methyl)-1,5-naphthyridine | 375.2 | $^1H$ NMR(400 MHz, DMSO) δ 8.80-8.83(m, 2H), 8.46(d, 1H), 7.79(d, 1H), 7.41-7.47(m, 2H), 6.73(d, 1H), 6.32(s, 1H), 4.59(s, 2H), 3.99(s, 3H), 3.89-3.92(m, 2H), 3.42-3.48(m,2H), 2.95-3.00( m, 1H), 1.84-1.87(m, 2H), 1.65-1.75(m, 2H) |

**Biochemical assays**

To perform the enzymatic step, working solutions were prepared in enzymatic buffer (12.5nM SEB, 1mM DTT, 5mM $MgCl_2$, 1mM $MnCl_2$). The reaction was carried out in a 384-well plate with 2.5μL inhibitor or enzymatic buffer, 2.5μL recombinant human protein (CSF-1R,c-Kit, PDGFRβ and FLT3) and 5μL substrate/ATP mixture at room temperature for 1 hour. After addition of detection mixture (5μl Sa-XL 665 and 5μl TK-antibody-Cryptate), the plate is sealed and incubated at room temperature for 1 hour. Detection was performed with Spark reader (Tecan) using standard HTRF protocol settings. $IC_{50}$ determinations were performed using GraphPad Prism software (GraphPad, Inc.). The selectivity fold was determined by the $IC_{50}$ of other kinases (cKit, FLT3, PDGFR-1β) and a multiple of the $IC_{50}$ of CSF-1R.

**Cell proliferation assay**

Ba/F3_CSF-1R(stably expressing human CSF-1R kinase) was cultured in RPMI1640 medium supplemented with 10% FBS and 100ng/ml recombinant human CSF-1. EOC2 was cultured in DMEM medium supplemented with 10% FBS and 100ng/ml recombinant mouse CSF-1. Cells were plated in 384-well plates (1 x $10^3$ cells/well for Ba/F3_CSF1R cell and 5 x $10^2$ cells/well for EOC2 cell) for 24 hours, and then treated with compounds for 72 hours. Cell growth rate was determined using the CellCounting-Lite luciferase-based ATP detection assay (Vazyme) following the manufacturer's protocol. $IC_{50}$ determinations were performed using GraphPad Prism software (GraphPad, Inc.).

Biological Activity Data

The enzymatic and cellular inhibitory activity for representative compounds disclosed herein are presented in Table 2 below.

Table 2

| Example No | CSF-1R $IC_{50}$ (nM) | CSF-1R BaF3 $EC_{50}$ (nM) | ECO2 $EC_{50}$ (nM) |
|---|---|---|---|
| 1 | 7.5 | 42.6 | 42 |
| 2 | 13.4 | 75 | 126 |
| 3 | 26 | 89.2 | N.A. |
| 4 | 8.7 | 44.6 | 22.6 |

(continued)

| Example No | CSF-1R $IC_{50}$ (nM) | CSF-1R BaF3 $EC_{50}$ (nM) | ECO2 $EC_{50}$ (nM) |
|---|---|---|---|
| 5 | 21 | 263 | 310 |
| 6 | 8.2 | 551 | 592 |
| 7 | 5.3 | 305 | 328 |
| 8 | 81.4 | 546 | N.A. |
| 9 | 169 | 901 | N.A. |
| 10 | 118 | >1000 | N.A. |
| 11 | 149 | >1000 | N.A. |
| 12 | 299 | > 1000 | N.A. |
| 13 | 9.8 | 176 | 151 |
| 14 | 118 | 157 | 132 |
| 15 | 9.8 | 437 | 253 |
| 16 | 2.1 | 61.7 | 39.2 |
| 17 | 5.7 | 185 | 180 |
| 18 | 9.4 | 196 | 272 |
| 19 | 47.6 | >1000 | N.A. |
| 20 | 11 | 134 | 130 |
| 21 | 12.6 | 509 | 492 |
| 22 | 64 | >1000 | N.A. |
| 23 | 8 | 323 | N.A. |
| 24 | 25 | 177 | 239 |
| 25 | 40 | 583 | N.A. |
| 26 | 5 | 283 | N.A. |
| 27 | 117 | 207 | N.A. |
| 28 | 25.7 | 356 | N.A.. |
| 29 | 73.9 | 873 | N.A.. |
| 30 | 7.9 | 214 | 249 |
| 31 | 21.3 | >1000 | >1000 |
| 32 | 20.1 | 267 | 129 |
| 33 | 203 | 218 | 89.5 |
| 34 | 23.2 | 493 | 462 |
| 35 | 16.1 | 449 | 392 |
| 36 | 39.8 | 637 | 486 |
| 37 | 20.4 | >1000 | > 1000 |
| 38 | 2.1 | 210 | 40.8 |
| 39 | 1 | 46.5 | 32.1 |
| 40 | 93.7 | >1000 | 956 |
| 41 | 39.9 | > 1000 | > 1000 |
| 42 | 14.7 | 510 | 408 |
| 43 | 2.8 | > 1000 | > 1000 |

(continued)

| Example No | CSF-1R $IC_{50}$ (nM) | CSF-1R BaF3 $EC_{50}$ (nM) | ECO2 $EC_{50}$ (nM) |
|---|---|---|---|
| 44 | 8.6 | 457 | 171 |
| 45 | 2.31 | 34.2 | 30.5 |
| 46 | 22.4 | 332 | 201 |
| 47 | 2 | > 1000 | > 1000 |
| 48 | 8.2 | 586 | 296 |
| 49 | 62.1 | >1000 | >1000 |
| 50 | 15.2 | 481 | 312 |
| 51 | 105 | 590 | 225 |
| 52 | 49.7 | 482 | 209 |
| 53 | 60.1 | 338 | 278 |
| 54 | 87.1 | 800 | 317 |
| 55 | 3.2 | 155 | 45.4 |
| 56 | 2.2 | 131 | 31.2 |
| 57 | 19 | 178 | 98.6 |
| 58 | 4.9 | 93.9 | 42.9 |
| 59 | 4.4 | 41.5 | 42.3 |
| 60 | 5.4 | 97.3 | 84.1 |
| 61 | 3.9 | 70.6 | 47.4 |
| 62 | 10.9 | 177.4 | 495.8 |
| 63 | 16.7 | 235.5 | 687.6 |
| 64 | 21.8 | 399.9 | 973.3 |
| 65 | 26.9 | 787.1 | > 1000 |
| 66 | 3.0 | 89.1 | 62.7 |
| 67 | 9.3 | >1000 | >1000 |
| 68 | 99.2 | >1000 | >1000 |
| 69 | 202.9 | > 1000 | > 1000 |
| 70 | 272.3 | > 1000 | > 1000 |
| 71 | 25.4 | 358.6 | 168.4 |
| 72 | 2.2 | 74.6 | 58.9 |
| 73 | 3.3 | 73.1 | 47.5 |
| 74 | 3.3 | 100 | 33.3 |
| 75 | 32 | 949.4 | 448.3 |
| 76 | 13.6 | 301.5 | 82.1 |
| 77 | 101.2 | 542.7 | 360.3 |
| 78 | 6.7 | 143.2 | 48.4 |
| 79 | 3.6 | 118.3 | 60.4 |
| 80 | 4 | 50.7 | 29.8 |
| 81 | 5.1 | 25.8 | 60.9 |
| 82 | 4.6 | 161.4 | 115.8 |

(continued)

| Example No | CSF-1R $IC_{50}$ (nM) | CSF-1R BaF3 $EC_{50}$ (nM) | ECO2 $EC_{50}$ (nM) |
|---|---|---|---|
| 83 | 1.2 | 26.7 | 37 |
| 84 | 28.4 | > 1000 | > 1000 |
| 85 | 109.8 | > 1000 | > 1000 |
| 86 | 4.4 | 290 | 182.8 |
| 87 | 0.83 | 86.3 | 92 |
| 88 | 4.9 | 76.7 | 116.3 |
| 89 | 286.2 | > 1000 | > 1000 |
| 90 | 17.4 | 282.1 | 154.2 |
| 91 | 40.6 | 753.4 | 387.9 |
| 92 | 1.8 | 67.9 | 26 |
| 93 | 2.1 | 72.9 | 18.3 |
| 94 | 14 | >1000 | >1000 |
| 95 | 3.4 | 42.2 | 54.3 |
| 96 | 16.2 | 234 | 190.4 |
| 97 | 2 | 34 | 82.7 |
| 98 | 4.7 | 57.3 | 54.7 |
| 99 | 5.6 | 59.1 | 51.7 |
| 100 | 2.2 | 17.1 | 10 |
| 101 | 1.9 | 77.7 | 48.3 |
| 102 | 4 | 134.2 | 77 |
| 103 | 1.9 | 41.7 | 26 |
| 104 | 2.4 | 53.2 | 41 |
| 105 | 2.4 | 107.6 | 65 |
| 106 | 2.6 | 95.9 | 76.3 |
| 107 | 1.6 | 65.9 | 37.1 |
| 108 | 1.9 | 37.6 | 23.7 |
| 109 | 1.1 | 102.4 | 43.7 |
| 110 | 3.4 | 109.1 | 36.2 |
| 111 | 2.2 | 35 | 17.4 |
| 112 | 4.7 | 167.6 | 101.6 |
| 113 | 12.3 | 348.1 | 143.2 |
| 114 | 1.7 | 75.7 | 37.9 |
| 115 | 4 | 158.4 | 81.9 |
| 116 | 3 | 33.8 | 10 |
| 117 | 21.1 | 343.5 | 170.5 |
| 118 | 3.6 | 96.9 | 34.5 |
| 119 | 0.73 | 15.9 | 8 |
| 120 | 1.9 | 43.7 | 18 |
| 121 | 19.9 | 41.1 | 15.2 |

(continued)

| Example No | CSF-1R $IC_{50}$ (nM) | CSF-1R BaF3 $EC_{50}$ (nM) | ECO2 $EC_{50}$ (nM) |
|---|---|---|---|
| 122 | 14 | 131 | 108 |
| 123 | 52.5 | 128 | 77 |
| 124 | 319.7 | >1000 | 442 |
| 125 | 521.6 | > 1000 | 950 |
| 126 | 12.2 | 3.8 | 2.4 |
| 127 | 22.3 | 28 | 25.4 |
| 128 | 4.9 | 21.5 | 4.9 |
| 129 | 42.7 | 307 | 166 |
| 130 | 1.6 | 2 | 1.5 |
| 131 | 5.4 | 9.9 | 6.7 |
| 132 | 879.2 | > 1000 | >1000 |
| 133 | 58.8 | 442.8 | 311.7 |
| 134 | 6.6 | 7.1 | 5.5 |
| 135 | 22.4 | 25.8 | 66.5 |
| 136 | 5.0 | 66.2 | 94.8 |
| 137 | 21.9 | 46.7 | 65 |
| 138 | 12.6 | 1.8 | 2.2 |
| 139 | 11.3 | 8.8 | 7.9 |
| 140 | 9.4 | 9.8 | 10.6 |
| 141 | 7.1 | 5.8 | 3.7 |
| 142 | 13.8 | 7.3 | 4.8 |
| 143 | 15.7 | 45.9 | 57.5 |
| 144 | 11.3 | 10.7 | 25.3 |
| 145 | 39.1 | 229.2 | 178.3 |
| 146 | 208.4 | >1000 | >1000 |
| 147 | 7.9 | 60.9 | 78.8 |
| 148 | 6.3 | 37.3 | 49.8 |
| 149 | 3.9 | 61.6 | 91.2 |
| 150 | 946.8 | >1000 | >1000 |
| 151 | 22.6 | 412.1 | 279.5 |
| 152 | >1000 | >1000 | >1000 |
| 153 | > 1000 | > 1000 | > 1000 |
| 154 | 28.6 | 328.8 | 199.3 |
| 155 | 257.5 | 607.2 | >1000 |
| 156 | >1000 | >1000 | >1000 |
| 157 | 543.2 | >1000 | >1000 |
| 158 | > 1000 | > 1000 | > 1000 |
| N.A. not available. | | | |

The selectivity data for selected compounds against CSF1R over cKit, FLT3, PDGFR-β are exhibited in Table 3 below.

Table 3

| Example No | CSF-1R $IC_{50}$ (nM) | cKit $IC_{50}$ (nM) Fold | FLT3 $IC_{50}$ (nM) Fold | PDGFR-1β $IC_{50}$ (nM) Fold |
|---|---|---|---|---|
| 1 | 7.5 | > 1000<br>> 100 fold | > 1000<br>> 100 fold | > 1000<br>> 100 fold |
| 13 | 9.8 | > 1000<br>> 100 fold | >1000<br>> 100 fold | >1000<br>> 100 fold |
| 15 | 9.8 | > 1000<br>> 100 fold | > 1000<br>>100 fold | > 1000<br>>100 fold |
| 16 | 2.1 | 881<br>> 100 fold | >1000<br>> 100 fold | >1000<br>>100 fold |
| 55 | 3.2 | 785<br>> 100 fold | > 1000<br>> 100 fold | 322<br>>100 fold |
| 56 | 2.2 | > 1000<br>> 100 fold | >1000<br>> 100 fold | 312<br>>100 fold |
| 59 | 4.4 | > 1000<br>> 100 fold | >1000<br>>100 fold | 349<br>79 fold |
| 66 | 3 | 948.3<br>> 100 fold | > 1000<br>>100 fold | 73.6<br>24.5 fold |
| 72 | 2.2 | >1000<br>> 100 fold | >1000<br>> 100 fold | >1000<br>> 100 fold |
| 73 | 3.3 | > 1000<br>> 100 fold | >1000<br>>100 fold | >1000<br>> 100 fold |
| 74 | 3.3 | > 1000<br>> 100 fold | > 1000<br>>100 fold | > 1000<br>> 100 fold |
| 78 | 6.7 | > 1000<br>> 100 fold | >1000<br>> 100 fold | 947<br>> 100 fold |
| 79 | 3.6 | > 1000<br>> 100 fold | >1000<br>> 100 fold | 683.7<br>>100 fold |
| 80 | 4 | 213.6<br>53.4 fold | > 1000<br>>100 fold | 49<br>12.3 fold |
| 81 | 5.1 | >1000<br>> 100 fold | >1000<br>> 100 fold | 371<br>72.7 fold |
| 82 | 4.6 | >1000<br>> 100 fold | >1000<br>> 100 fold | >1000<br>> 100 fold |
| 83 | 1.2 | > 1000<br>> 100 fold | > 1000<br>>100 fold | 158<br>> 100 fold |
| 87 | 0.83 | > 1000<br>> 100 fold | >1000<br>>100 fold | 207.6<br>>100 fold |
| 88 | 4.9 | > 1000<br>> 100 fold | >1000<br>> 100 fold | >1000<br>> 100 fold |
| 93 | 2.1 | > 1000<br>> 100 fold | > 1000<br>>100 fold | > 1000<br>> 100 fold |

(continued)

| Example No | CSF-1R $IC_{50}$ (nM) | cKit $IC_{50}$ (nM) Fold | FLT3 $IC_{50}$ (nM) Fold | PDGFR-1β $IC_{50}$ (nM) Fold |
|---|---|---|---|---|
| 100 | 2.2 | 875<br>> 100 fold | > 1000<br>> 100 fold | 377.8<br>> 100 fold |
| 101 | 1.9 | 715.2<br>>100 fold | >1000<br>> 100 fold | 839.3<br>> 100 fold |
| 112 | 4.7 | > 1000<br>>100 fold | > 1000<br>>100 fold | 873.6<br>> 100 fold |
| 116 | 3 | > 1000<br>> 100 fold | > 1000<br>> 100 fold | > 1000<br>> 100 fold |
| 119 | 0.73 | 680.9<br>>100 fold | >1000<br>> 100 fold | 43.2<br>59.2 fold |
| 121 | 19.9 | >1000<br>>50 fold | >1000<br>>50 fold | >1000<br>>50 fold |
| 134 | 6.6 | N.A | N.A | 394.3<br>59.7 fold |
| 136 | 5.0 | >1000<br>> 100 fold | N.A | >1000<br>> 100 fold |
| GW2580 | 7.9 | >1000<br>> 100 fold | >1000<br>> 100 fold | >1000<br>> 100 fold |
| PLX5622 | 9.8 | >1000<br>> 100 fold | 562<br>57 fold | >1000<br>> 100 fold |
| BLZ945 | 7.7 | >1000<br>>100 fold | >1000<br>> 100 fold | 408<br>53 fold |
| Comparator A | 6.1 | >1000<br>>100 fold | >1000<br>> 100 fold | >1000<br>> 100 fold |

The structures of reference compounds GW2580, PLX5622, BLZ945, and Comparator A are provided below.

GW2580:

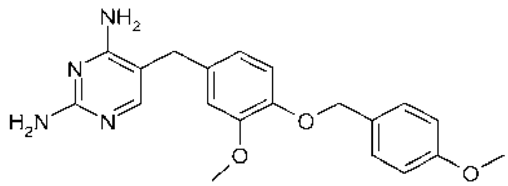

PLX5622:

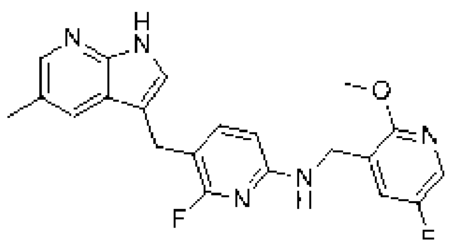

BLZ945:

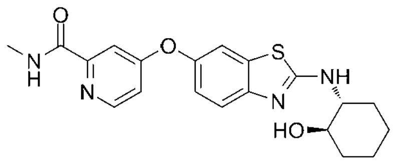

Comparator A

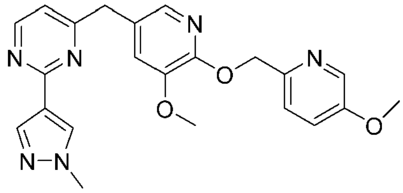

***In Vivo* PK Study**

The PK profiles were determined in CD1 mouse, and all the procedures and protocols were in accordance with the National Institutes of Health Guide for the Care and Use of Laboratory Animals. The CD1 mouse were purchased from JH Laboratory Animal Co. LTD. The CD1 mouse (23-30 g, 6-8 weeks, male) were randomized and divided into five groups consisting of 21 mouse per group. The test compounds that have been formulated were subjected to PK studies. The test compounds were administrated via p.o. at 45 mg/kg and i.v. at 2 mg/kg. After administration, blood samples and brain samples were collected at 0.25, 0.5, 1, 2, 4, 8 and 24h. While the brain sample were collected at 1, 4, 8 and 24h. The blood samples were placed on wet ice, and serum was collected after centrifugation. Serum samples were frozen and stored at -70 °C. The brain samples were were dried on filtrate paper, and snap frozen on dry ice and further stored at -70 °C. Then, the blood samples and brain samples were further analyzed by LC-MS/MS (Triple Quad 6500+).

The formulation for selected compounds and reported inhibitor are presented in Table 4 below.

Table 4

| Example No. | Formulation |
|---|---|
| 16 | 80% PEG400 + 20% Water. |
| 45 | 80% PEG400 + 20% Water. |
| 73 | 80% PEG400 + 20% Water. |
| 116 | 80% PEG400 + 20% Water. |
| 134 | 80% PEG400 + 20% Water. |
| 136 | 80% PEG400 + 20% Water. |
| PLX-5622 | 5% DMSO + 10% Solutol HS15 + 85% (20%HP-β-CD in saline) |
| BLZ-945 | 80% PEG400 + 20% Water. |
| GW-2580 | 80% PEG400 + 20% Water. |
| Comparator A | 80% PEG400 + 20% Water. |

The *in vivo* ADMET data for selected compounds and reported inhibitor are presented in Table 5 and Table 6 below.

Table 5

| Title | Example 16 | Example 45 | Example 73 | Example 116 | Example 134 | Example 136 |
|---|---|---|---|---|---|---|
| IV-2mpk CL (L/hr/kg) | 0.448 | 0.134 | 0.220 | 3.46 | 1.06 | 0.405 |
| IV-2mpk $V_{SS}$ (L/kg) | 0.955 | 2.49 | 0.461 | 9.54 | 1.45 | 0.274 |
| IV-2mpk $T_{1/2}$ (hr) | 2.52 | 14.2 | 2.56 | 6.42 | 1.39 | 0.777 |
| IV-2mpk $AUC_{last}$ (hr*ng/mL) | 4461 | 10672 | 9088 | 566 | 1860 | 4941 |
| IV-2mpk $AUC_{Inf}$ (hr*ng/mL) | 4465 | 14920 | 9093 | 578 | 1892 | 4943 |
| PO-45mpk $T_{1/2}$ (hr) | 1.90 | N.A. | 1.57 | 4.87 | 1.86 | N.A |
| PO-45mpk $C_{max}$ (ng/mL) | 31367 | 8943 | 100367 | 2307 | 18100 | 51167 |
| PO-45mpk $T_{max}$ (hr) | 1.0 | 0.5 | 0.5 | 0.25 | 0.5 | 0.5 |
| PO-45mpk $AUC_{last}$ (hr*ng/mL) | 215853 | 132073 | 202068 | 5368 | 96442 | 85732 |
| PO-45mpk $AUC_{Inf}$ (hr*ng/mL) | 215894 | N.A. | 202071 | 5477 | 96456 | N.A |
| F (%) | 215 | 55 | 98.8 | 42.1 | 227 | 77.1 |

Table 6

| Title | PLX-5622 | BLZ-945 | GW-2580 | Comparator A |
|---|---|---|---|---|
| IV-2mpk CL (L/hr/kg) | 0.326 | 0.267 | 2.20 | 0.430 |
| IV-2mpk $V_{SS}$ (L/kg) | 0.98 | 1.29 | 3.09 | 0.635 |
| IV-2mpk $T_{1/2}$ (hr) | 3.69 | 4.36 | 1.21 | 1.34 |
| IV-2mpk $AUC_{last}$ (hr*ng/mL) | 6098 | 7381 | 902 | 4586 |
| IV-2mpk $AUC_{Inf}$ (hr*ng/mL) | 6133 | 7405 | 911 | 4656 |
| PO-45mpk $T_{1/2}$ (hr) | 2.43 | 3.24 | 2.28 | 1.64 |
| PO-45mpk $C_{max}$ (ng/mL) | 24933 | 43933 | 5843 | 31967 |
| PO-45mpk $T_{max}$ (hr) | 1.0 | 0.5 | 1.0 | 1.0 |
| PO-45mpk $AUC_{last}$ (hr*ng/mL) | 162598 | 105369 | 27678 | 181355 |
| PO-45mpk $AUC_{Inf}$ (hr*ng/mL) | 162713 | 105734 | 27695 | 181365 |
| F (%) | 118 | 62.6 | 135 | 173 |

Based on the in vivo ADME data, Example **16** shows moderate clearance (CL=0.448 L/hr/kg) with $T_{1/2}$ of 2.52 hr, moderate distribution and excellent absorption with F 215%. Example **45** exhibits low clearance (CL=0.134 L/hr/kg) with $T_{1/2}$ of 14.2 hr (2 mpk, IV) when dosed as free base to mouse. Example **45** also shows moderate absorption with F 55% after oral treatment to mice (45 mpk). Example **73** shows moderate clearance with $T_{1/2}$ of 2.52 hr, moderate distribution with Vss of 0.461 L/kg, and excellent high Cmax of 100367 ng/mL. Example **116** displays very high distribution with a $V_{SS}$ of 9.54 L/kg, which might lead to the low Cmax. Example **134** shows moderate Clearance, exposure, and high absorption with F of 227%. Example **136** displays comparable ADME properties with Example **073.**

**Plasma protein binding study**

On the day of experiment, the plasma was thawed under running cold tap water and centrifuged at 3220×g for 5 min to remove any clots. The pH value was checked and recorded. Only plasma within range of pH 7.0 to pH 8.0 was used. Pretreat the dialysis membrane according to the manufacturer's instructions: the dialysis membrane strips were soaked in ultra pure water at room temperature for approximately 1 hr. After that, each membrane strip that contains 2 membranes was separated and soaked in ethanol:water (20:80 v:v) for approximately 20 min, after which it was ready for use or was stored in the solution at 2-8°C for up to 1 month. Prior to the experiment, the membrane was rinsed and soaked for 20 min in ultra pure water for use. Working solutions (400 μM) of test compound and control compound were prepared. Aliquots of working solutions (3 μL) were spiked into blank matrix (597 μL) to achieve final concentrations and mixed throughly.

Aliquots of 50 μL loading matrix containing test compound or control compound in triplicate was transferred to Sample Collection Plate, respectively. The samples were matched with opposite blank buffer to obtain a final volume of 100 μL with a volume ratio of matrix: Dialysis Buffer (1:1, v:v) in each well immediately. The stop solution was added to these T0 samples of test compound and control compound. The plate was sealed and shaked at 800 rpm for 10 min. Then these T0 samples were stored at 2-8°C pending further process along with other post-dialysis samples. The dialysis instrument was assembled following manufacture's instructions. An aliquot of 100 μL of the loading matrix containing test compound or control compound was transferred to the donor side of each dialysis well in triplicate and 100 μL of the Dialysis Buffer was loaded to the receiver side of the well. Then the plate was rotated at approximately 100 rpm in a humidified incubator with 5% $CO_2$ at 37±1°C for 4 hours. At the end of the dialysis, aliquots of 50 μL samples from the buffer side and matrix side of the dialysis device were taken into new 96-well plates (Sample Collection Plates). An equal volume of opposite blank matrix (buffer or matrix) in each sample was added to reach a final volume of 100 μL with volume ratio of matrix: Dialysis Buffer at 1:1 (v:v) in each well. All samples were urther processed by protein precipitation for LC/MS/MS analysis.

**Brain protein binding study**

On the day of experiment, brain homogenate was thawed in water bath at room temperature and then incubated at 37 °C for 10 min before use. Pretreat the dialysis membrane according to the manufacturer's instructions: the dialysis membrane strips were soaked in ultra pure water at room temperature for approximately 1 hr. After that, each membrane strip that contains 2 membranes was separated and soaked in ethanol:water (20:80 v:v) for approximately 20 min, after which it was ready for use or was stored in the solution at 2-8°C for up to 1 month. Prior to the experiment, the membrane was rinsed and soaked for 20 min in ultra pure water for use. Then working solutions (400 μM) of test compound and control compound were prepared. Aliquots of working solutions (3 μL) were spiked into blank matrix (597 μL) to achieve final concentrations and mixed throughly. Aliquots of 50 μL loading matrix containing test compound or control compound in triplicate was transferred to Sample Collection Plate, respectively. The samples were matched with opposite blank buffer to obtain a final volume of 100 μL with a volume ratio of matrix: Dialysis Buffer (1:1, v:v) in each well immediately. The stop solution was added to these T0 samples of test compound and control compound. The plate was sealed and shaked at 800 rpm for 10 min. Then these T0 samples were stored at 2-8°C pending further process along with other post-dialysis samples. The dialysis instrument was assembled following manufacture's instructions. An aliquot of 100 μL of the loading matrix containing test compound or control compound was transferred to the donor side of each dialysis well in triplicate and 100 μL of the Dialysis Buffer was loaded to the receiver side of the well. Then the plate was rotated at approximately 100 rpm in a humidified incubator with 5% $CO_2$ at 37±1°C for 4 hours. At the end of the dialysis, aliquots of 50 μL samples from the buffer side and matrix side of the dialysis device were taken into new 96-well plates (Sample Collection Plates). An equal volume of opposite blank matrix (buffer or matrix) in each sample was added to reach a final volume of 100 μL with volume ratio of matrix: Dialysis Buffer at 1:1 (v:v) in each well. All samples were urther processed by protein precipitation for LC/MS/MS analysis.

The BBB penetration data for selected compounds and reported inhibitor are presented in table 7 and Tabke 8 below.

Table 7

| Title | Example 16 | Example 45 | Example 73 | Example 116 | Example 134 | Example 136 |
|---|---|---|---|---|---|---|
| PO-45mpk $AUC_{last^-plasma}$ (hr*ng/mL) | 210236 | 131545 | 213544 | 4623 | 88358 | 93167 |
| PO-45mpk $AUC_{last^-brain}$ (hr*ng/mL) | 76809 | 94772 | 31839 | 4251 | 44350 | 16173 |
| $AUC_{last^-brain}$ /$AUC_{last^-plasma}$ | 0.365 | 0.720 | 0.149 | 0.920 | 0.502 | 0.174 |
| PPB-mouse (Bound) (%) | 99.37 | 99.70 | 99.0 | 95.8 | 99.2 | 99.5 |
| BPB-mouse (Bound) (%) | 98.10 | 99.6 | 95.5 | 95.5 | 98.8 | 97.3 |
| Kp, uu | 1.10 | 0.96 | 0.67 | 0.99 | 0.75 | 0.94 |

Table 8

| Title | PLX-5622 | BLZ-945 | GW-2580 | Comparator A |
|---|---|---|---|---|
| PO-45mpk $AUC_{last^-plasma}$ (hr*ng/mL) | 162598 | 101554 | 27049 | 181355 |
| PO-45mpk $AUC_{last^-brain}$ (hr*ng/mL) | 37627 | 11326 | 83613 | 53674 |
| $AUC_{last^-brain}$ /$AUC_{last^-plasma}$ | 0.231 | 0.112 | 3.090 | 0.308 |

(continued)

| Title | PLX-5622 | BLZ-945 | GW-2580 | Comparator A |
|---|---|---|---|---|
| PPB-mouse (Bound) (%) | 97.70 | 98.77 | 93.34 | 95.85 |
| BPB-mouse (Bound) (%) | 99.97 | 96.94 | 98.72 | 88.37 |
| Kp, uu | 0.003 | 0.28 | 0.59 | 0.86 |

When given orally to mouse, both example 16 and example 45 showed high exposure in brain and high BBB penetration with Kp,uu values of 1.10 and 0.96, respectively.

**p-CSF1R ELISA assay**

1.1 Materials

| MATERIALS | PART NUMBER | VENDOR |
|---|---|---|
| 96-well plate | 3599 | Corning |
| Reagents | | |
| RPMI-1640 Medium | L220KJ | BasalMedia |
| 2-Mercaptoethanol | 31350010 | Gibco |
| Foetal bovine serum | 10091-148 | Gibco |
| DPBS | 21-031-CV | Corning |
| Penicillin/Streptomycin Solution (P/S) | SV300310 | Hyclone |
| Human CSF-1/M-CSF Recombinant protein | 8929SC | CST |
| PathScan® Phospho-M-CSF Receptor (panTyr) Sandwich ELISA Kit | 13491CA | CST |
| Halt(tm)Protease&PhosphataseInhibitor Cocktail (100X) | PIERCE-78440 | Thermo |

1.2 Cell Culture

1. Prepare medium for THP-1 cell culture according to ATCC instructions. The base medium for this cell line is RPMI-1640 Medium. To make the complete growth medium, add the following components to the base medium: 2-mercaptoethanol to a final concentration of 0.05 mM; fetal bovine serum to a final concentration of 10%; P/S to a final concentration of 1%.
2. Cultures can be maintained by the addition of fresh medium or replacement of medium. Cultures can be established by centrifugation with subsequent resuspension at 2 x $10^5$ viable cells/mL. Subculture when cell concentration reaches 8x$10^5$ cells/mL. Do not allow the cell concentration to exceed 1.5 x $10^6$ cells/mL. Medium Renewal: Every 3 to 4 days.

1.3 Reagent preparation

1. Dissolve Recombinant human M-CSF lyophilized powder to a concentration of 5ug/ml ac cording to instructions, dispense and store in -80°C refrigerator.

2. Prepare lysis buffer immediately before use. (Lysis Buffer complete: 1ml 10X Lysis Buff er incomplete from ELISA kit, 8.9ml DPBS and 100ul of the Halt (tm) Protease & Phosph atase Inhibitor Cocktail (100X) )

1.4 Procedures

1. Before seed cells, centrifuge the cells at 1000rpm for 5 min. After centrifugation, the cell pellet is resuspended with pre-warmed culture medium and cells are separated by pipetting 6 times up and down with a 10 ml serological pipette. The cell suspension are diluted to a final concentration of $2.5 \times 10^6$ cells/ml with pre-warmed culture medium. 80ul of cell suspension ($2 \times 10^5$ cells) is added to the well of the 96-well plate.
2. Cpds stock solution are dissolved in DMSO at 10mM. The compounds start at 5 mM (5μl + 5μl DMSO) and are diluted with 4-fold dilution, 8 dose point. For the control of 100% inhibition, the reference compound PLX3397 concentration is 2.5mM. Then dilute cpds by the medium in the mid plate (2μl cpd + 198μl medium). Transfer 20ul diluted cpds from mid plate to the cell plate. Cell-plates are incubated at 37 °C/5% $CO_2$ for 1h.
3. After 1h compounds treatment, add 25ul of the 5-fold M-CSF (250 ng/ml) to 96-well plate; The final concentration of Human M-CSF is 50 ng/ml;
4. After 2-5 min M-CSF treatment, centrifuge the cell plate at 4000 rpm (cool) for 10min, remove the culture medium and wash cells once with 200ul/well ice-cold 1X PBS, gently. Then centrifuge the cell plate at 4000 rpm (cool) for 10min, remove PBS and add 100ul/well ice-cold 1X Cell Lysis Buffer to 96-well plate. The plate is gently shaked at 4 °C for 2h.
5. After the micro-well strips have reached room temperature, add 90μl of cell lysate to the appropriate well. Seal with tape and press firmly onto top of micro-wells. Incubate the plate at 37°C for 2h.
6. Gently remove the tape and wash wells 5 times with 1X Wash Buffer, 200μl each time for each well.
7. Add 100μl of reconstituted Detection Antibody to each well. Seal with tape and incubate the plate at 37°C for 1h.
8. Gently remove the tape and wash wells 5 times with 1X Wash Buffer, 200μl each time for each well.
9. Add 100μl of reconstituted HRP-Linked secondary antibody to each well. Seal with tape and incubate the plate at 37°C for 30min.
10. Gently remove the tape and wash wells 5 times with 1X Wash Buffer, 200μl each time for each well.
11. Add 100μl of TMB Substrate to each well. Seal with tape and incubate the plate at 37°C for 15min.
12. Add 100μl of STOP Solution to each well. Shake gently for a few seconds. Read absorbance at 450 nm within 30 min after adding STOP Solution.

1.5 Data analysis

Calculations and Formulas: The following calculations and formulas are used for data analysis using XL Fit software:

1. The average of the "M-CSF stimulated cells with DMSO" are set to 0%.
2. The average of the "M-CSF stimulated cells with 5uM PLX3397" are set to 100%.
3. All other values are expressed as percent of this positive control.
4. Calculate the average of the 0% inhibition and 100% inhibition of the phospho-CSF1R, and the same for Stdev.
5. Calculate the Cv of 0% inhibition and 100% inhibition (Cv=Stdev/Average*100)
6. Calculate the Z factor, Z factor=1-3(StdevMAX+StdevMIN)/ (AverageMAX-AverageMIN)
7.

The inhibition %=( Average 0% inhibition-Sample values)/ (Average 0% inhibition-Average 100% inhibition)

8. Calculate S/B, S/B=Average 0% inhibition/Average 100% inhibition.
9. The curve fitting was made using the Fit model (205), the detail information is: 4 Parameter Logistic Model or Sigmoidal Dose-Response Model.

$$\text{fit} = (A+((B-A)/(1+((C/x)^\wedge D))))$$

$$\text{inv} = (C/((((B-A)/(y-A))-1)^\wedge(1/D)))$$

$$\text{res} = (y-\text{fit})$$

10. Data acceptance criteria: The following criteria are used for the data QC:

10.1 Z factor: >0.50 based on 0% and 100% inhibition controls.
10.2 The reference compounds IC50 values are consistent between each run.

**p-CSF1R and p-ERK1/2 Western Blot assays**

Reagents and cell-line were purchased through the vendor, Fisher Scientific. Murine microglia cell line - BV2 cells (Accegen) - were cultured in T75 flasks, in DMEM/F12 (Gibco) supplemented with 10% FBS, 1% penicillin-streptomycin and 0.1% amphotericin B, and split twice a week. The same medium formulation is used throughout unless otherwise stated. One day before the experiment, BV2 cells were trypsinized and counted. A total of $1x10^6$ cells were plated in 12-well PDL coated plates and returned to culture overnight in a $CO_2$ incubator at 37°C. At the start of the experiment, the cells were serum starved for 4 hours with pre-warmed DMEM/F12 only. Inhibitors were dissolved in DMSO and cells were pretreated with the inhibitors for 30 minutes at indicated concentrations in DMSO volume not exceeding 0.1% of the volume of the medium, followed by stimulation with recombinant mouse CSF1 (R&D Systems) at 100ng/mL for 5 minutes. The cells were lysed with RIPA buffer supplemented with protease and phosphatase inhibitor cocktail (Halt-Pierce) and benzonase (EMD Millipore). Lysates were incubated at room temperature for 15 minutes and centrifuged at 16200g for 10 minutes at 4°C to pellet the insoluble fraction. Supernatants were collected and protein concentrations were determined using the Rapid BCA kit (Pierce) and absorbances were measured on the SpectramaxM5 (Molecular Devices). 20ug total protein in each condition were loaded and electrophoresed through a 4-12% Bis-Tris SDS-PAGE gel (Invitrogen) and transferred onto a PVDF membrane using the iBlot2 system (Invitrogen). Immunoblotting for phospho-CSF1R (Cell signaling, 3155S, diluted 1:1000) and phospho-ERK1/2 (Cell Signaling, 9101S, diluted 1:1000) were performed followed by stripping with NewBlot IR Stripping Buffer (Li-Cor) for 15 minutes and reprobed for CSF1R (Cell Signaling, 3152S, diluted 1:2000), ERK1/2 (Cell Signaling, 9107S, diluted 1:1000), and beta-tubulin (Abcam, ab179513, diluted 1:5000).

**CSF1Ri effects on BV2 cell proliferation**

Reagents and cell-line were purchased through the vendor, Fisher Scientific. BV2 cells (Accegen) were cultured in T75 flasks, in DMEM/F12 (Gibco) supplemented with 10% FBS, 1% penicillin-streptomycin and 0.1% amphotericin B, and split twice a week. The same medium formulation is used throughout unless otherwise stated. 1000 BV2 cells per well were seeded in a 96-well tissue-culture treated plate (Corning). Inhibitors and/or CSF1 (100ng/mL) were added and cultured for 72 hours. To perform the Cell Titer Glo assay, the medium was aspirated from the wells. 100uL of fresh growth medium + 100uL of Cell Titer Glo reagent (Promega) were added and the plate was incubated for 15 minutes at room temperature. 100uL from each sample was transferred to a white solid-bottom plate (Corning) for luminescence read (560nm) on the SpectramaxM5 (Molecular Devices).

**RESULTS**

**Effect of CSF1Ri on CSF1R signaling pathway**

To better understand the pharmacodynamic effect of our lead compound on the CSF1R signaling pathway, we analyzed the levels of phosphorylated versions of CSF1R (p-CSF1R) and ERK1/2 (p-ERK1/2), a downstream MAP Kinase, in murine microglia-like BV2 cells stimulated with recombinant mouse CSF1. BV2 cells were pretreated for 30 minutes with Example 16 or reference compounds (1000nM - 0.3 nM) and stimulated with CSF1 (100ng/mL) for 5 minutes before lysing the cells and running the lysates on SDS-PAGE gels. CSF1 treatment consistently increased CSF1R and ERK1/2 phosphorylation in BV2 cells (Figure 1A, B, C - compare lane 1 to lane 2). Preincubation of the cells with GW2580 (Figure 1A) and Comparator A (Figure 1B) abolished CSF1R signaling in a concentration-dependent manner. Example 16 also abolished CSF1 signaling in a concentration-dependent manner (Figure 1C). Quantification of p-CSF1R and p-ERKI /2 immunosignals from the western blots are shown in Figure 2 (GW2580 - Figure 2A; Comparator A - Figure 2B, Example 16 - Figure 2C). Example 16 inhibited CSF1R signaling with an average EC50 values of 47.2nM for p-CSF1R and 21.4nM for p-ERK1/2 inhibition (Table A). Thus, Example 16 is at least as effective as the reference compounds in blocking CSF1R signaling pathway in BV2 cells.

Since BV2 cells are of murine origin, we decided to set up a pharmacodynamic assay in human THP-1 cell line using a well-established p-CSF1R ELISA assay. THP1 cells were pre-treated with compounds in a 10-point concentration-response curves (CRC) with a 3-fold dilution steps for one hour before stimulating with hCSF1. Cells were lysed and pCSF1R levels were quantified as described in the Materials and Methods section. Individual CRCs are presented in Figure 3 and calculated EC50 values are presented in Table B. Therefore, Example 16 shows concentration-dependent blockade of CSF1R phosphorylation in human THP1 cells with a calculated EC50 of 10.8nM.

Table A

| BV2 Western Blot | | |
|---|---|---|
| Compound ID | p-CSF1R EC50 (nM) | p-ERK1/2 EC50 (nM) |
| GW2580 | 49 | 47.6 |
| Comparatore A | 77.6 | 37.6 |
| Example 16 | 47.2 | 21.4 |

Table B

| THP1 pCSF1R ELISA | |
|---|---|
| Compound ID | EC50 (nM) |
| GW2580 | 61.8 |
| BLZ945 | 94.3 |
| Comparator A | 21.3 |
| Example 16 | 10.8 |

**Effect of CSF1Ri on microglia proliferation and survival**

The functional output of CSF1R signaling in microglia is cell proliferation and survival. We set up an assay to measure the effect of our compounds on microglia proliferation. We stimulated BV2 cells with CSF1 (100ng/mL) for 72 h, which led to a ~2-fold enhancement of cell proliferation compared to vehicle treated cells as assessed by the Cell Titer Glow assay (Figure 4A). CSF1 induced proliferation was abolished by CSF1R inhibitors, GW2580 and BLZ945, in a concentration-dependent manner (Figure 4B, C). Example 16 also showed a concentration-dependent inhibition of BV2 cell proliferation (Figure 4D). The calculated EC50 values in this assay are shown in Table C.

Table C

| BV2 cell proliferation | |
|---|---|
| Compound ID | EC50 (nM) |
| GW2580 | 89.2 |
| BLZ945 | 132.5 |
| Example 16 | 45.4 |

To assess the potency of CSF1R inhibitors disclosed herein we isolated and expanded primary brain glia from rat cortices in-vitro. This glial culture is about 20% microglia that is supported mostly by astrocytes that make up the rest of the population. The brain glia was exposed to CSF1R inhibitors for 3 days followed by paraformaldehyde fixation and immunofluorescence staining for the microglia marker, Iba1. The glial was imaged using a high-content-imager and the changes in microglia content were quantified. Using GraphPad Prism, a four-parameter logistic regression was applied to determine the potency of the CSF1R inhibitors. CSF1R inhibitor, Example 16 demonstrates a dose-dependent inhibition of primary rat microglia survival after 3 days of exposure (Figure 5A). CSF1R inhibitors, Examples 73, 134, and 136 demonstrate a dose-dependent inhibition of primary rat microglia survival after 3 days of exposure (Figure 5B).

**Effect of CSF1Ri on inhibition of CSF1R phosphorylation**

The THP-1 human monocyte-like cell line has intact CSF1R signaling that allows for the probing of the activation status of this pathway when stimulated by recombinant CSF. THP-1 cells were expanded in-vitro and pretreated with CSF1R inhibitors for an hour prior to stimulation with recombinant human M-CSF for 5 minutes. The cells were lysed, and the lysate was probed for activated CSF1R (pCSF1R) using ELISA. Using GraphPad Prism, a four-parameter logistic regression was applied to determine the potency of the CSF1R inhibitors. The calculated $IC_{50}$ values are shown in Table D. CSF1R inhibitor, Example 16 demonstrates a dose-dependent inhibition of CSF1R phosphorylation in CSF1R-stimulated THP-1 monocytes (Figure 6).

Table D

| Compound ID | Mean $IC_{50}$ (nM)* |
|---|---|
| PLX3397 | 45.4 |
| Example 134 | 5.4 |
| Example 136 | 32.5 |

**Effect of CSF1Ri on hiPSC-MG survival**

To demonstrate compound efficacy in a highly translatable human model, we set up human induced pluripotent stem cell-derived microglia (hiPSC-MG) cultures and examined the effect of our compounds on hiPSC-MG survival. In brief, hiPSC-MG was stabilized for three days after thawing and treated with CSF1R inhibitors for 48 h. Microglia survival was evaluated at the end using Presto-Blue viability assay (Figures 7A and 7B). Percent survival values representing cell viability were calculated from each Presto-Blue fluorescence value normalized to DMSO treated controls (i.e., DMSO set as 100%). CSF1R-dependent cell survival was inhibited in a concentration-dependent manner by all CSF1R inhibitors used herein (Figures 7A and 7B). In particular, $IC_{50}$ values of Example 16 (Figure 7A), Example 73 (Figure 7B), and Example 134 (Figure 7B) were lower than that of BLZ945 (Figure 7B), demonstrating the superior potency of our compounds in human microglia.

**In vivo Microglial Depletion Effects of Example 134**

To measure the *in vivo* pharmacodynamic effect, we quantified microglial depletion following repeated dosing of our compound in naïve animals. Female C57BL/6 mice were given Example 134 (0, 50, 100, or 200 mg/kg/day, respectively) by oral gavage BID for 7 days. Following the final dose, mice were perfused with PBS followed by 4% paraformaldehyde (PFA). Brains were post-fixed overnight in 4% PFA and then transferred to 30% sucrose solution. Sagittal sections were sliced and stained for the microglial marker Iba1 and counterstained for DAPI. Brain sections were then imaged under 20X wide-field imaging, and the microglia density was quantified in the cortex and hippocampus.

The microglial depletion effect of Example 134 in the brain is shown in Figures 8A, Figure 8B, and Table E. We observed a dose-dependent decrease in microglial density for Example 134. In the cortex, microglia densities were significantly decreased (>22%, >91%, and >98%) at doses of 50, 100 and 200 mg/kg, respectively compared to vehicle control (Figure 8A). In the hippocampus, microglia densities were significantly lower in both 100 mg/kg (>83%) and 200 mg/kg groups (>93%) relative to vehicle control (see Figure 8B). To sum up, Example 134 shows a strong depletion effect in the brain at a dosage as low as 50 mg/kg/day in the cortex and 100 mg/kg/day in the hippocampus.

Table E

| Microglial Density following treatment with Example 134 | | | |
|---|---|---|---|
| Dose Group | No. of Animals | Mean Cortical Iba1+ Cell Density $\pm$ SEM ($mm^{-2}$) | Mean Hippocampal Iba1+ Cell Density $\pm$ SEM ($mm^{-2}$) |
| Vehicle | 8 | 82.3 $\pm$ 4.4 | 83.7 $\pm$ 6.1 |
| 50 mg/kg Example 134 | 8 | 63.6 $\pm$ 8.1 | 73.3 $\pm$ 7.8 |
| 100 mg/kg Example 134 | 8 | 6.9 $\pm$ 1.2 | 13.6 $\pm$ 2.2 |
| 200 mg/kg Example 134 | 8 | 1.5 $\pm$ 0.3 | 5.6 $\pm$ 0.9 |

**SUMMARY**

Example 16 was tested in the above referenced pharmacodynamic assays (p-CSF1R ELISA assay, p-CSF1R and p-ERK1/2 Western Blot assay), and cell-based efficacy assays (BV2 cell proliferation asssy, rat microglia survival assey, CSF1R phosphorylation inhibition assay, hiPSC-MG survival assay). Example 134 was tested in the *in vivo* pharmacodynamics study. The pharmacodynamic assays showed that Example 16 is a potent inhibitor of CSF1R and is comparable to, if not more potent than, reference compounds GW2580, BLZ945 and Comparator A. The efficacy assay has shown that Example 16 is a potent inhibitor of CSF1 directed proliferation of the microglia like BV2 cell line with a calculated EC50 value that is consistent with Example 16 exerting an on-target effect. Additionally, the efficacy assay has shown that Example 16 deminstrates a strong inhibition of CSF1R phosphorylation. The microglia survival assey has shown that Examples 16, 73, 134, and 136 demonstrate a strong inhibition of primary rat microglia survival. The hiPSC-MG

survival asseys demonstrated the superior potency of our compounds in the function of human microglia, with calculated $IC_{50}$ values of Example 16, 73, and 134 being lower compared to BLZ945. The *in vivo* study showed that Example 134 deminstrates a strong depletion effect in the brain at a dosage as low as 50 mg/kg/day in the cortex and 100 mg/kg/day in the hippocampus.

**Claims**

**1.** A compound represented by Formula (I):

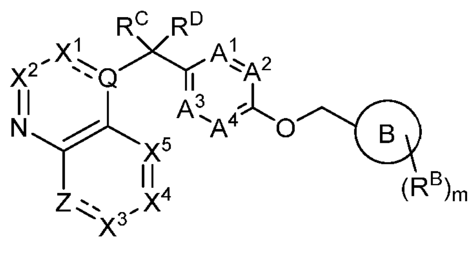

(I)

a pharmaceutically acceptable salt, or a tautomer thereof, wherein:

Q is C or N;
when Q is N, Q-$X^1$ is a single bond and $X^1$ is C=O;
when Q is C, Q-$X^1$ is a double bond and $X^1$ is N or $CR^1$;
Z is CH or NH;
when Z is NH, Z-$X^3$ is a single bond and $X^3$ is C=O;
when Z is CH, Z-$X^3$ is a double bond and $X^3$ is N or $CR^3$;
$X^2$ is N or $CR^2$;
$X^4$ is N or $CR^4$;
$X^5$ is N or $CR^5$;
each $R^1$, $R^2$, $R^3$, $R^4$, and $R^5$ is independently selected from H, halogen, -CN, -OH, $C_{1-6}$alkyl optionally substituted with one to three deuterium, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^1$, $R^2$, $R^3$, $R^4$, or $R^5$ is optionally substituted by one or two groups selected from CN, halogen, $C_{1-6}$alkyl, $C_{1-6}$haloalkyl, $C_{1-6}$alkoxy, and $C_{1-6}$haloalkoxy;
each $A^1$, $A^2$, $A^3$, and $A^4$ is independently N or $CR^A$; and no more than two of $A^1$, $A^2$, $A^3$, and $A^4$ are N;
each $R^A$ is independently selected from halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^A$ is optionally substituted by one or two groups selected from CN, halogen, $C_{1-6}$alkyl, $C_{1-6}$haloalkyl, $C_{1-6}$alkoxy, and $C_{1-6}$haloalkoxy;
Ring B is phenyl, or 5 or 6-membered monocyclic heteroaryl;
each $R^B$ is independently selected from halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^B$ is optionally substituted by one or two groups selected from CN, halogen, $C_{1-6}$alkyl, $C_{1-6}$haloalkyl, $C_{1-6}$alkoxy, and $C_{1-6}$haloalkoxy; and/or
two $R^B$ together with the ring B atoms which they attached, form 5-7 membered monocyclic ring or 6-9 membered bicyclic ring fused to Ring B, said 5-7 membered monocyclic or 6-9 membered bicyclic ring is optionally

substituted with one or two groups selected from CN, halogen, $C_{1-6}$alkyl, $C_{1-6}$haloalkyl, $C_{1-6}$alkoxy, $C_{1-6}$haloalkoxy, and 3-6 membered cycloalkyl;

each $R^C$ and $R^D$ is independently selected from hydrogen, deuterium, F, and methyl;

each R* is independently selected from the group consisting of H, $C_{1-6}$alkyl, $C_{1-6}$haloalkyl, $C_{2-6}$alkenyl, $C_{2-6}$alkynyl, 3-6 membered carbocyclyl, or 4-6 membered heterocyclyl; and

m is 0, 1, 2, 3, or 4.

**2.** The compound of claim 1, or a pharmaceutically acceptable salt thereof, wherein:

each $R^1$, $R^2$, $R^3$, $R^4$, and $R^5$ is independently selected from H, halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^1$, $R^2$, $R^3$, $R^4$, or $R^5$ is optionally substituted by one or two groups selected from CN, halogen, $C_{1-6}$alkyl, $C_{1-6}$haloalkyl, $C_{1-6}$alkoxy, and $C_{1-6}$haloalkoxy;

each $R^A$ is independently selected from H, halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^A$ is optionally substituted by one or two groups selected from CN, halogen, $C_{1-6}$alkyl, $C_{1-6}$haloalkyl, $C_{1-6}$alkoxy, and $C_{1-6}$haloalkoxy;

each $R^B$ is independently selected from halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^B$ is optionally substituted by one or two groups selected from CN, halogen, $C_{1-6}$alkyl, $C_{1-6}$haloalkyl, $C_{1-6}$alkoxy, and $C_{1-6}$haloalkoxy; or

two $R^B$ together with the ring B atoms which they attached, form 5,6-membered ring fused to Ring B, said 5,6-membered ring is optionally substituted with one or two groups selected from CN, halogen, $C_{1-6}$alkyl, $C_{1-6}$haloalkyl, $C_{1-6}$alkoxy, and $C_{1-6}$haloalkoxy; and

each $R^C$ and $R^D$ is independently selected from hydrogen, F, and methyl; optionally wherein the compound is represented by any one of Formula (II-A), Formula (III-A-1), Formula (III-A-2), Formula (III-A-3), Formula (III-A-4), Formula (III-A-5), Formula (III-A-6), Formula (III-A-7), Formula (III-B-1), Formula (III-B-2), Formula (III-C-1), Formula (III-C-2), or Formula (III-C-3):

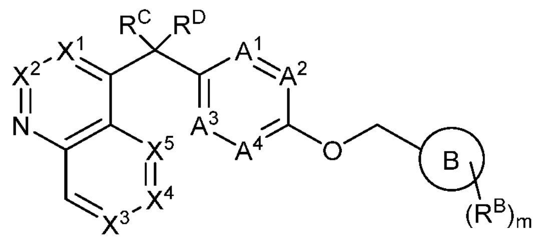
(II-A),

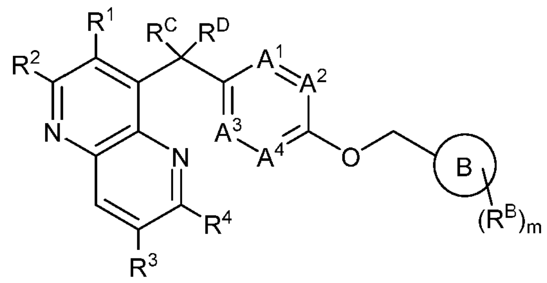

(III-A-1),

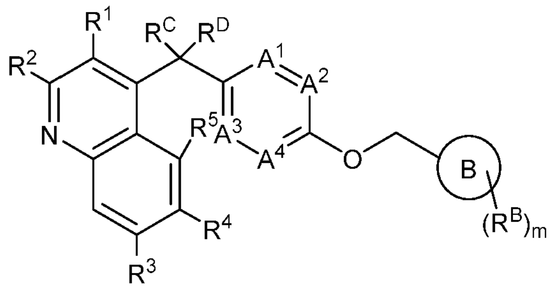

(III-A-2),

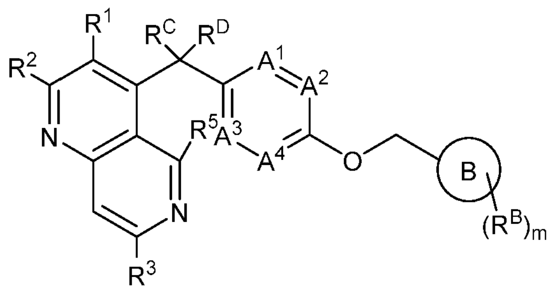

(III-A-3),

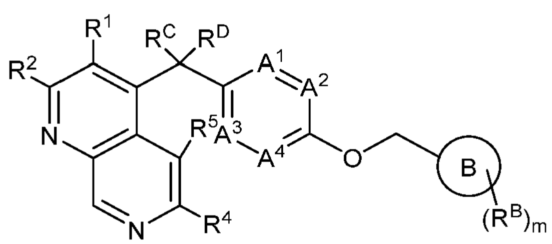

(III-A-4),

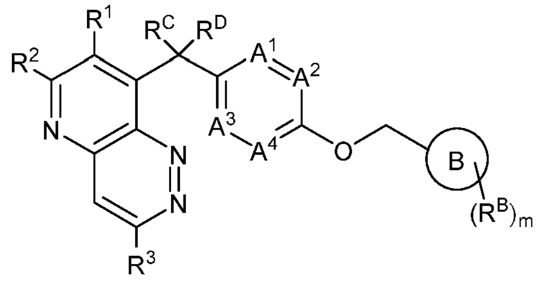

(III-A-5),

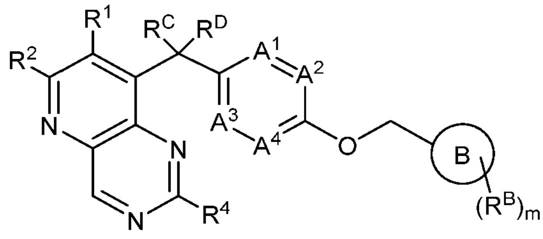

(III-A-6),

or

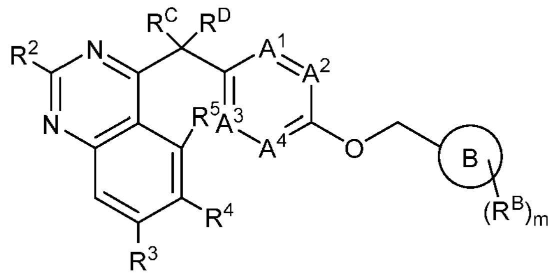

(III-A-7),

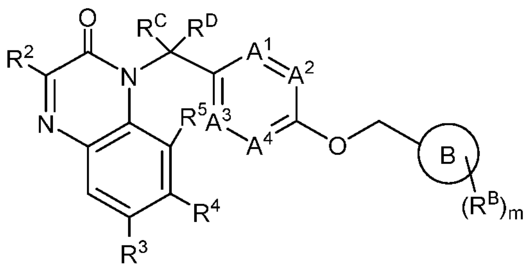

(III-B-1),

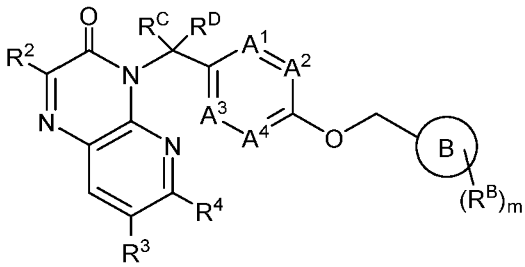

(III-B-2);

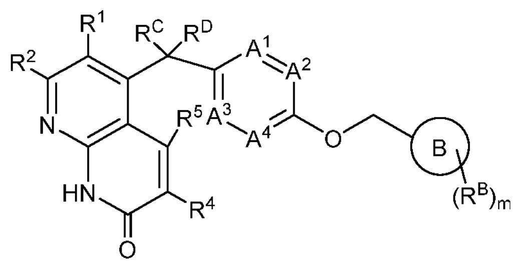

(III-C-1),

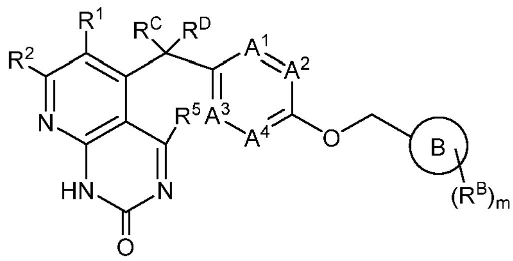

(III-C-2),

or

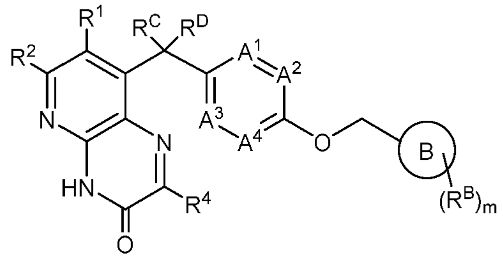

(III-C-3);

or a pharmaceutically acceptable salt thereof.

**3.** The compound of claim 2 or a pharmaceutically acceptable salt thereof, wherein:

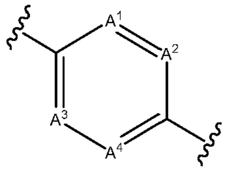

is phenyl or pyridyl, each of which is optionally substituted by one or two $R^A$; and/or
ring B is:

(i) phenyl optionally substituted by one or two $R^B$, each $R^B$ is independently selected from halogen, -CN, -OH, $C_{1-6}$alkyl, $C_{1-6}$haloalkyl,
$C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, -OR*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^B$ is optionally substituted by one or two halogen or $C_{1-6}$alkyl; or
(ii) 5, 6-membered monocyclic heteroaryl optionally substituted by one or two $R^B$, each $R^B$ is independently selected from halogen, -CN, -OH, $C_{1-6}$alkyl, $C_{1-6}$haloalkyl, $C_{1-6}$hydroxylalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, -OR*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^B$ is optionally substituted by one or two halogen or $C_{1-6}$alkyl; or
(iii) phenyl or 5, 6-membered monocyclic heteroaryl, each of which is optionally substituted by one to four $R^B$, and two $R^B$ together with the Ring B atoms to which they attached, form 5,6-membered ring fused to Ring B, said 5,6-membered fused ring is optionally substituted with one or two CN, halogen, $C_{1-4}$alkyl,
$C_{1-4}$haloalkyl, $C_{1-4}$alkoxy, or $C_{1-4}$haloalkoxy; or
(iv) pyrazolyl, isoxazolyl, 1,2,4-oxadiazolyl, thiazolyl, phenyl, pyridyl, pyrimidyl, pyrazinyl, imidazo[1,2-a]pyridinyl, benzo[d]imidazolyl, pyrazolo[1,5-a]pyridinyl, indazolyl, [1,2,4]triazolo[1,5-a]pyridinyl, 4,5,6,7-tetrahydropyrazolo[1,5-a]pyrazinyl, 6,7-dihydro-4H-pyrazolo[5,1-c][1,4]oxazinyl, 5,6-dihydro-4H-pyrrolo[1,2-b]pyrazolyl, or

benzo[d][1,3]dioxolyl.

**4.** The compound of claim 2 or 3 or a pharmaceutically acceptable salt thereof, wherein:

each $R^1$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, -C(O)R*, -C(O)OR*, -C(O)NR*R*, $-(CH_2)_{0-4}$NR*R*, -NR*C(O)R*, and -NR*C(O)OR*; and/or
each $R^2$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, -C(O)R*, -C(O)OR*, -C(O)NR*R*, $-(CH_2)_{0-4}$NR*R*, -NR*C(O)R*, and -NR*C(O)OR*; and/or
each $R^3$ is independently selected from H, halogen, -CN, -OH, $C_{1-4}$alkyl, $C_{1-4}$haloalkyl, $C_{1-4}$hydroxylalkyl, $-(CH_2)_{0-2}C_{1-4}$alkoxy optionally substituted with one to three deuterium, $C_{1-4}$haloalkoxy, $C_{1-4}$hydroxyalkoxy, -C(O)R*, -C(O)OR*, -C(O)NR*R*, $-(CH_2)_{0-4}$NR*R*, -NR*C(O)R*, and -NR*C(O)OR*; and/or
each $R^4$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, -C(O)R*, -C(O)OR*, -C(O)NR*R*, $-(CH_2)_{0-4}$NR*R*, -NR*C(O)R*, and -NR*C(O)OR*; and/or
each $R^5$ is independently selected from H, halogen, -CN, -OH, $C_{1-4}$alkyl, $C_{1-4}$haloalkyl, $C_{1-4}$hydroxylalkyl, $-(CH_2)_{0-4}C_{1-4}$alkoxy, $C_{1-4}$haloalkoxy, $C_{1-4}$hydroxyalkoxy, -C(O)R*, -C(O)OR*, -C(O)NR*R*, -NR*R*, -NR*C(O)R*, and -NR*C(O)OR*; and/or
each $R^A$ is independently selected from H, halogen, -CN, -OH, $C_{1-4}$alkyl, $C_{1-4}$haloalkyl, $C_{1-4}$hydroxylalkyl, $C_{1-4}$alkoxy optionally substituted with one to three deuterium, $C_{1-4}$haloalkoxy, $C_{1-4}$hydroxyalkoxy, -C(O)R*, -C(O)OR*, -C(O)NR*R*, $-(CH_2)_{0-4}$NR*R*, -NR*C(O)R*, and -NR*C(O)OR*; and/or
each $R^B$ is independently selected from halogen, -CN, -OH, $C_{1-4}$alkyl, $C_{1-4}$haloalkyl, $C_{1-4}$hydroxylalkyl, $-(CH_2)_{0-2}C_{1-4}$alkoxy optionally substituted with one to three deuterium, $C_{1-4}$haloalkoxy, $C_{1-4}$hydroxyalkoxy, -C(O)R*, -C(O)OR*, -C(O)NR*R*, $-(CH_2)_{0-4}$NR*R*, -NR*C(O)R*, -NR*C(O)OR*, and $-(CH_2)_{0\text{ or }1}$-3-6 membered cycloalkyl optionally substituted with one or two halogen; and/or
each R* is independently H or $C_{1-4}$alkyl (preferably H or $C_{1-2}$alkyl).

**5.** The compound of any one of claims 2-4 or a pharmaceutically acceptable salt thereof, wherein:

each $R^1$ is independently selected from H, halogen, $C_{1-4}$alkyl, $C_{1-4}$haloalkyl, $C_{1-4}$alkoxy, and $C_{1-4}$haloalkoxy (preferably H); and
each $R^2$ is independently selected from H, halogen, $C_{1-4}$alkyl, $C_{1-4}$haloalkyl, $C_{1-4}$alkoxy, and $C_{1-4}$haloalkoxy (preferably H); and/or
each $R^3$ is independently selected from H, halogen, $C_{1-4}$alkyl, $C_{1-4}$haloalkyl, $C_{1-4}$alkoxy optionally substituted with one to three deuterium, and $C_{1-4}$haloalkoxy (preferably H, CN, halogen, $C_{1-4}$alkyl, or $C_{1-4}$alkoxy optionally substituted with one to three deuterium); and/or
each $R^4$ is independently selected from H, halogen, $C_{1-4}$alkyl, $C_{1-4}$haloalkyl, $C_{1-4}$alkoxy, and $C_{1-4}$haloalkoxy (preferably H, F, or $C_{1-4}$alkoxy); and/or
each $R^5$ is independently selected from H, halogen, $C_{1-4}$alkyl, $C_{1-4}$haloalkyl, $C_{1-4}$alkoxy, and $C_{1-4}$haloalkoxy (preferably H); and/or
each $R^A$ is independently selected from H, $C_{1-2}$alkyl, or $C_{1-2}$alkoxy optionally substituted with one to three deuterium (preferably H, $-OCH_3$, or $-OCD_3$); and/or
each $R^B$ is independently selected from halogen, -OH, $C_{1-3}$alkyl, $C_{1-3}$haloalkyl, $-(CH_2)_{0-2}C_{1-2}$alkoxy optionally substituted with one to three deuterium, $C_{1-2}$haloalkoxy, $-(CH_2)_{0-2}$NR*R*, or 3-6 membered cycloalkyl optionally substituted with one or two halogen (preferably F, Cl, -OH, $-CH_3$, $-CH_2CH_3$, $-CH(CH_3)_2$, $-CHF_2$, $-CF_3$, $-CH_2N(CH_3)_2$,$-CH_2OCH_3$, $-OCH_3$, $-OCHF_2$, $-OCD_3$, cyclopropyl optionally substituted with one or two fluoro); and/or

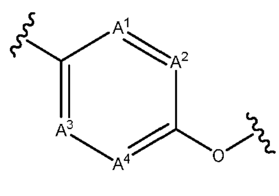 is 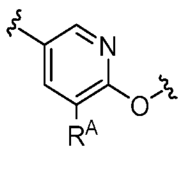 or 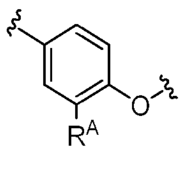 .

**6.** The compound of claim 2, 4, or 5, wherein the compound is represented by Formula (IV-A-1) or (IV-A-2):

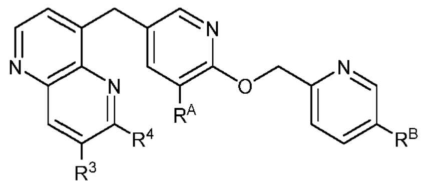

(IV-A-1),

or

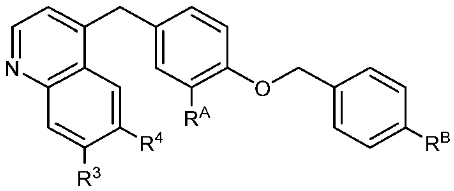

(IV-A-2),

or a pharmaceutically acceptable salt thereof.

**7.** The compound of any one of claims 1-3 and 6, a pharmaceutically acceptable salt, or a tautomer thereof, wherein:

(i) each $R^3$ is independently selected from H, halogen, -CN, $C_{1-6}$alkyl optionally substituted with one to three deuterium,
$C_{1-6}$haloalkyl, $C_{1-6}$hydroxylalkyl, -$C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, -NR*R*, 3-6 membered carbocyclyl, 3-6 membered monocyclic heterocyclyl, 7-10 membered bridged heterocyclyl, phenyl, or 5-6 membered heteroaryl; wherein said carbocyclyl, heterocyclyl, phenyl, or heteroaryl in the group represented by $R^3$ is optionally substituted by one or two CN, halogen, $C_{1-4}$alkyl, $C_{1-4}$haloalkyl, $C_{1-4}$alkoxy, or $C_{1-4}$haloalkoxy; or
(ii) each $R^3$ is independently selected from H, halogen, CN, $C_{1-4}$alkyl optionally substituted with one to three deuterium, $C_{1-4}$alkoxy optionally substituted with one to three deuterium, NR*R*, -C(O)NR*R*, 3-6 membered cycloalkyl, 3-6 membered monocyclic heterocyclyl, 5-6 membered heteroaryl, or 6-oxa-3-azabicyclo[3.1.1] heptanyl, wherein said cycloalkyl, heterocyclyl, or heteroaryl in the group represented by $R^3$ is optionally substituted by one or two halogen, $C_{1-2}$alkyl, $C_{1-2}$haloalkyl, $C_{1-2}$alkoxy, or $C_{1-2}$haloalkoxy; or
(iii) each $R^3$ is independently selected from H, F, Cl, CN, $CH_3$, $OCH_3$, $OCD_3$,
-$N(CH_3)_2$, -$CON(CH_3)_2$, cyclopropanyl, azetidinyl optionally substituted with one or two fluoro or $OCH_3$, imidazole optionally substituted with $CH_3$, morpholinyl, pyridyl, piperazinyl optionally substituted with $CH_3$, pyrrolidinyl optionally substituted with $OCH_3$, pyrazolyl optionally substituted with $CH_3$, thiazole optionally substituted with $CH_3$, or 6-oxa-3-azabicyclo[3.1.1]heptanyl; or
(iv) each $R^3$ is independently selected from H or $OCH_3$.

**8.** The compound of any one of claims 1, 2, 4, 5 or 7, a pharmaceutically acceptable salt, or a tautomer thereof, wherein:

(i) ring B is phenyl, 5 or 6-membered monocyclic heteroaryl optionally substituted by one or two $R^B$, each $R^B$ is independently selected from halogen, -OH, $C_{1-4}$alkyl, $C_{1-4}$haloalkyl, $C_{1-4}$alkoxyalkyl,

$C_{1-4}$alkoxy optionally substituted with one to three deuterium, $C_{1-4}$haloalkoxy, -C(O)NR*R*, -$(CH_2)_{0-1}$NR*R*, -NR*C(O)R*, -3-6 membered monocyclic carbocyclyl, -3-6 membered monocyclic heterocyclyl; wherein said carbocyclyl or heterocyclyl in the group represented by $R^B$ is optionally substituted by one or two halogen or $C_{1-4}$alkyl; or
each $R^B$ is independently selected from F, Cl, -OH, $C_{1-4}$alkyl, $C_{1-4}$haloalkyl, $C_{1-4}$alkoxyalkyl, $C_{1-4}$alkoxy optionally substituted with one to three deuterium, $C_{1-4}$haloalkoxy, -C(O)NR*R*, -$CH_2$NR*R*, -NR*C(O)R*, -3-5 membered monocyclic cycloalkyl, -3-5 membered monocyclic heterocyclyl; wherein said cycloalkyl or heterocyclyl in the group represented by $R^B$ is optionally substituted by one or two F or $C_{1-2}$alkyl; or

(ii) ring B is phenyl or 5, 6-membered monocyclic heteroaryl, each of which is optionally substituted by one to four $R^B$, and two $R^B$, together with the ring B atoms to which they attached, form 5-7 membered monocyclic ring or 6-9

membered bicyclic ring fused to ring B, said 5-7 membered monocyclic ring or 6-9 membered bicyclic ring is optionally substituted with one or two CN, halogen, $C_{1-4}$alkyl, $C_{1-4}$haloalkyl, $C_{1-4}$alkoxy, $C_{1-4}$haloalkoxy, or 3-6 membered cycloalkyl; ortwo $R^B$, together with the ring B atoms to which they attached, form a ring selected from

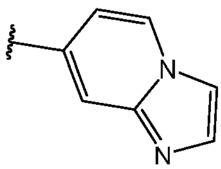, 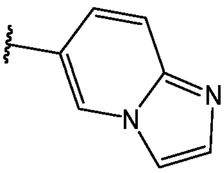, 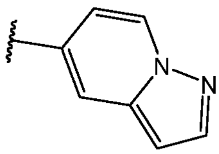, 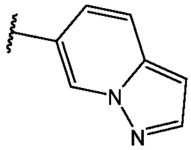, 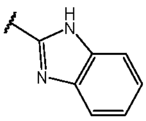,

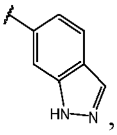, 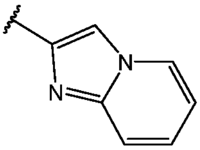, 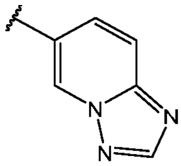, 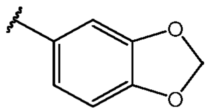, 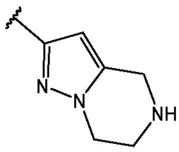,

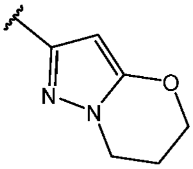, 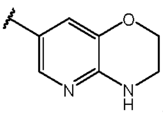, 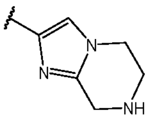, 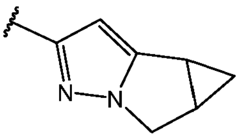, 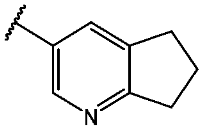,

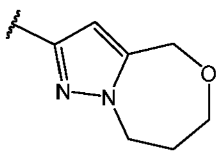, 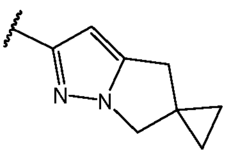, 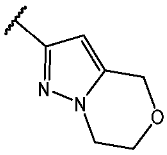, 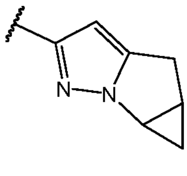, 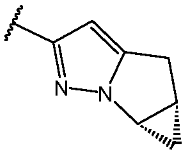,

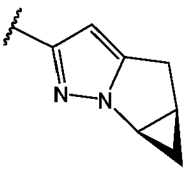, 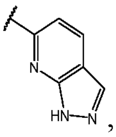, 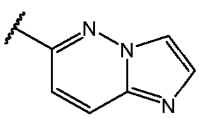, 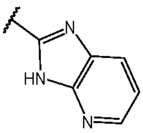, 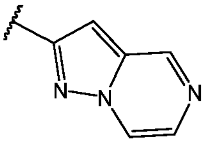,

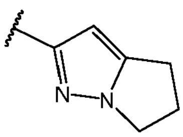, and 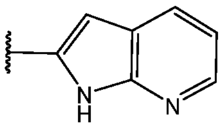,

each of which is optionally substituted with one or two groups selected from halogen, $C_{1-4}$alkyl, $C_{1-4}$haloalkyl, $C_{1-4}$alkoxy, and $C_{1-4}$haloalkoxy; or

two $R^B$, together with the ring B atoms to which they attached, form a ring selected from

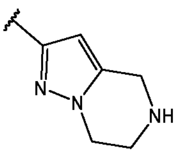, 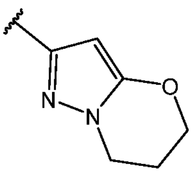, 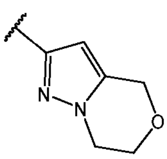, 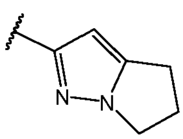

and

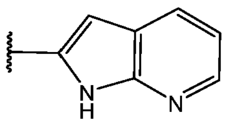

**9.** The compound of any one of claims 1-8, a pharmaceutically acceptable salt, or a tautomer thereof, wherein $R^C$ is H, and $R^D$ is H.

**10.** A compound represented by Formula (I'):

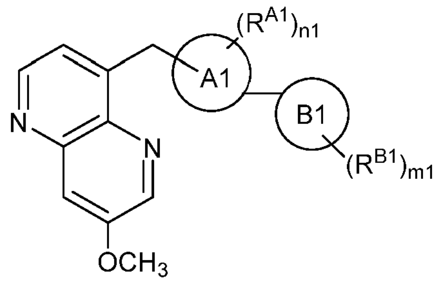

(I')

or a pharmaceutically acceptable salt thereof, wherein:

ring A1 is 5,6 or 6,6-membered nitrogen containing bicyclic heteroaryl;
ring B1 is 3-6 membered monocyclic carbocyclyl or 3-6 membered monocyclic heterocyclyl;
each $R^{A1}$ is independently selected from halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^{A1}$ is optionally substituted by one or two CN, halogen, $C_{1-6}$alkyl, $C_{1-6}$haloalkyl, $C_{1-6}$alkoxy, or $C_{1-6}$haloalkoxy;
each $R^{B1}$ is independently selected from halogen, -CN, -OH, $C_{1-6}$alkyl, $C_{1-6}$haloalkyl, $C_{1-6}$hydroxylalkyl, $C_{1-6}$alkoxyalkyl, $C_{2-6}$alkenyl, $C_{2-6}$alkynyl, $-(CH_2)_{0-4}C_{1-6}$alkoxy optionally substituted with one to three deuterium, $C_{1-6}$haloalkoxy, $C_{1-6}$hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ or }1}$-3-12 membered carbocyclyl, $-(CH_2)_{0\text{ or }1}$-3-12 membered heterocyclyl, $-(CH_2)_{0\text{ or }1}$-6-10 membered aryl, or $-(CH_2)_{0\text{ or }1}$-5-10 membered heteroaryl; wherein said carbocyclyl, heterocyclyl, aryl, or heteroaryl in the group represented by $R^{B1}$ is optionally substituted by one or two CN, halogen, $C_{1-6}$alkyl, $C_{1-6}$haloalkyl, $C_{1-6}$alkoxy, or $C_{1-6}$haloalkoxy; and/or
two $R^{B1}$ together with the ring B1 atoms which they attached, form 5,6-membered monocyclic ring or 6-9-membered bicyclic ring fused to ring B1, said 5,6-membered ring or 6-9-membered bicyclic ring is optionally substituted with one or two CN, halogen, $C_{1-6}$alkyl, $C_{1-6}$haloalkyl, $C_{1-6}$alkoxy, $C_{1-6}$haloalkoxy, or 3-6 membered cycloalkyl;
each R* is independently selected from the group consisting of H, $C_{1-6}$alkyl, $C_{1-6}$haloalkyl, $C_{2-6}$alkenyl, $C_{2-6}$alkynyl, 3-6 membered carbocyclyl, or 4-6 membered heterocyclyl;
m1 is 0, 1, 2, 3, or 4; and
n1 is 0, 1, 2, or 3.

**11.** The compound of claim 10 or a pharmaceutically acceptable salt thereof, wherein:

(i)

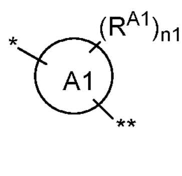

is

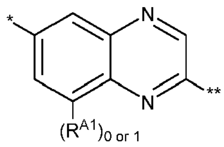 , 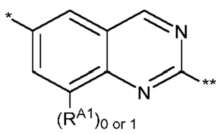 , 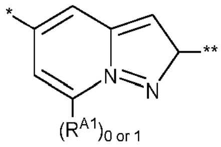 , or 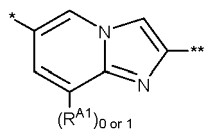 ,

wherein -* represents the point to which -$CH_2$- attaches; -** represents the point to which ring B1 attaches; and $R^{A1}$ is $C_{1-4}$alkyl or $C_{1-4}$alkoxy; and/or

(ii) ring B1 is

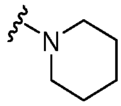 , 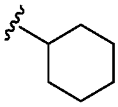 , or 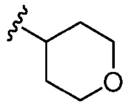 ;

$R^{B1}$ is $C_{1-4}$alkyl or $C_{1-4}$alkoxy; and
n1 is 0 or 1.

**12.** A compound selected from:

| Example No | Structure | Example No | Structure |
|---|---|---|---|
| 1 | 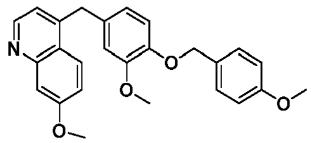 | 80 | 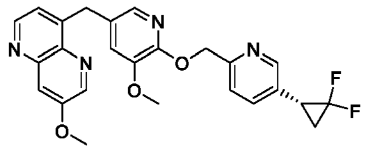 or 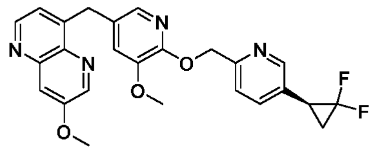 |
| 2 | 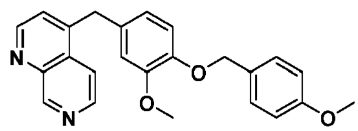 | 81 | 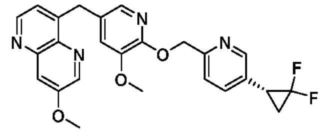 or 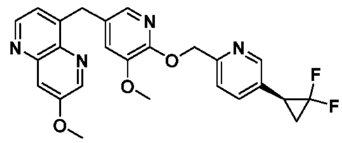 |
| 3 | 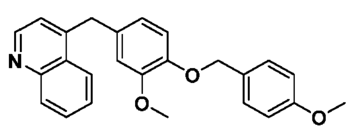 | 82 | 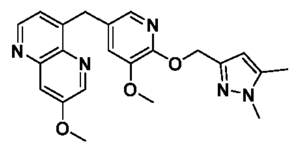 |
| 4 | 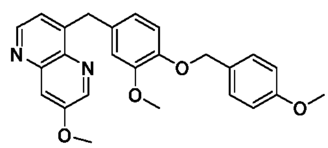 | 83 | 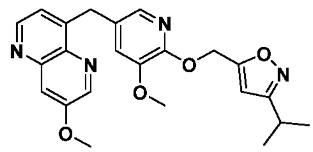 |

(continued)

| Example No | Structure | Example No | Structure |
|---|---|---|---|
| 5 | 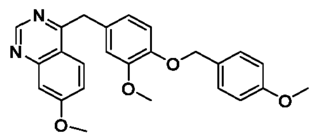 | 84 | 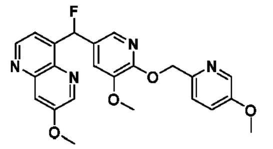 |
| 6 | 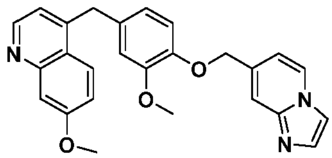 | 85 | 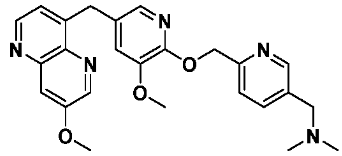 |
| 7 | 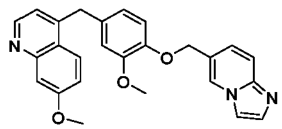 | 86 | 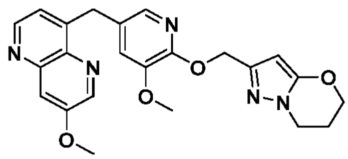 |
| 8 | 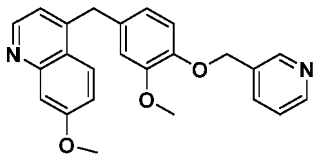 | 87 | 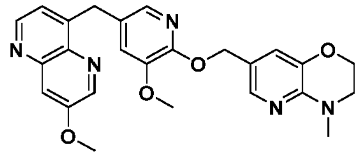 |
| 9 | 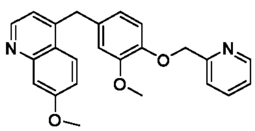 | 88 | 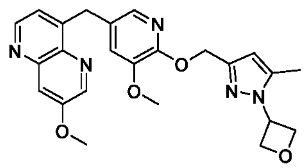 |
| 10 | 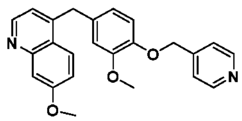 | 89 | 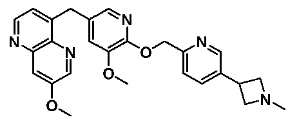 |
| 11 | 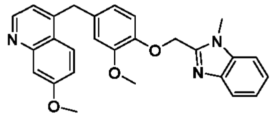 | 90 | 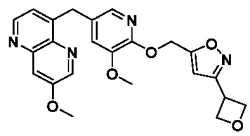 |
| 12 | 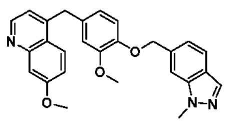 | 91 | 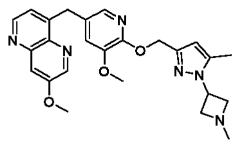 |
| 13 | 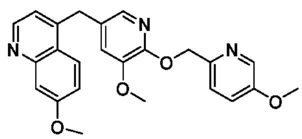 | 92 | 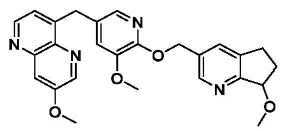 |
| 14 | 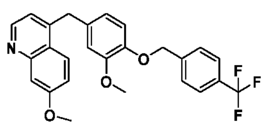 | 93 | 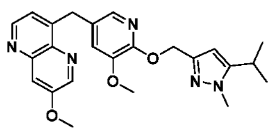 |

(continued)

| Example No | Structure | Example No | Structure |
|---|---|---|---|
| 15 | 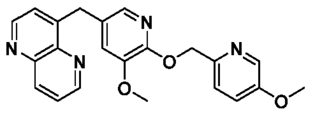 | 94 | 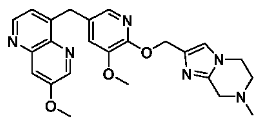 |
| 16 | 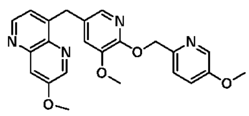 | 95 | 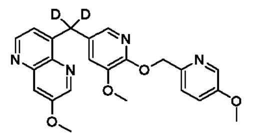 |
| 17 | 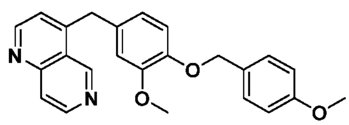 | 96 | 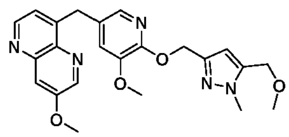 |
| 18 | 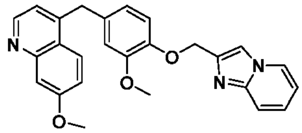 | 97 | 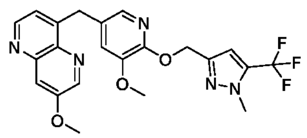 |
| 19 | 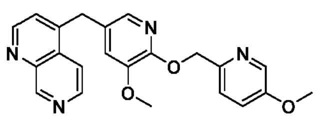 | 98 | 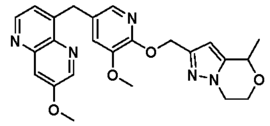 |
| 20 | 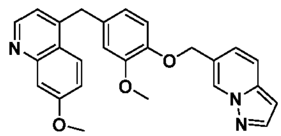 | 99 | 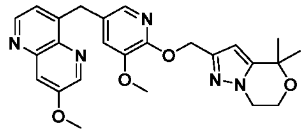 |
| 21 | 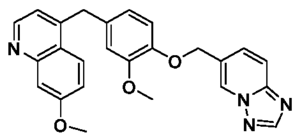 | 100 | 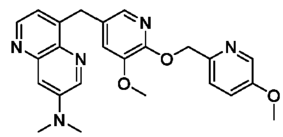 |
| 22 | 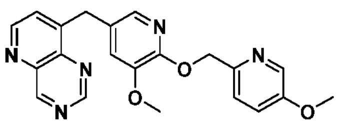 | 101 | 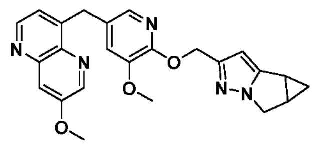 |
| 23 | 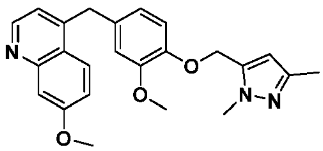 | 102 | 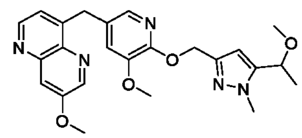 |
| 24 | 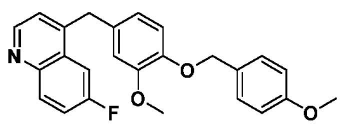 | 103 | 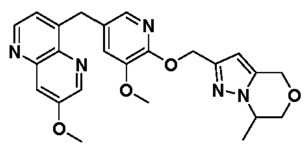 |

(continued)

| Example No | Structure | Example No | Structure |
|---|---|---|---|
| 25 | 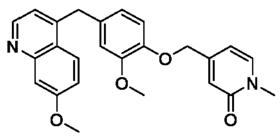 | 104 | 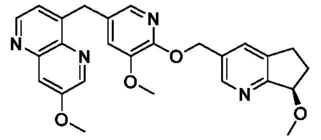 or 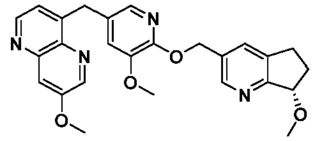 |
| 26 | 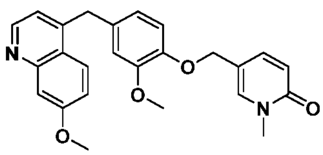 | 105 | 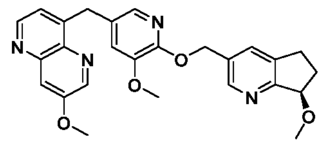 or 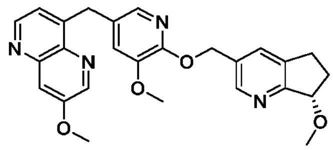 |
| 27 | 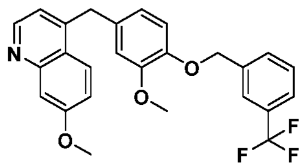 | 106 | 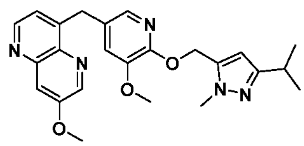 |
| 28 | 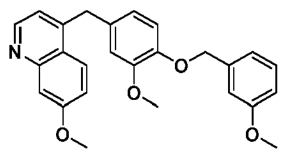 | 107 | 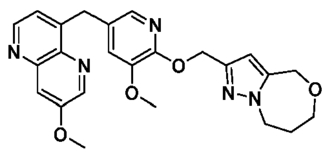 |
| 29 | 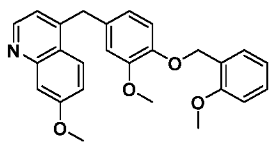 | 108 | 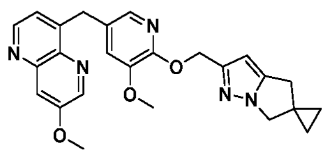 |
| 30 | 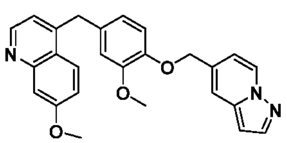 | 109 | 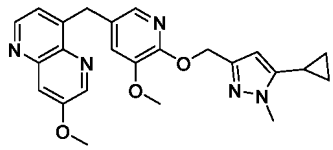 |
| 31 | 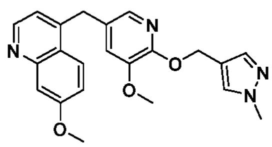 | 110 | 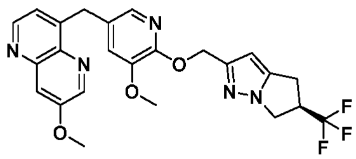 |

(continued)

| Example No | Structure | Example No | Structure |
|---|---|---|---|
| 32 | 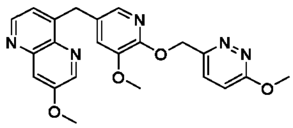 | 111 | 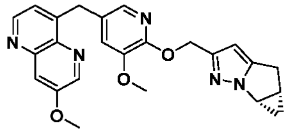 |
| 33 | 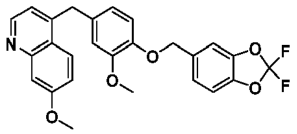 | 112 | 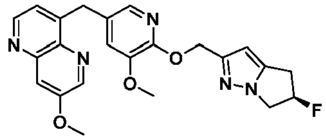 |
| 34 | 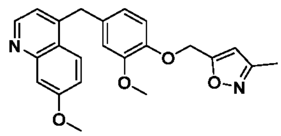 | 113 | 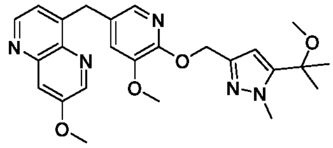 |
| 35 | 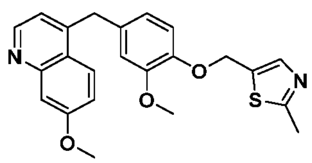 | 114 | 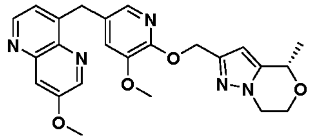 or 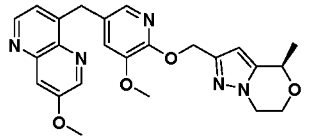 |
| 36 | 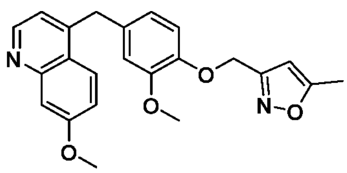 | 115 | 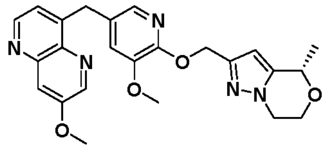 or 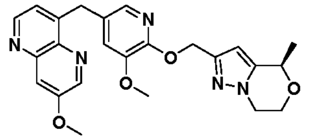 |
| 37 | 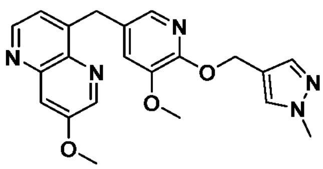 | 116 | 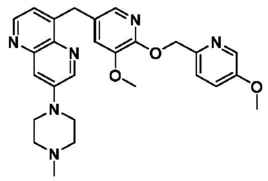 |
| 38 | 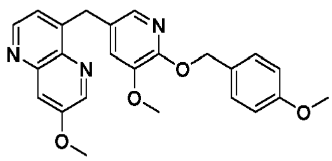 | 117 | 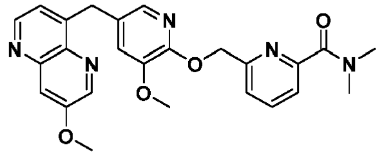 |

(continued)

| Example No | Structure | Example No | Structure |
|---|---|---|---|
| 39 | 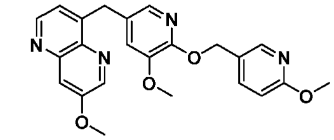 | 118 | 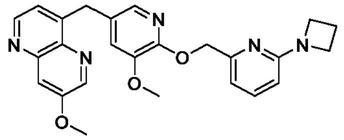 |
| 40 | 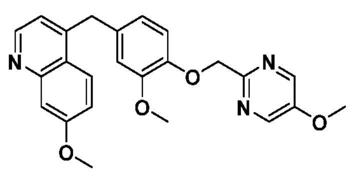 | 119 | 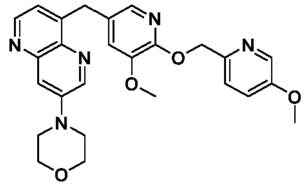 |
| 41 | 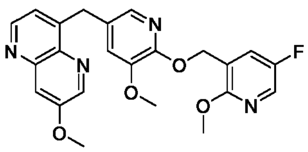 | 120 | 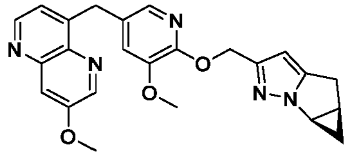 |
| 42 | 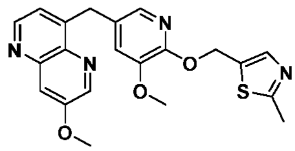 | 121 | 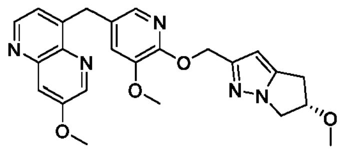 |
| 43 | 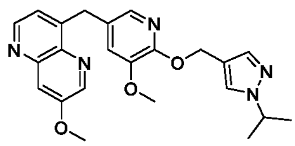 | 122 | 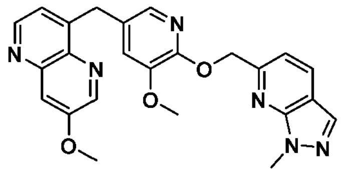 |
| 44 | 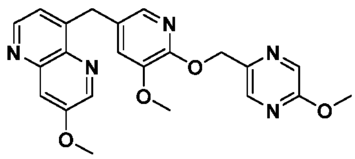 | 123 | 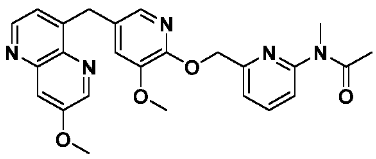 |
| 45 | 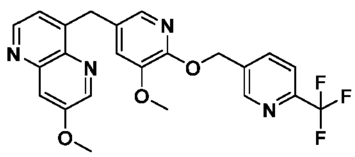 | 124 | 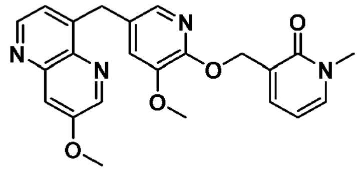 |
| 46 | 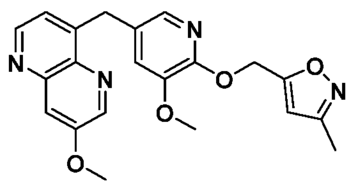 | 125 | 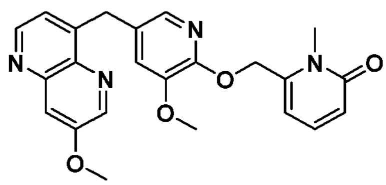 |
| 47 | 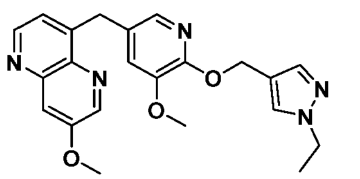 | 126 | 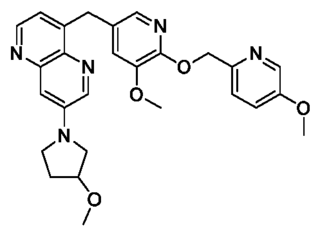 |

(continued)

| Example No | Structure | Example No | Structure |
|---|---|---|---|
| 48 | 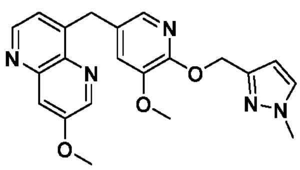 | 127 | 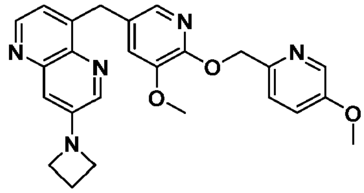 |
| 49 | 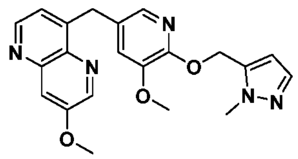 | 128 | 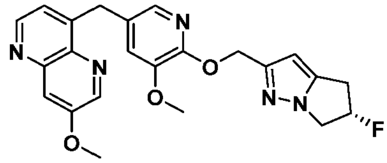 |
| 50 | 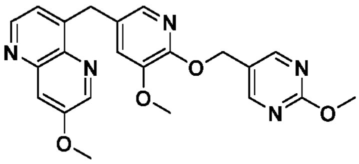 | 129 | 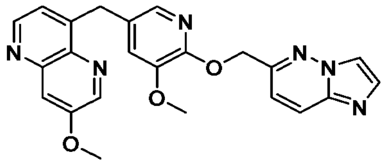 |
| 51 | 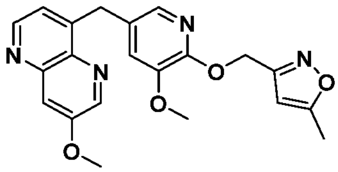 | 130 | 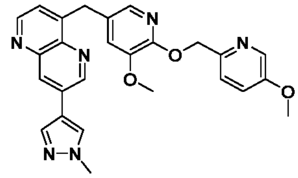 |
| 52 | 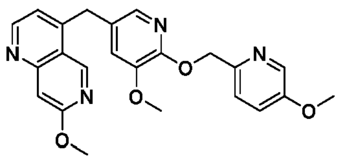 | 131 | 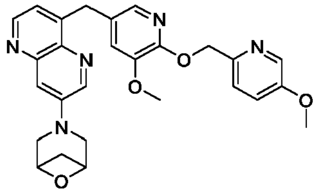 |
| 53 | 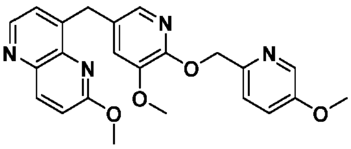 | 132 | 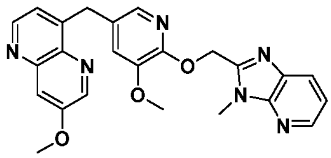 |
| 54 | 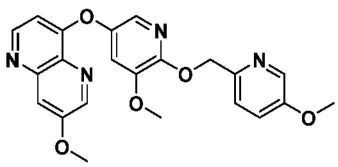 | 133 | 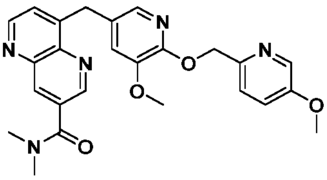 |
| 55 | 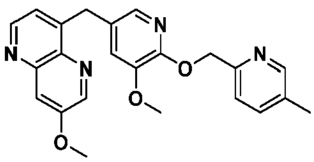 | 134 | 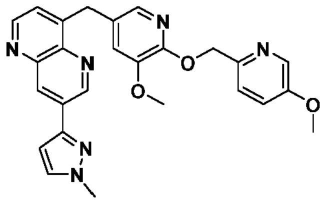 |

(continued)

| Example No | Structure | Example No | Structure |
|---|---|---|---|
| 56 | 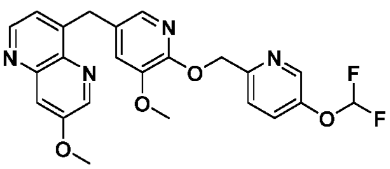 | 135 | 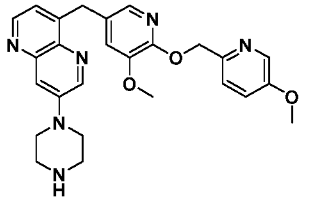 |
| 57 | 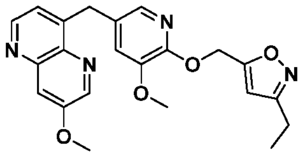 | 136 | 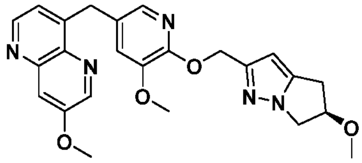 |
| 58 | 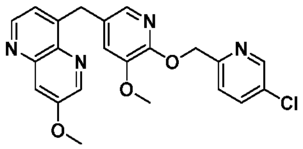 | 137 | 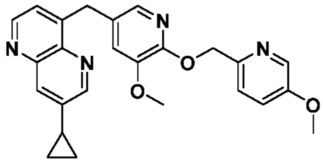 |
| 59 | 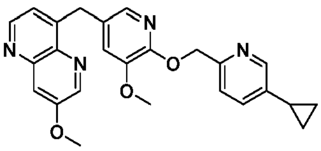 | 138 | 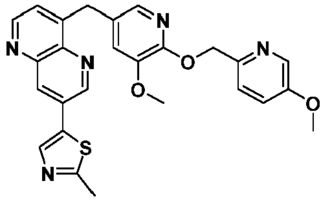 |
| 60 | 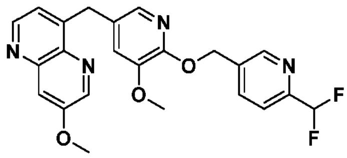 | 139 | 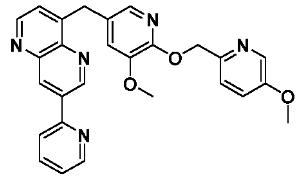 |
| 61 | 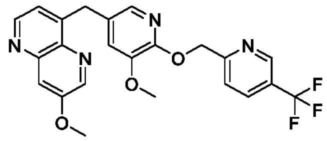 | 140 | 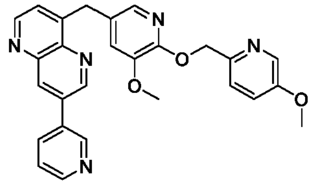 |
| 62 | 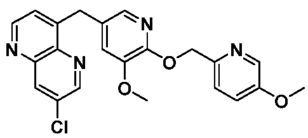 | 141 | 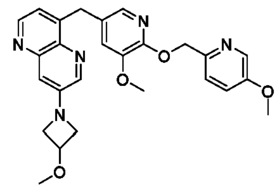 |
| 63 | 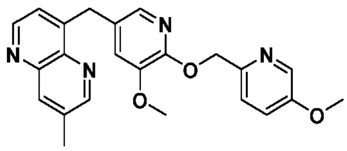 | 142 | 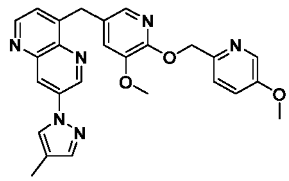 |

(continued)

| Example No | Structure | Example No | Structure |
|---|---|---|---|
| 64 | 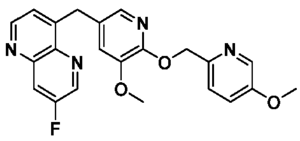 | 143 | 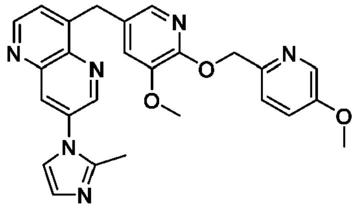 |
| 65 | 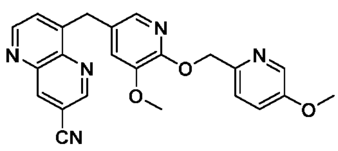 | 144 | 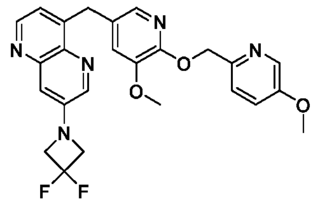 |
| 66 | 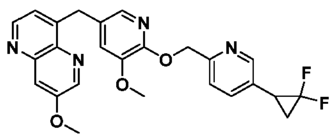 | 145 | 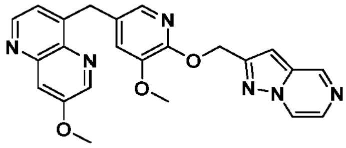 |
| 67 | 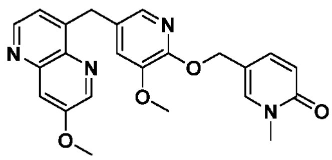 | 146 | 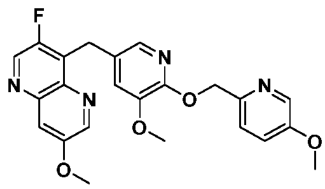 |
| 68 | 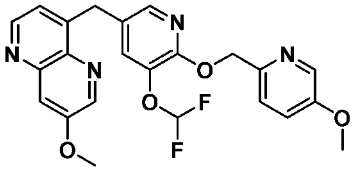 | 147 | 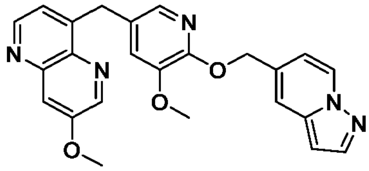 |
| 69 | 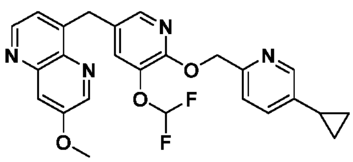 | 148 | 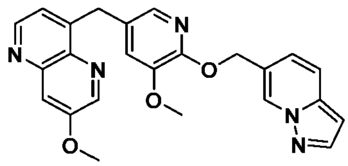 |
| 70 | 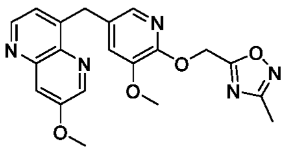 | 149 | 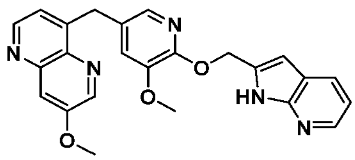 |
| 71 | 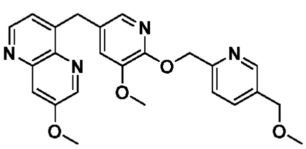 | 150 | 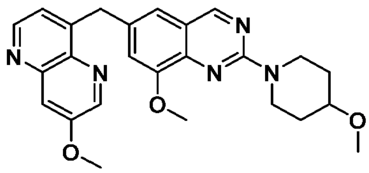 |

(continued)

| Example No | Structure | Example No | Structure |
|---|---|---|---|
| 72 | 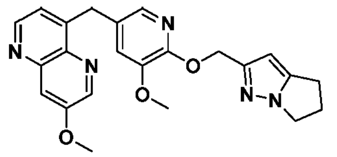 | 151 | 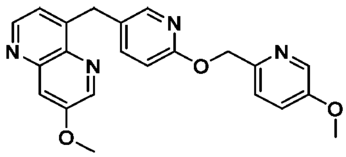 |
| 73 | 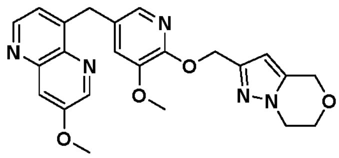 | 152 | 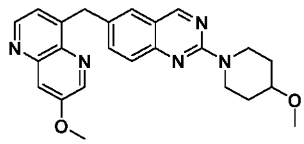 |
| 74 | 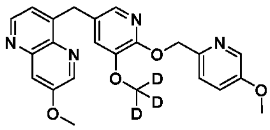 | 153 | 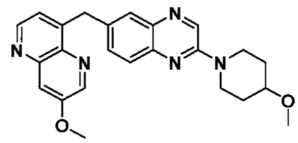 |
| 75 | 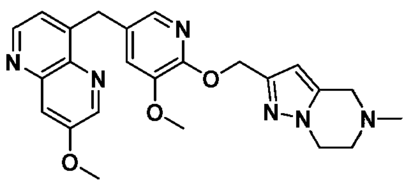 | 154 | 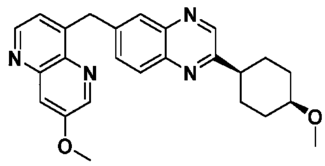 or 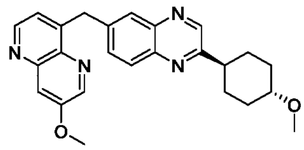 |
| 76 | 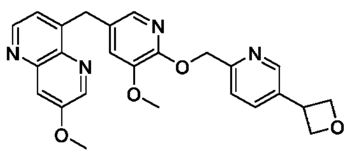 | 155 | 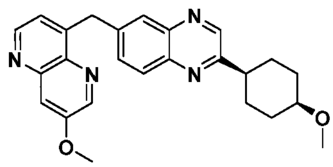 or 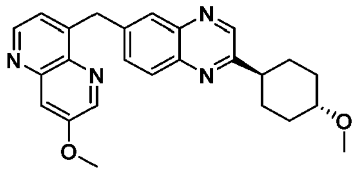 |
| 77 | 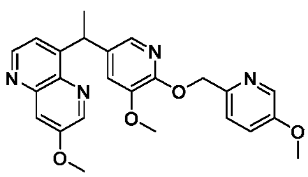 | 156 | 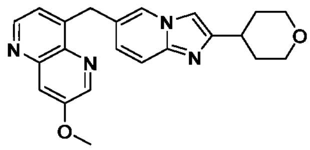 |
| 78 | 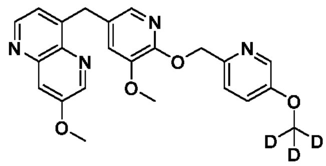 | 157 | 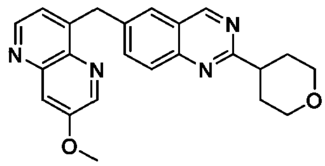 |

(continued)

| Example No | Structure | Example No | Structure |
|---|---|---|---|
| 79 | 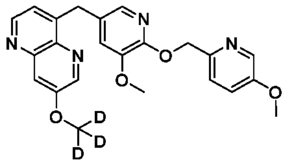 | 158 | 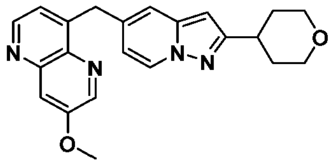 |

or a pharmaceutically acceptable salt thereof.

**13.** The compound of claim 12 or a pharmaceutically acceptable salt thereof, wherein the compound is

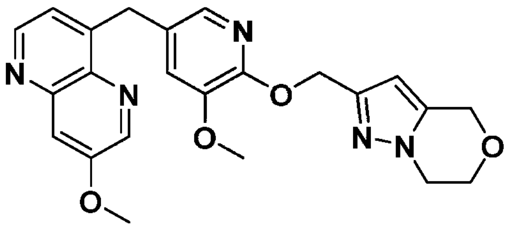

**14.** A pharmaceutical composition comprising the compound of any one of claims 1-13 or a pharmaceutically acceptable salt thereof, and a pharmaceutically acceptable carrier or excipient.

**15.** A compound of any one of claims 1-13, or a pharmaceutically acceptable salt thereof, for use in a method of treating a disease mediated by CSF-1R or at least in part by CSF-1R in a subject, wherein the disease is an autoimmune disease, an inflammatory disease, a neurodegenerative disease, cancer, a metabolic disease, obesity, or an obesity-related disease.

**16.** The compound for use of claim 15, wherein

the autoimmune disease or inflammatory disease is chosen from rheumatoid arthritis, collagen-induced arthritis, osteoarthritis, pigmented villonodular synovitis (PVNS), systemic lupus erythematosus, multiple sclerosis, systemic scleroderma, autoimmune nephritis, inflammatory bowel disease, Crohn's disease, ulcerative colitis, psoriasis, atopic dermatitis, asthma, chronic obstructive pulmonary disease, Behcet's disease, idiopathic thrombocytopenic purpura, spinal arthritis, systemic juvenile idiopathic arthritis (SoJIA), pancreatitis, ischemia reperfusion injury of parenchymatous organs, organ-graft rejection, septicemia, systemic inflammatory response syndrome, and chemotherapy drugs induced organ injury;

the neurodegenerative disease is chosen from Parkinson's disease (PD), multiple system atrophy, Alzheimer's disease (AD), frontotemporal lobar dementia, Huntington's disease (HD), corticobasal degeneration, spinocerebellar ataxia, amyotrophic lateral sclerosis (ALS), spinal muscular atrophy (SMA), and hereditary motor and sensory neuropathy (CMT); and

the cancer is solid tumor or hematologic malignancy, such as ovarian cancer, lung cancer (including non-small cell lung cancer), brain tumor (including glioblastoma (GBM)), tenosynovial giant cell tumor, gastrointestinal stromal tumor (GIST), gastric cancer, esophageal cancer, colon cancer, colorectal cancer, pancreatic cancer, prostate cancer, breast cancer, cervical cancer, melanoma, mesothelioma, mesothelial carcinoma, renal cancer, liver cancer, thyroid carcinoma, head and neck cancer, urothelial carcinoma, bladder cancer, endometrial cancer, choriocarcinoma, adrenal carcinoma, sarcoma, leukemia, lymphoma, or myeloma.

**17.** A compound of any one of claims 1-13 or a pharmaceutically acceptable salt thereof, for use in a method of treating Alzheimer's disease, progressive supranuclear palsy, a tau-mediated neurodegenerative disorder, or a disease or disorder involving microglia-mediated inflammation in a subject.

## Patentansprüche

**1.** Verbindung, dargestellt durch Formel (I):

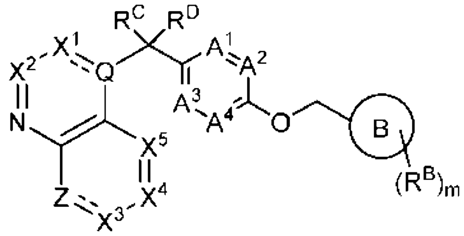

(I)

pharmazeutisch unbedenkliches Salz oder Tautomer davon, wobei:

Q C oder N ist;
wenn Q N ist, Q-$X^1$ eine Einfachbindung ist und $X^1$ C=O ist;
wenn Q C ist, Q-$X^1$ eine Doppelbindung ist und $X^1$ N oder $CR^1$ ist;
Z CH oder NH ist;
wenn Z NH ist, Z-$X^3$ eine Einfachbindung ist und $X^3$ C=O ist;
wenn Z CH ist, Z-$X^3$ eine Doppelbindung ist und $X^3$ N oder $CR^3$ ist;
$X^2$ N oder $CR^2$ ist;
$X^4$ N oder $CR^4$ ist;
$X^5$ N oder $CR^5$ ist;
jedes $R^1$, $R^2$, $R^3$, $R^4$ und $R^5$ unabhängig ausgewählt ist aus H, Halogen, -CN, -OH, $C_{1-6}$-Alkyl optional substituiert mit einem bis drei Deuterium, $C_{1-6}$-Haloalkyl, $C_{1-6}$-Hydroxylalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0 \text{ oder } 1}$-3-12-gliedrigem Carbocyclyl, $-(CH_2)_{0 \text{ oder } 1}$-3-12-gliedrigem Heterocyclyl, $-(CH_2)_{0 \text{ oder } 1}$-6-10-gliedrigem Aryl oder $-(CH_2)_{0 \text{ oder } 1}$-5-10-gliedrigem Heteroaryl; wobei das Carbocyclyl, Heterocyclyl, Aryl oder Heteroaryl in der Gruppe dargestellt durch $R^1$, $R^2$, $R^3$, $R^4$ $_{\text{oder}}$ $R^5$ optional substituiert ist durch eine oder zwei Gruppen ausgewählt aus CN, Halogen, $C_{1-6}$-Alkyl, $C_{1-6}$-Haloalkyl, $C_{1-6}$-Alkoxy und $C_{1-6}$-Haloalkoxy;
jedes $A^1$, $A^2$, $A^3$ und $A^4$ unabhängig N oder $CR^A$ ist; und nicht mehr als zwei von $A^1$, $A^2$, $A^3$ und $A^4$ N sind;
jedes $R^A$ unabhängig ausgewählt ist aus Halogen, -CN, -OH, $C_{1-6}$-Alkyl, $C_{1-6}$-Haloalkyl, $C_{1-6}$-Hydroxylalkyl, $C_{1-6}$-Alkoxyalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, - C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, - NR*SO2NR*R*, -P(O)R*R*, $-(CH_2)_{0 \text{ oder } 1}$-3-12-gliedrigem Carbocyclyl, $-(CH_2)_{0 \text{ oder } 1}$-3-12-gliedrigem Heterocyclyl, $-(CH_2)_{0 \text{ oder } 1}$-6-10-gliedrigem Aryl oder $-(CH_2)_{0 \text{ oder } 1}$-5-10-gliedrigem Heteroaryl; wobei das Carbocyclyl, Heterocyclyl, Aryl oder Heteroaryl in der Gruppe dargestellt durch $R^A$ optional substituiert ist durch eine oder zwei Gruppen ausgewählt aus CN, Halogen, $C_{1-6}$-Alkyl, $C_{1-6}$-Haloalkyl, $C_{1-6}$-Alkoxy und $C_{1-6}$-Haloalkoxy;
Ring B Phenyl oder 5- oder 6-gliedriges monocyclisches Heteroaryl ist;
jedes $R^B$ unabhängig ausgewählt ist aus Halogen, -CN, -OH, $C_{1-6}$-Alkyl, $C_{1-6}$-Haloalkyl, $C_{1-6}$-Hydroxylalkyl, $C_{1-6}$-Alkoxyalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, - C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, - NR*SO2NR*R*, -P(O)R*R*, $-(CH_2)_{0 \text{ oder } 1}$-3-12-gliedrigem Carbocyclyl, $-(CH_2)_{0 \text{ oder } 1}$-3-12-gliedrigem Heterocyclyl, $-(CH_2)_{0 \text{ oder } 1}$-6-10-gliedrigem Aryl oder $-(CH_2)_{0 \text{ oder } 1}$-5-10-gliedrigem Heteroaryl; wobei das Carbocyclyl, Heterocyclyl, Aryl oder Heteroaryl in der Gruppe dargestellt durch $R^B$ optional substituiert ist durch eine oder zwei Gruppen ausgewählt aus CN, Halogen, $C_{1-6}$-Alkyl, $C_{1-6}$-Haloalkyl, $C_{1-6}$-Alkoxy und $C_{1-6}$-Haloalkoxy; und/oder
zwei $R^B$ zusammen mit den Ring-B-Atomen, an die sie gebunden sind, 5-7-gliedrigen monocyclischen Ring oder 6-9-gliedrigen bicyclischen Ring kondensiert an Ring B bilden, wobei der 5-7-gliedrige monocyclische oder 6-9-gliedrige bicyclische Ring optional substituiert ist mit einer oder zwei Gruppen ausgewählt aus CN, Halogen, $C_{1-6}$-Alkyl, $C_{1-6}$-Haloalkyl, $C_{1-6}$-Alkoxy, $C_{1-6}$-Haloalkoxy und 3-6-gliedrigem Cycloalkyl;
jedes $R^C$ und $R^D$ unabhängig ausgewählt ist aus Wasserstoff, Deuterium, F und Methyl;
jedes R* unabhängig ausgewählt ist aus der Gruppe bestehend aus H, $C_{1-6}$-Alkyl, $C_{1-6}$-Haloalkyl, $C_{2-6}$-Alkenyl, $C_{2-6}$-Alkinyl, 3-6-gliedrigem Carbocyclyl oder 4-6-gliedrigem Heterocyclyl; und
m 0, 1, 2, 3 oder 4 ist.

**2.** Verbindung nach Anspruch 1 oder pharmazeutisch unbedenkliches Salz davon, wobei:

jedes $R^1$, $R^2$, $R^3$, $R^4$ und $R^5$ unabhängig ausgewählt ist aus H, Halogen, -CN, -OH, $C_{1-6}$-Alkyl, $C_{1-6}$-Haloalkyl, $C_{1-6}$-Hydroxylalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, - C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, - NR*SO2NR*R*, -P(O)R*R*, $-(CH_2)_{0\ oder\ 1}$-3-12-gliedrigem Carbocyclyl, $-(CH_2)_{0\ oder\ 1}$-3-12-gliedrigem Heterocyclyl, $-(CH_2)_{0\ oder\ 1}$-6-10-gliedrigem Aryl oder $-(CH_2)_{0\ oder\ 1}$-5-10-gliedrigem Heteroaryl; wobei das Carbocyclyl, Heterocyclyl, Aryl oder Heteroaryl in der Gruppe dargestellt durch $R^1$, $R^2$, $R^3$, $R^4$ oder $R^5$ optional substituiert ist durch eine oder zwei Gruppen ausgewählt aus CN, Halogen, $C_{1-6}$-Alkyl, $C_{1-6}$-Haloalkyl, $C_{1-6}$-Alkoxy und $C_{1-6}$-Haloalkoxy;

jedes $R^A$ unabhängig ausgewählt ist aus H, Halogen, -CN, -OH, $C_{1-6}$-Alkyl, $C_{1-6}$-Haloalkyl, $C_{1-6}$-Hydroxylalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, -C(O)OR*, - C(O)NR*R*, $-SO_2R^*$, $-(CH_2)_{0-4}NR^*R^*$, -NR*C(O)R*, -NR*CO) OR*, $-NR^*SO_2R^*$, $-NR^*SO_2NR^*R^*$, -P(O)R*R*, $-(CH_2)_{0\ oder\ 1}$-3-12-gliedrigem Carbocyclyl, $-(CH_2)_{0\ oder\ 1}$-3-12-gliedrigem Heterocyclyl, $-(CH_2)_{0\ oder\ 1}$-6-10-gliedrigem Aryl oder $-(CH_2)_{0\ oder\ 1}$-5-10-gliedrigem Heteroaryl; wobei das Carbocyclyl, Heterocyclyl, Aryl oder Heteroaryl in der Gruppe dargestellt durch $R^A$ optional substituiert ist durch eine oder zwei Gruppen ausgewählt aus CN, Halogen, $C_{1-6}$-Alkyl, $C_{1-6}$-Haloalkyl, $C_{1-6}$-Alkoxy und $C_{1-6}$-Haloalkoxy;

jedes $R^B$ unabhängig ausgewählt ist aus Halogen, -CN, -OH, $C_{1-6}$-Alkyl, $C_{1-6}$-Haloalkyl, $C_{1-6}$-Hydroxylalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, -C(O)OR*, - C(O)NR*R*, $-SO_2R^*$, $-(CH_2)_{0-4}NR^*R^*$, -NR*C(O)R*, -NR*C(O)OR*, $-NR^*SO_2R^*$, - NR*SO2NR*R*, -P(O)R*R*, $-(CH_2)_{0\ oder\ 1}$-3-12-gliedrigem Carbocyclyl, $-(CH_2)_{0\ oder\ 1}$-3-12-gliedrigem Heterocyclyl, $-(CH_2)_{0\ oder\ 1}$-6-10-gliedrigem Aryl oder $-(CH_2)_{0\ oder\ 1}$-5-10-gliedrigem Heteroaryl; wobei das Carbocyclyl, Heterocyclyl, Aryl oder Heteroaryl in der Gruppe dargestellt durch $R^B$ optional substituiert ist durch eine oder zwei Gruppen ausgewählt aus CN, Halogen, $C_{1-6}$-Alkyl, $C_{1-6}$-Haloalkyl, $C_{1-6}$-Alkoxy und $C_{1-6}$-Haloalkoxy; oder

zwei $R^B$ zusammen mit den Ring-B-Atomen, an die sie gebunden sind, 5,6-gliedrigen Ring kondensiert an Ring B bilden, wobei der 5,6-gliedrige Ring optional substituiert ist mit einer oder zwei Gruppen ausgewählt aus CN, Halogen, $C_{1-6}$-Alkyl, $C_{1-6}$-Haloalkyl, $C_{1-6}$-Alkoxy und $C_{1-6}$-Haloalkoxy; und

jedes $R^C$ und $R^D$ unabhängig ausgewählt ist aus Wasserstoff, F und Methyl; wobei optional die Verbindung dargestellt ist durch eine beliebige von Formel (II-A), Formel (III-A-1), Formel (III-A-2), Formel (III-A-3), Formel (III-A-4), Formel (III-A-5), Formel (III-A-6), Formel (III-A-7), Formel (III-B-1), Formel (III-B-2), Formel (III-C-1), Formel (III-C-2) oder Formel (III-C-3):

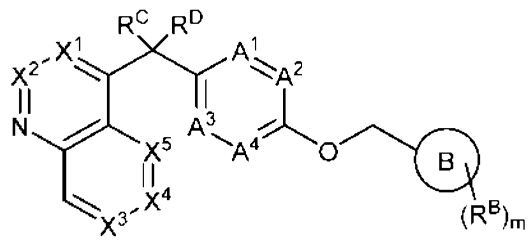
(II-A),

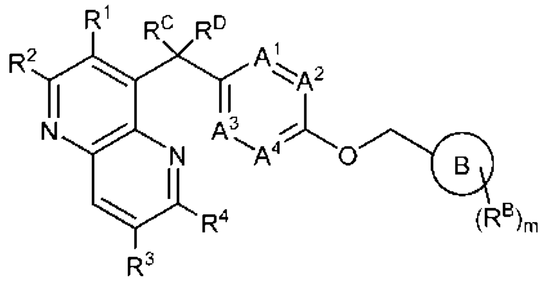
(III-A-1),

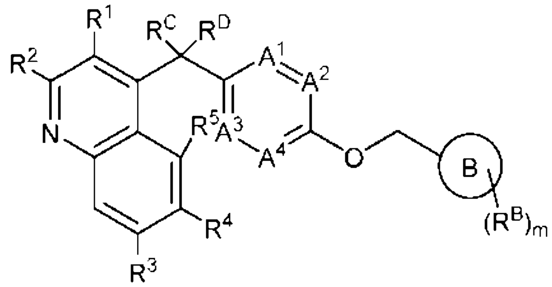

(III-A-2),

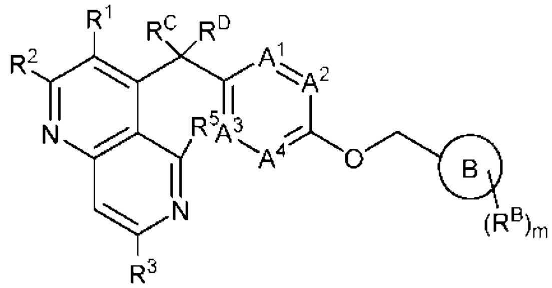

(III-A-3),

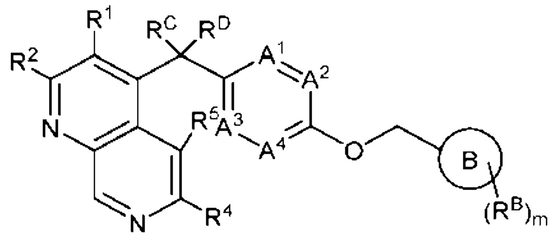

(III-A-4),

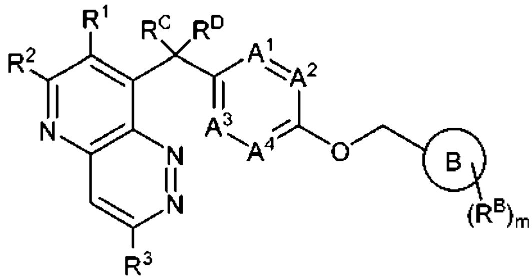

(III-A-5),

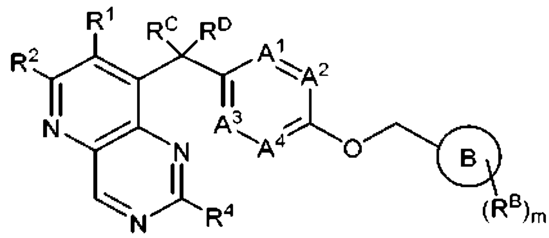

(III-A-6),

oder

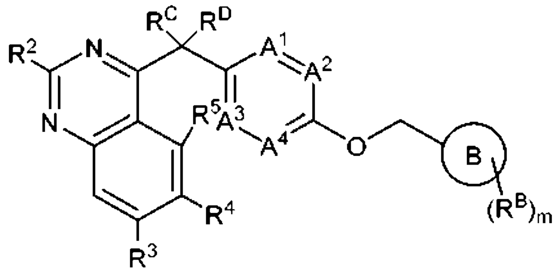

(III-A-7),

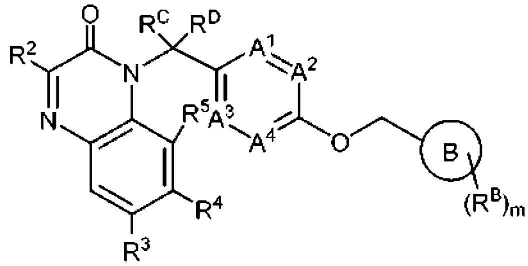

(III-B-1),

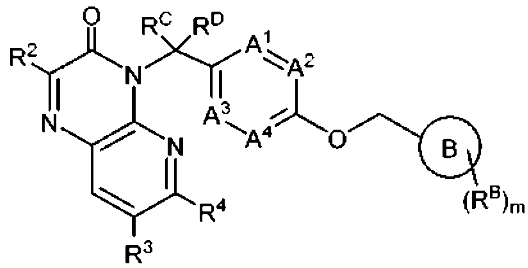

(III-B-2);

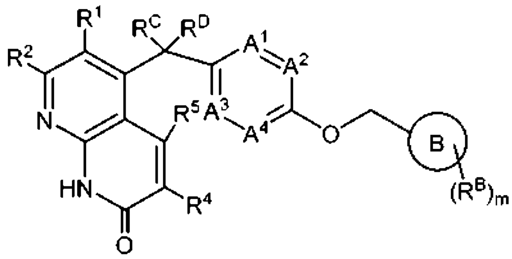

(III-C-1),

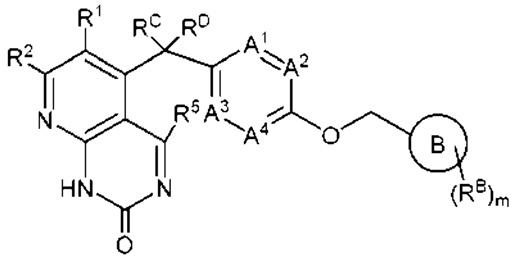

(III-C-2),

oder

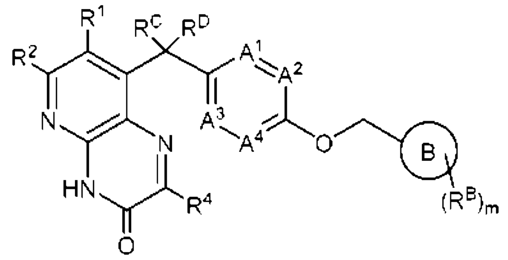

(III-C-3);

oder pharmazeutisch unbedenkliches Salz davon.

**3.** Verbindung nach Anspruch 2 oder pharmazeutisch unbedenkliches Salz davon, wobei:

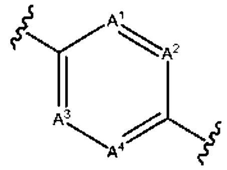

Phenyl oder Pyridyl ist, von denen jedes optional substituiert ist durch ein oder zwei $R^A$; und/oder Ring B wie folgt ist:

(i) Phenyl optional substituiert durch ein oder zwei $R^B$, wobei jedes $R^B$ unabhängig ausgewählt ist aus Halogen, -CN, -OH, $C_{1-6}$-Alkyl, $C_{1-6}$-Haloalkyl, $C_{1-6}$-Hydroxylalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, -C(O)OR*, -C(O) NR*R*, -OR*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ oder }1}$-3-12-gliedrigem Carbocyclyl, $-(CH_2)_{0\text{ oder }1}$-3-12-gliedrigem Heterocyclyl, $-(CH_2)_{0\text{ oder }1}$-6-10-gliedrigem Aryl oder $-(CH_2)_{0\text{ oder }1}$-5-10-gliedrigem Heteroaryl; wobei das Carbocyclyl, Heterocyclyl, Aryl oder Heteroaryl in der Gruppe dargestellt durch $R^B$ optional substituiert ist durch ein oder zwei Halogen oder $C_{1-6}$-Alkyl; oder

(ii) 5, 6-gliedriges monocyclisches Heteroaryl optional substituiert durch ein oder zwei $R^B$, wobei jedes $R^B$ unabhängig ausgewählt ist aus Halogen, -CN, -OH, $C_{1-6}$-Alkyl, $C_{1-6}$-Haloalkyl, $C_{1-6}$-Hydroxylalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, -C(O)OR*, -C(O)NR*R*, - OR*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, - P(O)R*R*, $-(CH_2)_{0\text{ oder }1}$-3-12-gliedrigem Carbocyclyl, $-(CH_2)_{0\text{ oder }1}$-3-12-gliedrigem Heterocyclyl, $-(CH_2)_{0\text{ oder }1}$-6-10-gliedrigem Aryl oder $-(CH_2)_{0\text{ oder }1}$-5-10-gliedrigem Heteroaryl; wobei das Carbocyclyl, Heterocyclyl, Aryl oder Heteroaryl in der Gruppe dargestellt durch $R^B$ optional substituiert ist durch ein oder zwei Halogen oder $C_{1-6}$-Alkyl; oder

(iii) Phenyl oder 5, 6-gliedriges monocyclisches Heteroaryl, von denen jedes optional substituiert ist durch ein bis vier $R^B$, und zwei $R^B$ zusammen mit den Ring-B-Atomen, an die sie gebunden sind, 5,6-gliedrigen Ring kondensiert an Ring B bilden, wobei der 5,6-gliedrige kondensierte Ring optional substituiert ist mit einem oder zwei CN, Halogen, $C_{1-4}$-Alkyl,

$C_{1-4}$-Haloalkyl, $C_{1-4}$-Alkoxy oder $C_{1-4}$-Haloalkoxy; oder

(iv) Pyrazolyl, Isoxazolyl, 1,2,4-Oxadiazolyl, Thiazolyl, Phenyl, Pyridyl, Pyrimidyl, Pyrazinyl, Imidazo[1,2-a] pyridinyl, Benzo[d]imidazolyl,

Pyrazolo[1,5-a]pyridinyl, Indazolyl, [1,2,4]Triazolo[1,5-a]pyridinyl,
4,5,6,7-Tetrahydropyrazolo[1,5-a]pyrazinyl,
6,7-Dihydro-4H-pyrazolo[5,1-c][1,4]oxazinyl,
5,6-Dihydro-4H-pyrrolo[1,2-b]pyrazolyl oder Benzo[d][1,3]dioxolyl.

**4.** Verbindung nach Anspruch 2 oder 3 oder pharmazeutisch unbedenkliches Salz davon, wobei: jedes $R^1$ unabhängig ausgewählt ist aus H, Halogen, -CN, -OH,

$C_{1-4}$-Alkyl, $C_{1-4}$-Haloalkyl, $C_{1-4}$-Hydroxylalkyl, $-(CH_2)_{0-4}C_{1-4}$-Alkoxy, $C_{1-4}$-Haloalkoxy, $C_{1-4}$-Hydroxyalkoxy, -C(O)R*, -C(O)OR*, -C(O)NR*R*, $-(CH_2)_{0-4}NR^*R^*$, -NR*C(O)R* und - NR*C(O)OR*; und/oder

jedes $R^2$ unabhängig ausgewählt ist aus H, Halogen, -CN, -OH, $C_{1-4}$-Alkyl, $C_{1-4}$-Haloalkyl, $C_{1-4}$-Hydroxylalkyl, $-(CH_2)_{0-4}C_{1-4}$-Alkoxy, $C_{1-4}$-Haloalkoxy, $C_{1-4}$-Hydroxyalkoxy, -C(O)R*, - C(O)OR*, -C(O)NR*R*, $-(CH_2)_{0-4}NR^*R^*$, -NR*C(O)R* und -NR*C(O)OR*; und/oder

jedes $R^3$ unabhängig ausgewählt ist aus H, Halogen, -CN, -OH, $C_{1-4}$-Alkyl, $C_{1-4}$-Haloalkyl, $C_{1-4}$-Hydroxylalkyl, $-(CH_2)_{0-2}C_{1-4}$-Alkoxy optional substituiert mit einem bis drei Deuterium, $C_{1-4}$-Haloalkoxy, $C_{1-4}$-Hydroxyalkoxy, -C(O)R*, -C(O)OR*, -C(O)NR*R*, $-(CH_2)_{0-4}NR^*R^*$, - NR*C(O)R* und -NR*C(O)OR*; und/oder

jedes $R^4$ unabhängig ausgewählt ist aus H, Halogen, -CN, -OH, $C_{1-4}$-Alkyl, $C_{1-4}$-Haloalkyl, $C_{1-4}$-Hydroxylalkyl, $-(CH_2)_{0-4}C_{1-4}$-Alkoxy, $C_{1-4}$-Haloalkoxy, $C_{1-4}$-Hydroxyalkoxy, -C(O)R*, - C(O)OR*, -C(O)NR*R*, $-(CH_2)_{0-4}NR^*R^*$, -NR*C(O)R* und -NR*C(O)OR*; und/oder

jedes $R^5$ unabhängig ausgewählt ist aus H, Halogen, -CN, -OH, $C_{1-4}$-Alkyl, $C_{1-4}$-Haloalkyl, $C_{1-4}$-Hydroxylalkyl, $-(CH_2)_{0-4}C_{1-4}$-Alkoxy, $C_{1-4}$-Haloalkoxy, $C_{1-4}$-Hydroxyalkoxy, -C(O)R*, - C(O)OR*, -C(O)NR*R*, -NR*R*, -NR*C(O)R* und -NR*C(O)OR*; und/oder

jedes $R^A$ unabhängig ausgewählt ist aus H, Halogen, -CN, -OH, $C_{1-4}$-Alkyl, $C_{1-4}$-Haloalkyl, $C_{1-4}$-Hydroxylalkyl, $C_{1-4}$-Alkoxy optional substituiert mit einem bis drei Deuterium, $C_{1-4}$-Haloalkoxy, $C_{1-4}$-Hydroxyalkoxy, -C(O)R*, -C(O)OR*, -C(O)NR*R*, $-(CH_2)_{0-4}NR^*R^*$, - NR*C(O)R* und -NR*C(O)OR*; und/oder

jedes $R^B$ unabhängig ausgewählt ist aus Halogen, -CN, -OH, $C_{1-4}$-Alkyl, $C_{1-4}$-Haloalkyl, $C_{1-4}$-Hydroxylalkyl, $-(CH_2)_{0-2}C_{1-4}$-Alkoxy optional substituiert mit einem bis drei Deuterium, $C_{1-4}$-Haloalkoxy, $C_{1-4}$-Hydroxyalkoxy, -C(O)R*, -C(O)OR*, -C(O)NR*R*, $-(CH_2)_{0-4}NR^*R^*$, - NR*C(O)R*, -NR*C(O)OR* und $-(CH_2)_{0\ oder\ 1}$-3-6-gliedrigem Cycloalkyl optional substituiert mit einem oder zwei Halogen; und/oder

jedes R* unabhängig H oder $C_{1-4}$-Alkyl (bevorzugt H oder $C_{1-2}$-Alkyl) ist.

**5.** Verbindung nach einem der Ansprüche 2-4 oder pharmazeutisch unbedenkliches Salz davon, wobei:

jedes $R^1$ unabhängig ausgewählt ist aus H, Halogen, $C_{1-4}$-Alkyl, $C_{1-4}$-Haloalkyl, $C_{1-4}$-Alkoxy und $C_{1-4}$-Haloalkoxy (bevorzugt H); und

jedes $R^2$ unabhängig ausgewählt ist aus H, Halogen, $C_{1-4}$-Alkyl, $C_{1-4}$-Haloalkyl, $C_{1-4}$-Alkoxy und $C_{1-4}$-Haloalkoxy (bevorzugt H); und/oder

jedes $R^3$ unabhängig ausgewählt ist aus H, Halogen, $C_{1-4}$-Alkyl, $C_{1-4}$-Haloalkyl, $C_{1-4}$-Alkoxy optional substituiert mit einem bis drei Deuterium und $C_{1-4}$-Haloalkoxy (bevorzugt H, CN, Halogen, $C_{1-4}$-Alkyl oder $C_{1-4}$-Alkoxy optional substituiert mit einem bis drei Deuterium); und/oder jedes $R^4$ unabhängig ausgewählt ist aus H, Halogen, $C_{1-4}$-Alkyl, $C_{1-4}$-Haloalkyl, $C_{1-4}$-Alkoxy und $C_{1-4}$-Haloalkoxy (bevorzugt H, F oder $C_{1-4}$-Alkoxy); und/oder

jedes $R^5$ unabhängig ausgewählt ist aus H, Halogen, $C_{1-4}$-Alkyl, $C_{1-4}$-Haloalkyl, $C_{1-4}$-Alkoxy und $C_{1-4}$-Haloalkoxy (bevorzugt H); und/oder

jedes $R^A$ unabhängig ausgewählt ist aus H, $C_{1-2}$-Alkyl oder $C_{1-2}$-Alkoxy optional substituiert mit einem bis drei Deuterium (bevorzugt H, $-OCH_3$ oder $-OCD_3$); und/oder

jedes $R^B$ unabhängig ausgewählt ist aus Halogen, -OH, $C_{1-3}$-Alkyl, $C_{1-3}$-Haloalkyl, $-(CH_2)_{0-2}C_{1-2}$-Alkoxy optional substituiert mit einem bis drei Deuterium, $C_{1-2}$-Haloalkoxy, $-(CH_2)_{0-2}NR^*R^*$ oder 3-6-gliedrigem Cycloalkyl optional substituiert mit einem oder zwei Halogen (bevorzugt F, Cl, -OH, $-CH_3$, $-CH_2CH_3$, $-CH(CH_3)_2$, $-CHF_2$, $-CF_3$, $-CH_2N(CH_3)_2$,$-CH_2OCH_3$, $-OCH_3$, $-OCHF_2$, $-OCD_3$, Cyclopropyl optional substituiert mit einem oder zwei Fluor); und/oder

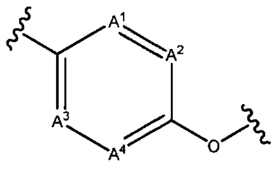

wie folgt ist

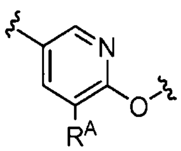

oder

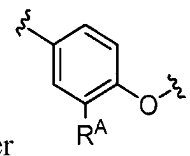

.

**6.** Verbindung nach Anspruch 2, 4 oder 5, wobei die Verbindung dargestellt ist durch Formel (IV-A-1) oder (IV-A-2):

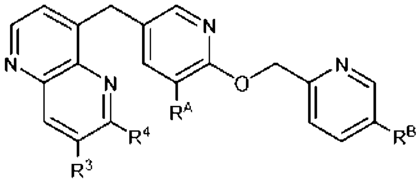

(IV-A-1),

oder

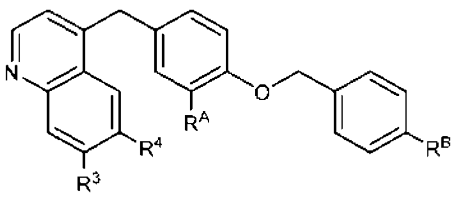

(IV-A-2),

oder pharmazeutisch unbedenkliches Salz davon.

**7.** Verbindung nach einem der Ansprüche 1-3 und 6, pharmazeutisch unbedenkliches Salz oder Tautomer davon, wobei:

(i) jedes $R^3$ unabhängig ausgewählt ist aus H, Halogen, -CN, $C_{1-6}$-Alkyl optional substituiert mit einem bis drei Deuterium, $C_{1-6}$-Haloalkyl, $C_{1-6}$-Hydroxylalkyl, -$C_{1-6}$-Alkoxy optional substituiert mit einem bis drei Deuterium, $C_{1-6}$-Haloalkoxy, $C_{1-6}$-Hydroxyalkoxy, - C(O)R*, -C(O)OR*, -C(O)NR*R*, -NR*R*, 3-6-gliedrigem Carbocyclyl, 3-6-gliedrigem monocyclischen Heterocyclyl, 7-10-gliedrigem verbrückten Heterocyclyl, Phenyl oder 5-6-gliedrigem Heteroaryl; wobei das Carbocyclyl, Heterocyclyl, Phenyl oder Heteroaryl in der Gruppe dargestellt durch $R^3$ optional substituiert ist durch ein oder zwei CN, Halogen, $C_{1-4}$-Alkyl, $C_{1-4}$-Haloalkyl, $C_{1-4}$-Alkoxy oder $C_{1-4}$-Haloalkoxy; oder
(ii) jedes $R^3$ unabhängig ausgewählt ist aus H, Halogen, CN, $C_{1-4}$-Alkyl optional substituiert mit einem bis drei Deuterium, $C_{1-4}$-Alkoxy optional substituiert mit einem bis drei Deuterium, NR*R*, -C(O)NR*R*, 3-6-gliedrigem Cycloalkyl, 3-6-gliedrigem monocyclischen Heterocyclyl, 5-6-gliedrigem Heteroaryl oder 6-Oxa-3-azabicyclo [3.1.1]heptanyl, wobei das Cycloalkyl, Heterocyclyl oder Heteroaryl in der Gruppe dargestellt durch $R^3$ optional substituiert ist durch ein oder zwei Halogen, $C_{1-2}$-Alkyl, $C_{1-2}$-Haloalkyl, $C_{1-2}$-Alkoxy oder $C_{1-2}$-Haloalkoxy; oder
(iii) jedes $R^3$ unabhängig ausgewählt ist aus H, F, Cl, CN, $CH_3$, $OCH_3$, $OCD_3$, -$N(CH_3)_2$, - $CON(CH_3)_2$, Cyclopropanyl, Azetidinyl optional substituiert mit einem oder zwei Fluor oder $OCH_3$, Imidazol optional substituiert mit $CH_3$, Morpholinyl, Pyridyl, Piperazinyl optional substituiert mit $CH_3$, Pyrrolidinyl optional substituiert mit $OCH_3$, Pyrazolyl optional substituiert mit $CH_3$, Thiazol optional substituiert mit $CH_3$ oder 6-Oxa-3-azabicyclo[3.1.1] heptanyl; oder
(iv) jedes $R^3$ unabhängig ausgewählt ist aus H oder $OCH_3$,

**8.** Verbindung nach einem der Ansprüche 1, 2, 4, 5 oder 7, pharmazeutisch unbedenkliches Salz oder Tautomer davon, wobei:

(i) Ring B Phenyl, 5- oder 6-gleidriges monocyclisches Heteroaryl optional substituiert durch ein oder zwei $R^B$ ist, jedes $R^B$ unabhängig ausgewählt ist aus Halogen, -OH, $C_{1-4}$-Alkyl, $C_{1-4}$-Haloalkyl, $C_{1-4}$-Alkoxyalkyl, $C_{1-4}$-Alkoxy optional substituiert mit einem bis drei Deuterium, $C_{1-4}$-Haloalkoxy, -C(O)NR*R*, -$(CH_2)_{0-1}$NR*R*, -NR*C(O)R*, -3-6-gliedrigem monocyclischen Carbocyclyl, -3-6-gliedrigem monocyclischen Heterocyclyl; wobei das Carbocyclyl oder Heterocyclyl in der Gruppe dargestellt durch $R^B$ optional substituiert ist durch ein oder zwei Halogen oder $C_{1-4}$-Alkyl; oder
jedes $R^B$ unabhängig ausgewählt ist aus F, Cl, -OH, $C_{1-4}$-Alkyl, $C_{1-4}$-Haloalkyl, $C_{1-4}$-Alkoxyalkyl, $C_{1-4}$-Alkoxy optional substituiert mit einem bis drei Deuterium, $C_{1-4}$-Haloalkoxy, - C(O)NR*R*, -$CH_2$NR*R*, -NR*C(O)R*, -3-5-gliedrigem monocyclischen Cycloalkyl, -3-5-gliedrigem monocyclischen Heterocyclyl; wobei das Cycloalkyl oder Heterocyclyl in der Gruppe dargestellt durch $R^B$ optional substituiert ist durch ein oder zwei F oder $C_{1-2}$-Alkyl; oder
(ii) Ring B Phenyl oder 5, 6-gliedriges monocyclisches Heteroaryl ist, von denen jedes optional substituiert ist durch ein bis vier $R^B$, und zwei $R^B$, zusammen mit den Ring-B-Atomen, an die sie gebunden sind, 5-7-gliedrigen monocyclischen Ring oder 6-9-gliedrigen bicyclischen Ring kondensiert an Ring B bilden, wobei der 5-7-gliedrige monocyclische Ring oder 6-9-gliedrige bicyclische Ring optional substituiert ist mit einem oder zwei CN, Halogen,

$C_{1-4}$-Alkyl, $C_{1-4}$-Haloalkyl, $C_{1-4}$-Alkoxy, $C_{1-4}$-Haloalkoxy oder 3-6-gliedrigem Cycloalkyl; oder zwei $R^B$, zusammen mit den Ring-B-Atomen, an die sie gebunden sind, einen Ring ausgewählt aus Folgendem bilden

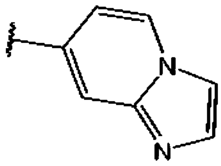, 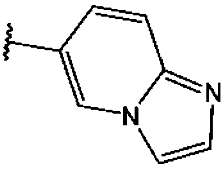, 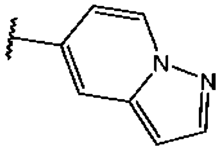, 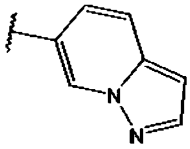, 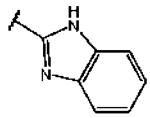,

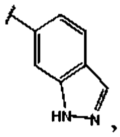, 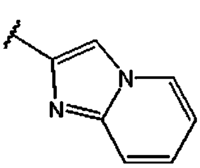, 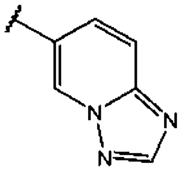, 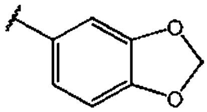, 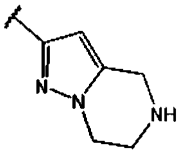,

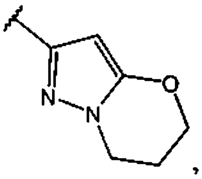, 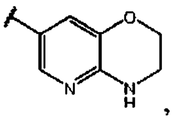, 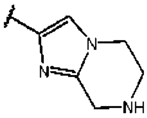, 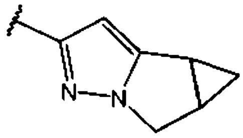, 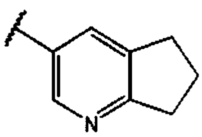,

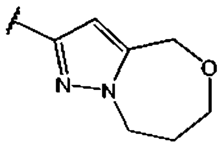, 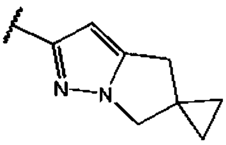, 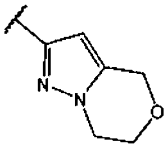, 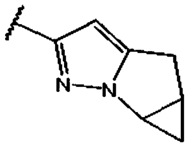, 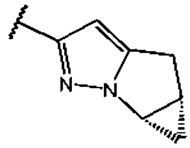,

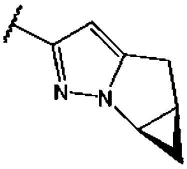, 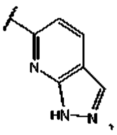, 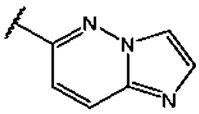, 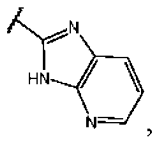, 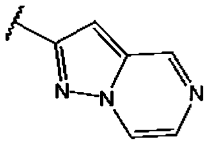,

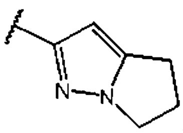, und 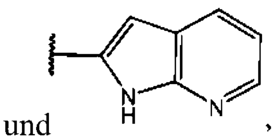,

von denen jedes optional substituiert ist mit einer oder zwei Gruppen ausgewählt aus Halogen, $C_{1-4}$-Alkyl, $C_{1-4}$-Haloalkyl, $C_{1-4}$-Alkoxy und $C_{1-4}$-Haloalkoxy; oder
zwei $R^B$, zusammen mit den Ring-B-Atomen, an die sie gebunden sind, einen Ring bilden, ausgewählt aus

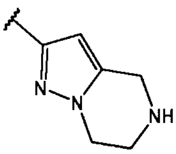, 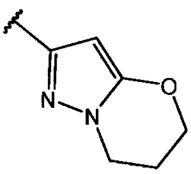, 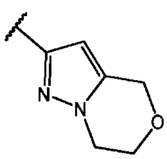, 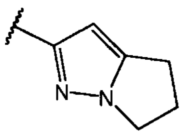 und 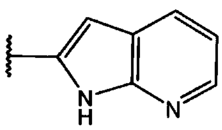.

**9.** Verbindung nach einem der Ansprüche 1-8, pharmazeutisch unbedenkliches Salz oder Tautomer davon, wobei $R^C$ H ist und $R^D$ H ist.

**10.** Verbindung, dargestellt durch Formel (I'):

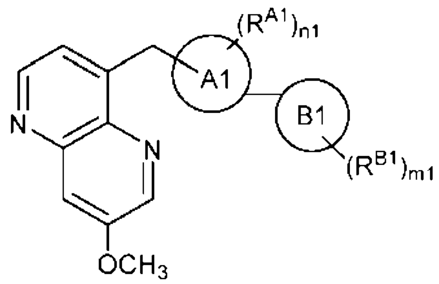

(I')

oder pharmazeutisch unbedenkliches Salz davon, wobei:

Ring A1 5,6- oder 6,6-gliedriges stickstoffhaltiges bicyclisches Heteroaryl ist;
Ring B1 3-6-gliedriges monocyclisches Carbocyclyl oder 3-6-gliedriges monocyclisches Heterocyclyl ist;
jedes $R^{A1}$ unabhängig ausgewählt ist aus Halogen, -CN, -OH, $C_{1-6}$-Alkyl, $C_{1-6}$-Haloalkyl, $C_{1-6}$-Hydroxylalkyl, $C_{1-6}$-Alkoxyalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, - C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, - NR*SO2NR*R*, -P(O)R*R*, $-(CH_2)_{0\ oder\ 1}$-3-12-gliedrigem Carbocyclyl, $-(CH_2)_{0\ oder\ 1}$-3-12-gliedrigem Heterocyclyl, $-(CH_2)_{0\ oder\ 1}$-6-10-gliedrigem Aryl oder $-(CH_2)_{0\ oder\ 1}$-5-10-gliedrigem Heteroaryl; wobei das Carbocyclyl, Heterocyclyl, Aryl oder Heteroaryl in der Gruppe dargestellt durch $R^{A1}$ optional substituiert ist durch ein oder zwei CN, Halogen, $C_{1-6}$-Alkyl, $C_{1-6}$-Haloalkyl, $C_{1-6}$-Alkoxy oder $C_{1-6}$-Haloalkoxy;
jedes $R^{B1}$ unabhängig ausgewählt ist aus Halogen, -CN, -OH, $C_{1-6}$-Alkyl, $C_{1-6}$-Haloalkyl, $C_{1-6}$-Hydroxylalkyl, $C_{1-6}$-Alkoxyalkyl, $C_{2-6}$-Alkenyl, $C_{2-6}$-Alkinyl, $-(CH_2)_{0-4}C_{1-6}$-Alkoxy optional substituiert mit einem bis drei Deuterium, $C_{1-6}$-Haloalkoxy, $C_{1-6}$-Hydroxyalkoxy, -C(O)R*, - C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, - $NR^*SO_2NR^*R^*$, -P(O)R*R*, $-(CH_2)_{0\ oder\ 1}$-3-12-gliedrigem Carbocyclyl, $-(CH_2)_{0\ oder\ 1}$-3-12-gliedrigem Heterocyclyl, $-(CH_2)_{0\ oder\ 1}$-6-10-gliedrigem Aryl oder $-(CH_2)_{0\ oder\ 1}$-5-10-gliedrigem Heteroaryl; wobei das Carbocyclyl, Heterocyclyl, Aryl oder Heteroaryl in der Gruppe dargestellt durch $R^{B1}$ optional substituiert ist durch ein oder zwei CN, Halogen, $C_{1-6}$-Alkyl, $C_{1-6}$-Haloalkyl, $C_{1-6}$-Alkoxy oder $C_{1-6}$-Haloalkoxy; und/oder
zwei $R^{B1}$ zusammen mit den Ring-B1-Atomen, an die sie gebunden sind, 5,6-gliedrigen monocyclischen Ring oder 6-9-gliedrigen bicyclischen Ring kondensiert an Ring B1 bilden, wobei der 5,6-gliedrige Ring oder 6-9-gliedrige bicyclische Ring optional substituiert ist mit einem oder zwei CN, Halogen, $C_{1-6}$-Alkyl, $C_{1-6}$-Haloalkyl, $C_{1-6}$-Alkoxy, $C_{1-6}$-Haloalkoxy oder 3-6-gliedrigem Cycloalkyl;
jedes R* unabhängig ausgewählt ist aus der Gruppe bestehend aus H, $C_{1-6}$-Alkyl, $C_{1-6}$-Haloalkyl, $C_{2-6}$-Alkenyl, $C_{2-6}$-Alkinyl, 3-6-gliedrigem Carbocyclyl oder 4-6-gliedrigem Heterocyclyl;
m1 0, 1, 2, 3 oder 4 ist; und
n1 0, 1, 2 oder 3 ist.

**11.** Verbindung nach Anspruch 10 oder pharmazeutisch unbedenkliches Salz davon, wobei:

(i)

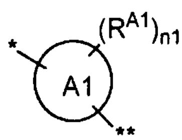

wie folgt ist

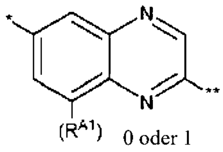 , 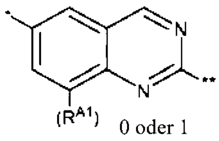 , 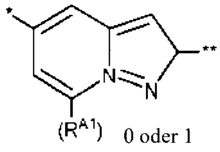 oder 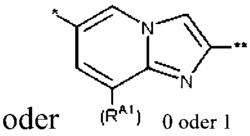 ,

wobei -* den Punkt darstellt, an den -$CH_2$- bindet; -** den Punkt darstellt, an den Ring B1 bindet; und $R^{A1}$ $C_{1-4}$-Alkyl oder $C_{1-4}$-Alkoxy ist; und/oder
(ii) Ring B1 wie folgt ist

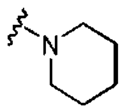 , 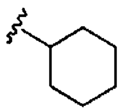 , oder 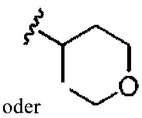 ;

$R^{B1}$ $C_{1-4}$-Alkyl oder $C_{1-4}$-Alkoxy ist; und
n1 0 oder 1 ist.

**12.** Verbindung, ausgewählt aus:

| Beispiel Nr. | Struktur | Beispiel Nr. | Struktur |
|---|---|---|---|
| 1 | 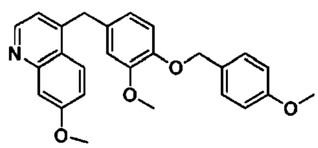 | 80 | 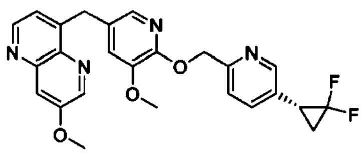 oder 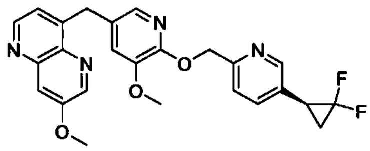 |
| 2 | 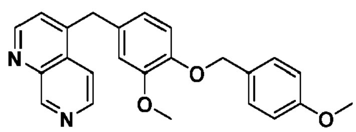 | 81 | 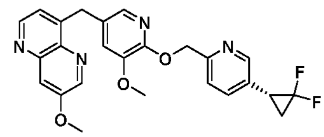 oder 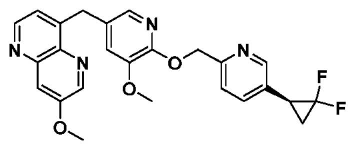 |
| 3 | 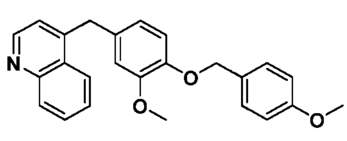 | 82 | 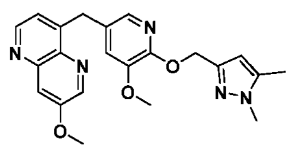 |
| 4 | 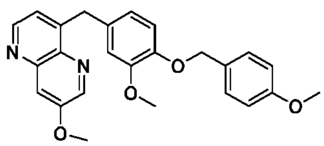 | 83 | 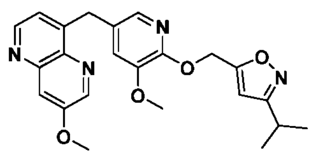 |

(continued)

| Beispiel Nr. | Struktur | Beispiel Nr. | Struktur |
|---|---|---|---|
| 5 | 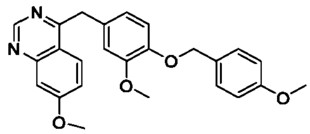 | 84 | 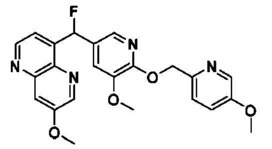 |
| 6 | 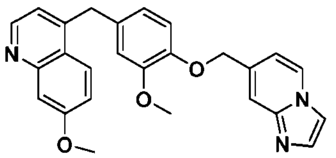 | 85 | 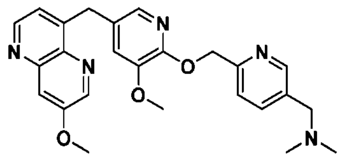 |
| 7 | 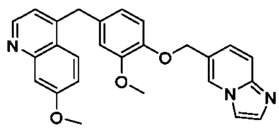 | 86 | 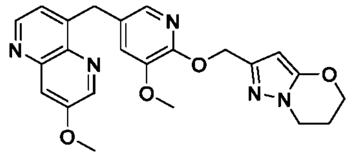 |
| 8 | 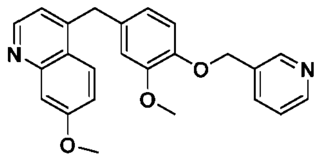 | 87 | 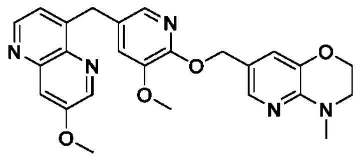 |
| 9 | 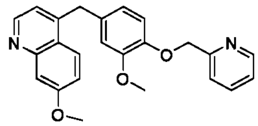 | 88 | 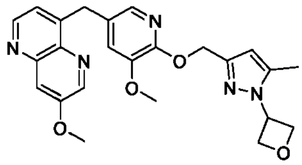 |
| 10 | 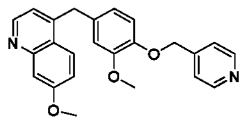 | 89 | 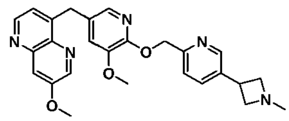 |
| 11 | 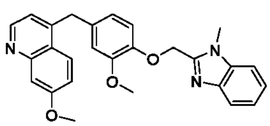 | 90 | 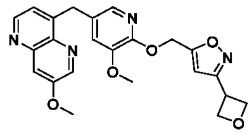 |
| 12 | 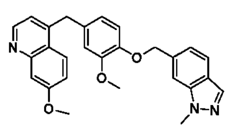 | 91 | 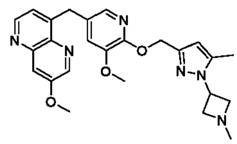 |
| 13 | 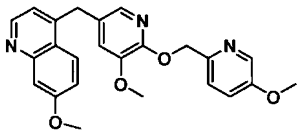 | 92 | 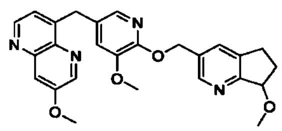 |
| 14 | 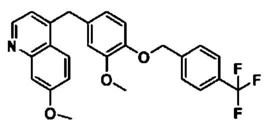 | 93 | 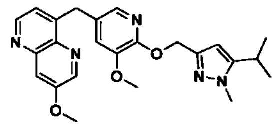 |

(continued)

| Beispiel Nr. | Struktur | Beispiel Nr. | Struktur |
|---|---|---|---|
| 15 | 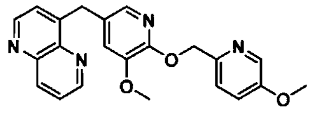 | 94 | 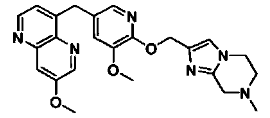 |
| 16 | 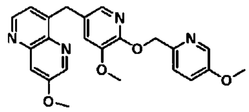 | 95 | 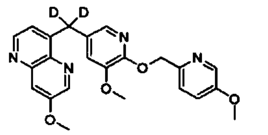 |
| 17 | 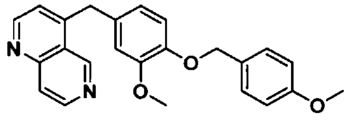 | 96 | 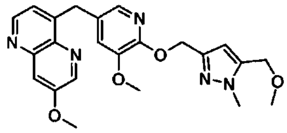 |
| 18 | 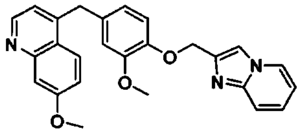 | 97 | 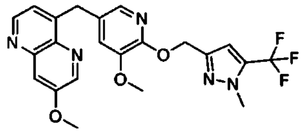 |
| 19 | 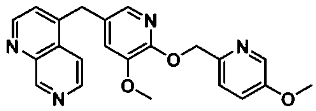 | 98 | 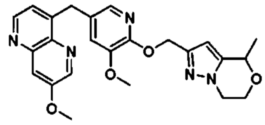 |
| 20 | 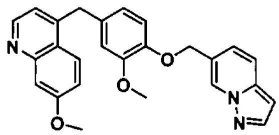 | 99 | 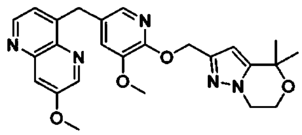 |
| 21 | 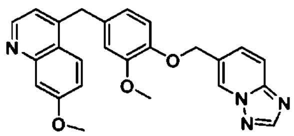 | 100 | 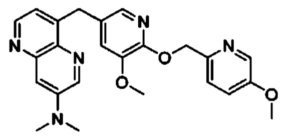 |
| 22 | 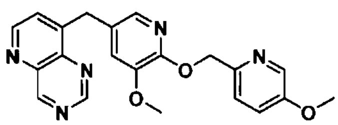 | 101 | 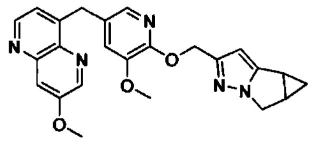 |
| 23 | 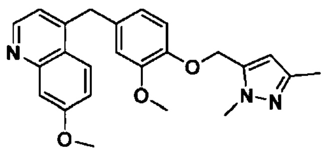 | 102 | 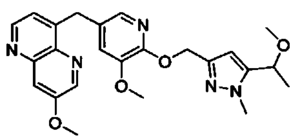 |
| 24 | 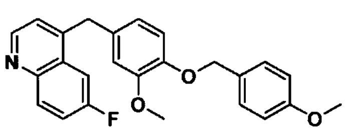 | 103 | 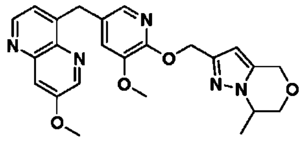 |

(continued)

| Beispiel Nr. | Struktur | Beispiel Nr. | Struktur |
|---|---|---|---|
| 25 | 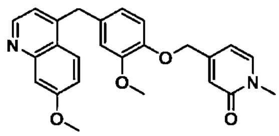 | 104 | 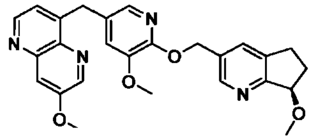 oder 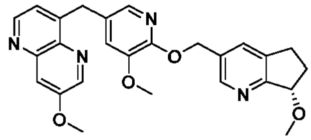 |
| 26 | 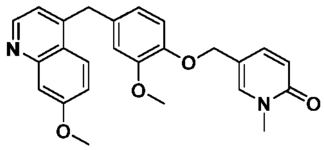 | 105 | 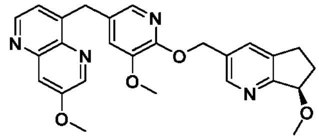 oder 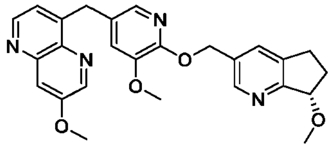 |
| 27 | 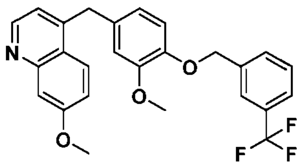 | 106 | 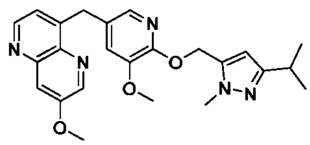 |
| 28 | 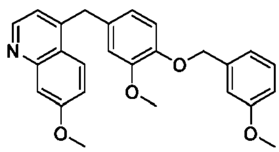 | 107 | 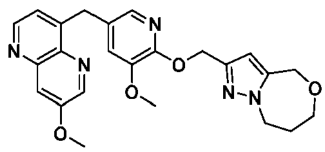 |
| 29 | 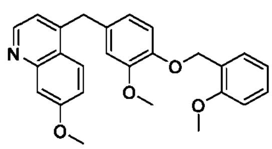 | 108 | 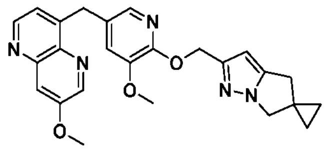 |
| 30 | 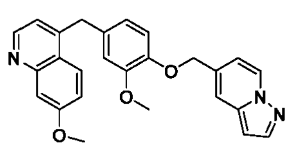 | 109 | 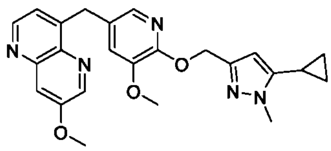 |
| 31 | 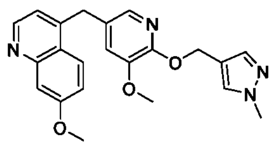 | 110 | 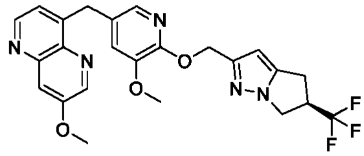 |

(continued)

| Beispiel Nr. | Struktur | Beispiel Nr. | Struktur |
|---|---|---|---|
| 32 | 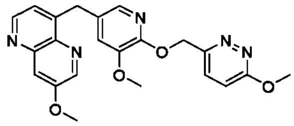 | 111 | 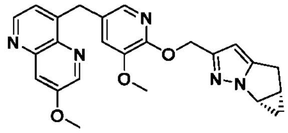 |
| 33 | 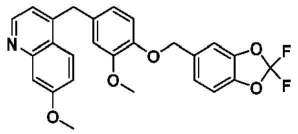 | 112 | 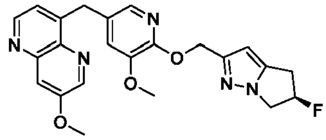 |
| 34 | 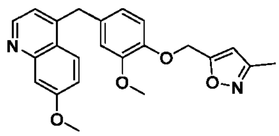 | 113 | 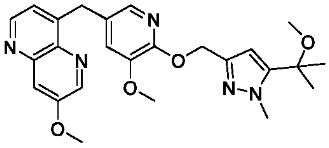 |
| 35 | 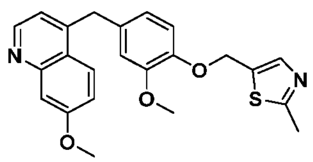 | 114 | 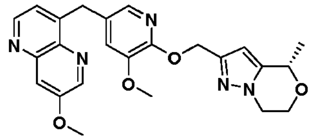 oder 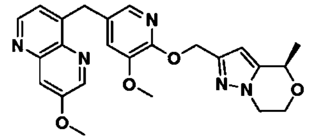 |
| 36 | 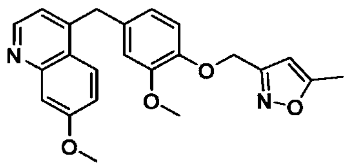 | 115 | 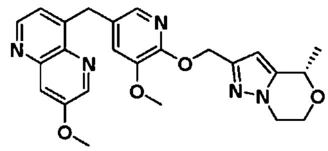 oder 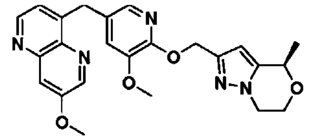 |
| 37 | 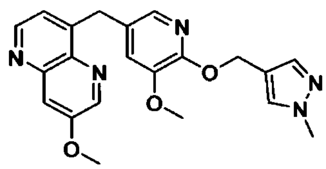 | 116 | 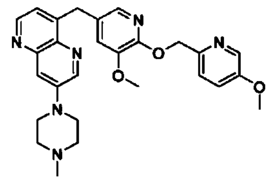 |
| 38 | 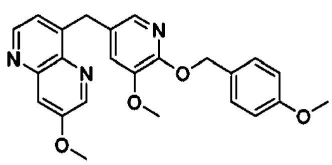 | 117 | 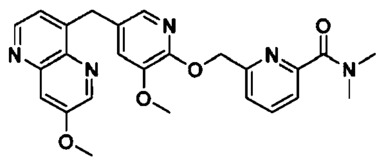 |

(continued)

| Beispiel Nr. | Struktur | Beispiel Nr. | Struktur |
|---|---|---|---|
| 39 | 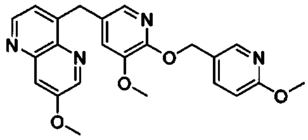 | 118 | 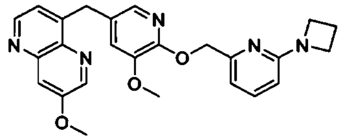 |
| 40 | 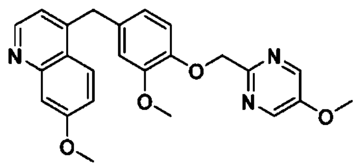 | 119 | 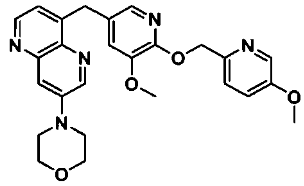 |
| 41 | 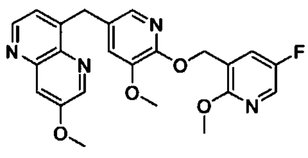 | 120 | 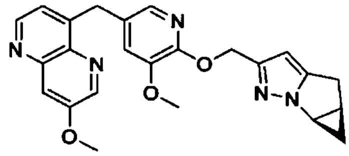 |
| 42 | 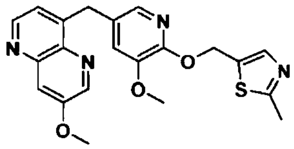 | 121 | 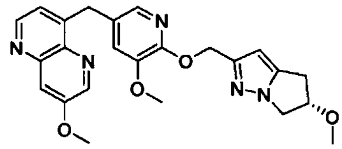 |
| 43 | 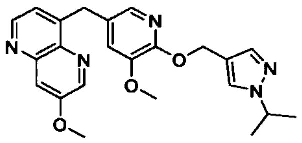 | 122 | 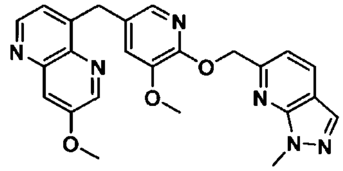 |
| 44 | 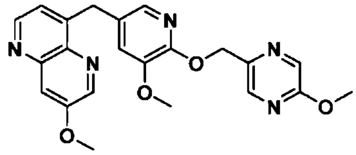 | 123 | 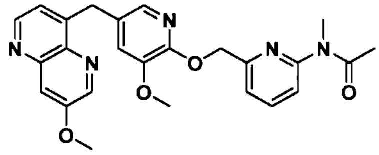 |
| 45 | 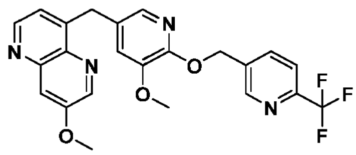 | 124 | 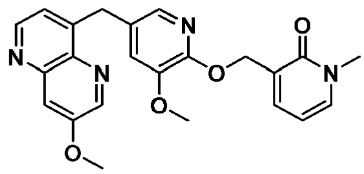 |
| 46 | 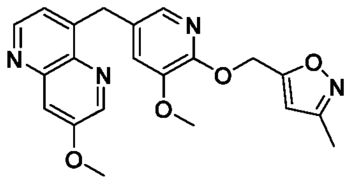 | 125 | 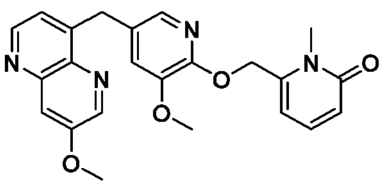 |
| 47 | 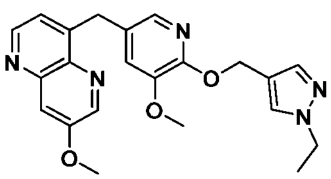 | 126 | 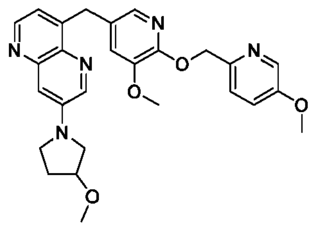 |

(continued)

| Beispiel Nr. | Struktur | Beispiel Nr. | Struktur |
|---|---|---|---|
| 48 | 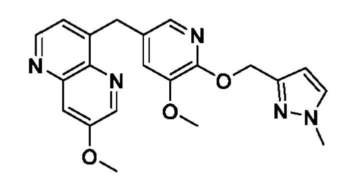 | 127 | 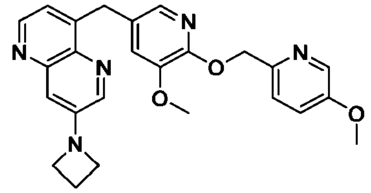 |
| 49 | 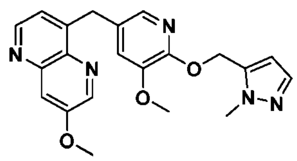 | 128 | 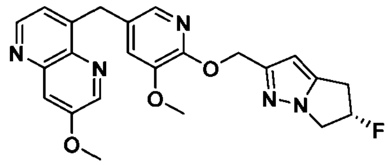 |
| 50 | 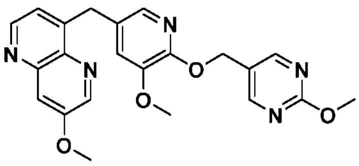 | 129 | 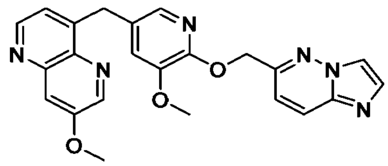 |
| 51 | 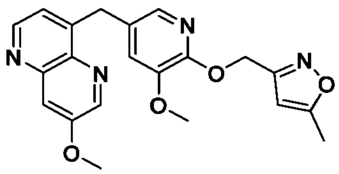 | 130 | 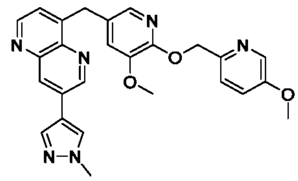 |
| 52 | 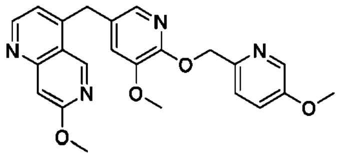 | 131 | 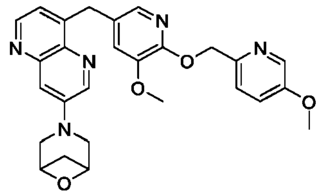 |
| 53 | 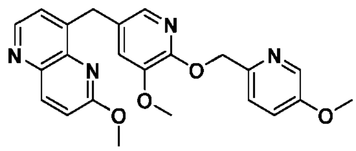 | 132 | 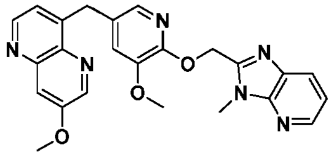 |
| 54 | 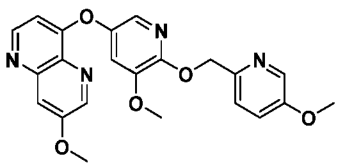 | 133 | 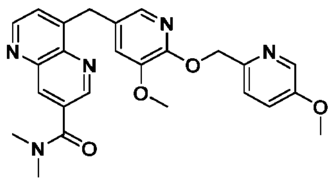 |
| 55 | 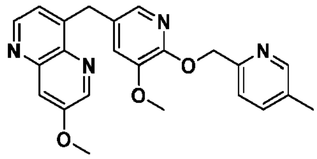 | 134 | 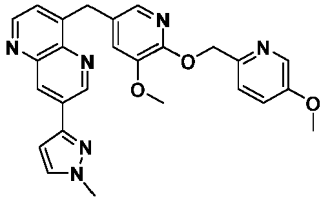 |

(continued)

| Beispiel Nr. | Struktur | Beispiel Nr. | Struktur |
|---|---|---|---|
| 56 | 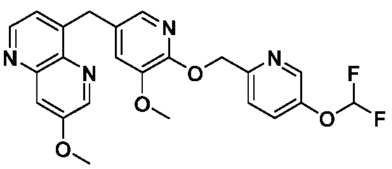 | 135 | 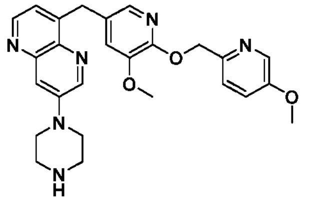 |
| 57 | 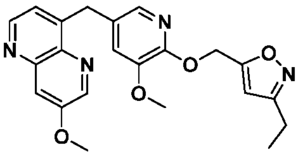 | 136 | 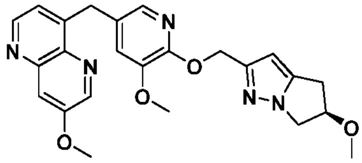 |
| 58 | 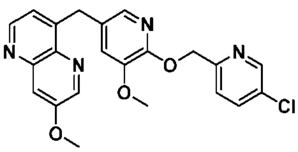 | 137 | 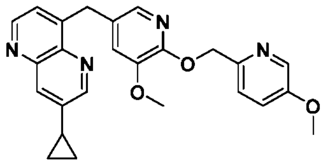 |
| 59 | 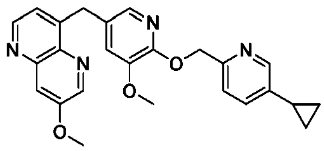 | 138 | 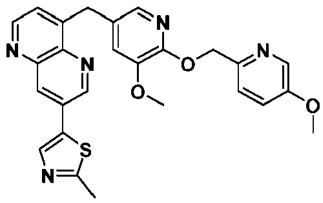 |
| 60 | 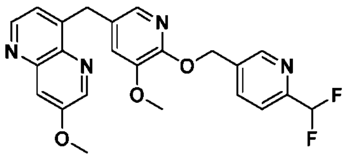 | 139 | 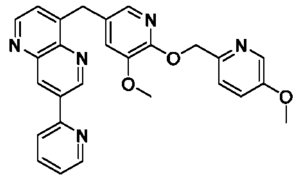 |
| 61 | 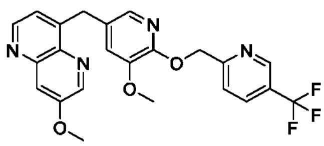 | 140 | 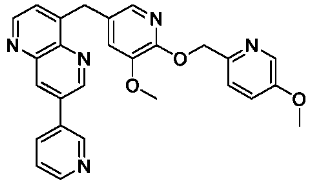 |
| 62 | 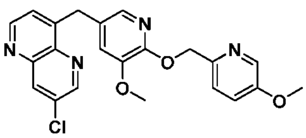 | 141 | 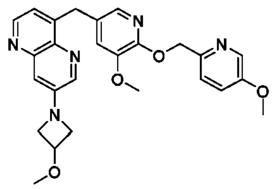 |
| 63 | 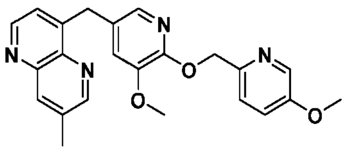 | 142 | 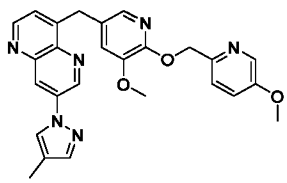 |

(continued)

| Beispiel Nr. | Struktur | Beispiel Nr. | Struktur |
|---|---|---|---|
| 64 | 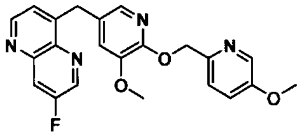 | 143 | 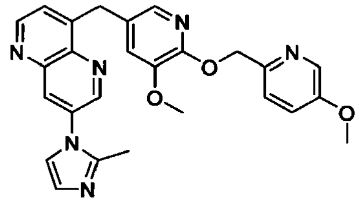 |
| 65 | 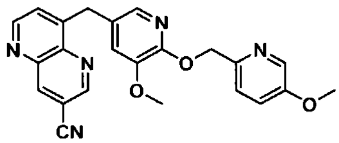 | 144 | 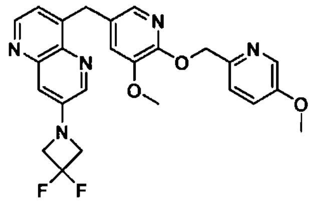 |
| 66 | 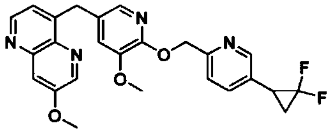 | 145 | 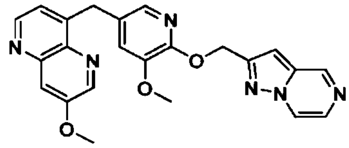 |
| 67 | 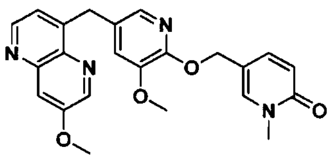 | 146 | 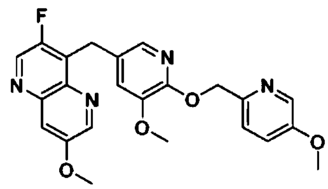 |
| 68 | 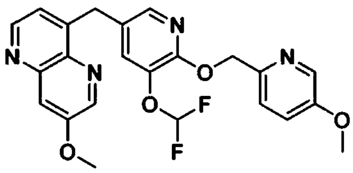 | 147 | 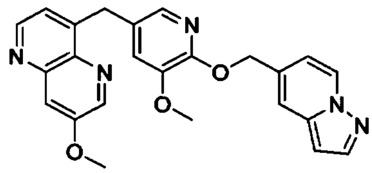 |
| 69 | 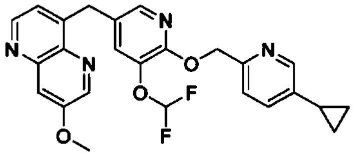 | 148 | 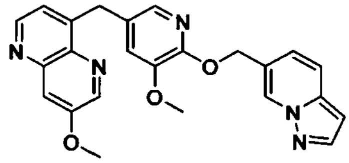 |
| 70 | 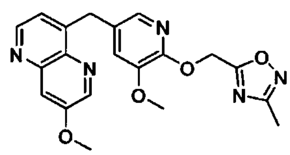 | 149 | 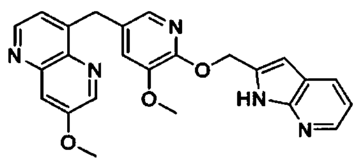 |
| 71 | 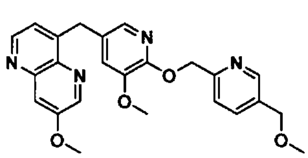 | 150 | 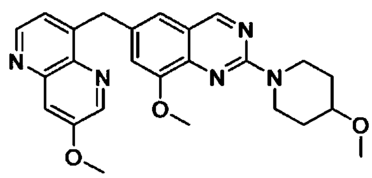 |

(continued)

| Beispiel Nr. | Struktur | Beispiel Nr. | Struktur |
|---|---|---|---|
| 72 | 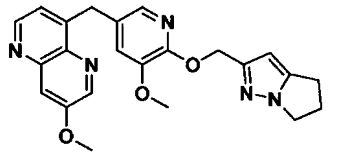 | 151 | 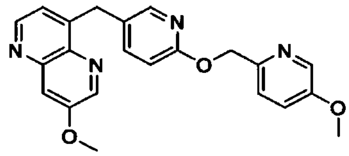 |
| 73 | 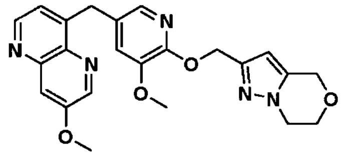 | 152 | 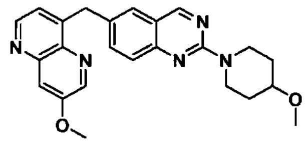 |
| 74 | 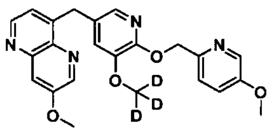 | 153 | 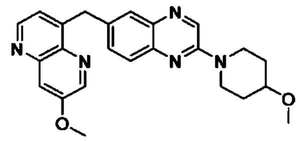 |
| 75 | 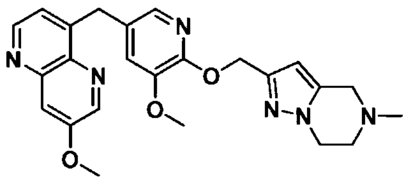 | 154 | 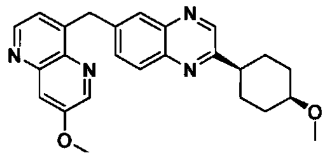 oder 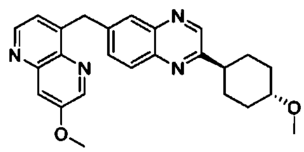 |
| 76 | 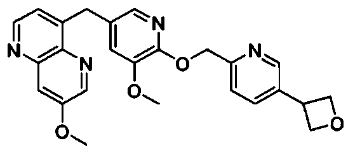 | 155 | 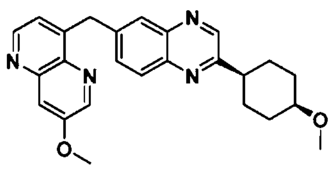 oder 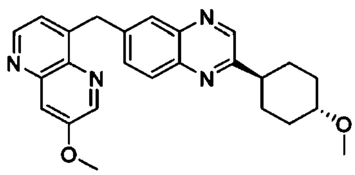 |
| 77 | 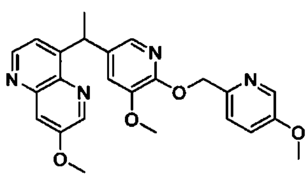 | 156 | 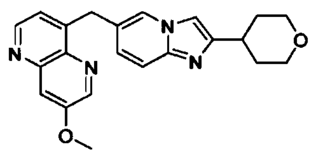 |
| 78 | 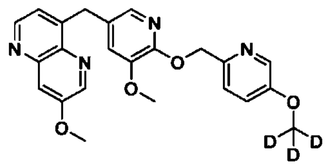 | 157 | 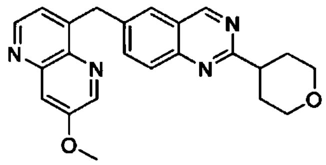 |

(continued)

| Beispiel Nr. | Struktur | Beispiel Nr. | Struktur |
|---|---|---|---|
| 79 | 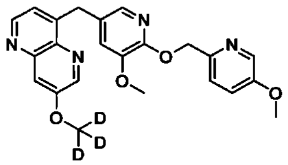 | 158 | 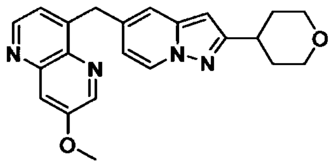 |

oder pharmazeutisch unbedenkliches Salz davon.

**13.** Verbindung nach Anspruch 12 oder pharmazeutisch unbedenkliches Salz davon, wobei die Verbindung wie folgt ist

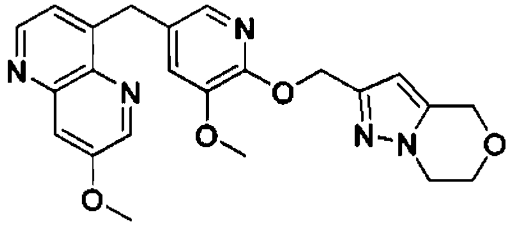

.

**14.** Pharmazeutische Zusammensetzung, umfassend die Verbindung nach einem der Ansprüche 1-13 oder ein pharmazeutisch unbedenkliches Salz davon und einen pharmazeutisch unbedenklichen Träger oder Hilfsstoff.

**15.** Verbindung nach einem der Ansprüche 1-13 oder pharmazeutisch unbedenkliches Salz davon zur Verwendung in einem Verfahren zum Behandeln einer Erkrankung, die durch CSF-IR oder zumindest teilweise durch CSF-1R vermittelt wird, bei einem Subjekt, wobei die Erkrankung eine Autoimmunerkrankung, eine entzündliche Erkrankung, eine neurodegenerative Erkrankung, Krebs, eine Stoffwechselerkrankung, Fettleibigkeit oder eine mit Fettleibigkeit zusammenhängende Erkrankung ist.

**16.** Verbindung zur Verwendung nach Anspruch 15, wobei

die Autoimmunerkrankung oder entzündliche Erkrankung gewählt ist aus rheumatoider Arthritis, kollageninduzierter Arthritis, Osteoarthritis, pigmentierter villonodulärer Synovitis (PVNS), systemischem Lupus erythematodes, multipler Sklerose, systemischer Sklerodermie, Autoimmunnephritis, entzündlicher Darmerkrankung, Crohn-Erkrankung, Colitis ulcerosa, Psoriasis, atopischer Dermatitis, Asthma, chronisch obstruktiver Lungenerkrankung, Behcet-Erkrankung, idiopathischer thrombozytopenischer Purpura, spinaler Arthritis, systemischer juveniler idiopathischer Arthritis (SoJIA), Pankreatitis, Ischämie-Reperfusionsverletzung von parenchymatischen Organen, Organtransplantatabstoßung, Septikämie, systemischem Entzündungsreaktionssyndrom und durch Chemotherapie-Arzneimittel induzierter Organverletzung;

wobei die neurodegenerative Erkrankung gewählt ist aus Parkinson-Erkrankung (PD), Multisystematrophie, Alzheimer-Erkrankung (AD), frontotemporaler Lobardemenz, Huntington-Erkrankung (HD), kortikobasaler Degeneration, spinozerebellärer Ataxie, amyotropher Lateralsklerose (ALS), spinaler Muskelatrophie (SMA) und hereditärer motorischer und sensorischer Neuropathie (CMT); und

der Krebs solider Tumor oder hämatologische Malignität ist, wie Eierstockkrebs, Lungenkrebs (beinhaltend nichtkleinzelligen Lungenkrebs), Himtumor (beinhaltend Glioblastom (GBM)), tenosynovialer Riesenzelltumor, gastrointestinaler Stromatumor (GIST), Magenkrebs, Speiseröhrenkrebs, Kolonkrebs, Kolorektalkrebs, Bauchspeicheldrüsenkrebs, Prostatakrebs, Brustkrebs, Gebärmutterhalskrebs, Melanom, Mesotheliom, Mesothelkarzinom, Nierenkrebs, Leberkrebs, Schilddrüsenkarzinom, Kopf- und Halskrebs, Urothelkarzinom, Blasenkrebs, Endometriumkrebs, Chorionokarzinom, Nebennierenkarzinom, Sarkom, Leukämie, Lymphom oder Myelom.

**17.** Verbindung nach einem der Ansprüche 1-13 oder pharmazeutisch unbedenkliches Salz davon zur Verwendung in einem Verfahren zum Behandeln von Alzheimer-Erkrankung, progressiver supranukleärer Lähmung, einer Tau-vermittelten neurodegenerativen Störung oder einer Erkrankung oder Störung, die mikrogliavermittelte Entzündung bei einem Subjekt involviert.

**Revendications**

1. Composé représenté par la Formule (I) :

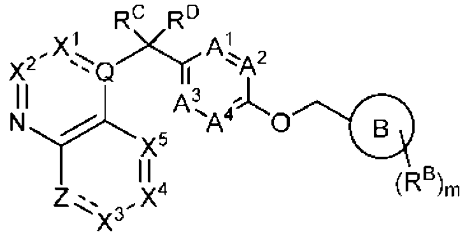

(I)

sel acceptable pharmaceutiquement ou tautomère de celui-ci, où :

Q est C ou N ;
lorsque Q est N, Q-$X^1$ est une liaison simple et $X^1$ est C=O ;
lorsque Q est C, Q-$X^1$ est une double liaison et $X^1$ est N ou $CR^1$ ;
Z est CH ou NH ;
lorsque Z est NH, Z-$X^3$ est une liaison simple et $X^3$ est C=O ;
lorsque Z est CH, Z-$X^3$ est une double liaison et $X^3$ est N ou $CR^3$ ;
$X^2$ est N ou $CR^2$ ;
$X^4$ est N ou $CR^4$ ;
$X^5$ est N ou $CR^5$ ;
chaque $R^1$, $R^2$, $R^3$, $R^4$ et $R^5$ est indépendamment choisi parmi H, un halogène, -CN, -OH, un alkyle en $C_{1-6}$ éventuellement substitué par un à trois deutérium, un halogénoalkyle en $C_{1-6}$, un hydroxylalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons ou $-(CH_2)_{0\text{ ou }1}$-hétéroaryle à 5-10 chaînons ; où ledit carbocyclyle, hétérocyclyle, aryle ou hétéroaryle dans le groupe représenté par $R^1$, $R^2$, $R^3$, $R^4$ ou $R^5$ est éventuellement substitué par un ou deux groupes choisis parmi CN, un halogène, un alkyle en $C_{1-6}$, un halogénoalkyle en $C_{1-6}$, un alcoxy en $C_{1-6}$ et un halogénoalcoxy en $C_{1-6}$ ;
chaque $A^1$, $A^2$, $A^3$ et $A^4$ est indépendamment N ou $CR^A$ ; et pas plus de deux de $A^1$, $A^2$, $A^3$ et $A^4$ sont N ;
chaque $R^A$ est indépendamment choisi parmi un halogène, -CN, -OH, un alkyle en $C_{1-6}$, un halogénoalkyle en $C_{1-6}$, un hydroxylalkyle en $C_{1-6}$, un alcoxyalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, - $(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons ou $-(CH_2)_{0\text{ ou }1}$-hétéroaryle à 5-10 chaînons ; où ledit carbocyclyle, hétérocyclyle, aryle ou hétéroaryle dans le groupe représenté par $R^A$ est éventuellement substitué par un ou deux groupes choisis parmi CN, un halogène, un alkyle en $C_{1-6}$, un halogénoalkyle en $C_{1-6}$, un alcoxy en $C_{1-6}$ et un halogénoalcoxy en $C_{1-6}$ ;
le cycle B est un phényle ou un hétéroaryle monocyclique à 5 ou 6 chaînons ;
chaque $R^B$ est indépendamment choisi parmi un halogène, -CN, -OH, un alkyle en $C_{1-6}$, un halogénoalkyle en $C_{1-6}$, un hydroxylalkyle en $C_{1-6}$, un alcoxyalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, - $(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons ou $-(CH_2)_{0\text{ ou }1}$-hétéroaryle à 5-10 chaînons ; où ledit carbocyclyle, hétérocyclyle, aryle ou hétéroaryle dans le groupe représenté par $R^B$ est éventuellement substitué par un ou deux groupes choisis parmi CN, un halogène, un alkyle en $C_{1-6}$, un halogénoalkyle en $C_{1-6}$, un alcoxy en $C_{1-6}$ et un halogénoalcoxy en $C_{1-6}$ ; et/ou
deux $R^B$ conjointement avec les atomes de cycle B auxquels ils sont liés, forment un cycle monocyclique à 5-7 chaînons ou un cycle bicyclique à 6-9 chaînons fusionné au cycle B, ledit cycle monocyclique à 5-7 chaînons ou ledit cycle bicyclique à 6-9 chaînons est éventuellement substitué par un ou deux groupes choisis parmi CN, un halogène, un alkyle en $C_{1-6}$, un halogénoalkyle en $C_{1-6}$, un alcoxy en $C_{1-6}$, un halogénoalcoxy en $C_{1-6}$ et un cycloalkyle à 3-6 chaînons ;

chaque $R^C$ et $R^D$ est indépendamment choisi parmi un hydrogène, un deutérium, F et un méthyle ;
chaque R* est indépendamment choisi dans le groupe constitué par H, un alkyle en $C_{1-6}$, un halogénoalkyle en $C_{1-6}$, un alcényle en $C_{2-6}$, un alcynyle en $C_{2-6}$, un carbocyclyle à 3-6 chaînons ou un hétérocyclyle à 4-6 chaînons ; et
m vaut 0, 1, 2, 3 ou 4.

**2.** Composé de la revendication 1, ou sel acceptable pharmaceutiquement de celui-ci, dans lequel : chaque $R^1$, $R^2$, $R^3$, $R^4$ et $R^5$ est indépendamment choisi parmi H, un halogène, -CN, -OH, un alkyle en $C_{1-6}$, un halogénoalkyle en $C_{1-6}$, un hydroxylalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, - $(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons ou $-(CH_2)_{0\text{ ou }1}$-hétéroaryle à 5-10 chaînons ; où ledit carbocyclyle, hétérocyclyle, aryle ou hétéroaryle dans le groupe représenté par $R^1$, $R^2$, $R^3$, $R^4$ ou $R^5$ est éventuellement substitué par un ou deux groupes choisis parmi CN, un halogène, un alkyle en $C_{1-6}$, un halogénoalkyle en $C_{1-6}$, un alcoxy en $C_{1-6}$ et un halogénoalcoxy en $C_{1-6}$ ;

chaque $R^A$ est indépendamment choisi parmi H, un halogène, -CN, -OH, un alkyle en $C_{1-6}$, un halogénoalkyle en $C_{1-6}$, un hydroxylalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons ou - $(CH_2)_{0\text{ ou }1}$-hétéroaryle à 5-10 chaînons ; où ledit carbocyclyle, hétérocyclyle, aryle ou hétéroaryle dans le groupe représenté par $R^A$ est éventuellement substitué par un ou deux groupes choisis parmi CN, un halogène, un alkyle en $C_{1-6}$, un halogénoalkyle en $C_{1-6}$, un alcoxy en $C_{1-6}$ et un halogénoalcoxy en $C_{1-6}$ ;
chaque $R^B$ est indépendamment choisi parmi un halogène, -CN, -OH, un alkyle en $C_{1-6}$, un halogénoalkyle en $C_{1-6}$, un hydroxylalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, $-(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons ou - $(CH_2)_{0\text{ ou }1}$-hétéroaryle à 5-10 chaînons ; où ledit carbocyclyle, hétérocyclyle, aryle ou hétéroaryle dans le groupe représenté par $R^B$ est éventuellement substitué par un ou deux groupes choisis parmi CN, un halogène, un alkyle en $C_{1-6}$, un halogénoalkyle en $C_{1-6}$, un alcoxy en $C_{1-6}$ et un halogénoalcoxy en $C_{1-6}$ ; ou deux $R^B$ conjointement avec les atomes de cycle B auxquels ils sont liés, forment un cycle à 5,6 chaînons fusionné au cycle B, ledit cycle à 5,6 chaînons est éventuellement substitué par un ou deux groupes choisis parmi CN, un halogène, un alkyle en $C_{1-6}$, un halogénoalkyle en $C_{1-6}$, un alcoxy en $C_{1-6}$ et un halogénoalcoxy en $C_{1-6}$ ; et
chaque $R^C$ et $R^D$ est indépendamment choisi parmi un hydrogène, F et un méthyle ; éventuellement où le composé est représenté par l'une quelconque de la Formule (II-A), la Formule (III-A-1), la Formule (III-A-2), la Formule (III-A-3), la Formule (III-A-4), la Formule (III-A-5), la Formule (III-A-6), la Formule (III-A-7), la Formule (III-B-1), la Formule (III-B-2), la Formule (III-C-1), la Formule (III-C-2) ou la Formule (III-C-3) :

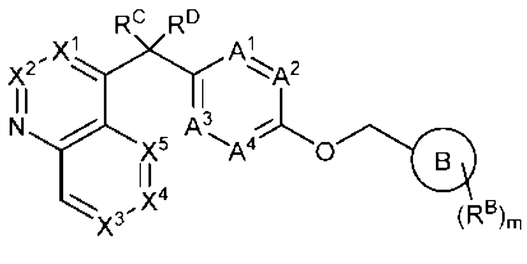
(II-A),

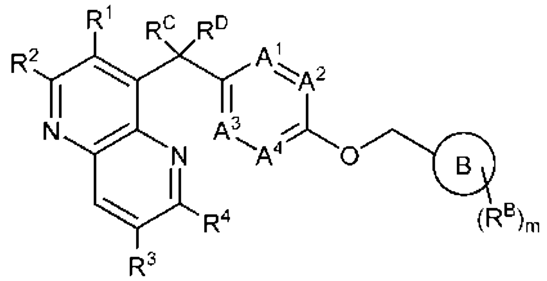

(III-A-1),

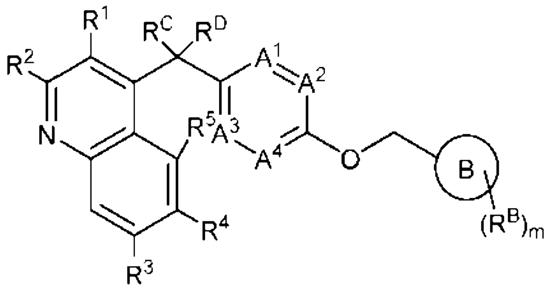

(III-A-2),

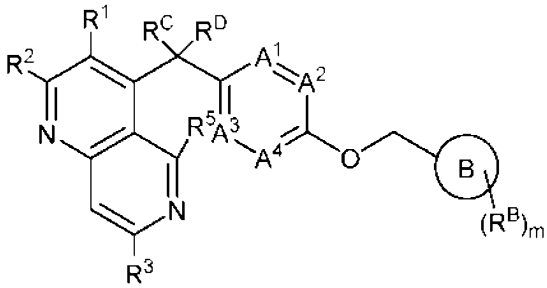

(III-A-3),

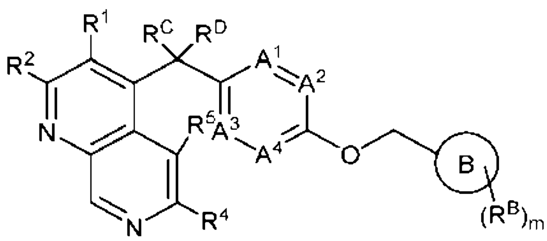

(III-A-4),

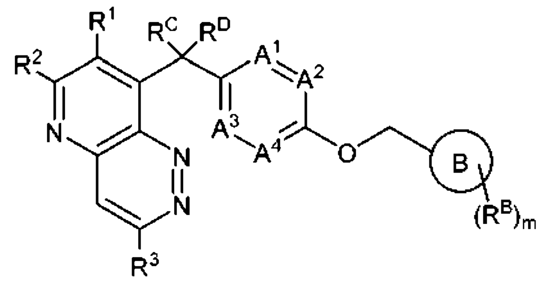

(III-A-5),

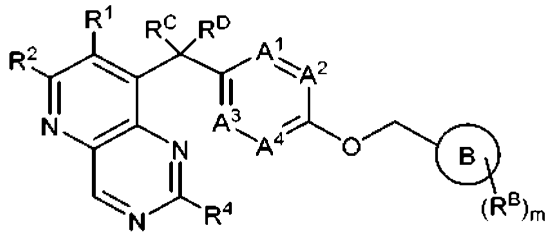
(III-A-6),

ou

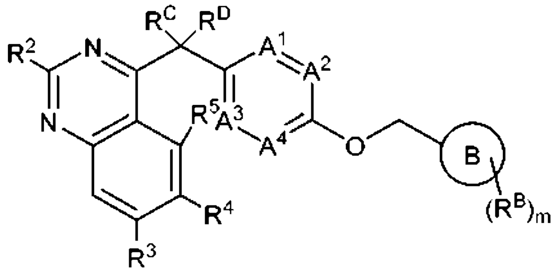
(III-A-7),

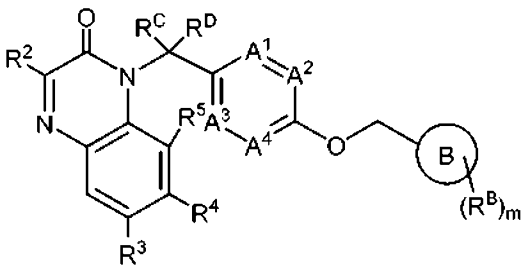
(III-B-1),

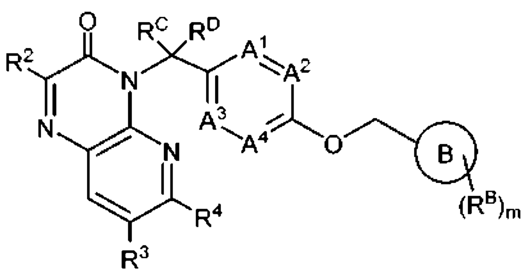
(III-B-2);

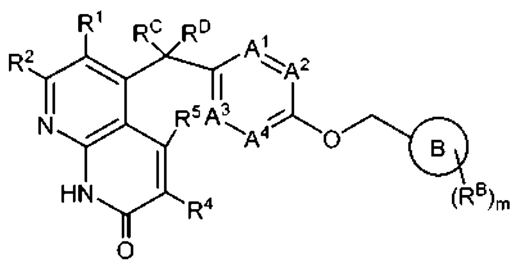
(III-C-1),

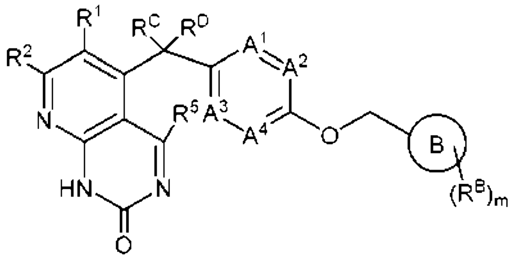

(III-C-2),

ou

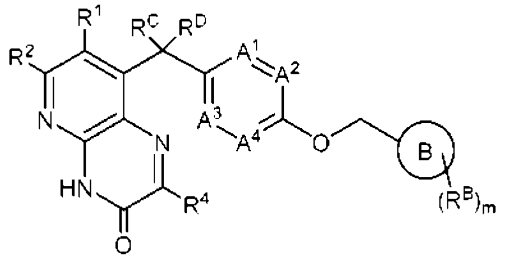

(III-C-3);

ou un sel acceptable pharmaceutiquement de celui-ci.

**3.** Composé de la revendication 2 ou sel acceptable pharmaceutiquement de celui-ci, dans lequel :

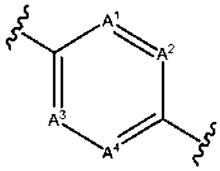

est un phényle ou un pyridyle, dont chacun est éventuellement substitué par un ou deux $R^A$ ; et/ou le cycle B est :

(i) un phényle éventuellement substitué par un ou deux $R^B$, chaque $R^B$ est indépendamment choisi parmi un halogène, -CN, -OH, un alkyle en $C_{1-6}$, un halogénoalkyle en $C_{1-6}$, un hydroxylalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, - C(O)OR*, -C(O)NR*R*, -OR*, $-SO_2R^*$, $-(CH_2)_{0-4}NR^*R^*$, -NR*C(O)R*, -NR*C(O)OR*, - $NR^*SO_2R^*$, $-NR^*SO_2NR^*R^*$, -P(O)R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons, ou $-(CH_2)_{0\text{ ou }1}$-hétéroaryle à 5-10 chaînons ; où ledit carbocyclyle, hétérocyclyle, aryle ou hétéroaryle dans le groupe représenté par $R^B$ est éventuellement substitué par un ou deux halogènes ou un alkyle en $C_{1-6}$ ; ou

(ii) un hétéroaryle monocyclique à 5, 6 chaînons éventuellement substitué par un ou deux $R^B$, chaque $R^B$ est indépendamment choisi parmi un halogène, -CN, -OH, un alkyle en $C_{1-6}$, un halogénoalkyle en $C_{1-6}$, un hydroxylalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, -OR*, $-SO_2R^*$, $-(CH_2)_{0-4}NR^*R^*$, - NR*C(O)R*, -NR*C(O)OR*, $-NR^*SO_2R^*$, $-NR^*SO_2NR^*R^*$, -P(O) R*R*, $-(CH_2)_{0\text{ ou }1}$-carbocyclyle à 3-12 chaînons, $-(CH_2)_{0\text{ ou }1}$-hétérocyclyle à 3-12 chaînons, $-(CH_2)_{0\text{ ou }1}$-aryle à 6-10 chaînons ou - $(CH_2)_{0\text{ ou }1}$-hétéroaryle à 5-10 chaînons ; où ledit carbocyclyle, hétérocyclyle, aryle ou hétéroaryle dans le groupe représenté par $R^B$ est éventuellement substitué par un ou deux halogènes ou un alkyle en $C_{1-6}$ ; ou

(iii) un phényle ou un hétéroaryle monocyclique à 5, 6 chaînons, dont chacun est éventuellement substitué par un à quatre $R^B$, et deux $R^B$ conjointement avec les atomes de cycle B auxquels ils sont liés, forment un cycle à 5,6 chaînons fusionné au cycle B, ledit cycle fusionné à 5,6 chaînons est éventuellement substitué par un ou deux CN, un halogène, un alkyle en $C_{1-4}$, un halogénoalkyle en $C_{1-4}$, un alcoxy en $C_{1-4}$ ou un halogénoalcoxy en $C_{1-4}$ ; ou

(iv) un pyrazolyle, un isoxazolyle, 1,2,4-oxadiazolyle, un thiazolyle, un phényle, un pyridyle, un pyrimidyle, un

pyrazinyle, un imidazo[1,2-a]pyridinyle, un benzo[d]imidazolyle, un pyrazolo[ 1,5-a]pyridinyle, un indazolyle, [1,2,4]triazolo[1,5-a]pyridinyle, 4,5,6,7-tétrahydropyrazolo[1,5-a]pyrazinyle, 6,7-dihydro-4H-pyrazolo[5,1-c][1,4]oxazinyle, 5,6-dihydro-4H-pyrrolo[1,2-b]pyrazolyle, ou un benzo[d][1,3]dioxolyle.

**4.** Composé de l'une des revendications 2 ou 3 ou sel acceptable pharmaceutiquement de celui-ci, dans lequel : chaque $R^1$ est indépendamment choisi parmi H, un halogène, -CN, -OH, un alkyle en $C_{1-4}$, un halogénoalkyle en $C_{1-4}$, un hydroxylalkyle en $C_{1-4}$, $-(CH_2)_{0-4}$-alcoxy en $C_{1-4}$, un halogénoalcoxy en $C_{1-4}$, un hydroxyalcoxy en $C_{1-4}$, -C(O)R*, -C(O)OR*, -C(O)NR*R*, $-(CH_2)_{0-4}$NR*R*, -NR*C(O)R* et -NR*C(O)OR* ; et/ou

chaque $R^2$ est indépendamment choisi parmi H, un halogène, -CN, -OH, un alkyle en $C_{1-4}$, un halogénoalkyle en $C_{1-4}$, un hydroxylalkyle en $C_{1-4}$, $-(CH_2)_{0-4}$-alcoxy en $C_{1-4}$, un halogénoalcoxy en $C_{1-4}$, un hydroxyalcoxy en $C_{1-4}$, -C(O)R*, -C(O)OR*, -C(O)NR*R*, $-(CH_2)_{0-4}$NR*R*, -NR*C(O)R* et -NR*C(O)OR* ; et/ou
chaque $R^3$ est indépendamment choisi parmi H, un halogène, -CN, -OH, un alkyle en $C_{1-4}$, un halogénoalkyle en $C_{1-4}$, un hydroxylalkyle en $C_{1-4}$, $-(CH_2)_{0-2}$-alcoxy en $C_{1-4}$ éventuellement substitué par un à trois deutérium, un halogénoalcoxy en $C_{1-4}$, un hydroxyalcoxy en $C_{1-4}$, -C(O)R*, -C(O)OR*, -C(O)NR*R*, $-(CH_2)_{0-4}$NR*R*, -NR*C(O)R* et -NR*C(O)OR* ; et/ou
chaque $R^4$ est indépendamment choisi parmi H, un halogène, -CN, -OH, un alkyle en $C_{1-4}$, un halogénoalkyle en $C_{1-4}$, un hydroxylalkyle en $C_{1-4}$, $-(CH_2)_{0-4}$-alcoxy en $C_{1-4}$, un halogénoalcoxy en $C_{1-4}$, un hydroxyalcoxy en $C_{1-4}$, -C(O)R*, -C(O)OR*, -C(O)NR*R*, $-(CH_2)_{0-4}$NR*R*, -NR*C(O)R* et -NR*C(O)OR* ; et/ou
chaque $R^5$ est indépendamment choisi parmi H, un halogène, -CN, -OH, un alkyle en $C_{1-4}$, un halogénoalkyle en $C_{1-4}$, un hydroxylalkyle en $C_{1-4}$, $-(CH_2)_{0-4}$-alcoxy en $C_{1-4}$, un halogénoalcoxy en $C_{1-4}$, un hydroxyalcoxy en $C_{1-4}$, -C(O)R*, -C(O)OR*, -C(O)NR*R*, -NR*R*, -NR*C(O)R* et -NR*C(O)OR* ; et/ou
chaque $R^A$ est indépendamment choisi parmi H, un halogène, -CN, -OH, un alkyle en $C_{1-4}$, un halogénoalkyle en $C_{1-4}$, un hydroxylalkyle en $C_{1-4}$, un alcoxy en $C_{1-4}$ éventuellement substitué par un à trois deutérium, un halogénoalcoxy en $C_{1-4}$, un hydroxyalcoxy en $C_{1-4}$, -C(O)R*, -C(O)OR*, - C(O)NR*R*, $-(CH_2)_{0-4}$NR*R*, -NR*C(O)R* et -NR*C(O)OR* ; et/ou
chaque $R^B$ est indépendamment choisi parmi un halogène, -CN, -OH, un alkyle en $C_{1-4}$, un halogénoalkyle en $C_{1-4}$, un hydroxylalkyle en $C_{1-4}$, $-(CH_2)_{0-2}$-alcoxy en $C_{1-4}$ éventuellement substitué par un à trois deutérium, un halogénoalcoxy en $C_{1-4}$, un hydroxyalcoxy en $C_{1-4}$, -C(O)R*, -C(O)OR*, -C(O)NR*R*, $-(CH_2)_{0-4}$NR*R*, -NR*C(O)R*, -NR*C(O)OR* et $-(CH_2)_{0\text{ ou }1}$-cycloalkyle à 3-6 chaînons éventuellement substitué par un ou deux halogènes ; et/ou
chaque R* est indépendamment H ou un alkyle en $C_{1-4}$ (de préférence H ou un alkyle en $C_{1-2}$).

**5.** Composé de l'une quelconque des revendications 2 à 4, ou sel acceptable pharmaceutiquement de celui-ci, dans lequel :

chaque $R^1$ est indépendamment choisi parmi H, un halogène, un alkyle en $C_{1-4}$, un halogénoalkyle en $C_{1-4}$, un alcoxy en $C_{1-4}$ et un halogénoalcoxy en $C_{1-4}$ (de préférence H) ; et
chaque $R^2$ est indépendamment choisi parmi H, un halogène, un alkyle en $C_{1-4}$, un halogénoalkyle en $C_{1-4}$, un alcoxy en $C_{1-4}$ et un halogénoalcoxy en $C_{1-4}$ (de préférence H) ; et/ou
chaque $R^3$ est indépendamment choisi parmi H, un halogène, un alkyle en $C_{1-4}$, un halogénoalkyle en $C_{1-4}$, un alcoxy en $C_{1-4}$ éventuellement substitués par un à trois deutérium, et un halogénoalcoxy en $C_{1-4}$ (de préférence H, CN, un halogène, un alkyle en $C_{1-4}$ ou un alcoxy en $C_{1-4}$ éventuellement substitués par un à trois deutérium) ; et/ou
chaque $R^4$ est indépendamment choisi parmi H, un halogène, un alkyle en $C_{1-4}$, un halogénoalkyle en $C_{1-4}$, un alcoxy en $C_{1-4}$ et un halogénoalcoxy en $C_{1-4}$ (de préférence H, F ou un alcoxy en $C_{1-4}$) ; et/ou
chaque $R^5$ est indépendamment choisi parmi H, un halogène, un alkyle en $C_{1-4}$, un halogénoalkyle en $C_{1-4}$, un alcoxy en $C_{1-4}$ et un halogénoalcoxy en $C_{1-4}$ (de préférence H) ; et/ou
chaque $R^A$ est indépendamment choisi parmi H, un alkyle en $C_{1-2}$ ou un alcoxy en $C_{1-2}$ éventuellement substitué par un à trois deutérium (de préférence H, $-OCH_3$ ou $-OCD_3$) ; et/ou
chaque $R^B$ est indépendamment choisi parmi un halogène, -OH, un alkyle en $C_{1-3}$, un halogénoalkyle en $C_{1-3}$, $-(CH_2)_{0-2}$-alcoxy en $C_{1-2}$ éventuellement substitué par un à trois deutérium, un halogénoalcoxy en $C_{1-2}$, $-(CH_2)_{0-2}$NR*R* ou un cycloalkyle à 3-6 chaînons éventuellement substitué par un ou deux halogènes (de préférence F, Cl, -OH, $-CH_3$, $-CH_2CH_3$, $-CH(CH_3)_2$, $-CHF_2$, $-CF_3$, $-CH_2N(CH_3)_2$,$-CH_2OCH_3$, $-OCH_3$, $-OCHF_2$, $-OCD_3$, un cyclopropyle éventuellement substitué par un ou deux fluoro) ; et/ou

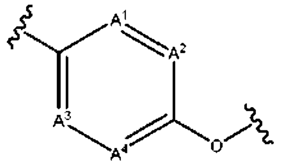 est 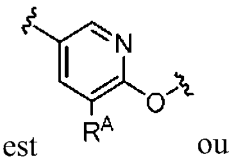 ou 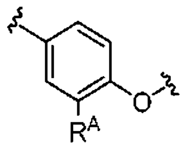

6. Composé de l'une des revendications 2, 4 ou 5, dans lequel le composé est présenté par la Formule (IV-A-1) ou (IV-A-2) :

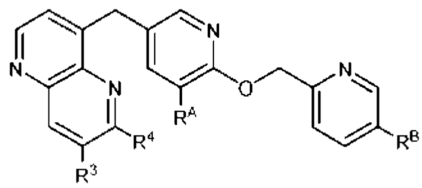

(IV-A-1),

ou

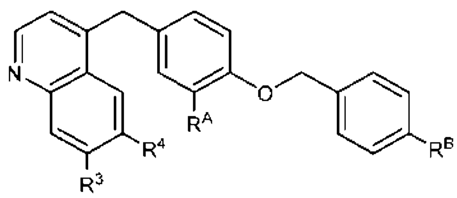

(IV-A-2),

ou un sel acceptable pharmaceutiquement de celui-ci.

7. Composé de l'une quelconque des revendications 1 à 3 et 6, sel acceptable pharmaceutiquement ou tautomère de celui-ci, dans lequel :

(i) chaque $R^3$ est indépendamment choisi parmi H, un halogène, -CN, un alkyle en $C_{1-6}$ éventuellement substitué par un à trois deutérium, un halogénoalkyle en $C_{1-6}$, un hydroxylalkyle en $C_{1-6}$, un alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, -NR*R*, un carbocyclyle à 3-6 chaînons, un hétérocyclyle monocyclique à 3-6 chaînons, un hétérocyclyle ponté à 7-10 chaînons, un phényle ou un hétéroaryle à 5-6 chaînons ; où ledit carbocyclyle, hétérocyclyle, phényle ou hétéroaryle dans le groupe représenté par $R^3$ est éventuellement substitué par un ou deux CN, un halogène, un alkyle en $C_{1-4}$, un halogénoalkyle en $C_{1-4}$, un alcoxy en $C_{1-4}$ ou un halogénoalcoxy en $C_{1-4}$ ; ou
(ii) chaque $R^3$ est indépendamment choisi parmi H, un halogène, CN, un alkyle en $C_{1-4}$ éventuellement substitué par un à trois deutérium, un alcoxy en $C_{1-4}$ éventuellement substitué par un à trois deutérium, NR*R*, -C(O)NR*R*, un cycloalkyle à 3-6 chaînons, un hétérocyclyle monocyclique à 3-6 chaînons, un hétéroaryle à 5-6 chaînons ou 6-oxa-3-azabicyclo[3.1.1]heptanyle, où ledit cycloalkyle, hétérocyclyle ou hétéroaryle dans le groupe représenté par $R^3$ est éventuellement substitué par un ou deux halogènes, un alkyle en $C_{1-2}$, un halogénoalkyle en $C_{1-2}$, un alcoxy en $C_{1-2}$ ou un halogénoalcoxy en $C_{1-2}$ ; ou
(iii) chaque $R^3$ est indépendamment choisi parmi H, F, Cl, CN, $CH_3$, $OCH_3$, $OCD_3$, $-N(CH_3)_2$, $-CON(CH_3)_2$, un cyclopropanyle, un azétidinyle éventuellement substitué par un ou deux fluoro ou $OCH_3$, un imidazole éventuellement substitué par $CH_3$, un morpholinyle, un pyridyle, un pipérazinyle éventuellement substitué par $CH_3$, un pyrrolidinyle éventuellement substitué par $OCH_3$, un pyrazolyle éventuellement substitué par $CH_3$, un thiazole éventuellement substitué par $CH_3$, ou 6-oxa-3-azabicyclo[3.1.1]heptanyle ; ou
(iv) chaque $R^3$ est indépendamment choisi parmi H ou $OCH_3$,

8. Composé de l'une quelconque des revendications 1, 2, 4, 5 ou 7, sel acceptable pharmaceutiquement ou tautomère de celui-ci, dans lequel :

(i) le cycle B est un phényle, un hétéroaryle monocyclique à 5 ou 6 chaînons éventuellement substitué par un ou deux $R^B$, chaque $R^B$ est indépendamment choisi parmi un halogène, -OH, un alkyle en $C_{1-4}$, un halogénoalkyle en

$C_{1-4}$, un alcoxyalkyle en $C_{1-4}$, un alcoxy en $C_{1-4}$ éventuellement substitué par un à trois deutérium, un halogénoalcoxy en $C_{1-4}$, -C(O)NR*R*, - $(CH_2)_{0-1}NR*R*$, -NR*C(O)R*, un carbocyclyle monocyclique à 3-6 chaînons, un hétérocyclyle monocyclique à 3-6 chaînons ; où ledit carbocyclyle ou hétérocyclyle dans le groupe représenté par $R^B$ est éventuellement substitué par un ou deux halogènes ou un alkyle en $C_{1-4}$ ; ou

chaque $R^B$ est indépendamment choisi parmi F, Cl, -OH, un alkyle en $C_{1-4}$, un halogénoalkyle en $C_{1-4}$, un alcoxyalkyle en $C_{1-4}$, un alcoxy en $C_{1-4}$ éventuellement substitué par un à trois deutérium, un halogénoalcoxy en $C_{1-4}$, -C(O)NR*R*, $-CH_2NR*R*$, -NR*C(O)R*, un cycloalkyle monocyclique à 3-5 chaînons, un hétérocyclyle monocyclique à 3-5 chaînons ; où ledit cycloalkyle ou hétérocyclyle dans le groupe représenté par $R^B$ est éventuellement substitué par un ou deux F ou un alkyle en $C_{1-2}$ ; ou

(ii) le cycle B est un phényle ou un hétéroaryle monocyclique à 5,6 chaînons, dont chacun est éventuellement substitué par un à quatre $R^B$, et deux $R^B$, conjointement avec les atomes de cycle B auxquels ils sont liés, forment un cycle monocyclique à 5-7 chaînons ou un cycle bicyclique à 6-9 chaînons fusionné au cycle B, ledit cycle monocyclique à 5-7 chaînons ou ledit cycle bicyclique à 6-9 chaînons est éventuellement substitué par un ou deux CN, un halogène, un alkyle en $C_{1-4}$, un halogénoalkyle en $C_{1-4}$, un alcoxy en $C_{1-4}$, un halogénoalcoxy en $C_{1-4}$ ou un cycloalkyle à 3-6 chaînons ; ou deux $R^B$, conjointement avec les atomes de cycle B auxquels ils sont liés, forment un cycle choisi parmi

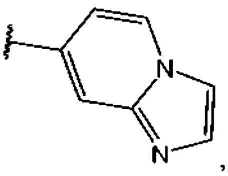, 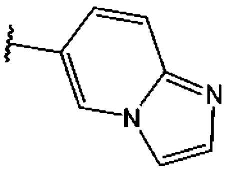, 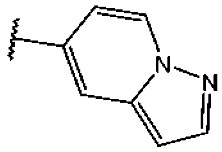, 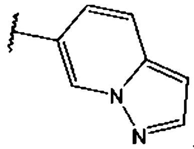, 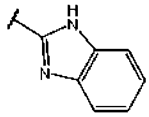,

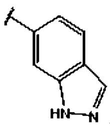, 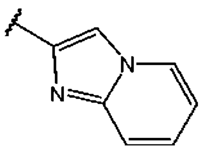, 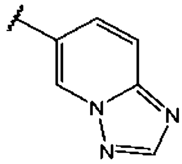, 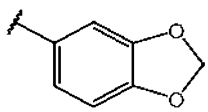, 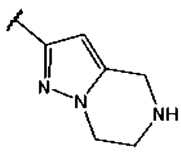,

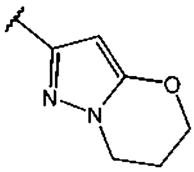, 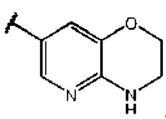, 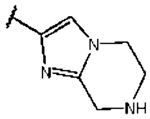, 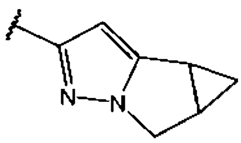, 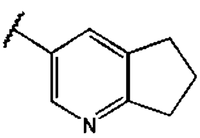,

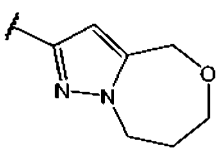, 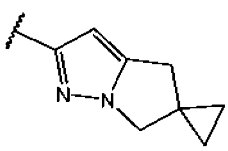, 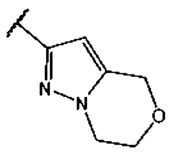, 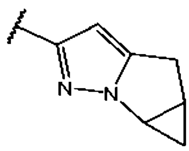, 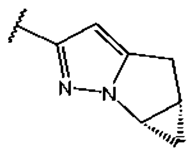,

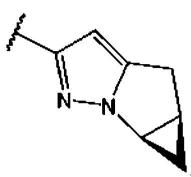, 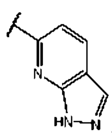, 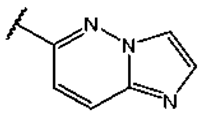, 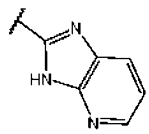, 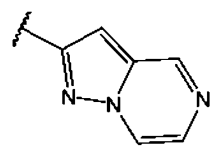,

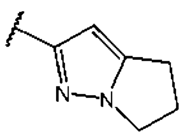, et 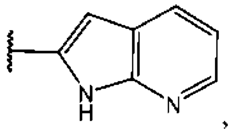,

dont chacun est éventuellement substitué par un ou deux groupes choisis parmi un halogène, un alkyle en $C_{1-4}$,

un halogénoalkyle en $C_{1-4}$, un alcoxy en $C_{1-4}$ et un halogénoalcoxy en $C_{1-4}$ ; ou
deux $R^B$, conjointement avec les atomes de cycle B auxquels ils sont liés, forment un cycle choisi parmi

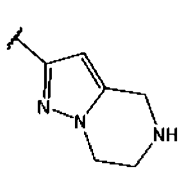,
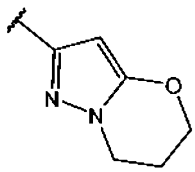,
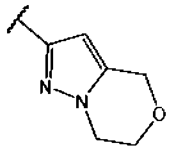,
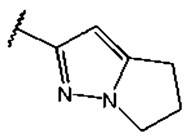

et

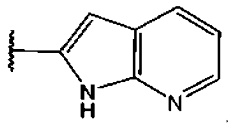

9. Composé de l'une quelconque des revendications 1 à 8, sel acceptable pharmaceutiquement, ou tautomère de celui-ci, dans lequel $R^C$ est H, et $R^D$ est H.

10. Composé représenté par la Formule (I') :

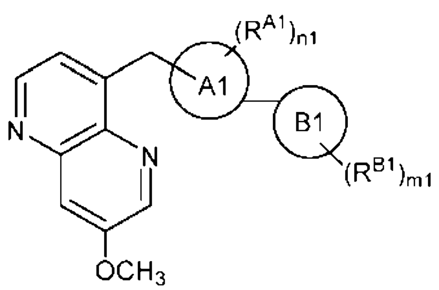

(I')

ou sel acceptable pharmaceutiquement de celui-ci, où :

le cycle A1 est un hétéroaryle bicyclique à 5,6 ou 6,6 chaînons contenant de l'azote ;
le cycle B1 est un carbocyclyle monocyclique à 3-6 chaînons ou un hétérocyclyle monocyclique à 3-6 chaînons ;
chaque $R^{A1}$ est indépendamment choisi parmi un halogène, -CN, -OH, un alkyle en $C_{1-6}$, un halogénoalkyle en $C_{1-6}$, un hydroxylalkyle en $C_{1-6}$, un alcoxyalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, - $(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\ ou\ 1}$-carbocyclyle à 3-12 chaînons, $-(CH_2)_{0\ ou\ 1}$-hétérocyclyle à 3-12 chaînons, $-(CH_2)_{0\ ou\ 1}$-aryle à 6-10 chaînons ou $-(CH_2)_{0\ ou\ 1}$-hétéroaryle à 5-10 chaînons ; où ledit carbocyclyle, hétérocyclyle, aryle ou hétéroaryle dans le groupe représenté par $R^{A1}$ est éventuellement substitué par un ou deux CN, un halogène, un alkyle en $C_{1-6}$, un halogénoalkyle en $C_{1-6}$, un alcoxy en $C_{1-6}$ ou un halogénoalcoxy en $C_{1-6}$ ;
chaque $R^{B1}$ est indépendamment choisi parmi un halogène, -CN, -OH, un alkyle en $C_{1-6}$, un halogénoalkyle en $C_{1-6}$, un hydroxylalkyle en $C_{1-6}$, un alcoxyalkyle en $C_{1-6}$, un alcényle en $C_{2-6}$, un alcynyle en $C_{2-6}$, $-(CH_2)_{0-4}$-alcoxy en $C_{1-6}$ éventuellement substitué par un à trois deutérium, un halogénoalcoxy en $C_{1-6}$, un hydroxyalcoxy en $C_{1-6}$, -C(O)R*, -C(O)OR*, -C(O)NR*R*, $-SO_2R^*$, - $(CH_2)_{0-4}NR^*R^*$, -NR*C(O)R*, -NR*C(O)OR*, $-NR^*SO_2R^*$, $-NR^*SO_2NR^*R^*$, -P(O)R*R*, $-(CH_2)_{0\ ou\ 1}$-carbocyclyle à 3-12 chaînons, $-(CH_2)_{0\ ou\ 1}$-hétérocyclyle à 3-12 chaînons, $-(CH_2)_{0\ ou\ 1}$-aryle à 6-10 chaînons ou $-(CH_2)_{0\ ou\ 1}$-hétéroaryle à 5-10 chaînons ; où ledit carbocyclyle, hétérocyclyle, aryle ou hétéroaryle dans le groupe représenté par $R^{B1}$ est éventuellement substitué par un ou deux CN, un halogène, un alkyle en $C_{1-6}$, un halogénoalkyle en $C_{1-6}$, un alcoxy en $C_{1-6}$ ou un halogénoalcoxy en $C_{1-6}$ ; et/ou
deux $R^{B1}$ conjointement avec les atomes de cycle B1 auxquels ils sont liés, forment un cycle monocyclique à 5,6 chaînons ou un cycle bicyclique à 6-9 chaînons fusionné au cycle B1, ledit cycle à 5,6 chaînons ou ledit cycle bicyclique à 6-9 chaînons est éventuellement substitué par un ou deux CN, un halogène, un alkyle en $C_{1-6}$, un

halogénoalkyle en $C_{1-6}$, un alcoxy en $C_{1-6}$, un halogénoalcoxy en $C_{1-6}$ ou un cycloalkyle à 3-6 chaînons ; chaque R* est indépendamment choisi dans le groupe constitué par H, un alkyle en $C_{1-6}$, un halogénoalkyle en $C_{1-6}$, un alcényle en $C_{2-6}$, un alcynyle en $C_{2-6}$, un carbocyclyle à 3-6 chaînons ou un hétérocyclyle à 4-6 chaînons ;
m1 vaut 0, 1, 2, 3 ou 4 ; et
n1 vaut 0, 1, 2 ou 3.

**11.** Composé de la revendication 10 ou sel acceptable pharmaceutiquement de celui-ci, dans lequel :

(i)

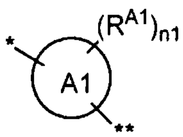

est

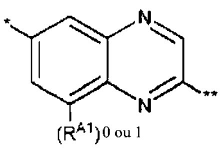 , 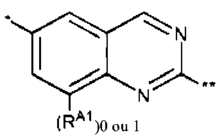 , 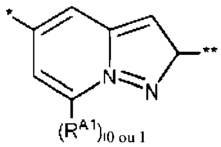 , ou 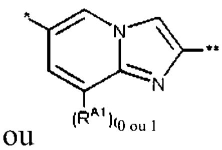 ,

où -* représente le point auquel $-CH_2-$ se fixe ; -** représente le point auquel le cycle B1 se fixe ; et $R^{A1}$ est un alkyle en $C_{1-4}$ ou un alcoxy en $C_{1-4}$ ; et/ou
(ii) le cycle B1 est

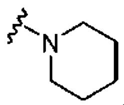 , 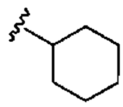 ou 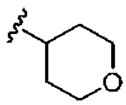 ;

$R^{B1}$ est un alkyle en $C_{1-4}$ ou un alcoxy en $C_{1-4}$ ; et
n1 vaut 0 ou 1.

**12.** Composé choisi parmi :

| Exemple n° | Structure | Exemple n° | Structure |
|---|---|---|---|
| 1 | 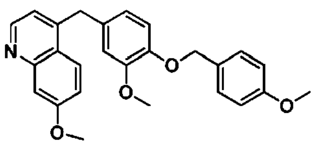 | 80 | 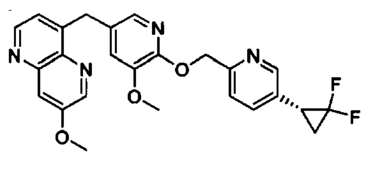 o u 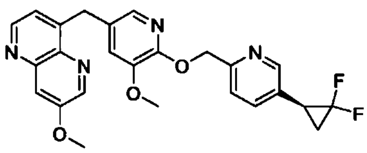 |

(continued)

| Exemple n° | Structure | Exemple n° | Structure |
|---|---|---|---|
| 2 | 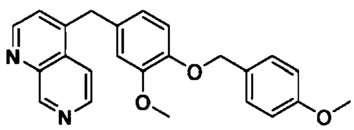 | 81 | 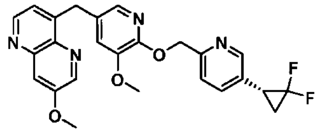 o u 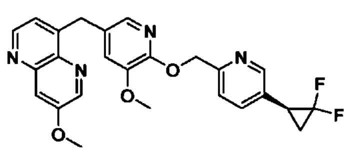 |
| 3 | 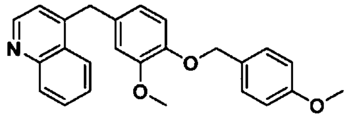 | 82 | 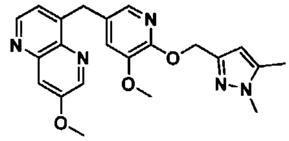 |
| 4 | 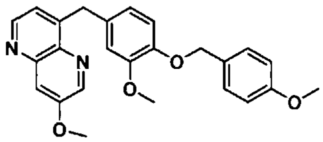 | 83 | 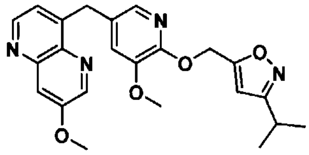 |
| 5 | 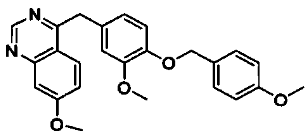 | 84 | 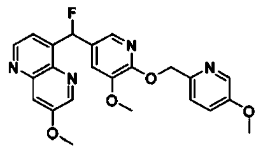 |
| 6 | 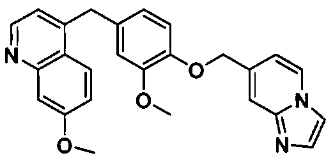 | 85 | 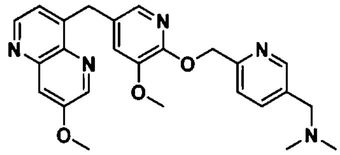 |
| 7 | 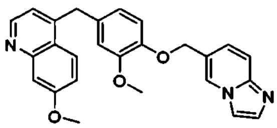 | 86 | 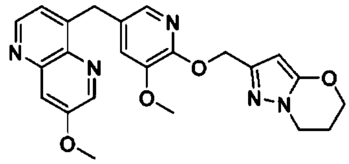 |
| 8 | 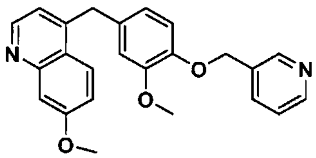 | 87 | 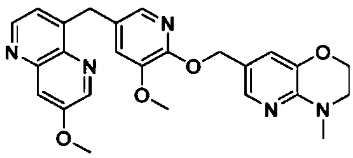 |
| 9 | 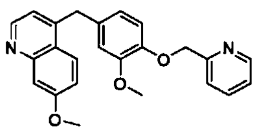 | 88 | 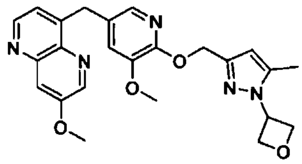 |
| 10 | 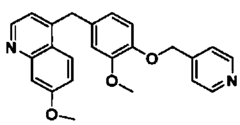 | 89 | 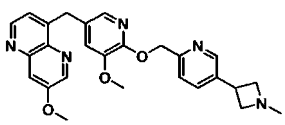 |

(continued)

| Exemple n° | Structure | Exemple n° | Structure |
|---|---|---|---|
| 11 | 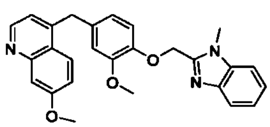 | 90 | 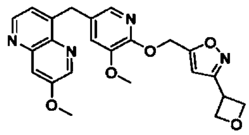 |
| 12 | 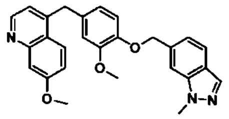 | 91 | 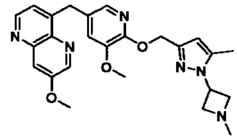 |
| 13 | 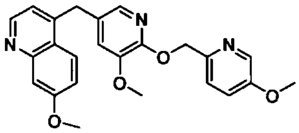 | 92 | 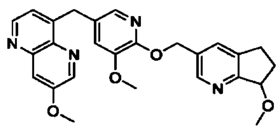 |
| 14 | 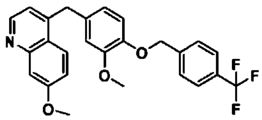 | 93 | 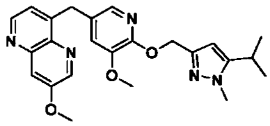 |
| 15 | 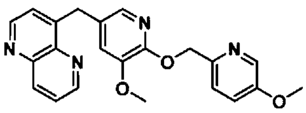 | 94 | 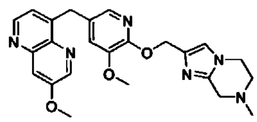 |
| 16 | 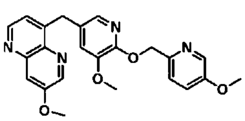 | 95 | 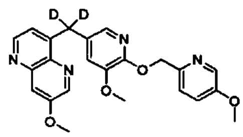 |
| 17 | 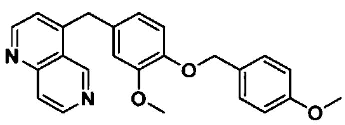 | 96 | 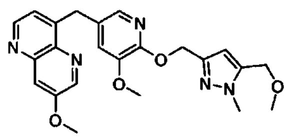 |
| 18 | 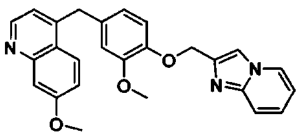 | 97 | 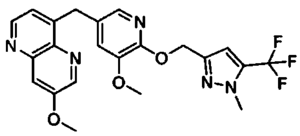 |
| 19 | 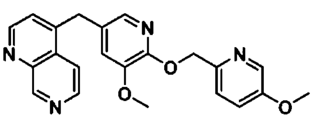 | 98 | 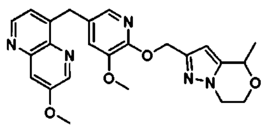 |
| 20 | 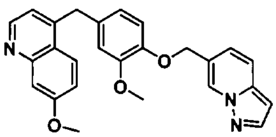 | 99 | 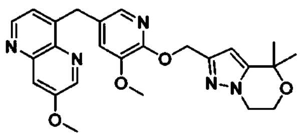 |
| 21 | 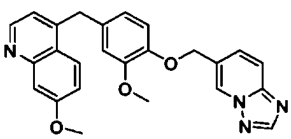 | 100 | 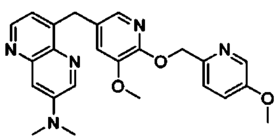 |

(continued)

| Exemple n° | Structure | Exemple n° | Structure |
|---|---|---|---|
| 22 | 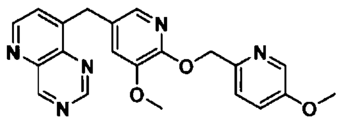 | 101 | 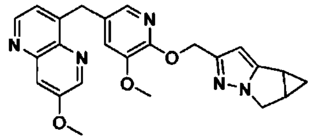 |
| 23 | 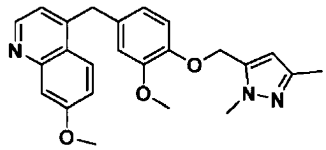 | 102 | 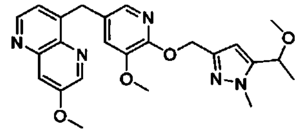 |
| 24 | 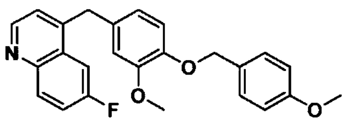 | 103 | 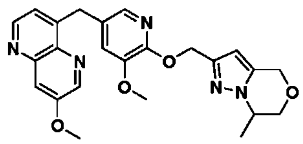 |
| 25 | 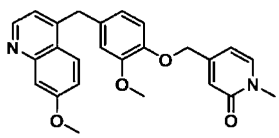 | 104 | 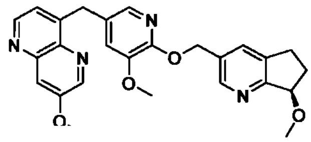 ou 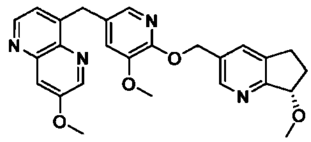 |
| 26 | 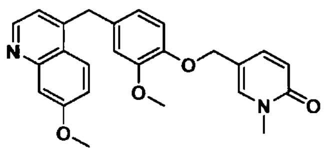 | 105 | 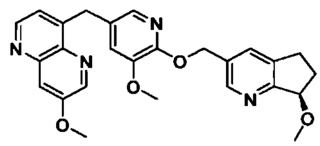 ou 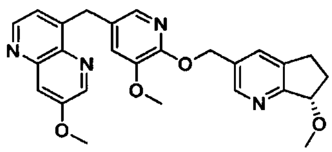 |
| 27 | 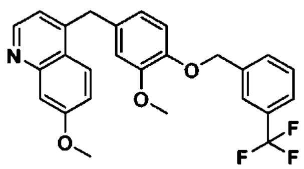 | 106 | 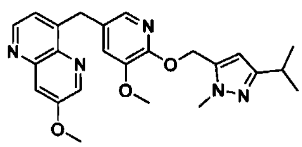 |
| 28 | 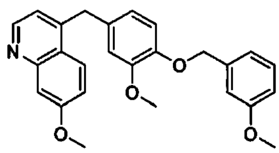 | 107 | 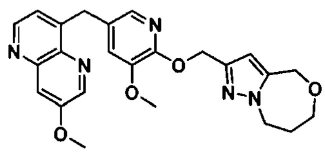 |

(continued)

| Exemple n° | Structure | Exemple n° | Structure |
|---|---|---|---|
| 29 | 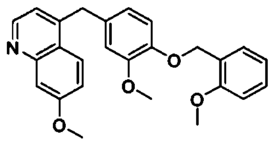 | 108 | 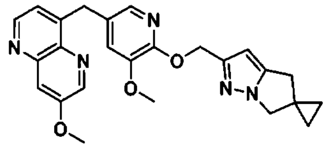 |
| 30 | 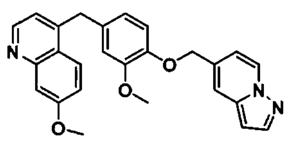 | 109 | 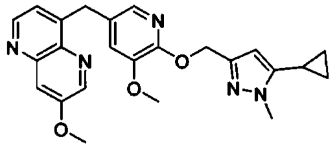 |
| 31 | 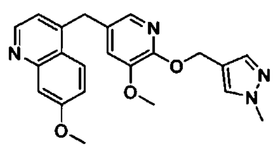 | 110 | 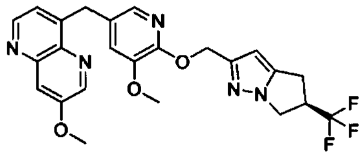 |
| 32 | 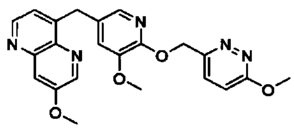 | 111 | 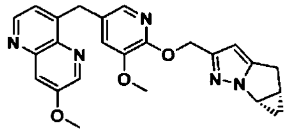 |
| 33 | 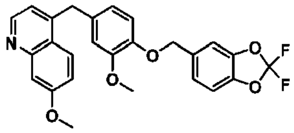 | 112 | 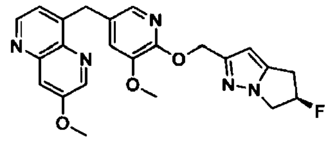 |
| 34 | 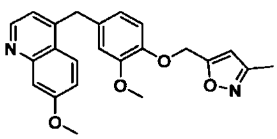 | 113 | 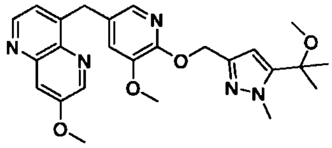 |
| 35 | 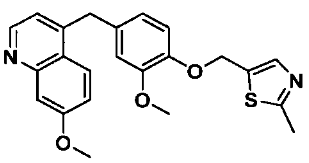 | 114 | 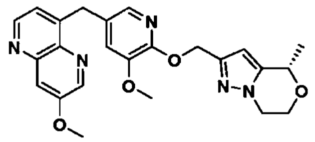 ou 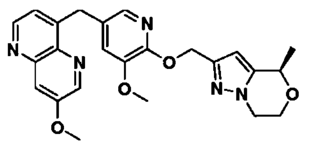 |
| 36 | 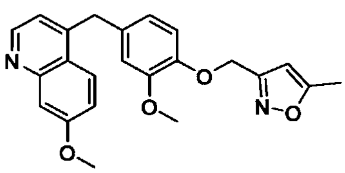 | 115 | 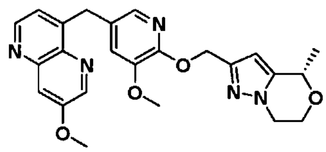 ou 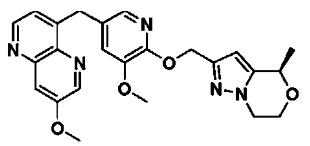 |

(continued)

| Exemple n° | Structure | Exemple n° | Structure |
|---|---|---|---|
| 37 | 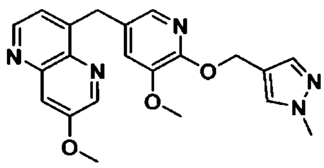 | 116 | 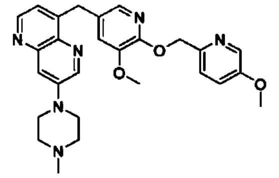 |
| 38 | 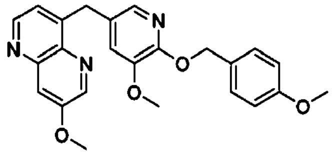 | 117 | 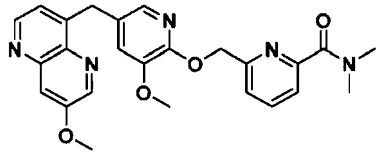 |
| 39 | 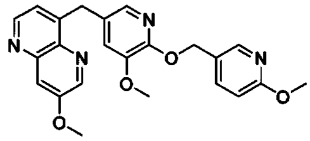 | 118 | 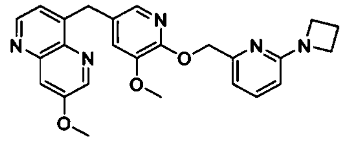 |
| 40 | 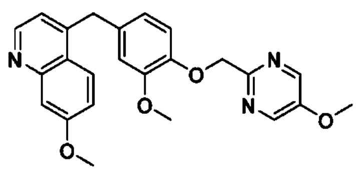 | 119 | 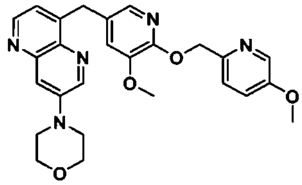 |
| 41 | 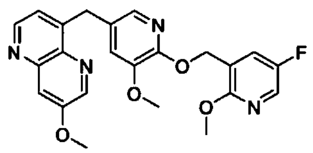 | 120 | 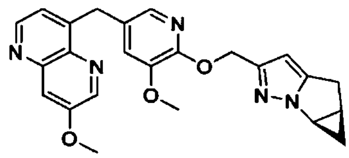 |
| 42 | 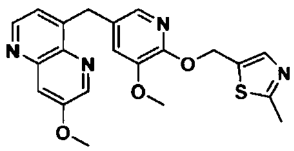 | 121 | 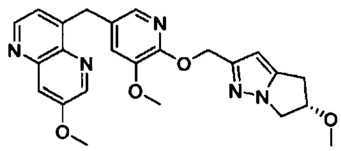 |
| 43 | 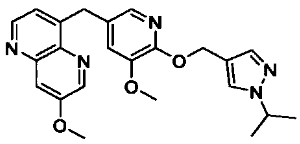 | 122 | 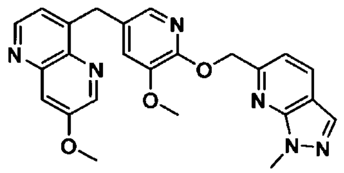 |
| 44 | 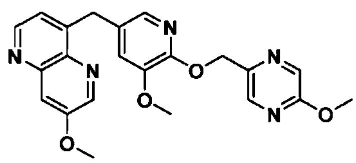 | 123 | 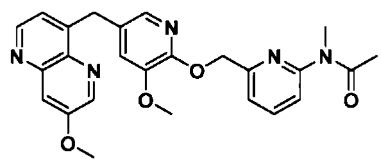 |
| 45 | 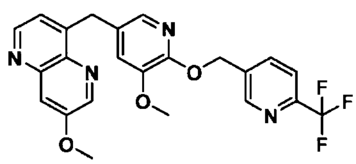 | 124 | 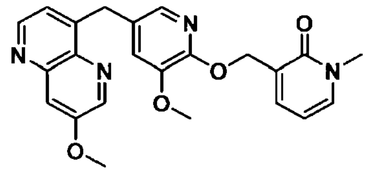 |

(continued)

| Exemple n° | Structure | Exemple n° | Structure |
|---|---|---|---|
| 46 | 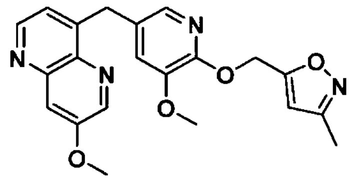 | 125 | 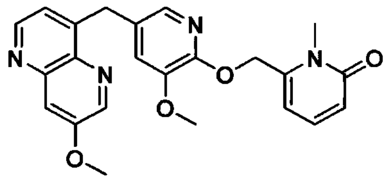 |
| 47 | 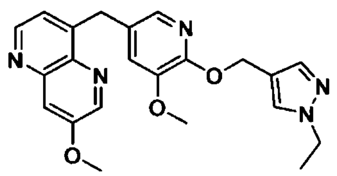 | 126 | 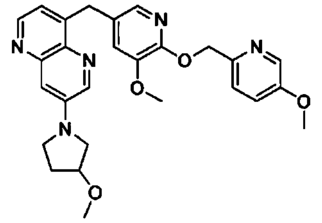 |
| 48 | 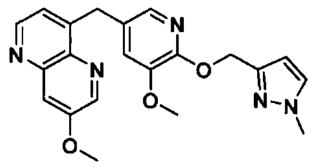 | 127 | 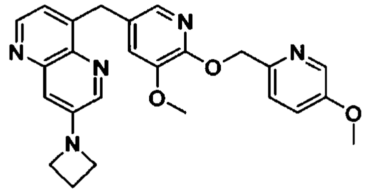 |
| 49 | 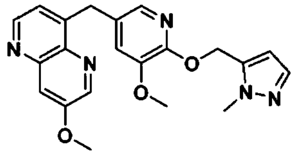 | 128 | 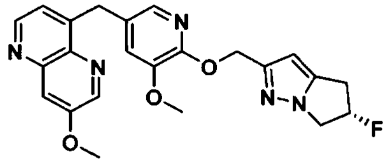 |
| 50 | 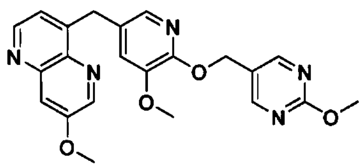 | 129 | 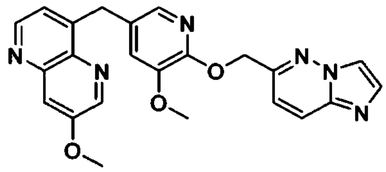 |
| 51 | 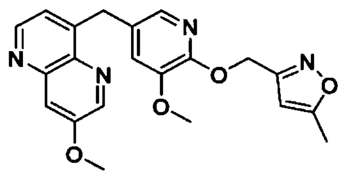 | 130 | 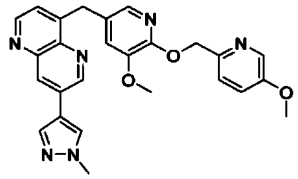 |
| 52 | 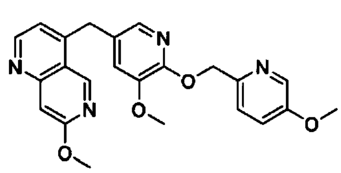 | 131 | 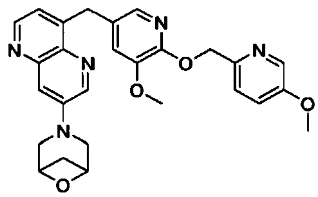 |
| 53 | 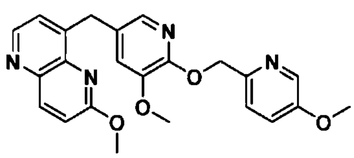 | 132 | 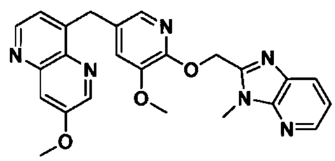 |

(continued)

| Exemple n° | Structure | Exemple n° | Structure |
|---|---|---|---|
| 54 | 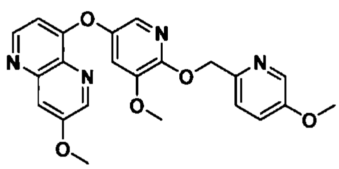 | 133 | 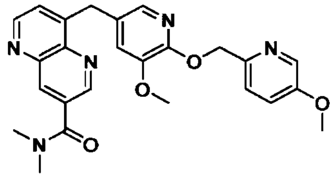 |
| 55 | 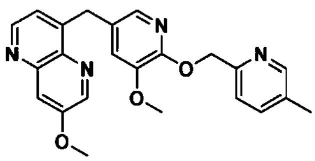 | 134 | 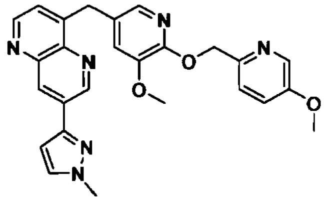 |
| 56 | 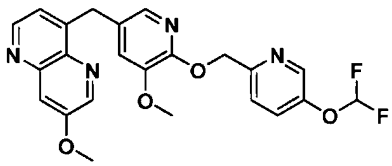 | 135 | 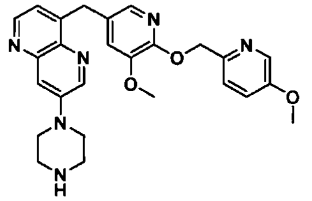 |
| 57 | 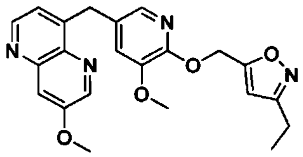 | 136 | 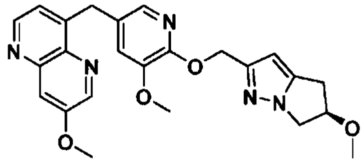 |
| 58 | 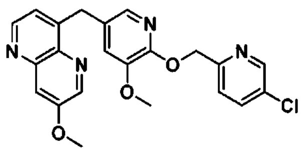 | 137 | 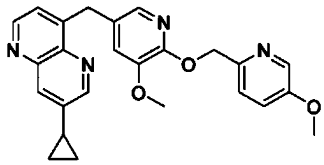 |
| 59 | 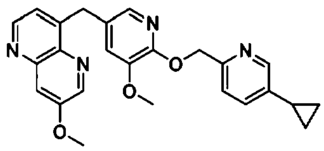 | 138 | 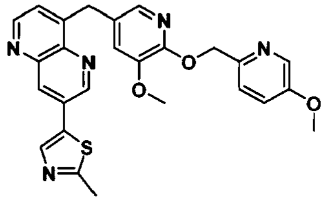 |
| 60 | 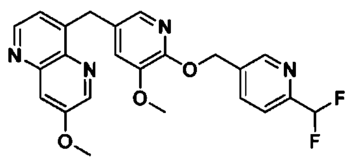 | 139 | 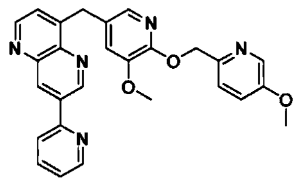 |
| 61 | 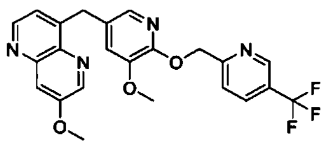 | 140 | 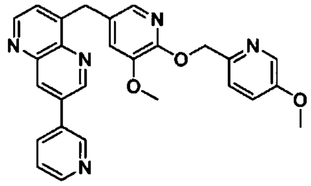 |

(continued)

| Exemple n° | Structure | Exemple n° | Structure |
|---|---|---|---|
| 62 | 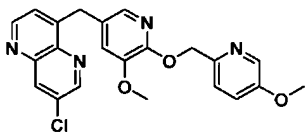 | 141 | 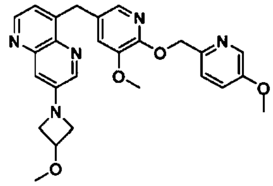 |
| 63 | 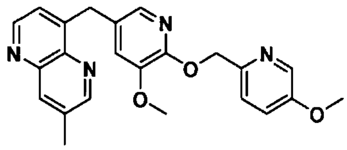 | 142 | 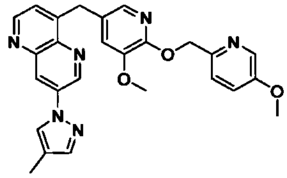 |
| 64 | 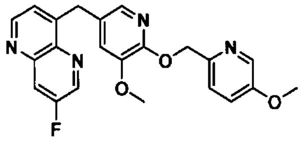 | 143 | 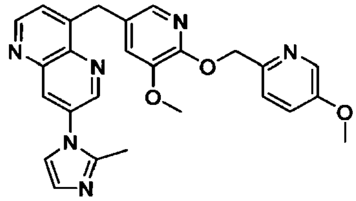 |
| 65 | 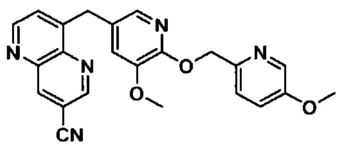 | 144 | 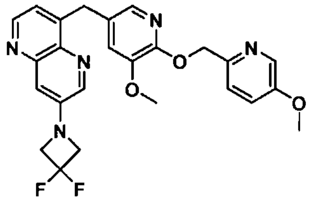 |
| 66 | 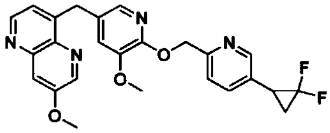 | 145 | 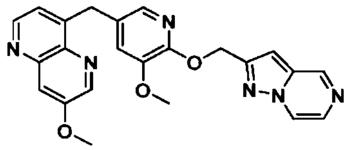 |
| 67 | 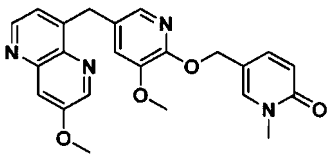 | 146 | 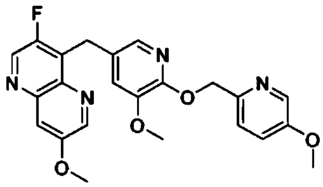 |
| 68 | 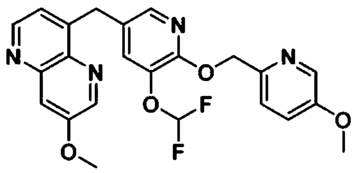 | 147 | 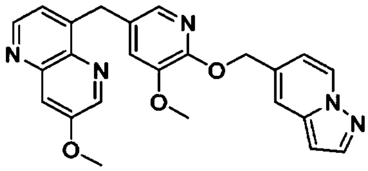 |
| 69 | 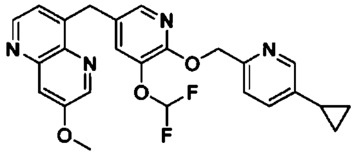 | 148 | 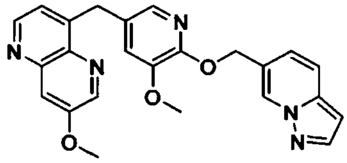 |

(continued)

| Exemple n° | Structure | Exemple n° | Structure |
|---|---|---|---|
| 70 | 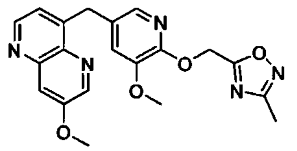 | 149 | 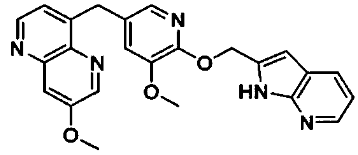 |
| 71 | 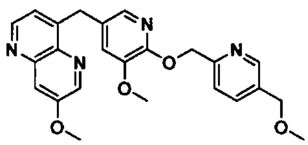 | 150 | 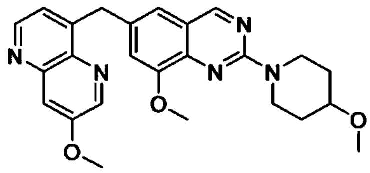 |
| 72 | 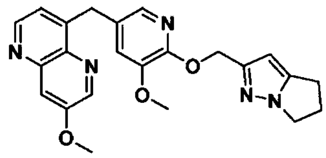 | 151 | 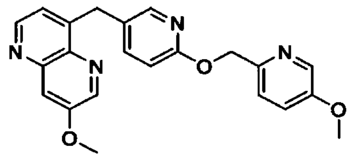 |
| 73 | 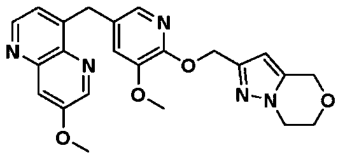 | 152 | 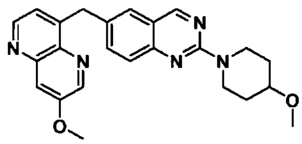 |
| 74 | 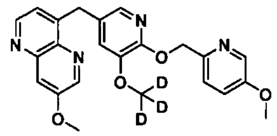 | 153 | 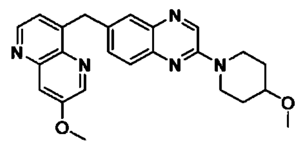 |
| 75 | 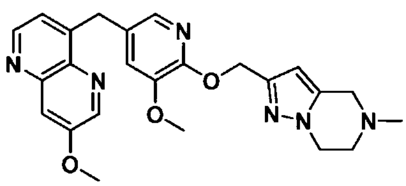 | 154 | 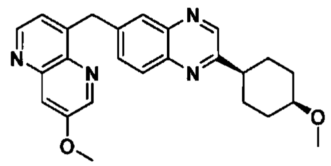 ou 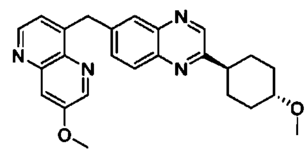 |
| 76 | 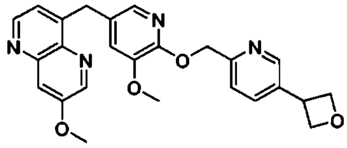 | 155 | 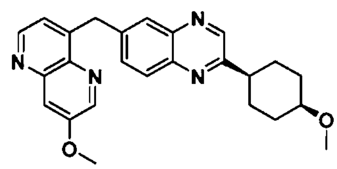 ou 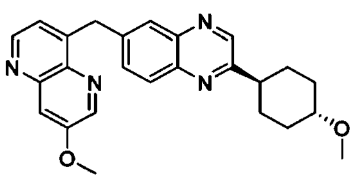 |

(continued)

| Exemple n° | Structure | Exemple n° | Structure |
|---|---|---|---|
| 77 | 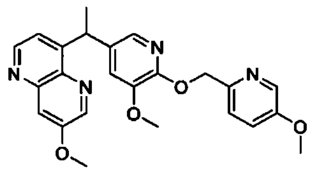 | 156 | 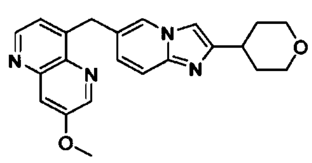 |
| 78 | 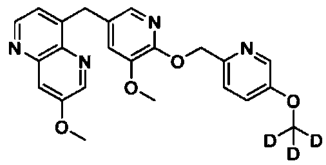 | 157 | 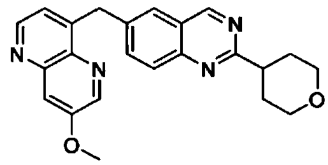 |
| 79 | 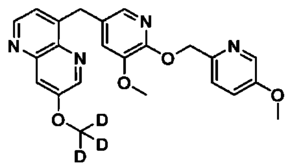 | 158 | 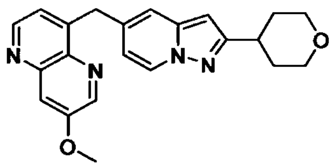 |

ou sel acceptable pharmaceutiquement de celui-ci.

**13.** Composé de la revendication 12, ou sel acceptable pharmaceutiquement de celui-ci, dans lequel le composé est

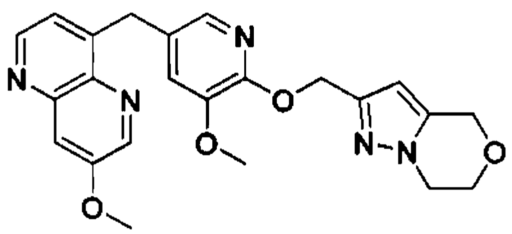

.

**14.** Composition pharmaceutique comprenant le composé de l'une quelconque des revendications 1 à 13 ou un sel acceptable pharmaceutiquement de celui-ci, et un support ou un excipient acceptable pharmaceutiquement.

**15.** Composé de l'une quelconque des revendications 1 à 13, ou sel acceptable pharmaceutiquement de celui-ci, destiné à être utilisé dans un procédé de traitement d'une maladie médiée par CSF-1R ou au moins en partie par CSF-1R chez un sujet, dans lequel la maladie est une maladie auto-immune, une maladie inflammatoire, une maladie neurodégénérative, un cancer, une maladie métabolique, l'obésité ou une maladie liée à l'obésité.

**16.** Composé destiné à être utilisé selon la revendication 15, dans lequel

la maladie auto-immune ou la maladie inflammatoire est choisie parmi la polyarthrite rhumatoïde, l'arthrite induite par le collagène, l'arthrose, la synovite villonodulaire pigmentée (PVNS), le lupus érythémateux systémique, la sclérose en plaques, la sclérodermie systémique, la néphrite auto-immune, les maladies inflammatoires chroniques de l'intestin, la maladie de Crohn, la colite ulcéreuse, le psoriasis, la dermatite atopique, l'asthme, la bronchopneumopathie chronique obstructive, la maladie de Behcet, le purpura thrombocytopénique idiopathique, l'arthrite spinale, l'arthrite juvénile idiopathique systémique (SoJIA), la pancréatite, les lésions d'ischémie-reperfusion des organes parenchymateux, le rejet de greffe d'organe, la septicémie, le syndrome de réponse inflammatoire systémique et les lésions d'organes induites par des médicaments de chimiothérapie ;

la maladie neurodégénérative est choisie parmi la maladie de Parkinson (PD), l'atrophie multisystématisée, la maladie d'Alzheimer (AD), la démence lobaire fronto-temporale, la maladie de Huntington (HD), la dégénérescence corticobasale, l'ataxie spinocérébelleuse, la sclérose latérale amyotrophique (ALS), l'atrophie musculaire spinale (SMA) et la neuropathie sensitivomotrice héréditaire (CMT) ; et

le cancer est une tumeur solide ou une malignité hématologique, telle que le cancer de l'ovaire, le cancer du

poumon (y compris le cancer du poumon non à petites cellules), une tumeur cérébrale (y compris le glioblastome (GBM)), la tumeur ténosynoviale à cellules géantes, la tumeur stromale gastro-intestinale (GIST), le cancer gastrique, le cancer de l'œsophage, le cancer du côlon, le cancer colorectal, le cancer du pancréas, le cancer de la prostate, le cancer du sein, le cancer du col de l'utérus, le mélanome, le mésothéliome, le carcinome mésothélial, le cancer rénal, le cancer du foie, le carcinome thyroïdien, le cancer de la tête et du cou, le carcinome urothélial, le cancer de la vessie, le cancer de l'endomètre, le choriocarcinome, le carcinome surrénal, le sarcome, la leucémie, le lymphome ou le myélome.

**17.** Composé de l'une quelconque des revendications 1 à 13 ou sel acceptable pharmaceutiquement de celui-ci, destiné à être utilisé dans un procédé de traitement de la maladie d'Alzheimer, de la paralysie supranucléaire progressive, d'un trouble neurodégénératif médié par tau, ou d'une maladie ou d'un trouble impliquant une inflammation médiée par la microglie chez un sujet.

# Figure 1

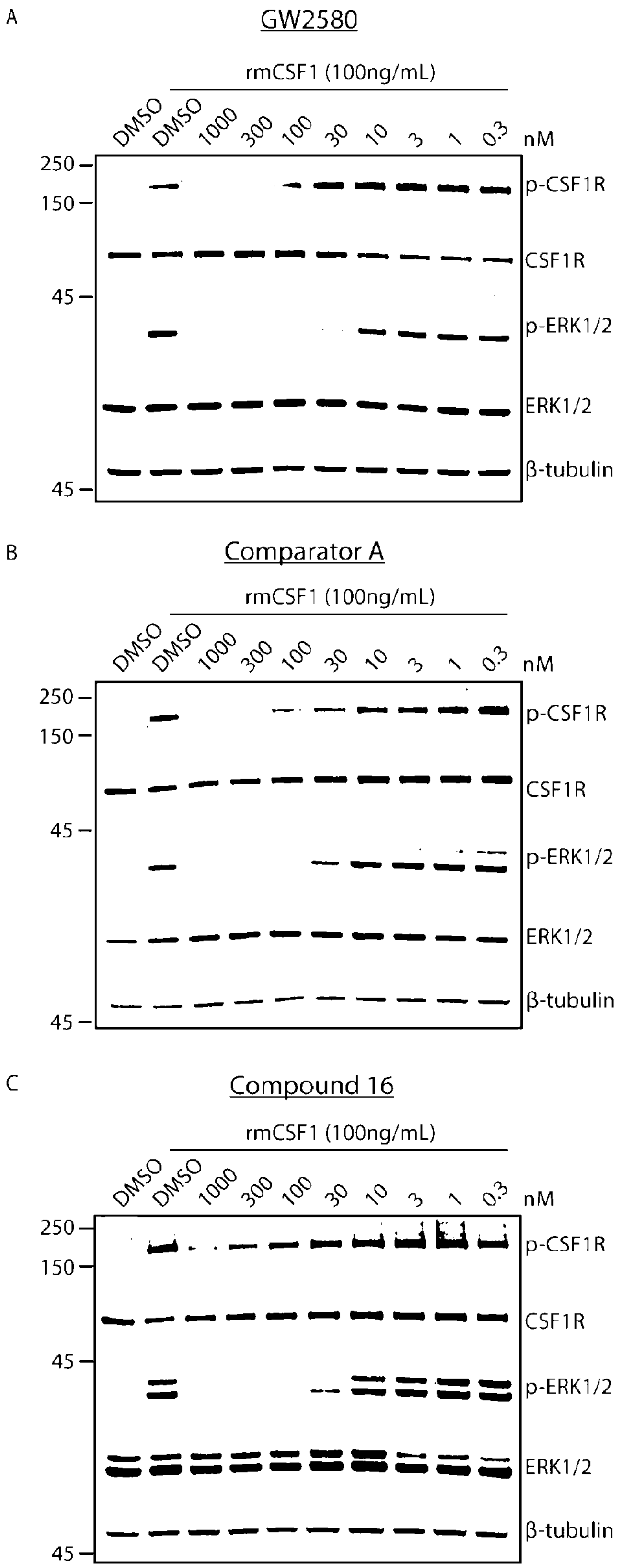
A
GW2580
rmCSF1 (100ng/mL)
DMSO DMSO 1000 300 100 30 10 3 1 0.3 nM
250
150
45
45
p-CSF1R
CSF1R
p-ERK1/2
ERK1/2
β-tubulin
B
Comparator A
rmCSF1 (100ng/mL)
DMSO DMSO 1000 300 100 30 10 3 1 0.3 nM
250
150
45
45
p-CSF1R
CSF1R
p-ERK1/2
ERK1/2
β-tubulin
C
Compound 16
rmCSF1 (100ng/mL)
DMSO DMSO 1000 300 100 30 10 3 1 0.3 nM
250
150
45
45
p-CSF1R
CSF1R
p-ERK1/2
ERK1/2
β-tubulin

**Figure 2**

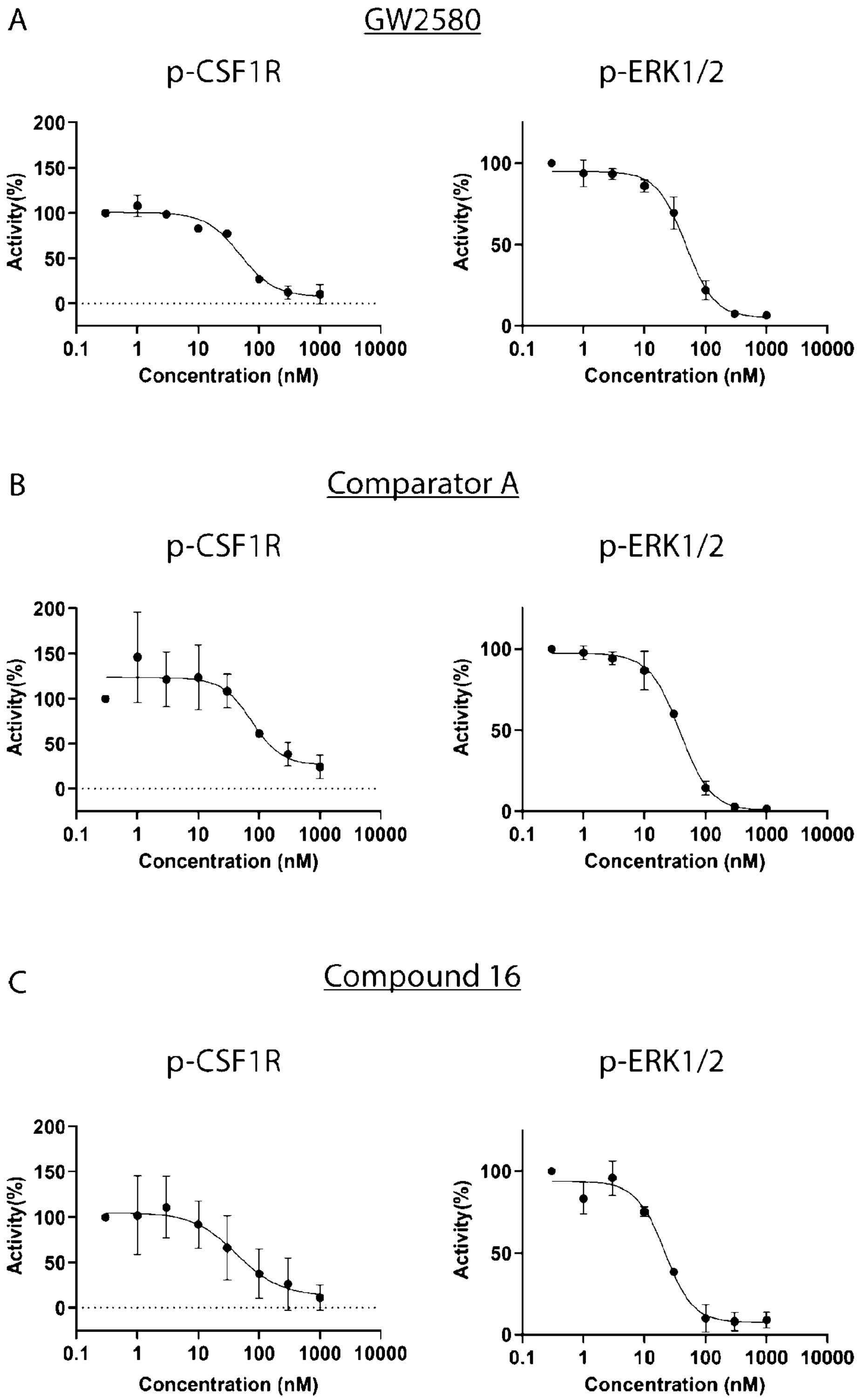
A
GW2580
p-CSF1R
p-ERK1/2
B
Comparator A
p-CSF1R
p-ERK1/2
C
Compound 16
p-CSF1R
p-ERK1/2
Activity(%)
Concentration (nM)
0.1
1
10
100
1000
10000
0
50
100
150
200

**Figure 3**

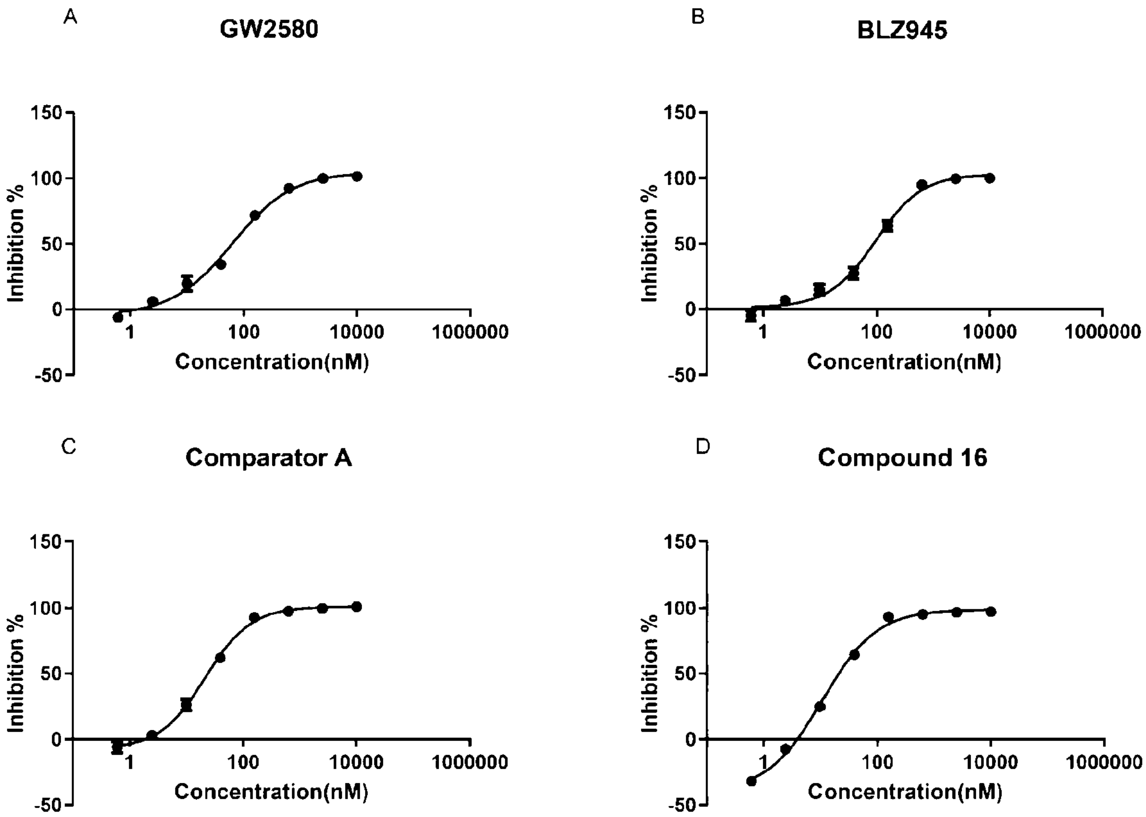
A
GW2580
B
BLZ945
C
Comparator A
D
Compound 16
Inhibition %
150
100
50
0
-50
1
100
10000
1000000
Concentration(nM)

# Figure 4

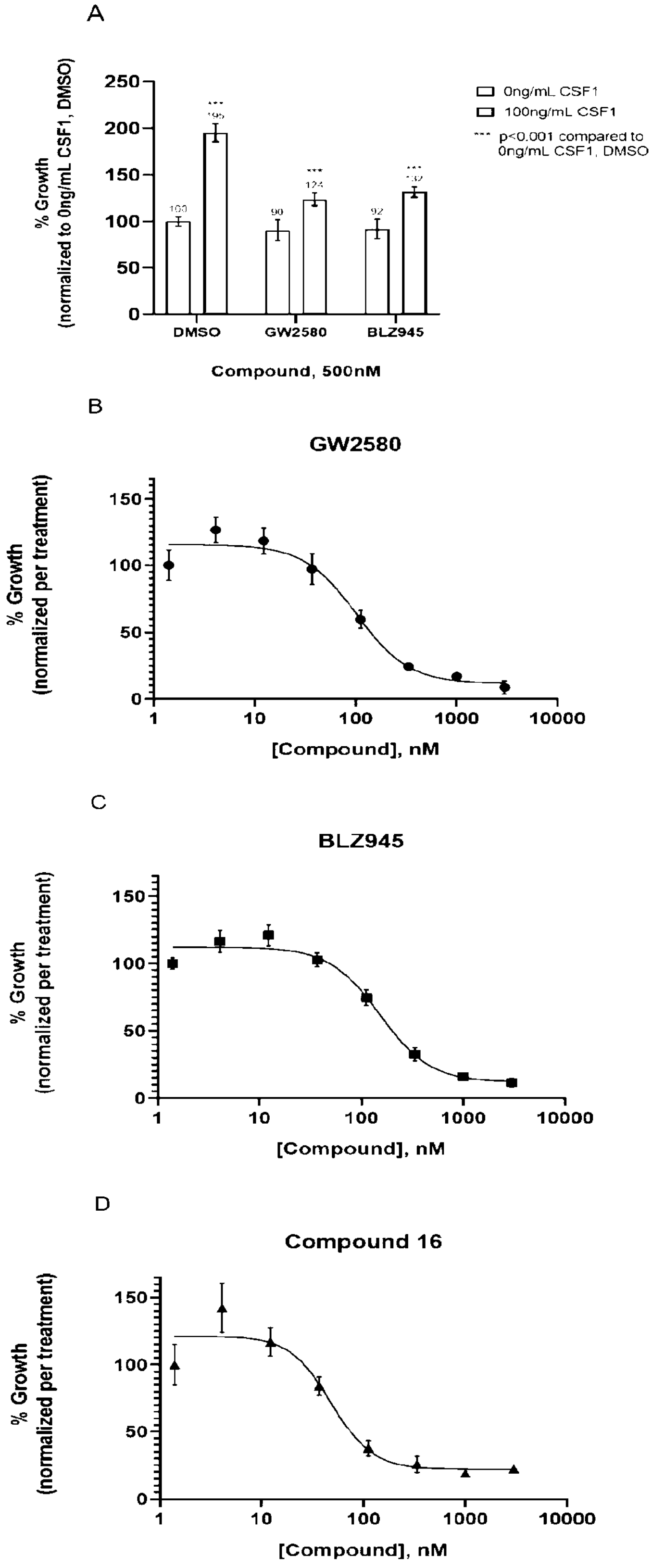
A
% Growth (normalized to 0ng/mL CSF1, DMSO)
0ng/mL CSF1
100ng/mL CSF1
*** p<0.001 compared to 0ng/mL CSF1, DMSO
100
90
124
92
DMSO
GW2580
BLZ945
Compound, 500nM
B
GW2580
% Growth (normalized per treatment)
[Compound], nM
C
BLZ945
% Growth (normalized per treatment)
[Compound], nM
D
Compound 16
% Growth (normalized per treatment)
[Compound], nM

Figure 5

5A

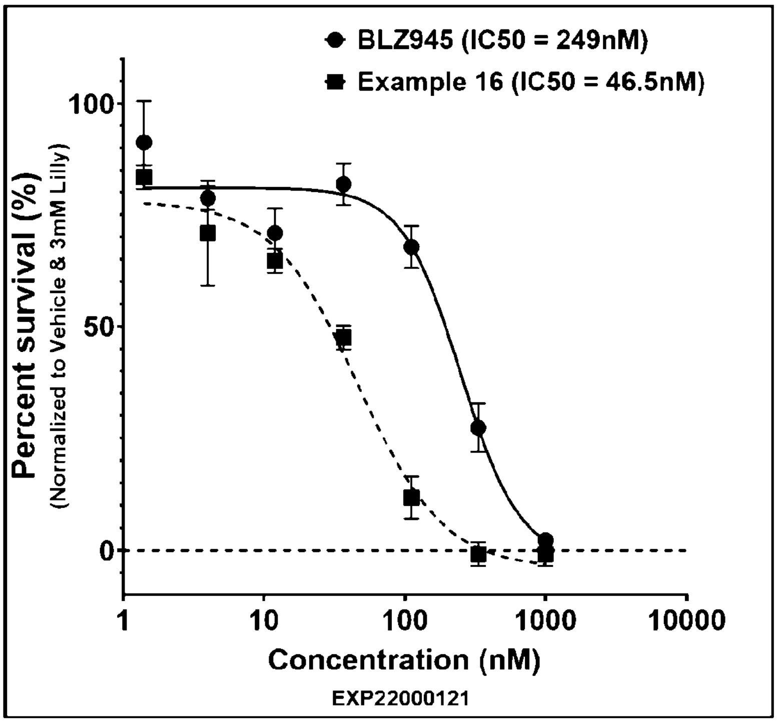


5B

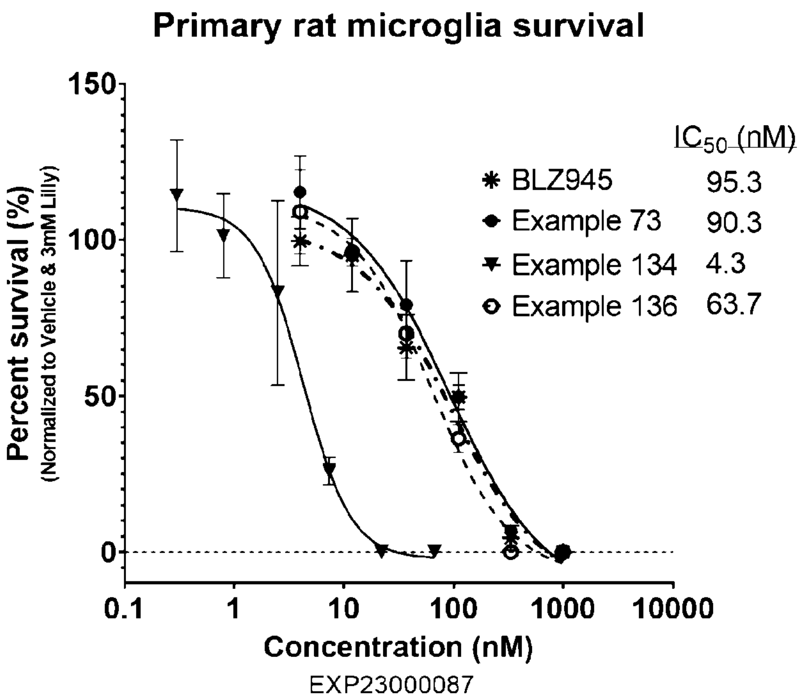

Figure 6

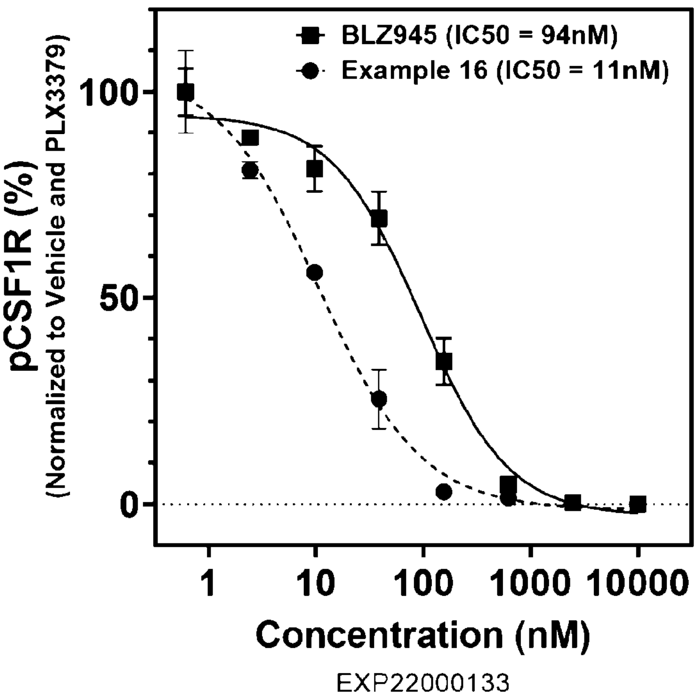
BLZ945 (IC50 = 94nM)
Example 16 (IC50 = 11nM)
pCSF1R (%)
(Normalized to Vehicle and PLX3379)
100
50
0
1
10
100
1000
10000
Concentration (nM)
EXP22000133

Figure 7

7A

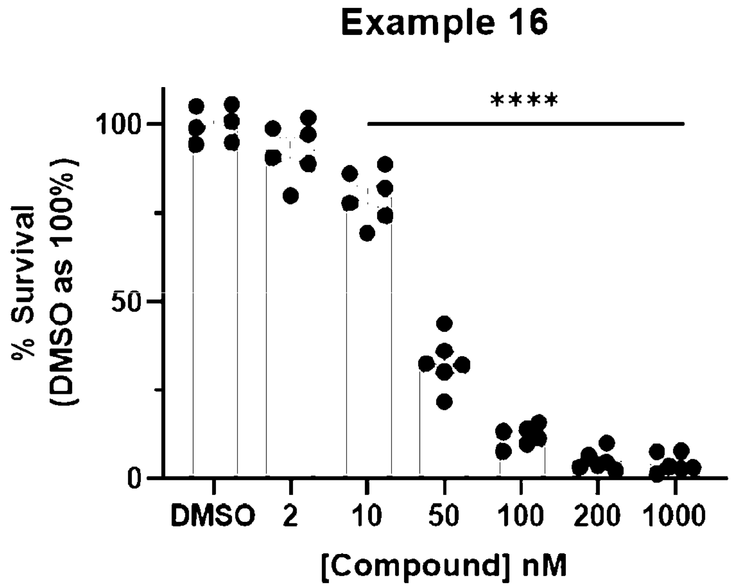


7B

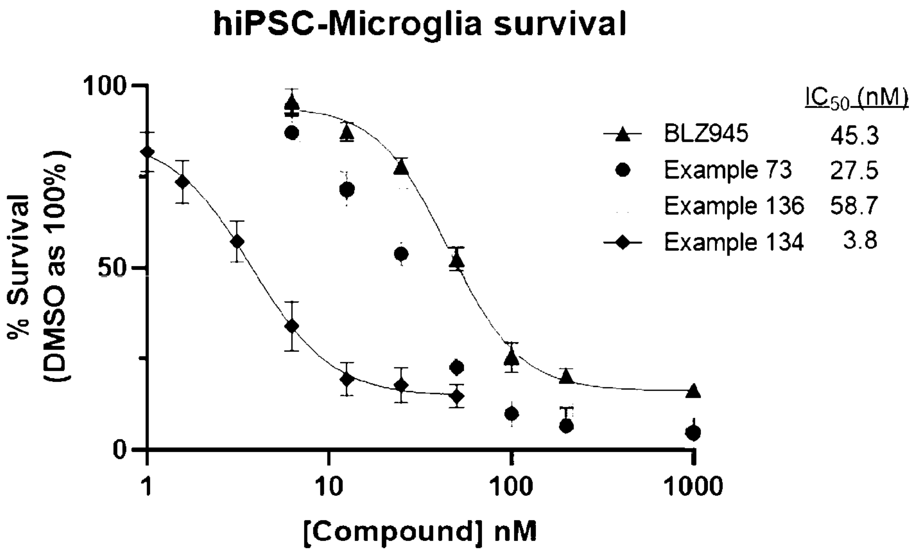

**Figure 8**

**8A** **8B**

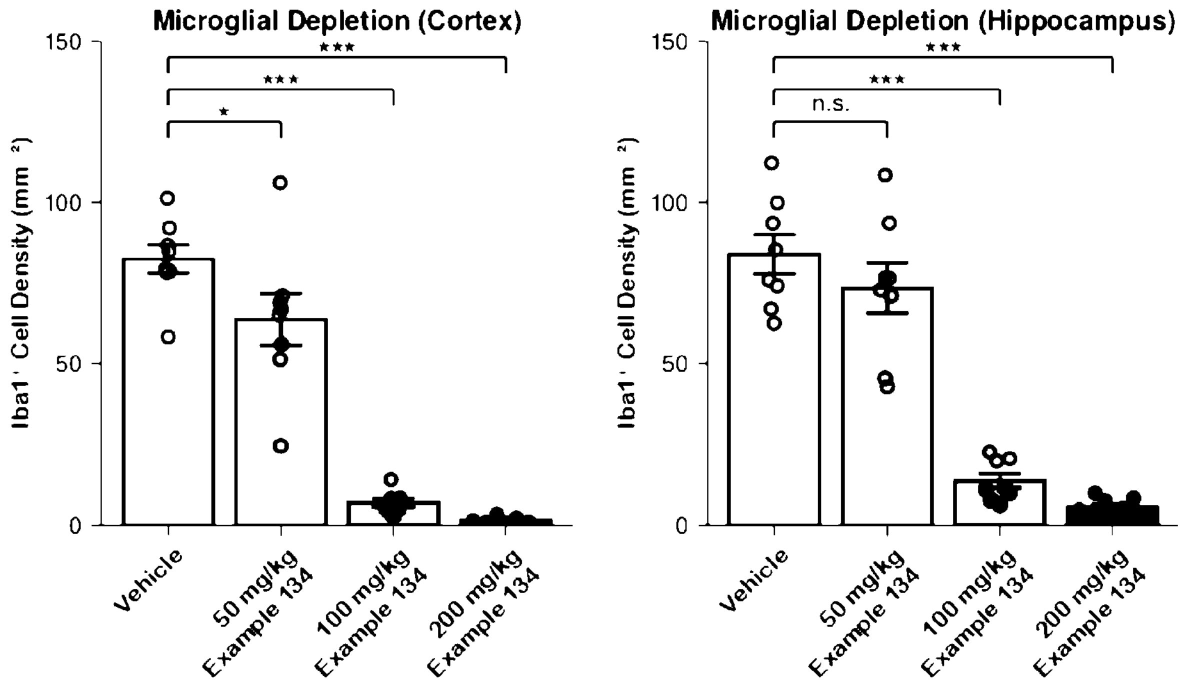

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022098943 W
- CN 2023093222 W


### Non-patent literature cited in the description


- *Cold Spring Harb Perspect Biol.*, 2014, vol. 6 (6)
- *J Cell Biochem.*, 1988, vol. 38 (3), 179-87
- *Immunity.*, 2014, vol. 41 (1), 49-61
- *Nat Med.*, 2013, vol. 19 (10), 1264-72
- *N Engl J Med.*, 2015, vol. 373 (5), 428-37
- *Arthritis Res Ther.*, 2016, vol. 18, 75
- *Nat Rev Immunol.*, 2008, vol. 8 (7), 533-44
- *J Immunother Cancer.*, 2017, vol. 5 (1), 53
- *Neurobiol Aging.*, 2000, vol. 21, 383-421
- *Brain Res.*, 1994, vol. 639, 171-4
- *Brain.*, 2016, vol. 139, 891-907
- *Sci Rep.*, 2016, vol. 6, 25663
- *J. Pharm. Sci.*, 1977, vol. 66, 1-19
- **FINNIN** ; **MORGAN**. *J. Pharm. Sci.*, 1999, vol. 88, 955-958
- **HOOVER, JOHN E.** Remington's Pharmaceutical Sciences. Mack Publishing Co., 1975
- Pharmaceutical Dosage Forms. Marcel Decke, 1980
- Handbook of Pharmaceutical Excipients. American Pharmaceutical Association, 1999
- **GOODMAN** ; **GILMAN**. The Pharmacological Basis of Therapeutics. Pergamon
- Remington's, Pharmaceutical Sciences. Mack Publishing Co.
- Comprehensive Organic Chemistry. Elsevier
- Comprehensive Organic Transformations: A Guide to Functional Group Preparations. John Wiley and Sons
- Compendium of Organic Synthetic Methods. Wiley-Interscience, vol. I-XII
- **GREENE**. Protective Groups in Organic Synthesis. John Wiley & Sons, 1991